(19) Europäisches Patentamt
European Patent Office
Office européen des brevets

(11) EP 1 377 577 B1

(12) EUROPEAN PATENT SPECIFICATION

(45) Date of publication and mention of the grant of the patent:
**14.06.2006 Bulletin 2006/24**

(21) Application number: **02732180.1**

(22) Date of filing: **22.01.2002**

(51) Int Cl.:
***C07D 471/04*** (2006.01) ***C07D 491/20*** (2006.01)
***A61K 31/4985*** (2006.01) ***A61P 29/00*** (2006.01)

(86) International application number:
**PCT/IB2002/000176**

(87) International publication number:
**WO 2002/057264 (25.07.2002 Gazette 2002/30)**

# (54) PYRROLE DERIVATES FOR TREATING CYTOKINE MEDIATED DISEASES

PYRROLDERIVATE ZUR BEHANDLUNG VON DURCH CYTOKINE VERMITTELTE KRANKHEITEN

DERIVES DE PYRROLE POUR TRAITER DES MALADIES DANS LESQUELLES INTERVIENNENT DES CYTOKINES

(84) Designated Contracting States:
**AT BE CH CY DE DK ES FI FR GB GR IE IT LI LU MC NL PT SE TR**

(30) Priority: **22.01.2001 JP 2001013817**
**12.03.2001 US 275005 P**

(43) Date of publication of application:
**07.01.2004 Bulletin 2004/02**

(73) Proprietor: **Sankyo Company Limited**
**Chuo-ku,**
**Tokyo 103-8426 (JP)**

(72) Inventors:
- **KIMURA, Tomio,**
**Sankyo Company Limited**
**Tokyo 140-8710 (JP)**
- **OHKAWA, Nobuyuki,**
**Sankyo Company Limited**
**Tokyo 140-8710 (JP)**
- **NAKAO, Akira,**
**Sankyo Company Limited**
**Tokyo 140-8710 (JP)**
- **NAGASAKI, Takayoshi,**
**Sankyo Company Limited**
**Tokyo 140-8710 (JP)**
- **YAMAZAKI, Takanori,**
**Sankyo Company Limited**
**Tokyo 140-8710 (JP)**

(74) Representative: **Gibson, Christian John Robert**
**Marks & Clerk**
**90 Long Acre**
**London WC2E 9RA (GB)**

(56) References cited:
**EP-A- 1 070 711**
**WO-A-97/05878**
**US-A- 5 792 778**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 377 577 B1

**Description**

The present invention relates to a series of heteroaryl-substituted pyrrole derivatives which have excellent inhibitory activity against the production of inflammatory cytokines such as interleukin (IL)-1, IL-6 and IL-8 and tumor necrosis factor (TNF), particularly IL-1β and TNFα. As a consequence, the compounds of the present invention have valuable anti-pyretic, analgesic, anti-viral and anti-inflammatory activity and are useful in the prophylaxis and treatment of autoimmune diseases such as chronic rheumatism, bone diseases such as osteoporosis and the many other diseases in which the above-described inflammatory cytokines take part. The invention also provides methods and compositions using these novel compounds and uses of these compounds as well as processes for their preparation.

**Background to the Invention**

Non-steroidal anti-inflammatory drugs (NSAIDs) have been widely used for the treatment and prophylaxis of various inflammatory diseases and in pain relief because they have, as their main pharmacological activity, anti-pyretic, analgesic, and anti-inflammatory activity which is based on their ability to inhibit the biosynthesis of prostaglandin (PG) through the inhibition of cyclooxygenase activity. Another class of compounds commonly used for the treatment of rheumatoid arthritis is the disease-modifying anti-rheumatic drugs (DMARDs), examples of which include methotrexate and sulphasalazine. This is a wide class of drugs in which the compounds have no common mechanism of action. For the treatment of chronic rheumatism, NSAIDs are used nosotropically and DMARDs are used etiotropically. There are a number of problems associated with these classes of drugs. Conventional NSAIDs can induce undesirable side effects including gastrointestinal disorders such as gastric ulcers and renal disorders, resulting in difficulties for any patient who has to take such a drug for an extended period of time. DMARDs can also induce undesirable side effects including nausea and diarrhoea and, furthermore, they have not yet been clearly shown to exhibit a stable, long-lasting effect.

A class of active substances generally called cytokines, which are produced in the body by immunocytes, has recently been found. One group of cytokines is known as the inflammatory cytokines and it includes interleukin (IL)-1, IL-6 and IL-8 and tumor necrosis factor (TNF). The inflammatory cytokines have been demonstrated to play a major role in a number of biological processes. These include action as an inflammatory mediator through the stimulation of the arachidonic acid metabolic pathway leading to the production of PG, the migration of leukocytes, the production of acute phase protein, and activation of osteoclasts.

It is believed that the inflammatory cytokines are associated with many diseases including inflammatory diseases and the induction of bone resorption. Due to their mechanism of action, which is different from that of conventional drugs such as those described above, compounds which are able to inhibit the production of inflammatory cytokines are expected to provide an improved new generation of anti-pyretic, analgesic and anti-inflammatory drugs and medicaments for the treatment of autoimmune diseases such as chronic rheumatism, bone diseases such as osteoporosis and the many other diseases in which the above-described inflammatory cytokines are believed to take part.

Compounds which are said to demonstrate inhibitory activity against the production of inflammatory cytokines include various heteroaryl compounds [see, for example, WO 96/21452, WO 97/5877, WO 97/23479 and WO 00/31063)]. Examples of compounds of this type include the following:

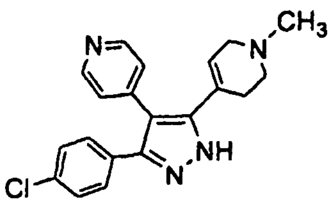

WO 00/31063
C-170

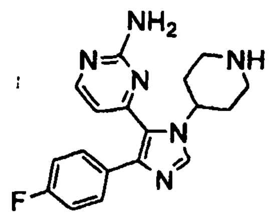

WO 97/23479
Compound of
Example 6

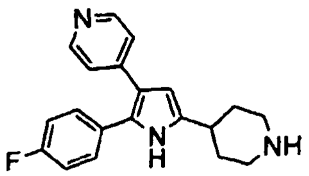

WO 97/5877
Compound of
Example 4

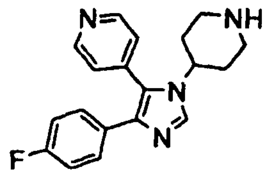

WO 96/21452
Compound of Example 23

There is a need for further compounds haying improved activity, pharmacokinetics and safety, and it is this need which is addressed by the present invention. Compounds having the characteristic bicyclic amino group of the compounds of the present invention have been neither disclosed nor suggested in the prior art.

## Brief Summary of the Invention

It is therefore an object of the present invention to provide a series of new pyrrole derivatives having a novel bicyclic amino substituent which inhibit the production of inflammatory cytokines and consequently show anti-pyretic, analgesic, anti-viral and anti-inflammatory activity and have utility in the prophylaxis and treatment of autoimmune diseases such as chronic rheumatism, bone diseases such as osteoporosis and the many other diseases in which the above-described inflammatory cytokines take part.

Other objects and advantages of the present invention will become apparent as the description proceeds.

The compounds of the present invention are compounds of the following formula (I-1), or a pharmacologically acceptable salt, ester or amide thereof:

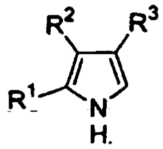

(I-1)

wherein:

$R^1$ is selected from the group consisting of aryl groups defined below which may optionally be substituted with at least one substituent selected from Substituent group $\alpha$ defined below and Substituent group $\beta$ defined below, and heteroaryl groups defined below which may optionally be substituted with at least one substituent selected from Substituent group $\alpha$ defined below and Substituent group $\beta$ defined below;

$R^2$ represents a heteroaryl group defined below which has at least one ring nitrogen atom, said heteroaryl group optionally being substituted with at least one substituent selected from Substituent group $\alpha$ defined below and Substituent group $\beta$ defined below; and

$R^3$ represents a group of general formula (IIa), (IIb) or (IIc) shown below:

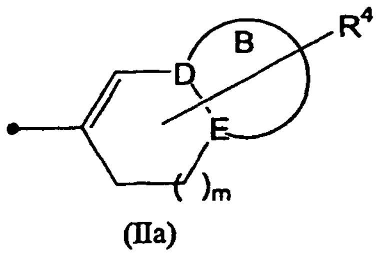

(IIa)

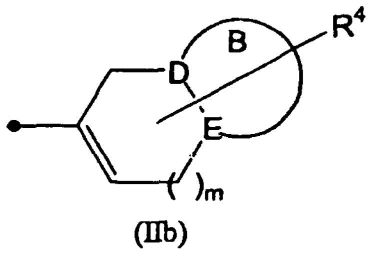

(IIb)

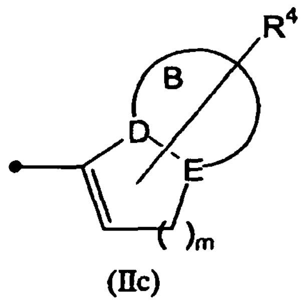

(IIc)

wherein
m represents 1 or 2,
one of D and E represents a nitrogen atom and the other represents a group of formula >C($R^5$)- (wherein $R^5$ is selected from the group consisting of hydrogen atoms, Substituent group α defined below and Substituent group β defined below),
B represents a 4- to 7-membered heterocyclic ring which has at least one ring nitrogen atom (said heterocyclic ring may be saturated or unsaturated, and may optionally be fused with a group selected from aryl groups defined below, heteroaryl groups defined below, cycloalkyl groups defined below and heterocyclyl groups defined below), and
$R^4$ represents from 1 to 3 substituents which are independently selected from the group consisting of Substituent group α defined below, Substituent group β defined below and Substituent group γ defined below, or
where B is a heterocyclic ring which is fused to an aryl group, a heteroaryl group, a cycloalkyl group or a heterocyclyl group, $R^4$ may be a hydrogen atom;

Substituent group α consists of hydroxyl groups, nitro groups, cyano groups, halogen atoms, lower alkoxy groups defined below, halogeno lower alkoxy groups defined below, lower alkylthio groups defined below, halogeno lower alkylthio groups defined below and groups of formula $-NR^aR^b$ (wherein $R^a$ and $R^b$ are the same or different from each other and each is independently selected from the group consisting of hydrogen atoms, lower alkyl groups defined below, lower alkenyl groups defined below, lower alkynyl groups defined below, aralkyl groups defined below and lower alkylsulfonyl groups defined below, or $R^a$ and $R^b$, taken together with the nitrogen atom to which they are attached, form a heterocyclyl group);

Substituent group β consists of lower alkyl groups defined below which may optionally be substituted with at least one substituent selected from Substituent group α defined above, lower alkenyl groups defined below which may optionally be substituted with at least one substituent selected from Substituent group α defined above, lower alkynyl groups defined below which may optionally be substituted with at least one substituent selected from Substituent group α defined above, aralkyl groups defined below and cycloalkyl groups defined below;

Substituents group γ consists of oxo groups, hydroxyimino groups, lower alkoxyimino groups defined below, lower alkylene groups defined below, lower alkylenedioxy groups defined below, lower alkylsulfinyl groups defined below, lower alkylsulfonyl groups defined below, aryl groups defined below which may optionally be substituted with at least one substituent selected from Substituent group α defined above and Substituent group β defined above, aryloxy groups defined below which may optionally be substituted with at least one substituent selected from Substituent group α defined above and Substituent group β defined above, lower alkylidenyl groups and aralkylidenyl groups.

The present invention also provides a pharmaceutical composition comprising an effective amount of a pharmacologically active compound together with a carrier or diluent therefor, wherein said pharmacologically active compound is a compound of formula (I-1) or a pharmacologically acceptable salt, ester or amide thereof.

The present invention also provides a compound of formula (I-1) or a pharmacologically acceptable salt, ester or amide thereof for use as a medicament.

The present invention also provides the use of at least one compound of formula (I-1) or a pharmacologically acceptable salt, ester or amide thereof in the manufacture of a medicament for inhibiting the production of inflammatory cytokines in a mammal, which may be human.

The present invention also provides the use of at least one compound of formula (I-1) or a pharmacologically acceptable salt, ester or amide thereof in the manufacture of a medicament for inhibiting bone resorption in a mammal, which may be human.

The present invention also provides the use of at least one compound of formula (I-1) or a pharmacologically acceptable salt, ester or amide thereof in the manufacture of a medicament for the treatment or prophylaxis of inflammatory diseases in a mammal, which may be human.

The present invention also provides the use of at least one compound of formula (I-1) or a pharmacologically acceptable salt, ester or amide thereof in the manufacture of a medicament for the treatment or prophylaxis of viral diseases in a mammal, which may be human.

The present invention also provides the use of at least one compound of formula (I-1) or a pharmacologically acceptable salt, ester or amide thereof in the manufacture of a medicament for relieving pain or pyrexia in a mammal, which may be human.

The present invention also provides the use of at least one compound of formula (I-1) or a pharmacologically acceptable salt, ester or amide thereof in the manufacture of a medicament for the treatment or prophylaxis of chronic rheumatoid arthritis in a mammal, which may be human.

The present invention also provides the use of at least one compound of formula (I-1) or a pharmacologically acceptable salt, ester or amide thereof in the manufacture of a medicament for the treatment or prophylaxis of osteoarthritis in a mammal, which may be human.

The present invention also provides the use of at least one compound of formula (I-1) or a pharmacologically

acceptable salt, ester or amide thereof in the manufacture of a medicament for the treatment or prophylaxis of cancer in a mammal, which may be human.

The present invention also provides the use of at least one compound of formula (I-1) or a pharmacologically acceptable salt, ester or amide thereof in the manufacture of a medicament for the treatment or prophylaxis of hepatitis in a mammal, which may be human.

The present invention also provides the use of at least one compound of formula (I-1) or a pharmacologically acceptable salt, ester or amide thereof in the manufacture of a medicament for the treatment or prophylaxis of a disease selected from the group consisting of allergic diseases, septicaemia, psoriasis, asthma, degenerative arthritis, Crohn's disease, systemic lupus erythematosus, osteoporosis, ulcerative colitis, diabetes, nephritis, ischemic heart disease, Alzheimer's disease and arteriosclerosis in a mammal, which may be human.

## Detailed Description of the Invention

In the general formula (I-1) above, the group $R^4$ in the groups of general formulae (IIa), (IIb) and (IIc) is defined as representing "from 1 to 3 substituents which are independently selected from the group consisting of Substituent group $\alpha$ defined below, Substituent group $\beta$ defined below and Substituent group $\gamma$ defined below, or where B is a heterocyclic ring which is fused with an aryl group, a heteroaryl group, a cycloalkyl group or a heterocyclyl group, $R^4$ may be a hydrogen atom." This means that the following options are covered: (i) compounds of formula (I) in which there are 1, 2 or 3 substituents selected from Substituent group $\alpha$, Substituent group $\beta$ and Substituent group $\gamma$ anywhere on the bicyclic ring systems of formulae (IIa), (IIb) and (IIc); and (ii) compounds of formula (I-1) in which the heterocyclic group B is fused with an aryl group, a heteroaryl group, a cycloalkyl group or a heterocyclyl group and there are 0 ($R^4$ represents hydrogen), 1, 2 or 3 substituents selected from Substituent group $\alpha$, Substituent group $\beta$ and Substituent group $\gamma$ anywhere on the ring systems of formulae (IIa), (IIb) and (IIc).

Where $R^1$ represents an aryl group which may optionally be substituted with at least one substituent selected from the group consisting of Substituent group $\alpha$ defined above and Substituent group $\beta$ defined above, or where Substituent $\gamma$ represents an aryl group which is optionally substituted with at least one substituent selected from the group consisting of Substituent group a defined above and Substituent group $\beta$ defined above, said aryl groups are aromatic hydrocarbon groups having from 6 to 14 carbon atoms in one or more rings, preferably from 6 to 10 carbon atoms, and examples include phenyl, naphthyl, phenanthryl and anthracenyl groups. Of these, we prefer phenyl and naphthyl groups, most preferably phenyl groups.

The aryl groups defined and exemplified above may be fused with a cycloalkyl group having from 3 to 10 carbon atoms. Examples of such a fused ring group include 5-indanyl groups.

Where $R^1$ or Substituent $\gamma$ represents an aryl group which is substituted with at least one substituent selected from the group consisting of Substituent group $\alpha$ defined above and Substituent group $\beta$ defined above, it is preferably an aryl group substituted with 1 to 4 substituents selected from the group consisting of Substituent group $\alpha$ and Substituent group $\beta$, and more preferably it is an aryl group substituted with 1 to 3 substituents selected from Substituent group $\alpha$ and Substituent group $\beta$. Examples of such substituted aryl groups include 4-fluorophenyl, 3-fluorophenyl, 4-chlorophenyl, 3-chlorophenyl, 3,4-difluorophenyl, 3,4-dichlorophenyl, 3,4,5-trifluorophenyl, 3-chloro-4-fluorophenyl, 3-difluoromethoxyphenyl, 3-trifluoromethoxyphenyl and 3-trifluoromethylphenyl.

Where $R^1$ represents a heteroaryl group which may optionally be substituted with at least one substituent selected from the group consisting of Substituent group $\alpha$ defined above and Substituent group $\beta$ defined above, said heteroaryl groups are 5- to 7-membered aromatic heterocyclic groups containing from 1 to 3 heteroatoms selected from the group consisting of sulfur atoms, oxygen atoms and nitrogen atoms. Examples of such heteroaryl groups include furyl, thienyl, pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl and pyrazinyl groups. We prefer 5- or 6-membered aromatic heterocyclic groups containing one or two heteroatoms selected from the group consisting of sulfur atoms, oxygen atoms and nitrogen atoms, examples of which include furyl, thienyl, pyrrolyl, pyrazolyl, imidazolyl, oxazolyl, isoxazolyl, thiazolyl, isothiazolyl, pyridyl, pyridazinyl, pyrimidinyl and pyrazinyl groups. Of these, furyl, thienyl, pyridyl and pyrimidinyl groups are particularly preferred.

The heteroaryl groups defined and exemplified above may be fused with another cyclic group selected from the group consisting of aryl groups defined above and cycloalkyl groups having from 3 to 10 carbon atoms. Examples of such a fused heteroaryl group include indolyl, benzofuranyl, benzothienyl, quinolyl, isoquinolyl, quinazolyl, tetrahydroquinolyl and tetrahydroisoquinolyl groups.

Where $R^1$ represents a heteroaryl group which is substituted with at least one substituent selected from the group consisting of Substituent group $\alpha$ and Substituent group $\beta$, said heteroaryl group is preferably a heteroaryl group substituted with from 1 to 3 substituents selected from the group consisting of Substituent group $\alpha$ and Substituent group $\beta$, and more preferably it is a heteroaryl group substituted with one or two substituents selected from the group consisting of Substituent group $\alpha$ and Substituent group $\beta$. Examples of such substituted heteroaryl groups include 5-fluoro-2-furyl,

4-chloro-2-thienyl, 5-difluoromethoxy-3-furyl, 5-trifluoromethyl-3-thienyl and 5-fluoro-2-oxazolyl groups.

Where $R^2$ represents a heteroaryl group having at least one ring nitrogen atom, said heteroaryl group optionally being substituted with at least one substituent selected from the group consisting of Substituent group α defined above and Substituent group β defined above, said heteroaryl groups are 5- to 7-membered aromatic heterocyclic groups containing at least one nitrogen atom and optionally containing one or two further heteroatoms selected from the group consisting of sulfur atoms, oxygen atoms and nitrogen atoms. Examples of such groups include pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl and pyrazinyl groups. Of these, we prefer 5- or 6-membered aromatic heterocyclic groups containing one nitrogen atom and optionally containing one further heteroatom selected from the group consisting of sulfur atoms, oxygen atoms and nitrogen atoms, examples of which include pyrrolyl, pyrazolyl, imidazolyl, oxazolyl, isoxazolyl, thiazolyl, isothiazolyl, pyridyl, pyridazinyl, pyrimidinyl and pyrazinyl groups. 5- or 6-membered aromatic heterocyclic groups containing one or two nitrogen atoms, such as imidazolyl, pyridyl, pyridazinyl, pyrimidinyl and pyrazinyl groups are more preferred and pyridyl and pyrimidinyl groups are particularly preferred.

Where $R^2$ represents a heteroaryl group having at least one ring nitrogen atom, 4-pyridyl and 4-pyrimidinyl groups are most preferred.

Where $R^2$ represents a heteroaryl group having at least one ring nitrogen atom, said heteroaryl group optionally being substituted with at least one substituent selected from the group consisting of Substituent group a and Substituent group β, said heteroaryl group is preferably a group substituted with 1 to 3 substituents selected from the group consisting of Substituent group α and Substituent group β, more preferably it is a heteroaryl group substituted with one or two substituents selected from the group consisting of Substituent group α and Substituent group β, still more preferably it is a heteroaryl group substituted with one substituent selected from the group consisting of Substituent group α and Substituent group β, and particularly preferably it is a 4-pyridyl or 4-pyrimidinyl group which is substituted at the 2-position of said group with one substituent selected from Substituent group α and Substituent group β. Most preferably, said heteroaryl group is a 4-pyridyl or 4-pyrimidinyl group which is substituted at the 2-position with a substituent of formula $-NR^aR^b$ (wherein $R^a$ and $R^b$ are same or different, and each independently represents a hydrogen atom, a lower alkyl group, a lower alkenyl group, a lower alkynyl group, an aralkyl group or a lower alkylsulfonyl group, or $R^a$ and $R^b$, taken together with the nitrogen atom to which they are attached, form a heterocyclyl group) or a lower alkyl group substituted with a substituent of formula $-NR^aR^b$ (wherein $R^a$ and $R^b$ have the same meaning as above). Preferred examples of such a group include 2-amino-4-pyridyl, 2-amino-4-pyrimidinyl, 2-methylamino-4-pyridyl, 2-methylamino-4-pyrimidinyl, 2-methoxy-4-pyridyl, 2-methoxy-4-pyrimidinyl, 2-benzylamino-4-pyridyl, 2-benzylamino-4-pyrimidinyl, 2-(α-methylbenzylamino)-4-pyridyl and 2-(α-methylbenzylamino)-4-pyrimidinyl.

The ring B is defined as a "4- to 7-membered heterocyclic ring which has at least one ring nitrogen atom", by which we mean a 4- to 7-membered heterocyclic ring which consists of group D, group E, and 2 to 5 atoms or groups selected from carbon atoms, nitrogen atoms, oxygen atoms, sulfur atoms, >SO and $>SO_2$, said heterocyclic ring containing at least one ring nitrogen atom. It will be appreciated that, where said ring B contains only one ring nitrogen atom, then this nitrogen atom is either group D or group E. The 4- to 7-membered heterocyclic ring of ring B may be a saturated heterocyclic ring or an unsaturated heterocyclic ring. Preferably, the ring B is a 5- or 6-membered heterocyclic ring which contains one nitrogen atom and which may optionally contain one further ring heteroatom or ring group selected from nitrogen atoms, oxygen atoms, sulfur atoms, >SO and $>SO_2$; more preferably it is pyrrolidine, pyrroline, imidazolidine, imidazoline, pyrazolidine, pyrazoline, oxazolidine, thiazolidine, piperidine, tetrahydropyridine, dihydropyridine, piperazine, morpholine or thiomorpholine; still more preferably it is pyrrolidine, pyrroline or imidazoline; and most preferably it is pyrrolidine or pyrroline.

The heterocyclic ring B defined and exemplified above may optionally be fused with an aryl group, a heteroaryl group, a cycloalkyl group or a heterocyclyl group, said aryl, heteroaryl and heterocyclyl groups optionally being substituted with at least one substituent selected from Substituent group α defined above and Substituent group β defined above. Said aryl groups are defined and exemplified above for substituent $R^1$ and Substituent group γ. Said heteroaryl groups are as defined and exemplified above for substituent $R^1$. Said cycloalkyl groups are cycloalkyl groups having from 3 to 7 carbon atoms, such as cyclopropyl, cyclobutyl, cyclopentyl, cyclohexyl and cycloheptyl groups, of which we prefer cycloalkyl groups having from 3 to 6 carbon atoms. Said heterocyclyl groups are 4- to 7-membered heterocyclyl groups which have from 1 to 3 ring sulfur atoms, oxygen atoms and/or nitrogen atoms, and preferably 4- to 7-membered heterocyclyl groups which have 1 or 2 heteroatoms selected from the group consisting of sulfur atoms, oxygen atoms and nitrogen atoms. Of these, 5- or 6-membered heterocyclyl groups which contain one ring nitrogen atom and which may optionally contain one further oxygen atom, sulfur atom or nitrogen atom are preferred, and examples of such groups include azetidinyl, pyrrolidinyl, pyrrolinyl, imidazolidinyl, imidazolinyl, pyrazolidinyl, pyrazolinyl, oxazolidinyl, thiazolidinyl, piperidyl, tetrahydropyridyl, dihydropyridyl, piperazinyl, morpholinyl, thiomorpholinyl and homopiperidyl.

Examples of such a fused ring system for ring B include tetrahydroquinoline, octahydroquinoline, decahydroquinoline, tetrahydroisoquinoline, octahydroisoquinoline, decahydroisoquinoline, indoline, octahydroindole, isoindoline and octahydroisoindole.

The lower alkyl groups in the definitions of $R^a$, $R^b$ and Substituent group β, and the lower alkyl moiety of the lower alkyl groups which may optionally be substituted with at least one substituent selected from Substituent group α in the definition of Substituent group β are straight or branched alkyl groups having from 1 to 6 carbon atoms. Examples of said lower alkyl groups include methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl groups. Alkyl groups having from 1 to 4 carbon atoms are preferred, methyl, ethyl, propyl, isopropyl and butyl groups are more preferred, and methyl, ethyl and propyl groups are most preferred.

The lower alkenyl groups in the definitions of $R^a$, $R^b$ and Substituent group β, and the lower alkenyl moiety of the lower alkenyl groups which may optionally be substituted with at least one substituent selected from Substituent group a in the definition of Substituent group β are straight or branched alkenyl groups having from 2 to 6 carbon atoms. Examples of said lower alkenyl groups include vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl and 5-hexenyl groups. Alkenyl groups having from 2 to 4 carbon atoms are preferred, and alkenyl groups having 2 or 3 carbon atoms are most preferred.

The lower alkynyl groups in the definitions of $R^a$, $R^b$ and Substituent group β, and the lower alkynyl moiety of the lower alkynyl groups which may optionally be substituted with at least one substituent selected from Substituent group a in the definition of Substituent group β are straight or branched alkynyl groups having from 2 to 6 carbon atoms. Examples of said lower alkynyl groups include ethynyl, 2-prqpynyl, 1-methyl-2-propynyl, 2-butynyl, 1-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl and 5-hexynyl groups. Alkynyl groups having from 2 to 4 carbon atoms are preferred, and alkynyl groups having 2 or 3 carbon atoms are most preferred.

The aralkyl group in the definitions of $R^a$, $R^b$ and Substituent group β is a lower alkyl group as defined above which is substituted with at least one aryl group as defined above which may optionally be substituted with from 1 to 3 substituents selected from Substituent group α defined above and Substituent group β defined above. Examples of such a group include benzyl, indenylmethyl, phenanthrylmethyl, anthrylmethyl, α-naphthylmethyl, β-naphthylmethyl, diphenylmethyl, triphenylmethyl, α-naphthyldiphenylmethyl, 9-anthrylmethyl, piperonyl, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl; 2-naphthylpentyl, 3-naphthylpentyl, 4-naphthylpentyl, 5-naphthylpenthyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl and 6-naphthylhexyl. Of these, benzyl, phenanthrylmethyl, anthrylmethyl, α-naphthylmethyl, β-naphthylmethyl, diphenylmethyl, triphenylmethyl, 9-anthrylmethyl, piperonyl, 1-phenethyl, 2-phenethyl, 1-phenylpropyl, 2-phenylpropyl, 3-phenylpropyl, 1-phenylbutyl, 2-phenylbutyl, 3-phenylbutyl and 4-phenylbutyl are preferred.

As noted above, the aryl moiety of the aralkyl groups may optionally be substituted with from 1 to 3 substituents selected from Substituent group a and Substituent group β defined above. Examples of such a substituted aralkyl group include aralkyl groups substituted with halogen atoms such as 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 3,5-difluorobenzyl, 2,5-difluorophenethyl, 2,6-difluorobenzyl, 2,4-difluorophenethyl, 3,5-dibromobenzyl, 2,5-dibromophenethyl, 2,6-dichlorobenzyl, 2,4-dichlorophenethyl, 2,3,6-trifluorobenzyl, 2,3,4-trifluorophenethyl, 3,4,5-trifluorobenzyl, 2,5,6-trifluorophenethyl, 2,4,6-trifluorobenzyl, 2,3,6-tribromophenetyl, 2,3,4-tribromobenzyl, 3,4,5-tribromophenethyl, 2,5,6-trichlorobenzyl, 2,4,6-trichlorophenethyl, 1-fluoro-2-naphthylmethyl, 2-fluoro-1-naphthylethyl, 3-fluoro-1-naphthylmethyl, 1-chloro-2-naphthylethyl, 2-chloro-1-naphthylmethyl, 3-bromo-1-naphthylethyl, 3,8-difluoro-1-naphthylmethyl, 2,3-difluoro-1-naphthylethyl, 4,8-difluoro-1-naphthylmethyl, 5,6-difluoro-1-naphthylethyl, 3,8-dichloro-1-naphthylmethyl, 2,3-dichloro-1-naphthylethyl, 4,8-dibromo-1-naphthylmethyl, 5,6-dibromo-1-naphthylethyl, 2,3,6-trifluoro-1-naphthylmethyl, 2,3,4-trifluoro-1-naphthylethyl, 3,4,5-trifluoro-1-naphthylmethyl, 4,5,6-trifluoro-1-naphthylethyl, 2,4,8-trifluoro-1-naphthylmethyl, bis(2-fluorophenyl)methyl, 3-fluorophenylphenylmethyl, bis(4-fluorophenyl)methyl, 4-fluorophenylphenylmethyl, bis(2-chlorophenyl)methyl, bis(3-chlorophenyl)methyl, bis(4-chlorophenyl)methyl, 4-chlorophenylphenylmethyl, 2-bromophenylphenylmethyl, 3-bromophenylphenylmethyl, bis(4-bromophenyl)methyl, bis(3,5-difluorophenyl)methyl, bis(2,5-difluorophenyl)methyl, bis(2,6-difluorophenyl)methyl, 2,4-difluorophenylphenylmethyl, bis(3,5-dibromophenyl)methyl, 2,5-dibromophenylphenylmethyl, 2,6-dichlorophenylphenylmethyl, bis(2,4-dichlorophenyl)methyl and bis(2,3,6-trifluorophenyl)methyl; aralkyl groups substituted with halogeno lower alkyl groups such as 2-trifluoromethylbenzyl, 3-trifluoromethylphenethyl, 4-trifluoromethylbenzyl, 2-trichloromethylphenethyl, 3-dichloromethylbenzyl, 4-trichloromethylphenethyl, 2-tribromomethylbenzyl, 3-dibromomethylphenethyl, 4-dibromomethylbenzyl, 3,5-bistrifluoromethylphenethyl, 2,5-bistrifluoromethylbenzyl, 2,6-bistrifluoromethylphenethyl, 2,4-bistrifluoromethylbenzyl, 3,5-bistribromomethylphenethyl, 2,5-bisdibromomethylbenzyl, 2,6-bisdichloromethylmethylphenethyl, 2,4-bisdichloromethylbenzyl, 2,3,6-tristrifluoromethylphenethyl, 2,3,4-tristrifluoromethylbenzyl, 3,4,5-tristrifluoromethylphenethyl, 2,5,6-tristrifluoromethylbenzyl, 2,4,6-tristrifluoromethylphenethyl, 2,3,6-tristribromomethylbenzyl, 2,3,4-trisdibromomethylphenethyl, 3,4,5-tristribromomethylbenzyl, 2,5,6-trisdichloromethylmethylphenethyl, 2,4,6-trisdichloromethylbenzyl, 1-trifluoromethyl-2-naphthylethyl, 2-trifluoromethyl-1-naphthylmethyl, 3-trifluoromethyl-1-naphthylethyl, 1-trichloromethyl-2-naphthylmethyl, 2-dichloromethyl-l-naphthylethyl, 3-tribromomethyl-1-naphthylmethyl, 3,8-bistrifluoromethyl-1-naphthylethyl, 3,8-bistrifluoromethyl-1-naphthylethyl, 2,3-bistrifluoromethyl-1-naphthylmethyl, 4,8-bistrifluoromethyl-1-naphthylethyl, 5,6-bistrifluoromethyl-1-naphthylmethyl, 3,8-bistrichloromethyl-1-naphthylethyl, 2,3-bisdichloromethyl-1-naphthylmethyl, 4,8-bisdibromomethyl-1-naphthylethyl, 5,6-bistribromomethyl-1-naphthylmethyl, 2,3,6-tristrifluoromethyl-1-naphthylethyl, 2,3,4-tristrifluoromethyl-1-naphthylmethyl, 3,4,5-tristrifluoromethyl-1-naphthylethyl, 4,5,6-tristrifluoromethyl-1-naphthylmethyl, 2,4,8-tristrifluoromethyl-1-naphthylmethyl, bis(4-trifluoromethylphenyl)methyl, 4-trifluoromethylphenylphenylmethyl, bis(2-trichloromethylphenyl)-methyl, bis(3-trichloromethylphenyl)methyl, bis(4-trichloromethylphenyl)piethyl, 2-tribromomethylphenylphenylmethyl, 3-tribromomethylphenylphenylmethyl, bis(4-tribromomethylphenyl)methyl, bis(3,5-bistrifluoromethylphenyl)methyl, bis(2,5-bistrifluoromethylphenyl)methyl, bis(2,6-bistrifluoromethylphenyl)methyl, 2,4-bistrifluoromethylphenylphenylmethyl, bis(3,5-bistribromomethylphenyl)methyl, 2,5-bistribromomethylphenylphenylmethyl, 2,6-bistrichloromethylphenylphenylmethyl, bis(2,4-bistrichloromethylphenyl)methyl and bis(2,3,6-tristrifluoromethylphenyl)-methyl; aralkyl groups substituted with lower alkyl groups such as 2-methylbenzyl, 3-methylbenzyl, 4-methylbenzyl, 2-methylphenethyl, 4-methylphenethyl, 2-ethylbenzyl, 3-propylphenethyl, 4-ethylbenzyl, 2-butylphenethyl, 3-pentylbenzyl, 4-pentylphenethyl, 3,5-dimethylbenzyl, 2,5-dimethylphenethyl, 2,6-dimethylbenzyl, 2,4-dimethylphenethyl, 3,5-dibutylbenzyl, 2,5-dipentylphenethyl, 2,6-dipropylbenzyl, 2,4-dipropylphenethyl, 2,3,6-trimethylbenzyl, 2,3,4-trimethylphenethyl, 3,4,5-trimethylbenzyl, 2,4,6-trimethylbenzyl, 2,5,6-trimethylphenethyl, 2,3,6-tributylphenethyl, 2,3,4-tripentylbenzyl, 3,4,5-tributylphenethyl, 2,5,6-tripropylbenzyl, 2,4,6-tripropylphenethyl, 1-methyl-2-naphthylmethyl, 2-methyl-1-naphthylethyl, 3-methyl-1-naphthylmethyl, 1-ethyl-2-naphthylethyl, 2-propyl-1-naphthylmethyl, 3-butyl-1-naphthylethyl, 3,8-dimethyl-1-naphthylmethyl, 2,3-dimethyl-1-naphthylethyl, 4,8-dimethyl-1-naphthylmethyl, 5,6-dimethyl-1-naphthylethyl, 3,8-diethyl-1-naphthylmethyl, 2,3-dipropyl-1-naphthylmethyl, 4,8-dipentyl-1-naphthylethyl, 5,6-dibutyl-1-naphthylmethyl, 2,3,6-trimethyl-1-naphthylmethyl, 2,3,4-trimethyl-1-naphthylethyl, 3,4,5-trimethyl-1-naphthylmethyl, 4,5,6-trimethyl-1-naphthylmethyl, 2,4,8-trimethyl-1-naphthylmethyl, bis(2-methylphenyl)methyl, 3-methylphenylphenylmethyl, bis(4-methylphenyl)methyl, 4-methylphenylphenylmethyl, bis(2-ethylphenyl)methyl, bis(3-ethylphenyl)methyl, bis(4-ethylphenyl)methyl, 2-propylphenylphenylmethyl, 3-propylphenylphenylmethyl, bis(4-propylphenyl)methyl, bis(3,5-dimethylphenyl)-methyl, bis(2,5-dimethylphenyl)methyl, bis(2,6-dimethylphenyl)methyl, 2,4-dimethylphenylphenylmethyl, bis(3,5-dipropylphenyl)methyl, 2,5-dipropylphenylphenylmethyl, 2,6-diethylphenylphenylmethyl, bis(2,4-diethylphenyl)methyl and bis(2,3,6-trimethylphenyl)methyl; aralkyl groups substituted with lower alkoxy groups such as 2-methoxybenzyl, 3-methoxybenzyl, 4-methoxybenzyl, 3-methoxyphenethyl, 2-ethoxyphenethyl, 3-propoxybenzyl, 4-ethoxyphenethyl, 2-butoxybenzyl, 3-pentoxyphenethyl, 4-pentoxybenzyl, 3,5-dimethoxyphenethyl, 2,5-dimethoxybenzyl, 2,6-dimethoxyphenethyl, 2,4-dimethoxybenzyl, 3,5-dibutoxyphenethyl, 2,5-dipentoxybenzyl, 2,6-dipropoxyphenethyl, 2,4-dipropoxybenzyl, 2,3,6-trimethoxyphenethyl, 2,3,4-trimethoxybenzyl, 3,4,5-trimethoxyphenethyl, 2,5,6-trimethoxybenzyl, 2,4,6-trimethoxyphenethyl, 2,3,6-tributoxybenzyl, 2,3,4-tripentoxyphenethyl, 3,4,5-tributoxybenzyl, 2,5,6-tripropoxyphenethyl, 2,4,6-tripropoxybenzyl, 1-methoxy-2-naphthylmethyl, 2-methoxy-1-naphthylmethyl, 3-methoxy-1-naphthylethyl, 1-ethoxy-2-naphthylmethyl, 2-propoxy-1-naphthylmethyl, 3-butoxy-1-naphthylethyl, 3,8-dimethoxy-1-naphthylmethyl, 2,3-dimethoxy-1-naphthylmethyl, 4,8-dimethoxy-1-naphthylethyl, 5,6-dimethoxy-1-naphthylmethyl, 3,8-diethoxy-1-naphthylmethyl, 2,3-dipropoxy-1-naphthylethyl, 4,8-dipentoxy-1-naphthytmethyl, 5,6-dibutoxy-1-naphthylmethyl, 2,3,6-trimethoxy-1-naphthylethyl, 2,3,4-trimethoxy-1-naphthylmethyl, 3,4,5-trimethoxy-1-naphthylmethyl, 4,5,6-trimethoxy-1-naphthylethyl, 2,4,8-trimethoxy-1-naphthylmethyl, bis(2-methoxyphenyl)methyl, 3-methoxyphenylphenylmethyl, bis(4-methoxyphenyl)methyl, 4-methoxyphenylphenylmethyl, bis(2-ethoxyphenyl)methyl, bis(3-ethoxyphenyl)methyl, bis(4-ethoxyphenyl)methyl, 2-propoxyphenylphenylmethyl, 3-propoxyphenylphenylmethyl, bis(4-propoxyphenyl)-methyl, bis(3,5-dimethoxyphenyl)methyl, bis(2,5-dimethoxyphenyl)methyl, bis(2,6-dimethoxyphenyl)methyl, 2,4-dimethoxyphenylphenylmethyl, bis(3,5-dipropoxyphenyl)methyl, 2,5-dipropoxyphenylphenylmethyl, 2,6-diethoxyphenylphenylmethyl, bis (2,4-diethoxyphenyl)methyl and bis(2,3,6-trimethoxyphenyl)methyl; aralkyl groups substituted with amino groups such as 2-aminophenethyl, 3-aminobenzyl, 4-aminophenethyl, 3,5-diaminobenzyl, 2,5-diaminophenethyl, 2,6-diaminobenzyl, 2,4-diaminophenethyl, 2,3,6-triaminobenzyl, 2,3,4-triaminophenethyl, 3,4,5-triaminobenzyl, 2,5,6-triaminophenethyl, 2,4,6-triaminobenzyl, 1-amino-2-naphthylmethyl, 2-amino-1-naphthylethyl, 3-amino-1-naphthylmethyl, 3,8-diamino-1-naphthylmethyl, 2,3-diamino-1-naphthylethyl, 4,8-diamino-1-naphthylmethyl, 5,6-diamino-1-naphthylmethyl, 2,3,6-triamino-1-naphthylethyl, 2,3,4-triamino-1-naphthylmethyl, 3,4,5-triamino-1-naphthylmethyl, 4,5,6-triamino-1-naphthylethyl, 2,4,8-triamino-1-naphthylmethyl, bis(2-aminophenyl)methyl, 3-aminophenylphenylmethyl, bis(4-aminophenyl)methyl, 4-aminophenylphenylmethyl, bis(3,5-diaminophenyl)methyl, bis(2,5-diaminophenyl)methyl, bis(2,6-diaminophenyl) methyl, 2,4-diaminophenylphenylmethyl and bis(2,3,6-triaminophenyl)methyl; aralkyl groups substituted with nitro groups such as 2-nitrophenethyl, 3-nitrobenzyl, 4-nitrobenzyl, 4-nitrophenethyl, 3,5-dinitrobenzyl, 2,5-dinitrophenethyl, 2,6-dinitrobenzyl, 2,4-dinitrophenethyl, 2,3,6-trinitrobenzyl, 2,3,4-trinitrophenethyl, 3,4,5-trinitrobenzyl, 2,5,6-trinitrophenethyl, 2,4,6-trinitrobenzyl, 1-nitro-2-naphthylmethyl, 2-nitro-l-naphthytethyt, 3-nitro-1-naphthylmethyl, 3,8-dinitro-1-naphthylmethyl, 2,3-dinitro-1-naphthylethyl, 4,8-dinitro-1-naphthylmethyl, 5,6-dinitro-1-naphthylmethyl, 2,3,6-trinitro-1-naphthylethyl, 2,3,4-trinitro-1-naphthylmethyl, 3,4,5-trinitro-1-naphthylmethyl, 4,5,6-trinitro-1-naphthylethyl, 2,4,8-trinitro-1-naphthylmethyl, bis(2-nitrophenyl)methyl, 3-nitrophenylphenylmethyl, bis(4-nitrophenyl)methyl, 4-nitrophenylphenylmethyl, bis(3,5-dinitrophenyl)methyl, bis(2,5-dinitrophenyl)methyl, bis(2,6-dinitrophenyl)methyl, 2,4-dinitrophenylphenylmethyl and bis(2,3,6-trinitrophenyl)methyl; and aralkyl groups substituted with cyano groups such as 2-cyanophenethyl, 3-cyanobenzyl, 4-cyanobenzyl, 4-cyanobenzyldiphenylmethyl, 4-cyanophenethyl, 3,5-dicyanobenzyl, 2,5-dicyanophenethyl, 2,6-dicyanobenzyl, 2,4-dicyanophenethyl, 2,3,6-tricyanobenzyl, 2,3,4-tricyanophenethyl, 3,4,5-tricyanobenzyl, 2,5,6-tricyanophenethyl, 2,4,6-tricyanobenzyl, 1-cyano-2-naphthylmethyl, 3-cyano-1-naphthylmethyl, 3,8-dicyano-1-naphthylmethyl, 2,3-dicyano-1-naphthylethyl, 4,8-dicyano-1-naphthylmethyl, 5,6-dicyano-1-naphthylmethyl, 2,3,6-tricyano-1-naphthylethyl, 2,3,4-tricyano-1-naphthylmethyl, 3,4,5-tricyano-1-naphthylmethyl, 4,5,6-tricyano-1-naphthylethyl, 2,4,8-tricyano-1-naphthylmethyl, bis(2-cyanophenyl)methyl, 3-cyanophenylphenylmethyl, bis(4-cyanophenyl)methyl, 4-cyanophenylphenylmethyl, bis(3,5-dicyanophenyl)-methyl, bis(2,5-dicyanophenyl)methyl, bis(2,6-dicyanophenyl)methyl, 2,4-dicyanophenylphenylmethyl and bis(2,3,6-tricyanophenyl)methyl.

Of the above, unsubstituted aralkyl groups and aralkyl groups substituted with at least one substituent selected from the group consisting of halogen atoms, lower alkyl groups and lower alkoxy groups are preferred, unsubstituted aralkyl groups and aralkyl groups substituted with at least one substituent selected from the group consisting of halogen atoms and lower alkyl groups are more preferred, and unsubstituted aralkyl groups are most preferred.

Where $R^a$, $R^b$ or Substituent $\gamma$ represent a lower alkylsulfonyl group, this is a group in which a lower alkyl group, defined and exemplified above, is bonded to a sulfonyl group ($-SO_2-$). The lower alkylsulfonyl group is preferably a straight or branched alkylsulfonyl group having from 1 to 4 carbon atoms, more preferably a methylsulfonyl, ethylsulfonyl, propylsulfonyl, isopropylsulfonyl or butylsulfonyl group, and most preferably a methylsulfonyl, ethylsulfonyl or propylsulfonyl group.

Where $R^a$ and $R^b$ together with the nitrogen atom to which they are attached represent a heterocyclyl group, said heterocyclyl group is a 4- to 7-membered heterocyclyl group which contains one nitrogen atom and which optionally contains one further heteroatom selected from the group consisting of oxygen atoms, sulfur atoms and nitrogen atoms. Examples of such heterocyclyl groups include 1-azetidinyl, 1-pyrrolidinyl, 1-pyrrolinyl, 1-imidazolidinyl, 1-imidazolinyl, 1-pyrazolidinyl, 1-pyrazolinyl, 3-oxazoldinyl, 3-thiazolidinyl, 1-piperidyl, tetrahydropyridin-1-yl, dihydropyridin-1-yl, 1-piperazinyl, 4-morpholinyl, 4-thiomorpholinyl, 1-homopiperidyl, 8-azabicyclo[3.2.1]octan-8-yl, 8-azabicyclo-[3.2.1]octen-8-yl, 9-azabicyclo[3.3.1]nonan-9-yl and 9-azabicyclo[3.3.1]nonen-9-yl.

Where substituents $R^a$ and $R^b$ together with the nitrogen atom to which they are bonded form a heterocyclyl group as defined and exemplified above, said heterocyclyl groups may be fused with another cyclic group selected from the group consisting of aryl groups defined above and heteroaryl groups defined above. Examples of such fused heterocyclyl groups include tetrahydroquinolin-1-yl and tetrahydroisoquinolin-2-yl.

The halogen atoms in the definition of Substituent group $\alpha$ include fluorine, chlorine, bromine and iodine atoms, of which fluorine and chlorine atoms are preferred.

Where the substituent in the definition of Substituent group $\alpha$ is a lower alkoxy group, this is a group in which an oxygen atom is bonded to a lower alkyl group as defined and exemplified above. The alkoxy groups are preferably straight or branched alkoxy groups having 1 to 4 carbon atoms, more preferably methoxy, ethoxy, propoxy, isopropoxy or butoxy groups, and particularly preferably methoxy, ethoxy or propoxy groups.

Where the substituent in the definition of Substituent group $\alpha$ is a halogeno lower alkoxy group this is a group in which a lower alkoxy group as defined above is substituted with at least one halogen atom as exemplified above. The halogeno lower alkoxy groups preferably have from 1 to 4 carbon atoms, and are more preferably selected from the group consisting of difluoromethoxy, trifluoromethoxy and 2,2,2-trifluoroethoxy groups. Difluoromethoxy groups are most preferred.

Where the substituent in the definition of Substituent group $\alpha$ is a lower alkylthio group this is a group in which a sulfur atom is bonded to a lower alkyl group as defined and exemplified above. The lower alkylthio groups are preferably straight or branched alkylthio groups having 1 to 4 carbon atoms, more preferably methylthio, ethylthio, propylthio, isopropylthio or butylthio groups, and particularly preferably methylthio, ethylthio or propylthio groups.

Where the substituent in the definition of Substituent group $\alpha$ is a halogeno lower alkylthio group this is a group in which a lower alkylthio group as defined above is substituted with at least one halogen atom as exemplified above. The halogeno lower alkylthio groups preferably have from I to 4 carbon atoms, and are more preferably selected from the group consisting of difluoromethylthio, trifluoromethylthio and 2,2,2-trifluoroethylthio groups.

Where the substituent in the definition of Substituent group $\beta$ represents a cycloalkyl group, said cycloalkyl group is a cycloalkyl group having from 3 to 7 carbon atoms, such as cyclopropyl, cyclobutyl, cyclopentyl, cyclohexyl and cycloheptyl groups, of which we prefer cycloalkyl groups having from 3 to 6 carbon atoms, especially cyclopentyl and cyclohexyl groups.

Where the substituent in the definition of Substituent group γ represents a lower alkoxyimino group this is a group wherein the hydrogen atom of a hydroxyimino group is replaced by a lower alkyl group as defined and exemplified above. It is preferably an alkoxyimino group having from 1 to 4 carbon atoms, and more preferably a methoxyimino, ethoxyimino or propoxyimino group.

Where the substituent in the definition of Substituent group γ represents a lower alkylene group it is a straight or branched chain alkylene group having from 2 to 6 carbon atoms, examples of which include ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene and hexamethylene groups. It is preferably a straight or branched chain alkylene group having from 2 to 4 carbon atoms, and more preferably it is an ethylene, trimethylene, propylene or tetramethylene group. It will be appreciated that the lower alkylene group together with the atom of the group of formula (IIa), (IIb) or (IIc) to which it is attached form a spiro group.

Where the substituent in the definition of Substituent group γ represents a lower alkylenedioxy group this is a group wherein the alkylene moiety, which is a straight or branched chain alkylene group having from 1 to 6 carbon atoms, such as a methylene, ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene and hexamethylene, is subsitituted with 2 oxy groups. Preferably, the alkylenedioxy group is a straight or branched chain alkylenedioxy group having from 1 to 4 carbon atoms, and more preferably it is a methylenedioxy, ethylenedioxy, trimethylenedioxy, propylenedioxy or tetramethylenedioxy group. It will be appreciated that the lower alkylenedioxy group together with the atom of the group of formula (IIa), (IIb) or (IIc) to which the 2 oxy groups are attached form a spiro group.

Where the substituent in the definition of Substituent group γ represents a lower alkylsulfinyl group, this is a group in which a lower alkyl group, defined and exemplified above, is bonded to a sulfinyl group (-SO-). The lower alkylsulfinyl group is preferably a straight or branched alkylsulfinyl group having from 1 to 4 carbon atoms, more preferably a methylsulfinyl, ethylsulfinyl, propylsulfinyl, isopropylsulfinyl or butylsulfinyl group, and most preferably a methylsulfinyl, ethylsulfinyl or propylsulfinyl group.

Where the substituent in the definition of Substituent group γ represents a lower alkylidenyl group, this is a straight or branched alkylidenyl group having from 1 to 6 carbon atoms, examples of which include methylidenyl, ethylidenyl, propylidenyl, 1-methylethylidenyl, butylidenyl and 1-methylpropylidenyl groups. The lower alkylidenyl group is preferably a straight or branched alkylidenyl group having from 1 to 4 carbon atoms, and most preferably it is a methylidenyl, ethylidenyl or propylidenyl group.

Where the substituent in the definition of Substituent group γ represents an aralkylidenyl group, this is a straight or branched alkylidenyl group as defined and exemplified above which is substituted with 1 or more aryl groups as defined and exemplified above. Examples of these lower aralkylidenyl groups include benzylidenyl, phenylethylidenyl, phenylpropylidenyl and naphthylmethylidenyl groups. The aralkylidenyl group is preferably a straight or branched alkylidenyl group having from 1 to 4 carbon atoms which is substituted with a phenyl group or a naphthyl group, and most preferably it is a benzylidenyl or phenylethylidenyl group.

Where Substituent γ represents an aryloxy group which is optionally substituted with at least one substituent selected from the group consisting of Substituent group α defined above and Substituent group β defined above, said aryloxy groups are oxygen atoms which are attached to an aryl group as defined and exemplified above, and examples include phenoxy, naphthyloxy, phenanthryloxy and anthracenyloxy groups. Of these, we prefer phenoxy and naphthyloxy groups, most preferably phenoxy groups.

A preferred group of substituents of Substituent group α is Substituent group $\alpha^1$ which consists of halogen atoms, lower alkoxy groups as defined above, halogeno lower alkoxy groups as defined above and groups of formula $-NR^aR^b$ (wherein one of $R^a$ and $R^b$ represents a hydrogen atom or a lower alkyl group as defined above, and the other represents a hydrogen atom, a lower alkyl group as defined above or an aralkyl group as defined above).

A preferred group of substituents of Substituent group β is Substituent group $\beta^1$ which consists of lower alkyl groups as defined above, halogeno lower alkyl groups as defined above, hydroxyl lower alkyl groups, nitro lower alkyl groups, amino lower alkyl groups, lower alkylamino lower alkyl groups, di(lower alkyl)amino lower alkyl groups and aralkylamino lower alkyl groups.

Where the substituent in the definition of Substituent group $\beta^1$ represents a halogeno lower alkyl group, this is a group in which a lower alkyl group as defined and exemplified above is substituted with at least one halogen atom as exemplified above. It is preferably a straight or branched halogenoalkyl group having from 1 to 4 carbon atoms; more preferably it is a trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl or 2,2-dibromoethyl group; still more preferably it is a trifluoromethyl, trichloromethyl, difluoromethyl or fluoromethyl group; and most preferably it is a trifluoromethyl group.

Where the substituent in the definition of Substituent group $\beta^1$ represents a hydroxy lower alkyl group, this is a group in which a lower alkyl group as defined and exemplified above is substituted with at least one hydroxy group. It is preferably a hydroxyalkyl group having from 1 to 4 carbon atoms, and most preferably it is a hydroxymethyl, 2-hydroxyethyl or 3-hydroxypropyl group.

Where the substituent in the definition of Substituent group $\beta^1$ represents a nitro lower alkyl group, this is a group in which a lower alkyl group as defined and exemplified above is substituted with at least one nitro group. It is preferably a nitroalkyl group having from I to 4 carbon atoms, and most preferably it is a nitromethyl, 2-nitroethyl or 3-nitropropyl group.

Where the substituent in the definition of Substituent group $\beta^1$ represents an amino lower alkyl group, a lower alkylamino lower alkyl group, a di(lower alkyl)-amino lower alkyl group or an aralkylamino lower alkyl group, this is a group in which a lower alkyl group as defined and exemplified above is substituted with a group of formula $-NR^aR^b$ (wherein one of $R^a$ and $R^b$ represents a hydrogen atom or a lower alkyl group as defined above, and the other represents a hydrogen atom, a lower alkyl group as defined above or an aralkyl group as defined above). Of these, substituents in which the alkyl moiety which is substituted with the group $-NR^aR^b$ has from 1 to 4 carbon atoms are preferred. Aminomethyl, 2-aminoethyl, 3-aminopropyl, methylaminomethyl, 2-(methylamino)ethyl, 3-(methylamino)propyl, ethylamino-methyl, 2-(ethylamino)ethyl, 3-(ethylamino)propyl, dimethylaminomethyl, 2-(dimethylamino)ethyl, 3-(dimethylamino)propyl, diethylaminomethyl, 2-(diethylamino)ethyl, 3-(diethylamino)propyl, benzylaminomethyl, 2-(benzylamino)-ethyl and 3-(benzylamino)propyl groups are more preferred.

The present invention encompasses esters and amides of the compounds of formula (I-1). These esters and amides are compounds of formula (I-1) in which a functional group (for example, a hydroxyl group, an amino group, an imino group or a sulfonamide group) of said compound of formula (I-1) is modified by the addition of a protecting group using conventional techniques well-known in the art (see, for example, "Protective Groups in Organic Synthesis, Second Edition, Theodora W. Greene and Peter G.M. Wuts, 1991, John Wiley & Sons, Inc.).

There is no particular restriction on the nature of this protecting group, provided that, where the ester or amide is intended for therapeutic purposes, it must be pharmacologically acceptable, i.e. the protecting group must be capable of being removed by a metabolic process (e.g. hydrolysis) on administration of said compound to the body of a live mammal to give a compound of formula (I-1) or a salt thereof. In other words, the pharmacologically acceptable esters or amides are pro-drugs of the compounds of formula (I-1) of the present invention. Where, however, the ester or amides of the compound of formula (I-1) of the present invention is intended for non-therapeutic purposes (e.g. as an intermediate in the preparation of other compounds), then the requirement that said ester or amide is pharmacologically acceptable does not apply.

Whether an ester or amide of a compound of formula (I-1) of the present invention is pharmacologically acceptable can be easily determined. The compound under investigation is intravenously administered to an experimental animal such as a rat or mouse and the body fluids of the animal are thereafter studied. If a compound of formula (I-1) or a pharmacologically acceptable salt thereof can be detected in the body fluids, the compound under investigation is judged to be a pharmacologically acceptable ester or amide.

The compounds of formula (I-1) of the present invention can be converted to an ester, examples of which include a compound of formula (I-1) in which a hydroxyl group present therein is esterified. The ester residue may be a general protecting group where the esterified compound is to be used as an intermediate or a protecting group which is capable of being removed by a metabolic process (e.g. hydrolysis) *in vivo* where the esterified compound is one which is pharmacologically acceptable.

The general protecting group referred to above is a protecting group which is removable by a chemical process such as hydrolysis, hydrogenolysis, electrolysis or photolysis. Preferred examples of such a general protecting group used to synthesise a compound of formula (I-1) in which a hydroxyl residue therein is modified include the following:

(i) aliphatic acyl groups, examples of which include
alkylcarbonyl groups having from 1 to 25 carbon atoms, examples of which include formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl groups,
halogenated alkylcarbonyl groups having from 1 to 25 carbons in which the alkyl moiety thereof is substituted by at least one halogen atom, examples of which include chloroacetyl, dichloroacetyl, trichloroacetyl and trifluoroacetyl groups,
lower alkoxyalkylcarbonyl groups which comprise an alkylcarbonyl group having from 1 to 25 carbon atoms in which the alkyl moiety thereof is substituted with at least one lower alkoxy group as defined above, examples of said lower alkoxyalkylcarbonyl groups including methoxyacetyl groups, and
unsaturated alkylcarbonyl groups having from 1 to 25 carbon atoms, examples of which include acryloyl, propioloyl, methacryloyl, crotonoyl, isocrotonoyl and (E)-2-methyl-2-butenoyl groups;
of these, alkylcarbonyl groups having from 1 to 6 carbon atoms are preferred;

(ii) aromatic acyl groups, examples of which include
arylcarbonyl groups which comprise a carbonyl group which is substituted with an aryl group as defined above, examples of which include benzoyl, α-naphthoyl and β-naphthoyl groups,
halogenated arylcarbonyl groups which comprise an arylcarbonyl group as defined above which is substituted with at least one halogen atom, examples of which include 2-bromobenzoyl, 4-chlorobenzoyl and 2,4,6-trifluorobenzoyl groups,
lower alkylated arylcarbonyl groups which comprise an arylcarbonyl group as defined above which is substituted with at least one lower alkyl group as defined above, examples of which include 2,4,6-trimethyl-benzoyl and 4-toluoyl groups,
lower alkoxylated arylcarbonyl groups which comprise an arylcarbonyl group as defined above which is substituted with at least one lower alkoxy group as defined above, examples of which include 4-anisoyl groups,
nitrated arylcarbonyl groups which comprise an arylcarbonyl group as defined above which is substituted with at least one nitro group, examples of which include 4-nitrobenzoyl and 2-nitrobenzoyl groups,
lower alkoxycarbonylated arylcarbonyl groups which comprise an arylcarbonyl group as defined above which is substituted with a carbonyl group which is itself substituted with a lower alkoxy group as defined above, examples of which include 2-(methoxycarbonyl)benzoyl groups, and
arylated arylcarbonyl groups which comprise an arylcarbonyl group as defined above which is substituted with at least one aryl group as defined above, examples of which include 4-phenylbenzoyl groups;

(iii) alkoxycarbonyl groups, examples of which include
lower alkoxycarbonyl groups which comprise a carbonyl group substituted with a lower alkoxy group as defined above, examples of which include methoxycarbonyl, ethoxycarbonyl, propoxycarbonyl, butoxycarbonyl, s-butoxycarbonyl, t-butoxycarbonyl and isobutoxycarbonyl groups, and
lower alkoxycarbonyl groups as defined above which are substituted with at least one substituent selected from the group consisting of halogen atoms and tri(lower alkyl)silyl groups (wherein said lower alkyl groups are as defined above), examples of which include 2,2,2-trichloroethoxycarbonyl and 2-trimethylsilylethoxycarbonyl groups;

(iv) tetrahydropyranyl or tetrahydrothiopyranyl groups which may optionally be substituted with at least one substituent selected from lower alkyl groups as defined above, halogen atoms and lower alkoxy groups as defined above, examples of which include tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl groups;

(v) tetrahydrofuranyl or tetrahydrothiofuranyl groups which may optionally be substituted with at least one substituent selected from lower alkyl groups as defined above, halogen atoms and lower alkoxy groups as defined above, examples of which include tetrahydrofuran-2-yl and tetrahydrothiofuran-2-yl groups;

(vi) silyl groups, examples of which include
tri(lower alkyl)silyl groups (wherein said lower alkyl groups are as defined above), examples of which include trimethylsilyl, triethylsilyl, isopropyldimethylsilyl, t-butyldimethylsilyl, methyldiisopropylsilyl, methyl-di-t-butylsilyl and triisopropylsilyl groups, and
tri(lower alkyl)silyl groups in which at least one of said lower alkyl groups is replaced with 1 or 2 aryl groups as defined above, examples of which include diphenylmethylsilyl, diphenylbutylsilyl, diphenylisopropylsilyl and phenyldiisopropylsilyl groups;

(vii) alkoxymethyl groups, examples of which include
lower alkoxymethyl groups which comprise a methyl group which is substituted with a lower alkoxy group as defined above, examples of which include methoxymethyl, 1,1-dimethyl-1-methoxymethyl, ethoxymethyl, propoxymethyl, isopropoxymethyl, butoxymethyl and t-butoxymethyl groups,
lower alkoxylated lower alkoxymethyl groups which comprise a lower alkoxymethyl group as defined above in which the alkoxy moiety thereof is substituted with a lower alkoxy group as defined above, examples of which include 2-methoxyethoxymethyl groups, and
lower halogeno alkoxymethyl groups which comprise a lower alkoxymethyl group as defined above in which the alkoxy moiety thereof is substituted with at least one halogen atom, examples of which include 2,2,2-trichloroethoxymethyl and bis(2-chloroethoxy)methyl groups;

(viii) substituted ethyl groups, examples of which include
lower alkoxylated ethyl groups which comprise an ethyl group which is substituted with a lower alkoxy group as defined above, examples of which include 1-ethoxyethyl and 1-(isopropoxy)ethyl groups, and

halogenated ethyl groups such as 2,2,2-trichloroethyl groups;

(ix) aralkyl groups as define above, examples of which include
lower alkyl groups as defined above which are substituted with from 1 to 3 aryl groups as defined above, examples of which include benzyl, α-naphthylmethyl, β-naphthylmethyl, diphenylmethyl, triphenylmethyl, α-naphthyldiphenylmethyl and 9-anthrylmethyl groups, and
lower alkyl groups as defined above which are substituted with from 1 to 3 aryl groups as defined above in which said aryl moiety is substituted with at least one substituent selected from the group consisting of lower alkyl groups as defined above, lower alkoxy groups as defined above, nitro groups, halogen atoms and cyano groups, examples of which include 4-methylbenzyl, 2,4,6-trimethylbenzyl, 3,4,5-trimethylbenzyl, 4-methoxybenzyl, 4-methoxyphenydiphenylmethyl, 2-nitrobenzyl, 4-nitrobenzyl, 4-chlorobenzyl, 4-bromobenzyl and 4-cyanobenzyl groups;

(x) "alkenyloxycarbonyl groups" which comprise a carbonyl group which is substituted with an alkenyloxy group having from 2 to 6 carbon atoms, examples of which include vinyloxycarbonyl and allyloxycarbonyl groups; and

(xi) aralkyloxycarbonyl groups which comprise a carbonyl group which is substituted with an aralkyloxy group (which is an oxygen atom substituted with an aralkyl group as defined above), in which the aryl moiety thereof may optionally be substituted with one or two substituents selected from lower alkoxy groups as defined above and nitro groups, examples of which include benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups.

The protecting group which is capable of being removed by a metabolic process (e.g. hydrolysis) *in vivo* is one, which on administration to the body of a live mammal is removable by a metabolic process (e.g. hydrolysis) to give a compound of formula (I) or a salt thereof. Preferred examples of such a protecting group include the following:

(i) 1-(acyloxy)lower alkyl groups, examples of which include
1-(aliphatic acyloxy)lower alkyl groups which comprise a lower alkyl group as defined above which is substituted with an alkylcarbonyloxy group having from 1 to 6 carbon atoms, examples of which include formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxy-propyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl groups,
1-(cycloalkylcarbonyloxy)lower alkyl groups which comprise a lower alkyl group as defined above which is substituted with a cycloalkylcarbonyloxy group in which a carbonyloxy group is substituted with a cycloalkyl group as defined above, examples of which include cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl and 1-cyclohexylcarbonyloxybutyl groups, and
1-(aromatic acyloxy)lower alkyl groups which comprise a lower alkyl group as defined above which is substituted with an arylcarbonyloxy group which comprises an oxygen atom which is substituted with an arylcarbonyl group, examples of which include benzoyloxymethyl groups;

(ii) substituted carbonyloxyalkyl groups, examples of which include
(lower alkoxycarbonyloxy)alkyl groups which comprise a lower alkyl group as defined above or a cycloalkyl group as defined above which is substituted with a lower alkoxycarbonyloxy group which comprises a carbonyloxy group substituted with a lower alkoxy group as defined above or a cycloalkoxy group, examples of which include methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)-methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy) ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy)-ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)-propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl groups, and oxodioxolenylmethyl groups, which comprise a methyl group which is substituted with an oxodioxolenyl group which itself may optionally be substituted with a group selected from the group consisting of lower alkyl groups as defined above and aryl groups as defined above which may optionally be substituted with at least one lower alkyl group as defined above, lower alkoxy group as defined above or halogen atom, examples of which include (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)-methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl groups;

(iii) phthalidyl groups which comprise a phthalidyl group which may optionally be substituted with a substituent selected from the group consisting of lower alkyl groups as defined above and lower alkoxy groups as defined above, examples of which include phthalidyl, dimethylphthalidyl and dimethoxyphthalidyl groups;

(iv) aliphatic acyl groups as defined and exemplified above in relation to the general protecting group for a hydroxyl group;

(v) aromatic acyl groups as defined and exemplified above in relation to the general protecting group for a hydroxyl group;

(vi) half-ester salt residues of succinic acid;

(vii) phosphate ester salt residues;

(viii) ester-forming residues of an amino acid;

(ix) carbamoyl groups which may optionally be substituted with 1 or 2 lower alkyl groups as defined above; and

(x) 1-(acyloxy)alkoxycarbonyl groups which comprise a lower alkoxycarbonyl group as defined above in which the lower alkoxy moiety is substituted with an aliphatic acyloxy group as defined above or an aromatic acyloxy group as defined above, examples of which include pivaloyloxymethyloxycarbonyl groups.

Of the above protecting groups which are capable of being removed by a metabolic process (e.g. hydrolysis) *in vivo* which are used to synthesise a compound of formula (I-1) in which a hydroxyl residue therein is modified, the substituted carbonyloxyalkyl groups are preferred.

In the case where the compound of formula (I-1) of the present invention has an amino group, an imino group and/or a sulfonamide group, the compound can be converted to an amide rather than the esters described above and the pharmacologically acceptable salts described below. Example of such amide derivatives include those in which an aliphatic acyl group defined and exemplified above or an aromatic acyl group defined and exemplified above is bonded to a nitrogen atom of an amino group, imino group and/or sulfonamide group present in said compound of formula (I-1). Where said derivative is a pharmacologically acceptable amide of a compound of formula (I-1) it must be capable of being removed by a metabolic process (e.g. hydrolysis) on administration of said compound to the body of a live mammal to give a compound of formula (I-1) or a salt thereof.

Where the compound of formula (I-1) of the present invention or a pharmacologically acceptable ester or amide thereof has a basic group, such as an amino group, the compound can be converted to a salt by reacting it with an acid, and in the case where the compound of formula (I-1) of the present invention or a pharmacologically acceptable ester or amide thereof has an acidic group, such as a sulfonamide group, the compound can be converted to a salt by reacting it with a base. The compounds of the present invention encompass such salts. Where said salts are to be used for a therapeutic use, they must be pharmacologically acceptable.

Preferred examples of the salts formed with a basic group present in the compound of formula (I-1) of the present invention include inorganic acid salts such as hydrohalogenated acid salts (e.g. hydrochlorides, hydrobromides and hydroiodides), nitrates, perchlorates, sulfates and phosphates; organic acid salts such as lower alkanesulfonates in which the lower alkyl moiety thereof is as defined above (e.g. methanesulfonates, trifluoromethanesulfonates and ethanesulfonates), arylsulfonates in which the aryl moiety thereof is as defined above (e.g. benzenesulfonate or p-toluenesulfonate), acetates, malates, fumarates, succinates, citrates, ascorbates, tartrates, oxalates and maleates; and amino acid salts such as glycine salts, lysine salts, arginine salts, ornithine salts, glutamates and aspartates.

Preferred example of the salts formed with an acidic group present in the compound of formula (I-1) of the

present invention include metal salts such as alkali metal salts (e.g. sodium salts, potassium salts and lithium salts), alkali earth metal salts (e.g. calcium salts and magnesium salts), aluminum salts and iron salts; amine salts such as inorganic amine salts (e.g. ammonium salts) and organic amine salts (e.g. t-octylamine salts, dibenzylamine salts, morpholine salts, glucosamine salts, phenylglycinealkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethylammonium salts and tris(hydroxymethyl)aminomethane salts; and amino acid salts such as glycine salts, lysine salts, arginine salts, onzithine salts, glutamates and aspartates.

The compound of formula (I-1) of the present invention can sometimes take up water upon exposure to the atmosphere or when recrystallized to absorb water or to form a hydrate and such hydrates are also included within the scope of the present invention. Additionally, certain other solvents may be taken up by the compounds of the present invention to produce solvates, which also form a part of the present invention.

The compounds of formula (I-1) of the present invention can sometimes exist in the form of geometrical isomers (cis and trans isomers, or E and Z isomers) and, where said compounds contain one or more asymmetric centres, optical isomers. For the compounds of the present invention, each of said isomers and mixture of said isomers are depicted by a single formula, i.e. the formula (I-1). Accordingly, the present invention covers both the individual isomers and mixtures thereof in any proportion, including racemic mixtures.

Preferred classes of compounds of the present invention are those compounds of formula (I-1) and pharmacologically acceptable salts, esters and amides thereof wherein:

(A) $R^1$ is an aryl group which may optionally be substituted with at least one substituent selected from Substituent group $\alpha$ defined above and Substituent group $\beta$ defined above;

(B) $R^1$ is a phenyl or naphthyl group, said groups optionally being substituted with at least one substituent selected from Substituent group $\alpha$ defined above and Substituent group $\beta$ defined above;

(C) $R^1$ is a phenyl group which may optionally be substituted with at least one substituent selected from Substituent group $\alpha^1$ defined above and Substituent group $\beta^1$ defined above;

(D) $R^1$ is a phenyl group which may optionally be substituted with at least one substituent selected from the group consisting of halogen atoms, halogeno lower alkyl groups defined above and halogeno lower alkoxy groups defined above;

(E) $R^1$ is a substituent selected from the group consisting of phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl and 3-trifluoromethylphenyl groups;

(F) $R^2$ is a 5- or 6-membered aromatic heterocyclic group which has one or two nitrogen atoms, said group optionally being substituted with at least one substituent selected from Substituent group $\alpha$ defined above and Substituent group $\beta$ defined above;

(G) $R^2$ is a pyridyl or pyrimidinyl group, said groups optionally being substituted with at least one substituent selected from the group consisting of Substituent group $\alpha$ defined above and Substituent group $\beta$ defined above;

(H) $R^2$ is a 4-pyridyl or 4-pyrimidinyl group, said groups optionally being substituted with at least one substituent selected from the group consisting of Substituent group $\alpha$ defined above and Substituent group $\beta$ defined above;

(I) $R^2$ is a 4-pyridyl or 4-pyrimidinyl group, said groups optionally being substituted at the 2-position thereof with a substituent selected from the group consisting of Substituent group $\alpha$ defined above and Substituent group $\beta$ defined above;

(J) $R^2$ is a 4-pyridyl or 4-pyrimidinyl group, said groups optionally being substituted at the 2-position thereof with a substituent selected from the group consisting ofmethoxy, amino, methylamino, benzylamino, and $\alpha$-methylbenzylamino groups;

(K) B is a 5- or 6-membered heterocyclic ring which has one ring nitrogen atom and optionally has one further ring heteroatom or ring group selected from a nitrogen atom, oxygen atom, sulfur atom, >SO and >$SO_2$ (said ring may be saturated or unsaturated and may optionally be fused with an aryl group defined above, a heteroaryl group

defined above, a cycloalkyl group defined above or a heterocyclyl group defined above);

(L) B is a 5- or 6-membered heterocyclic ring which consists of the group D, the group E and three or four carbon atoms (said ring may be saturated or unsaturated and may optionally be fused with an aryl group defined above, a heteroaryl group defined above, a cycloalkyl group defined above or a heterocyclyl group defined above);

(M) B is a pyrrolidinyl ring or a pyrrolinyl ring;

(N) $R^3$ is a group of general formula (IIa) or general formula (IIb);

(O) $R^3$ is a group of general formula (IIa);

(P) m is 1;

(Q) $R^4$ is 1 or 2 substituents which are independently selected from the group consisting of Substituent group $\alpha$ defined above, Substituent group $\beta$ defined above and Substituent group $\gamma^1$, wherein Substituent group $\gamma^1$ consists of oxo groups, hydroxyimino groups, lower alkoxyimino groups defined above, lower alkylene groups defined above, lower alkylenedioxy groups defined above, lower alkylsulfinyl groups defined above, lower alkylsulfonyl groups defined above and aryl groups defined above which may optionally be substituted with at least one substituent selected from Substituent group $\alpha$ defined above and Substituent group $\beta$ defined above;

(R) $R^4$ is a substituent selected from the group consisting of hydroxy groups, halogen atoms, lower alkoxy groups defined above, lower alkylthio groups defined above, halogeno lower alkoxy groups defined above, lower alkyl groups defined above, halogeno lower alkyl groups defined above, oxo groups, aryl groups defined above optionally substituted with at lest one substituent selected from Substituent group $\alpha$ defined above and Substituent group $\beta$ defined above, lower alkylenedioxy groups defined above, lower alkylene groups defined above and lower alkylsulfonyl groups defined above;

(S) $R^4$ is a substituent selected from the group consisting of hydroxy groups, fluorine atoms, chlorine atoms, methoxy groups, ethoxy groups, propoxy groups, methyl groups, ethyl groups, propyl groups and phenyl groups which may optionally be substituted with at least one substituent selected from Substituent group $\alpha$ defined above and Substituent group $\beta$ defined above;

(T) $R^4$ is a substituent selected from the group consisting of methoxy groups, methyl groups, ethyl groups, propyl groups and phenyl groups;

(U) $R^4$ is a substituent selected from the group consisting of aryloxy groups defined above which may optionally be substituted with at least one substituent selected from Substituent group $\alpha$ defined above and Substituent group $\beta$ defined above, alkylidene groups defined above and aralkylidene groups defined above;

(V) $R^4$ is a substituent selected from the group consisting of phenoxy, methylidene, ethylidene, propylidene and benzylidene groups ;

(W) D is a group of formula >C($R^5$)- (wherein $R^5$ is selected from the group consisting of hydrogen atoms, Substituent group $\alpha$ defined above and Substituent group $\beta$ defined above) and E is a nitrogen atom;

Compounds of formula (I-1) which comprise any combination of the factors selected freely from the eight groups consisting of (A) to (E) above; (F) to (J) above; (K) to (M) above; (N) and (O) above; (P) above; (Q) to (V) above; and (W) above are preferred.

More preferred compounds of the present invention are compounds of formula (I-1) and pharmacologically acceptable salts, esters and amides thereof, wherein:

(i) $R^1$ is as defined in (A) above, $R^2$ is as defined in (F) above and $R^3$ is as defined in (N) above wherein m is as defined in (P) above, D and E are as defined in (W) above $R^4$ is as defined in (Q) above;

(ii) $R^1$ is as defined in (B) above, $R^2$ is as defined in (G) above and $R^3$ is as defined in (N) above wherein m is as defined in (P) above, D and E are as defined in (W) above and $R^4$ is as defined in (R) above;

(iii) $R^1$ is as defined in (C) above, $R^2$ is as defined in (H) above and $R^3$ is as defined in (N) above wherein m is as defined in (P) above, D and E are as defined in (W) above and $R^4$ is as defined in (R) above;

(iv) $R^1$ is as defined in (D) above, $R^2$ is as defined in (I) above and $R^3$ is as defined in (O) above wherein m is as defined in (P) above, D and E are as defined in (W) above and $R^4$ is as defined in (S) above;

(v) $R^1$ is as defined in (E) above, $R^2$ is as defined in (J) above and $R^3$ is as defined in (O) above wherein m is as defined in (P) above, D and E are as defined in (W) above and $R^4$ is as defined in (T) above;

(vi) $R^1$ is as defined in (A) above, $R^2$ is as defined in (F) above and $R^3$ is as defined in (N) above wherein m is as defined in (P) above, D and E are as defined in (W) above and $R^4$ is as defined in (U) above;

(vii) $R^1$ is as defined in (B) above, $R^2$ is as defined in (G) above and $R^3$ is as defined in (N) above wherein m is as defined in (P) above, D and E are as defined in (W) above and $R^4$ is as defined in (U) above;

(viii) $R^1$ is as defined in (C) above, $R^2$ is as defined in (H) above and $R^3$ is as defined in (N) above wherein m is as defined in (P) above, D and E are as defined in (W) above and $R^4$ is as defined in (U) above;

(ix) $R^1$ is as defined in (D) above, $R^2$ is as defined in (I) above and $R^3$ is as defined in (O) above wherein m is as defined in (P) above, D and E are as defined in (W) above and $R^4$ is as defined in (V) above; and

(x) $R^1$ is as defined in (E) above, $R^2$ is as defined in (J) above and $R^3$ is as defined in (O) above wherein m is as defined in (P) above, D and E are as defined in (W) above and $R^4$ is as defined in (V) above.

Of the above, preferred compounds of the present invention are compounds of formula (I-1) selected from the following group of compounds, and pharmacologically acceptable salts, esters and amides thereof:

2-(3-fluorophenyl)-4-[2-methyl-1,2,3,5,6,8a-hexahydroindolizin-7-yl]-3-(pyridin-4-yl)-1*H*-pyrrole,
2-(3-fluorophenyl)-4-[2-phenyl-1,2,3,5,6,8a-hexahydroindolizin-7-yl]-3-(pyridin-4-yl)-1*H*-pyrrole,
2-(3-fluorophenyl)-4-[2-hydroxy-1,2,3,5,6,8a-hexahydroindolizin-7-yl]-3-(pyridin-4-yl)-1*H*-pyrrole,
2-(3-fluorophenyl)-4-[2-methoxy-1,2,3,5,6,8a-hexahydroindolizin-7-yl]-3-(pyridin-4-yl)-1*H*-pyrrole,
4-[2-fluoro-1,2,3,5,6,8a-hexahydroindolizin-7-yl]-2-(3-fluorophenyl)-3-(pyridin-4-yl)-1*H*-pyrrole,
2-(4-fluorophenyl)-4-[2-methyl-1,2,3,5,6,8a-hexahydroindolizin-7-yl]-3-(pyridin-4-yl)-1*H*-pyrrole,
2-(4-fluorophenyl)-4-[2-phenyl-1,2,3,5,6,8a-hexahydroindolizin-7-yl]-3-(pyridin-4-yl)-1*H*-pyrrole,
2-(4-fluorophenyl)-4-[2-hydroxy-1,2,3,5,6,8a-hexahydroindolizin-7-yl]-3-(pyridin-4-yl)-1*H*-pyrrole,
2-(4-fluorophenyl)-4-[2-methoxy-1,2,3,5,6,8a-hexahydroindolizin-7-yl]-3-(pyridin-4-yl)-1*H*-pyrrole,
4-[2,2-ethylenedioxy-1,2,3,5,6,8a-hexahydroindolizin-7-yl]-2-(4-fluorophenyl)-3-(pyridin-4-yl)-1*H*-pyrrole,
2-(4-nuorophenyl)-4-[2-oxo-1,2,3,5,6,8a-hexahydroindotizin-7-yl]-3-(pyridin-4-yl)-1*H*-pyrrole,
4-[2-fluoro-1,2,3,5,6,8a-hexahydroindolizin-7-yl]-2-(4-fluorophenyl)-3-(pyridin-4-yl)-1*H*-pyrrole,
4-[2-chloro-1,2,3,5,6,8a-hexahydroindolizin-7-yl]-2-(4-fluorophenyl)-3-(pyridin-4-yl)-1*H*-pyrrole,
4-[2,2-difluoro-1,2,3,5,6,8a-hexahydroindolizin-7-yl]-2-(4-fluorophenyl)-3-(pyridin-4-yl)-1*H*-pyrrole,
2-(3-chlorophenyl)-4-[2-methyl-1,2,3,5,6,8a-hexahydroindolizin-7-yl]-3-(pyridin-4-yl)-1*H*-pyrrole,
2-(3-chlorophenyl)-4-[2-phenyl-1,2,3,5,6,8a-hexahydroindolizin-7-yl]-3-(pyridin-4-yl)-1*H*-pyrrole,
2-(3-chlorophenyl)-4-[2-hydroxy-1,2,3,5,6,8a-hexahydroindolizin-7-yl]-3-(pyridin-4-yl)-1*H*-pyrrole,
2-(3-chlorophenyl)-4-[2-methoxy-1,2,3,5,6,8a-hexahydroindolizin-7-yl]-3-(pyridin-4-yl)-1*H*-pyrrole,
2-(3-chlorophenyl)-4-[2-fluoro-1,2,3,5,6,8a-hexahydroindolizin-7-yl]-3-(pyridin-4-yl)-1*H*-pyrrole,
4-[2-chloro-1,2,3,5,6,8a-hexahydroindolizin-7-yl]-2-(3-chlorophenyl)-3-(pyridin-4-yl)-1*H*-pyrrole,
4-[2-methyl-1,2,3,5,6,8a-hexahydroindolizin-7-yl]-3-(pyridin-4-yl)-2-(3-trifluoromethylphenyl)-1*H*-pyrrole,
4-[2-phenyl-1,2,3,5,6,8a-hexahydroindolizin-7-yl]-3-(pyridin-4-yl)-2-(3-trifluoromethylphenyl)-1*H*-pyrrole,
4-[2-hydroxy-1,2,3,5,6,8a-hexahydroindolizin-7-yl]-3-(pyridin-4-yl)-2-(3-trifluoromethylphenyl)-1*H*-pyrrole,
4-[2-methoxy-1,2,3,5,6,8a-hexahydroindolizin-7-yl]-3-(pyridin-4-yl)-2-(3-trifluoromethylphenyl)-1*H*-pyrrole,
4-[2-fluoro-1,2,3,5,6,8a-hexahydroindolizin-7-yl]-3-(pyridin-4-yl)-2-(3-trifluoromethylphenyl)-1*H*-pyrrole,
4-[2-chloro-1,2,3,5,6,8a-hexahydroindolizin-7-yl]-3-(pyridin-4-yl)-2-(3-trifluoromethylphenyl)-1*H*-pyrrole,
4-[2,8-dimethyl-1,2,3,5,6,8a-hexahydroindolizin-7-yl]-2-(4-fluorophenyl)-3-(pyridin-4-yl)-1*H*-pyrrole,
2-(4-fluorophenyl)-4-[2-hydroxy-8-methyl-1,2,3,5,6,8a-hexahydroindolizin-7-yl]-3-(pyridin-4-yl)-1*H*-pyrrole,
2-(4-fluorophenyl)-4-[2-methoxy-8-methyl-1,2,3,5,6,8a-hexahydroindolizin-7-yl]-3-(pyridin-4-yl)-1*H*-pyrrole,
4-[2-fluoro-8-methyl-1,2,3,5,6,8a-hexahydroindolizin-7-yl]-2-(4-fluorophenyl)-3-(pyridin-4-yl)-1*H*-pyrrole,
4-[2-chloro-8-methyl-1,2,3,5,6,8a-hexahydroindolizin-7-yl]-2-(4-fluorophenyl)-3-(pyridin-4-yl)-1*H*-pyrrole,

2-(4-fluorophenyl)-4-[2-methyl-3,5,6,8a-tetrahydroindolizin-7-yl]-3-(pyridin-4-yl)-1*H*-pyrrole,
2-(4-fluorophenyl)-4-[2-hydroxy-1,2,3,5,8,8a-hexahydroindolizin-7-yl]-3-(pyridin-4-yl)-1*H*-pyrrole,
2-(4-fluorophenyl)-4-[2-methoxy-1,2,3,5,8,8a-hexahydroindolizin-7-yl]-3-(pyridin-4-yl)-1*H*-pyrrole,
4-[2-chloro-1,2,3,5,8,8a-hexahydroindolizin-7-yl]-2-(4-fluorophenyl)-3-(pyridin-4-yl)-1*H*-pyrrole,
4-[2,2-difluoro-1,2,3,5,8,8a-hexahydroindolizin-7-yl]-2-(4-fluorophenyl)-3-(pyridin-4-yl)-1*H*-pyrrole,
4-[cyclopropanespiro-6'-(1',2',3',5',6',8a'-hexahydroindolizin)-7'-yl]-2-(4-fluorophenyl)-3-(pyridin-4-yl)-1*H*-pyrrole,
2-(4-fluorophenyl)-4-[2-methylidene-1,2,3,5,6,8a-hexahydroindolizin-7-yl]-3-(pyridin-4-yl)-1*H*-pyrrole,
4-[2,2-dimethyl-1,2,3,5,6,8a-hexahydroindolizin-7-yl]-2-(4-fluorophenyl)-3-(pyridin-4-yl)-1*H*-pyrrole,
2-(4-fluorophenyl)-4-[2-methylthio-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-2-(4-fluorophenyl)-3-(pyridin-4-yl)-1*H*-pyrrole,
4-[2-ethyl-1,2,3,5,6,8a-hexahydroindolizin-7-yl]-2-(3-fluorophenyl)-3-(pyridin-4-yl)-1*H*-pyrrole,
2-(3-chlorophenyl)-4-[2-ethyl-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-3-(pyridin-4-yl)-2-(3-trifluoromethylphenyl)-1*H*-pyrrole,
2-(4-chlorophenyl)-4-[2-ethyl-1,2,3,5,6,8a-hexahydroindolizin-7-yl]-3-(pyridin-4-yl)-1*H*-pyrrole,
4-[2-butylthio-1,2,3,5,6,8a-hexahydroindolizin-7-yl]-2-(4-fluorophenyl)-3-(pyridin-4-yl)-1*H*-pyrrole,
4-[2-ethylthio-1,2,3,5,6,8a-hexahydroindolizin-7-yl]-2-(4-fluorophenyl)-3-(pyridin-4-yl)-1*H*-pyrrole,
4-[2-ethylidene-1,2,3,5,6,8a-hexahydroindolizin-7-yl]-2-(4-fluorophenyl)-3-(pyridin-4-yl)-1*H*-pyrrole,
2-(4-fluorophenyl)-4-[2-propyl-1,2,3,5,6,8a-hexahydroindolizin-7-yl]-3-(pyridin-4-yl)-1*H*-pyrrole,
2-(3-fluorophenyl)-4-[2-propyl-1,2,3,5,6,8a-hexahydroindolizin-7-yl]-3-(pyridin-4-yl)-1*H*-pyrrole,
2-(3-chlorophenyl)-4-[2-propyl-1,2,3,5,6,8a-hexahydroindolizin-7-yl]-3-(pyridin-4-yl)-1*H*-pyrrole,
4-[2-propyl-1,2,3,5,6,8a-hexahydroindolizin-7-yl]-3-(pyridin-4-yl)-2-(3-trifluoromethylphenyl)-1*H*-pyrrole,
2-(4-chlorophenyl)-4-[2-propyl-1,2,3,5,6,8a-hexahydroindolizin-7-yl]-3-(pyridin-4-yl)-1*H*-pyrrole,
4-[2-ethoxy-1,2,3,5,6,8a-hexahydroindolizin-7-yl]-2-(4-fluorophenyl)-3-(pyridin-4-yl)-1*H*-pyrrole,
4-[cyclopentanespiro-2'-(1',2',3',5',6',8a'-hexahydroindolizin)-7'-yl]-2-(4-fluorophenyl)-3-(pyridin-4-yl)-1*H*-pyrrole,
4-[2-benzylidene-1,2,3,5,6,8a-hexahydroindolizin-7-yl]-2-(4-fluorophenyl)-3-(pyridin-4-yl)-1*H*-pyrrole,
2-(4-fluorophenyl)-4-[2-propylidene-1,2,3,5,6,8a-hexahydroindolizin-7-yl]-3-(pyridin-4-yl)-1*H*-pyrrole,
4-[5,5-dimethyl-1,2,3,5,6,8a-hexahydroindolizin-7-yl]-2-(4-fluorophenyl)-3-(pyridin-4-yl)-1*H*-pyrrole,
4-[2-ethyl-3,5,6,8a-tetrahydroindolizin-7-yl]-2-(4-fluorophenyl)-3-(pyridin-4-yl)-1*H*-pyrrole,
2-(4-fluorophenyl)-4-[2-propyl-3,5,6,8a-tetrahydroindolizin-7-yl]-3-(pyridin-4-yl)-1*H*-pyrrole,
2-(4-fluorophenyl)-4-[2-phenyl-3,5,6,8a-tetrahydroindolizin-7-yl]-3-(pyridin-4-yl)-1*H*-pyrrole,
2-(4-chlorophenyl)-4-[2-methoxy-1,2,3,5,6,8a-hexahydroindolizin-7-yl]-3-(pyridin-4-yl)-1*H*-pyrrole,
2-(4-chlorophenyl)-4-[2-methyl-1,2,3,5,6,8a-hexahydroindolizin -7-yl]-3-(pyridin-4-yl)-1*H*-pyrrole, and
2-(4-chlorophenyl)-4-[2-phenyl-1,2,3,5,6,8a-hexahydroindolizin -7-yl]-3-(pyridin-4-yl)-1*H*-pyrrole.

Of the above, more preferred compounds of the present invention are compounds of formula (I-1) selected from the following group of compounds, and pharmacologically acceptable salts and amides thereof:

2-(3-fluorophenyl)-4-[2-methyl-1,2,3,5,6,8a-hexahydroindolizin-7-yl]-3-(pyridin-4-yl)-1*H*-pyrrole,
2-(3-fluorophenyl)-4-[2-phenyl-1,2,3,5,6,8a-hexahydroindolizin-7-yl]-3-(pyridin-4-yl)-1*H*-pyrrole,
2-(3-fluorophenyl)-4-[2-methoxy-1,2,3,5,6,8a-hexahydroindolizin-7-yl]-3-(pyridin-4-yl)-1*H*-pyrrole,
2-(4-fluorophenyl)-4-[2-methyl-1,2,3,5,6,8a-hexahydroindolizin-7-yl]-3-(pyridin-4-yl)-1*H*-pyrrole,
2-(4-fluorophenyl)-4-[2-phenyl-1,2,3,5,6,8a-hexahydroindolizin-7-yl]-3-(pyridin-4-yl)-1*H*-pyrrole,
2-(4-fluorophenyl)-4-[2-methoxy-1,2,3,5,6,8a-hexahydroindolizin-7-yl]-3-(pyridin-4-yl)-1*H*-pyrrole,
2-(3-chlorophenyl)-4-[2-methyl-1,2,3,5,6,8a-hexahydroindolizin-7-yl]-3-(pyridin-4-yl)-1*H*-pyrrole,
2-(3-chlorophenyl)-4-[2-phenyl-1,2,3,5,6,8a-hexahydroindolizin-7-yl]-3-(pyridin-4-yl)-1*H*-pyrrole,
2-(3-chlorophenyl)-4-[2-methoxy-1,2,3,5,6,8a-hexahydroindolizin-7-yl]-3-(pyridin-4-yl)-1*H*-pyrrole,
4-[2-methyl-1,2,3,5,6,8a-hexahydroindolizin-7-yl]-3-(pyridin-4-yl)-2-(3-trifluoromethylphenyl)-1*H*-pyrrole,
4-[2-phenyl-1,2,3,5,6,8a-hexahydroindolizin-7-yl]-3-(pyridin-4-yl)-2-(3-trifluoromethylphenyl)-1*H*-pyrrole,
4-[2-methoxy-1,2,3,5,6,8a-hexahydroindolizin-7-yl]-3-(pyridin-4-yl)-2-(3-trifluoromethylphenyl)-1*H*-pyrrole,
2-(4-fluorophenyl)-4-[2-methyl-3,5,6,8a-tetrahydroindolizin-7-yl]-3-(pyridin-4-yl)-1H-pyrrole,
2-(4-fluorophenyl)-4-[2-methylidene-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-2-(4-fluorophenyl)-3-(pyridin-4-yl)-1*H*-pyrrole,
4-[2-ethyl-1,2,3,5,6,8a-hexahydroindolizin-7-yl]-2-(3-fluorophenyl)-3-(pyridin-4-yl)-1*H*-pyrrole,
2-(3-chlorophenyl)-4-[2-ethyl-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-3-(pyridin-4-yl)-2-(3-trifluoromethylphenyl)-1*H*-pyrrole,
2-(4-fluorophenyl)-4-[2-propyl-1,2,3,5,6,8a-hexahydroindolizin-7-yl]-3-(pyridin-4-yl)-1*H*-pyrrole,
2-(3-fluorophenyl)-4-[2-propyl-1,2,3,5,6,8a-hexahydroindolizin-7-yl]-3-(pyridin-4-yl)-1*H*-pyrrole,
2-(3-chlorophenyl)-4-[2-propyl-1,2,3,5,6,8a-hexahydroindolizin-7-yl]-3-(pyridin-4-yl)-1*H*-pyrrole,

4-[2-propyl-1,2,3,5,6,8a-hexahydroindolizin-7-yl]-3-(pyridin-4-yl)-2-(3-trifluoromethylphenyl)-1*H*-pyrrole,
4-[2-ethyl-3,5,6,8a-tetrahydroindolizin-7-yl]-2-(4-fluorophenyl)-3-(pyridin-4-yl)-1*H*-pyrrole,
2-(4-fluorophenyl)-4-[2-propyl-3,5,6,8a-tetrahydroindolizin-7-yl]-3-(pyridin-4-yl)-1*H*-pyrrole, and
2-(4-fluorophenyl)-4-[2-phenyl-3,5,6,8a-tetrahydroindolizin-7-yl]-3-(pyridin-4-yl)-1*H*-pyrrole.

Of the above, the most preferred compounds of the present invention are compounds of formula (I-1) selected from the following group of compounds, and pharmacologically acceptable salts and amides thereof:

2-(4-fluorophenyl)-4-[(2R,8aS)-2-phenyl-1,2,3,5,6,8a-hexahydroindolizin-7-yl]-3-(pyridin-4-yl)-1*H*-pyrrole,
2-(4-fluorophenyl)-4-[(8aS)-2-methyl-1,2,3,5,6,8a-hexahydroindolizin-7-yl]-3-(pyridin-4-yl)-1*H*-pyrrole,
2-(4-fluorophenyl)-4-[(8aS)-2-methylidene-1,2,3,5,6,8a-hexahydroindolizin-7-yl]-3-(pyridin-4-yl)-1*H*-pyrrole,
2-(4-fluorophenyl)-4-[(8aS)-2-methyl-3,5,6,8a-tetrahydroindolizin-7-yl]-3-(pyridin-4-yl)-1*H*-pyrrole,
4-[(2S,8aS)-2-ethyl-1,2,3,5,6,8a-hexahydroindolizin-7-yl)-2-(4-fluorophenyl)-3-(pyridin-4-yl)-1*H*-pyrrole, and
2-(4-fluorophenyl)-4-[(2S,8aS)-2-propyl-1,2,3,5,6,8a-hexahydroindolizin-7-yl]-3-(pyridin-4-yl)-1*H*-pyrrole.

Specific examples of compounds of formula (I-1) of the present invention include the following compounds in Tables 1 to 6 below.

**Table 1**

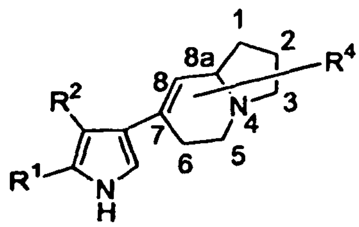

| Compound No. | $R^1$ | $R^2$ | $R^4$ |
|---|---|---|---|
| 1-1 | Ph | 4-Pyr | 1-Me |
| 1-2 | Ph | 4-Pyr | 1-Et |
| 1-3 | Ph | 4-Pyr | 1-Pr |
| 1-4 | Ph | 4-Pyr | 1,1-diMe |
| 1-5 | Ph | 4-Pyr | 2-Me |
| 1-6 | Ph | 4-Pyr | 2-Et |
| 1-7 | Ph | 4-Pyr | 2-Pr |
| 1-8 | Ph | 4-Pyr | 2-Bu |
| 1-9 | Ph | 4-Pyr | 2-Allyl |
| 1-10 | Ph | 4-Pyr | 2-Ph |
| 1-11 | Ph | 4-Pyr | 2-Bn |
| 1-12 | Ph | 4-Pyr | 2-Phet |
| 1-13 | Ph | 4-Pyr | 2,2-diMe |
| 1-14 | Ph | 4-Pyr | 2-OH |
| 1-15 | Ph | 4-Pyr | 2-MeO |
| 1-16 | Ph | 4-Pyr | 2-EtO |
| 1-17 | Ph | 4-Pyr | 2-PrO |
| 1-18 | Ph | 4-Pyr | 2,2-di(MeO) |
| 1-19 | Ph | 4-Pyr | 2,2-di(EtO) |

Table continued

| Compound No. | $R^1$ | $R^2$ | $R^4$ |
|---|---|---|---|
| 1-20 | Ph | 4-Pyr | 2,2-$OCH_2CH_2O$- |
| 1-21 | Ph | 4-Pyr | 2-Oxo |
| 1-22 | Ph | 4-Pyr | 2-F |
| 1-23 | Ph | 4-Pyr | 2-Cl |
| 1-24 | Ph | 4-Pyr | 2-Pr |
| 1-25 | Ph | 4-Pyr | 2-1 |
| 1-26 | Ph | 4-Pyr | 2,2-diF |
| 1-27 | Ph | 4-Pyr | 2,2-diCl |
| 1-28 | Ph | 4-Pyr | 2,2-diBr |
| 1-29 | Ph | 4-Pyr | 3-Me |
| 1-30 | Ph | 4-Pyr | 3-Et |
| 1-31 | Ph | 4-Pyr | 3-Pr |
| 1-32 | Ph | 4-Pyr | 3,3-diMe |
| 1-33 | Ph | 4-Pyr | 5-Me |
| 1-34 | Ph | 4-Pyr | 5-Et |
| 1-35 | Ph | 4-Pyr | 5-Pr |
| 1-36 | Ph | 4-Pyr | 5,5-diMe |
| 1-37 | Ph | 4-Pyr | 6-Me |
| 1-38 | Ph | 4-Pyr | 6-Et |
| 1-39 | Ph | 4-Pyr | 6-Pr |
| 1-40 | Ph | 4-Pyr | 6,6-diMe |
| 1-41 | Ph | 4-Pyr | 6-Oxo |
| 1-42 | Ph | 4-Pyr | 8-Me |
| 1-43 | Ph | 4-Pyr | 8-Et |
| 1-44 | Ph | 4-Pyr | 8-Pr |
| 1-45 | Ph | 4-Pyr | 8-Ph |
| 1-46 | Ph | 4-Pyr | 8a-Me |
| 1-47 | Ph | 4-Pyr | 8a-Et |
| 1-48 | Ph | 4-Pyr | 8a-Pr |
| 1-49 | Ph | 2-$NH_2$-4-Pym | 1-Me |
| 1-50 | Ph | 2-$NH_2$-4-Pym | 1-Et |
| 1-51 | Ph | 2-$NH_2$-4-Pym | 1-Pr |
| 1-52 | Ph | 2-$NH_2$-4-Pym | 1,1-diMe |
| 1-53 | Ph | 2-$NH_2$-4-Pym | 2-Me |
| 1-54 | Ph | 2-$NH_2$-4-Pym | 2-Et |
| 1-55 | Ph | 2-$NH_2$-4-Pym | 2-Pr |
| 1-56 | Ph | 2-$NH_2$-4-Pym | 2-Bu |
| 1-57 | Ph | 2-$NH_2$-4-Pym | 2-Allyl |

Table continued

| Compound No. | $R^1$ | $R^2$ | $R^4$ |
|---|---|---|---|
| 1-58 | Ph | 2-$NH_2$-4-Pym | 2-Ph |
| 1-59 | Ph | 2-$NH_2$-4-Pym | 2-Bn |
| 1-60 | Ph | 2-$NH_2$-4-Pym | 2-Phet |
| 1-61 | Ph | 2-$NH_2$-4-Pym | 2,2-diMe |
| 1-62 | Ph | 2-$NH_2$-4-Pym | 2-OH |
| 1-63 | Ph | 2-$NH_2$-4-Pym | 2-MeO |
| 1-64 | Ph | 2-$NH_2$-4-Pym | 2-EtO |
| 1-65 | Ph | 2-$NH_2$-4-Pym | 2-PrO |
| 1-66 | Ph | 2-$NH_2$-4-Pym | 2,2-di(MeO) |
| 1-67 | Ph | 2-$NH_2$-4-Pym | 2,2-di(EtO) |
| 1-68 | Ph | 2-$NH_2$-4-Pym | 2,2-$OCH_2CH_2O$- |
| 1-69 | Ph | 2-$NH_2$-4-Pym | 2-Oxo |
| 1-70 | Ph | 2-$NH_2$-4-Pym | 2-F |
| 1-71 | Ph | 2-$NH_2$-4-Pym | 2-Cl |
| 1-72 | Ph | 2-$NH_2$-4-Pym | 2-Br |
| 1-73 | Ph | 2-$NH_2$-4-Pym | 2-I |
| 1-74 | Ph | 2-$NH_2$-4-Pym | 2,2-diF |
| 1-75 | Ph | 2-$NH_2$-4-Pym | 2,2-diCl |
| 1-76 | Ph | 2-$NH_2$-4-Pym | 2,2-diBr |
| 1-77 | Ph | 2-$NH_2$-4-Pym | 3-Me |
| 1-78 | Ph | 2-$NH_2$-4-Pym | 3-Et |
| 1-79 | Ph | 2-$NH_2$-4-Pym | 3-Pr |
| 1-80 | Ph | 2-$NH_2$-4-Pym | 3,3-diMe |
| 1-81 | Ph | 2-$NH_2$-4-Pym | 5-Me |
| 1-82 | Ph | 2-$NH_2$-4-Pym | 5-Et |
| 1-83 | Ph | 2-$NH_2$-4-Pym | 5-Pr |
| 1-84 | Ph | 2-$NH_2$-4-Pym | 5,5-diMe |
| 1-85 | Ph | 2-$NH_2$-4-Pym | 6-Me |
| 1-86 | Ph | 2-$NH_2$-4-Pym | 6-Et |
| 1-87 | Ph | 2-$NH_2$-4-Pym | 6-Pr |
| 1-88 | Ph | 2-$NH_2$-4-Pym | 6,6,-diMe |
| 1-89 | Ph | 2-$NH_2$-4-Pym | 6-Oxo |
| 1-90 | Ph | 2-$NH_2$-4-Pym | 8-Me |
| 1-91 | Ph | 2-$NH_2$-4-Pym | 8-Et |
| 1-92 | Ph | 2-$NH_2$-4-Pym | 8-Pr |
| 1-93 | Ph | 2-$NH_2$-4-Pym | 8-Ph |
| 1-94 | Ph | 2-$NH_2$-4-Pym | 8a-Me |
| 1-95 | Ph | 2-$NH_2$-4-Pym | 8a-Et |

Table continued

| Compound No. | $R^1$ | $R^2$ | $R^4$ |
|---|---|---|---|
| 1-96 | Ph | $2-NH_2-4-Pym$ | 8a-Pr |
| 1-97 | Ph | 2-MeNH-4-Pym | 1-Me |
| 1-98 | Ph | 2-MeNH-4-Pym | 1-Et |
| 1-99 | Ph | 2-MeNH-4-Pym | 1-Pr |
| 1-100 | Ph | 2-MeNH-4-Pym | 1,1-diMe |
| 1-101 | Ph | 2-MeNH-4-Pym | 2-Me |
| 1-102 | Ph | 2-MeNH-4-Pym | 2-Et |
| 1-103 | Ph | 2-MeNH-4-Pym | 2-Pr |
| 1-104 | Ph | 2-MeNH-4-Pym | 2-Bu |
| 1-105 | Ph | 2-MeNH-4-Pym | 2-Allyl |
| 1-106 | Ph | 2-MeNH-4-Pym | 2-Ph |
| 1-107 | Ph | 2-MeNH-4-Pym | 2-Bn |
| 1-108 | Ph | 2-MeNH-4-Pym | 2-Phet |
| 1-109 | Ph | 2-MeNH-4-Pym | 2,2-diMe |
| 1-110 | Ph | 2-MeNH-4-Pym | 2-OH |
| 1-111 | Ph | 2-MeNH-4-Pym | 2-MeO |
| 1-112 | Ph | 2-MeNH-4-Pym | 2-EtO |
| 1-113 | Ph | 2-MeNH-4-Pym | 2-PrO |
| 1-114 | Ph | 2-MeNH-4-Pym | 2,2-di(MeO) |
| 1-115 | Ph | 2-MeNH-4-Pym | 2,2-di(EtO) |
| 1-116 | Ph | 2-MeNH-4-Pym | $2,2-OCH_2CH_2O-$ |
| 1-117 | Ph | 2-MeNH-4-Pym | 2-Oxo |
| 1-118 | Ph | 2-MeNH-4-Pym | 2-F |
| 1-119 | Ph | 2-MeNH-4-Pym | 2-Cl |
| 1-120 | Ph | 2-MeNH-4-Pym | 2-Br |
| 1-121 | Ph | 2-MeNH-4-Pym | 2-1 |
| 1-122 | Ph | 2-MeNH-4-Pym | 2,2-diF |
| 1-123 | Ph | 2-MeNH-4-Pym | 2,2-diCl |
| 1-124 | Ph | 2-MeNH-4-Pym | 2,2-diBr |
| 1-125 | Ph | 2-MeNH-4-Pym | 3-Me |
| 1-126 | Ph | 2-MeNH-4-Pym | 3-Et |
| 1-127 | Ph | 2-MeNH-4-Pym | 3-Pr |
| 1-128 | Ph | 2-MeNH-4-Pym | 3,3-diMe |
| 1-129 | Ph | 2-MeNH-4-Pym | 5-Me |
| 1-130 | Ph | 2-MeNH-4-Pym | 5-Et |
| 1-131 | Ph | 2-MeNH-4-Pym | 5-Pr |
| 1-132 | Ph | 2-MeNH-4-Pym | 5,5-diMe |
| 1-133 | Ph | 2-MeNH-4-Pym | 6-Me |

Table continued

| Compound No. | $R^1$ | $R^2$ | $R^4$ |
|---|---|---|---|
| 1-134 | Ph | 2-MeNH-4-Pym | 6-Et |
| 1-135 | Ph | 2-MeNH-4-Pym | 6-Pr |
| 1-136 | Ph | 2-MeNH-4-Pym | 6,6-diMe |
| 1-137 | Ph | 2-MeNH-4-Pym | 6-Oxo |
| 1-138 | Ph | 2-MeNH-4-Pym | 8-Me |
| 1-139 | Ph | 2-MeNH-4-Pym | 8-Et |
| 1-140 | Ph | 2-MeNH-4-Pym | 8-Pr |
| 1-141 | Ph | 2-MeNH-4-Pym | 8-Ph |
| 1-142 | Ph | 2-MeNH-4-Pym | 8a-Me |
| 1-143 | Ph | 2-MeNH-4-Pym | 8a-Et |
| 1-144 | Ph | 2-MeNH-4-Pym | 8a-Pr |
| 1-145 | 3-F-Ph | 4-Pyr | 1-Me |
| 1-146 | 3-F-Ph | 4-Pyr | 1-Et |
| 1-147 | 3-F-Ph | 4-Pyr | 1-Pr |
| 1-148 | 3-F-Ph | 4-Pyr | 1,1-diMe |
| 1-149 | 3-F-Ph | 4-Pyr | 2-Me |
| 1-150 | 3-F-Ph | 4-Pyr | 2-Et |
| 1-151 | 3-F-Ph | 4-Pyr | 2-Pr |
| 1-152 | 3-F-Ph | 4-Pyr | 2-Bu |
| 1-153 | 3-F-Ph | 4-Pyr | 2-Allyl |
| 1-154 | 3-F-Ph | 4-Pyr | 2-Ph |
| 1-155 | 3-F-Ph | 4-Pyr | 2-Bn |
| 1-156 | 3-F-Ph | 4-Pyr | 2-Phet |
| 1-157 | 3-F-Ph | 4-Pyr | 2,2-diMe |
| 1-158 | 3-F-Ph | 4-Pyr | 2-OH |
| 1-159 | 3-F-Ph | 4-Pyr | 2-MeO |
| 1-160 | 3-F-Ph | 4-Pyr | 2-EtO |
| 1-161 | 3-F-Ph | 4-Pyr | 2-PrO |
| 1-162 | 3-F-Ph | 4-Pyr | 2,2-di(MeO) |
| 1-163 | 3-F-Ph | 4-Pyr | 2,2-di(EtO) |
| 1-164 | 3-F-Ph | 4-Pyr | 2,2-$OCH_2CH_2O$- |
| 1-165 | 3-F-Ph | 4-Pyr | 2-Oxo |
| 1-166 | 3-F-Ph | 4-Pyr | 2-F |
| 1-167 | 3-F-Ph | 4-Pyr | 2-Cl |
| 1-168 | 3-F-Ph | 4-Pyr | 2-Br |
| 1-169 | 3-F-Ph | 4-Pyr | 2-I |
| 1-170 | 3-F-Ph | 4-Pyr | 2,2-diF |
| 1-171 | 3-F-Ph | 4-Pyr | 2,2-diCl |

Table continued

| Compound No. | $R^1$ | $R^2$ | $R^4$ |
|---|---|---|---|
| 1-172 | 3-F-Ph | 4-Pyr | 2,2-diBr |
| 1-173 | 3-F-Ph | 4-Pyr | 3-Me |
| 1-174 | 3-F-Ph | 4-Pyr | 3-Et |
| 1-175 | 3-F-Ph | 4-Pyr | 3-Pr |
| 1-176 | 3-F-Ph | 4-Pyr | 3,3-diMe |
| 1-177 | 3-F-Ph | 4-Pyr | 5-Me |
| 1-178 | 3-F-Ph | 4-Pyr | 5-Et |
| 1-179 | 3-F-Ph | 4-Pyr | 5-Pr |
| 1-180 | 3-F-Ph | 4-Pyr | 5,5-diMe |
| 1-181 | 3-F-Ph | 4-Pyr | 6-Me |
| 1-182 | 3-F-Ph | 4-Pyr | 6-Et |
| 1-183 | 3-F-Ph | 4-Pyr | 6-Pr |
| 1-184 | 3-F-Ph | 4-Pyr | 6,6-diMe |
| 1-185 | 3-F-Ph | 4-Pyr | 6-Oxo |
| 1-186 | 3-F-Ph | 4-Pyr | 8-Me |
| 1-187 | 3-F-Ph | 4-Pyr | 8-Et |
| 1-188 | 3-F-Ph | 4-Pyr | 8-Pr |
| 1-189 | 3-F-Ph | 4-Pyr | 8-Ph |
| 1-190 | 3-F-Ph | 4-Pyr | 8a-Me |
| 1-191 | 3-F-Ph | 4-Pyr | 8a-Et |
| 1-192 | 3-F-Ph | 4-Pyr | 8a-Pr |
| 1-193 | 3-F-Ph | 2-$NH_2$-4-Pym | 1-Me |
| 1-194 | 3-F-Ph | 2-$NH_2$-4-Pym | 1-Et |
| 1-195 | 3-F-Ph | 2-$NH_2$-4-Pym | 1-Pr |
| 1-196 | 3-F-Ph | 2-$NH_2$-4-Pym | 1,1-diMe |
| 1-197 | 3-F-Ph | 2-$NH_2$-4-Pym | 2-Me |
| 1-198 | 3-F-Ph | 2-$NH_2$-4-Pym | 2-Et |
| 1-199 | 3-F-Ph | 2-$NH_2$-4-Pym | 2-Pr |
| 1-200 | 3-F-Ph | 2-$NH_2$-4-Pym | 2-Bu |
| 1-201 | 3-F-Ph | 2-$NH_2$-4-Pym | 2-Allyl |
| 1-202 | 3-F-Ph | 2-$NH_2$-4-Pym | 2-Ph |
| 1-203 | 3-F-Ph | 2-$NH_2$-4-Pym | 2-Bn |
| 1-204 | 3-F-Ph | 2-$NH_2$-4-Pym | 2-Phet |
| 1-205 | 3-F-Ph | 2-$NH_2$-4-Pym | 2,2-diMe |
| 1-206 | 3-F-Ph | 2-$NH_2$-4-Pym | 2-OH |
| 1-207 | 3-F-Ph | 2-$NH_2$-4-Pym | 2-MeO |
| 1-208 | 3-F-Ph | 2-$NH_2$-4-Pym | 2-EtO |
| 1-209 | 3-F-Ph | 2-$NH_2$-4-Pym | 2-PrO |

Table continued

| Compound No. | $R^1$ | $R^2$ | $R^4$ |
|---|---|---|---|
| 1-210 | 3-F-Ph | 2-$NH_2$-4-Pym | 2,2-di(MeO) |
| 1-211 | 3-F-Ph | 2-$NH_2$-4-Pym | 2,2-di(EtO) |
| 1-212 | 3-F-Ph | 2-$NH_2$-4-Pym | 2,2-$OCH_2CH_2O$- |
| 1-213 | 3-F-Ph | 2-$NH_2$-4-Pym | 2-Oxo |
| 1-214 | 3-F-Ph | 2-$NH_2$-4-Pym | 2-F |
| 1-215 | 3-F-Ph | 2-$NH_2$-4-Pym | 2-Cl |
| 1-216 | 3-F-Ph | 2-$NH_2$-4-Pym | 2-Br |
| 1-217 | 3-F-Ph | 2-$NH_2$-4-Pym | 2-1 |
| 1-218 | 3-F-Ph | 2-$NH_2$-4-Pym | 2,2-diF |
| 1-219 | 3-F-Ph | 2-$NH_2$-4-Pym | 2,2-diCl |
| 1-220 | 3-F-Ph | 2-$NH_2$-4-Pym | 2,2-diBr |
| 1-221 | 3-F-Ph | 2-$NH_2$-4-Pym | 3-Me |
| 1-222 | 3-F-Ph | 2-$NH_2$-4-Pym | 3-Et |
| 1-223 | 3-F-Ph | 2-$NH_2$-4-Pym | 3-Pr |
| 1-224 | 3-F-Ph | 2-$NH_2$-4-Pym | 3,3-diMe |
| 1-225 | 3-F-Ph | 2-$NH_2$-4-Pym | 5-Me |
| 1-226 | 3-F-Ph | 2-$NH_2$-4-Pym | 5-Et |
| 1-227 | 3-F-Ph | 2-$NH_2$-4-Pym | 5-Pr |
| 1-228 | 3-F-Ph | 2-$NH_2$-4-Pym | 5,5-diMe |
| 1-229 | 3-F-Ph | 2-$NH_2$-4-Pym | 6=Me |
| 1-230 | 3-F-Ph | 2-$NH_2$-4-Pym | 6-Et |
| 1-231 | 3-F-Ph | 2-$NH_2$-4-Pym | 6-Pr |
| 1-232 | 3-F-Ph | 2-$NH_2$-4-Pym | 6,6-diMe |
| 1-233 | 3-F-Ph | 2-$NH_2$-4-Pym | 6-Oxo |
| 1-234 | 3-F-Ph | 2-$NH_2$-4-Pym | 8-Me |
| 1-235 | 3-F-Ph | 2-$NH_2$-4-Pym | 8-Et |
| 1-236 | 3-F-Ph | 2-$NH_2$-4-Pym | 8-Pr |
| 1-237 | 3-F-Ph | 2-$NH_2$-4-Pym | 8-Ph |
| 1-238 | 3-F-Ph | 2-$NH_2$-4-Pym | 8a-Me |
| 1-239 | 3-F-Ph | 2-$NH_2$-4-Pym | 8a-Et |
| 1-240 | 3-F-Ph | 2-$NH_2$-4-Pym | 8a-Pr |
| 1-241 | 3-F-Ph | 2-MeNH-4-Pym | 1-Me |
| 1-242 | 3-F-Ph | 2-MeNH-4-Pym | 1-Et |
| 1-243 | 3-F-Ph | 2-MeNH-4-Pym | 1-Pr |
| 1-244 | 3-F-Ph | 2-MeNH-4-Pym | 1,1-diMe |
| 1-245 | 3-F-Ph | 2-MeNH-4-Pym | 2-Me |
| 1-246 | 3-F-Ph | 2-MeNH-4-Pym | 2-Et |
| 1-247 | 3-F-Ph | 2-MeNH-4-Pym | 2-Pr |

Table continued

| Compound No. | $R^1$ | $R^2$ | $R^4$ |
|---|---|---|---|
| 1-248 | 3-F-Ph | 2-MeNH-4-Pym | 2-Bu |
| 1-249 | 3-F-Ph | 2-MeNH-4-Pym | 2-Allyl |
| 1-250 | 3-F-Ph | 2-MeNH-4-Pym | 2-Ph |
| 1-251 | 3-F-Ph | 2-MeNH-4-Pym | 2-Bn |
| 1-252 | 3-F-Ph | 2-MeNH-4-Pym | 2-Phet |
| 1-253 | 3-F-Ph | 2-MeNH-4-Pym | 2,2-diMe |
| 1-254 | 3-F-Ph | 2-MeNH-4-Pym | 2-OH |
| 1-255 | 3-F-Ph | 2-MeNH-4-Pym | 2-MeO |
| 1-256 | 3-F-Ph | 2-MeNH-4-Pym | 2-EtO |
| 1-257 | 3-F-Ph | 2-MeNH-4-Pym | 2-PrO |
| 1-258 | 3-F-Ph | 2-MeNH-4-Pym | 2,2-di(MeO) |
| 1-259 | 3-F-Ph | 2-MeNH-4-Pym | 2,2-di(EtO) |
| 1-260 | 3-F-Ph | 2-MeNH-4-Pym | 2,2-$OCH_2CH_2O$- |
| 1-261 | 3-F-Ph | 2-MeNH-4-Pym | 2-Oxo |
| 1-262 | 3-F-Ph | 2-MeNH-4-Pym | 2-F |
| 1-263 | 3-F-Ph | 2-MeNH-4-Pym | 2-Cl |
| 1-264 | 3-F-Ph | 2-MeNH-4-Pym | 2-Br |
| 1-265 | 3-F-Ph | 2-MeNH-4-Pym | 2-I |
| 1-266 | 3-F-Ph | 2-MeNH-4-Pym | 2,2-diF |
| 1-267 | 3-F-Ph | 2-MeNH-4-Pym | 2,2-diCl |
| 1-268 | 3-F-Ph | 2-MeNH-4-Pym | 2,2-diBr |
| 1-269 | 3-F-Ph | 2-MeNH-4-Pym | 3-Me |
| 1-270 | 3-F-Ph | 2-MeNH-4-Pym | 3-Et |
| 1-271 | 3-F-Ph | 2-MeNH-4-Pym | 3-Pr |
| 1-272 | 3-F-Ph | 2-MeNH-4-Pym | 3,3-diMe |
| 1-273 | 3-F-Ph | 2-MeNH-4-Pym | 5-Me |
| 1-274 | 3-F-Ph | 2-MeNH-4-Pym | 5-Et |
| 1-275 | 3-F-Ph | 2-MeNH-4-Pym | 5-Pr |
| 1-276 | 3-F-Ph | 2-MeNH-4-Pym | 5,5-diMe |
| 1-277 | 3-F-Ph | 2-MeNH-4-Pym | 6-Me |
| 1-278 | 3-F-Ph | 2-MeNH-4-Pym | 6-Et |
| 1-279 | 3-F-Ph | 2-MeNH-4-Pym | 6-Pr |
| 1-280 | 3-F-Ph | 2-MeNH-4-Pym | 6,6-diMe |
| 1-281 | 3-F-Ph | 2-MeNH-4-Pym | 6-Oxo |
| 1-282 | 3-F-Ph | 2-MeNH-4-Pym | 8-Me |
| 1-283 | 3-F-Ph | 2-MeNH-4-Pym | 8-Et |
| 1-284 | 3-F-Ph | 2-MeNH-4-Pym | 8-Pr |
| 1-285 | 3-F-Ph | 2-MeNH-4-Pym | 8-Ph |

Table continued

| Compound No. | $R^1$ | $R^2$ | $R^4$ |
|---|---|---|---|
| 1-286 | 3-F-Ph | 2-MeNH-4-Pym | 8a-Me |
| 1-287 | 3-F-Ph | 2-MeNH-4-Pym | 8a-Et |
| 1-288 | 3-F-Ph | 2-MeNH-4-Pym | 8a-Pr |
| 1-289 | 4-F-Ph | 4-Pyr | 1-Me |
| 1-290 | 4-F-Ph | 4-Pyr | 1-Et |
| 1-291 | 4-F-Ph | 4-Pyr | 1-Pr |
| 1-292 | 4-F-Ph | 4-Pyr | 1,1-diMe |
| 1-293 | 4-F-Ph | 4-Pyr | 2-Me |
| 1-294 | 4-F-Ph | 4-Pyr | 2-Et |
| 1-295 | 4-F-Ph | 4-Pyr | 2-Pr |
| 1-296 | 4-F-Ph | 4-Pyr | 2-Bu |
| 1-297 | 4-F-Ph | 4-Pyr | 2-Allyl |
| 1-298 | 4-F-Ph | 4-Pyr | 2-Ph |
| 1-299 | 4-F-Ph | 4-Pyr | 2-Bn |
| 1-300 | 4-F-Ph | 4-Pyr | 2-Phet |
| 1-301 | 4-F-Ph | 4-Pyr | 2,2-diMe |
| 1-302 | 4-F-Ph | 4-Pyr | 2-OH |
| 1-303 | 4-F-Ph | 4-Pyr | 2-MeO |
| 1-304 | 4-F-Ph | 4-Pyr | 2-EtO |
| 1-305 | 4-F-Ph | 4-Pyr | 2-PrO |
| 1-306 | 4-F-Ph | 4-Pyr | 2,2-di(MeO) |
| 1-307 | 4-F-Ph | 4-Pyr | 2,2-di(EtO) |
| 1-308 | 4-F-Ph | 4-Pyr | 2,2-$OCH_2CH_2O$- |
| 1-309 | 4-F-Ph | 4-Pyr | 2-Oxo |
| 1-310 | 4-F-Ph | 4-Pyr | 2-F |
| 1-311 | 4-F-Ph | 4-Pyr | 2-Cl |
| 1-312 | 4-F-Ph | 4-Pyr | 2-Br |
| 1-313 | 4-F-Ph | 4-Pyr | 2-1 |
| 1-314 | 4-F-Ph | 4-Pyr | 2,2-diF |
| 1-315 | 4-F-Ph | 4-Pyr | 2,2-diCl |
| 1-316 | 4-F-Ph | 4-Pyr | 2,2-diBr |
| 1-317 | 4-F-Ph | 4-Pyr | 3-Me |
| 1-318 | 4-F-Ph | 4-Pyr | 3-Et |
| 1-319 | 4-F-Ph | 4-Pyr | 3-Pr |
| 1-320 | 4-F-Ph | 4-Pyr | 3,3-diMe |
| 1-321 | 4-F-Ph | 4-Pyr | 5-Me |
| 1-322 | 4-F-Ph | 4-Pyr | 5-Et |
| 1-323 | 4-F-Ph | 4-Pyr | 5-Pr |

Table continued

| Compound No. | $R^1$ | $R^2$ | $R^4$ |
|---|---|---|---|
| 1-324 | 4-F-Ph | 4-Pyr | 5,5-diMe |
| 1-325 | 4-F-Ph | 4-Pyr | 6-Me |
| 1-326 | 4-F-Ph | 4-Pyr | 6-Et |
| 1-327 | 4-F-Ph | 4-Pyr | 6-Pr |
| 1-328 | 4-F-Ph | 4-Pyr | 6,6-diMe |
| 1-329 | 4-F-Ph | 4-Pyr | 6-Oxo |
| 1-330 | 4-F-Ph | 4-Pyr | 8-Me |
| 1-331 | 4-F-Ph | 4-Pyr | 8-Et |
| 1-332 | 4-F-Ph | 4-Pyr | 8-Pr |
| 1-333 | 4-F-Ph | 4-Pyr | 8-Ph |
| 1-334 | 4-F-Ph | 4-Pyr | 8a-Me |
| 1-335 | 4-F-Ph | 4-Pyr | 8a-Et |
| 1-336 | 4-F-Ph | 4-Pyr | 8a-Pr |
| 1-337 | 4-F-Ph | $2-NH_2-4-Pym$ | 1-Me |
| 1-338 | 4-F-Ph | $2-NH_2-4-Pym$ | 1-Et |
| 1-339 | 4-F-Ph | $2-NH_2-4-Pym$ | 1-Pr |
| 1-340 | 4-F-Ph | $2-NH_2-4-Pym$ | 1,1-diMe |
| 1-341 | 4-F-Ph | $2-NH_2-4-Pym$ | 2-Me |
| 1-342 | 4-F-Ph | $2-NH_2-4-Pym$ | 2-Et |
| 1-343 | 4-F-Ph | $2-NH_2-4-Pym$ | 2-Pr |
| 1-344 | 4-F-Ph | $2-NH_2-4-Pym$ | 2-Bu |
| 1-345 | 4-F-Ph | $2-NH_2-4-Pym$ | 2-Allyl |
| 1-346 | 4-F-Ph | $2-NH_2-4-Pym$ | 2-Ph |
| 1-347 | 4-F-Ph | $2-NH_2-4-Pym$ | 2-Bn |
| 1-348 | 4-F-Ph | $2-NH_2-4-Pym$ | 2-Phet |
| 1-349 | 4-F-Ph | $2-NH_2-4-Pym$ | 2,2-diMe |
| 1-350 | 4-F-Ph | $2-NH_2-4-Pym$ | 2-OH |
| 1-351 | 4-F-Ph | $2-NH_2-4-Pym$ | 2-MeO |
| 1-352 | 4-F-Ph | $2-NH_2-4-Pym$ | 2-EtO |
| 1-353 | 4-F-Ph | $2-NH_2-4-Pym$ | 2-PrO |
| 1-354 | 4-F-Ph | $2-NH_2-4-Pym$ | 2,2-di(MeO) |
| 1-355 | 4-F-Ph | $2-NH_2-4-Pym$ | 2,2-di(EtO) |
| 1-356 | 4-F-Ph | $2-NH_2-4-Pym$ | $2,2-OCH_2CH_2O-$ |
| 1-357 | 4-F-Ph | $2-NH_2-4-Pym$ | 2-Oxo |
| 1-358 | 4-F-Ph | $2-NH_2-4-Pym$ | 2-F |
| 1-359 | 4-F-Ph | $2-NH_2-4-Pym$ | 2-Cl |
| 1-360 | 4-F-Ph | $2-NH_2-4-Pym$ | 2-Br |
| 1-361 | 4-F-Ph | $2-NH_2-4-Pym$ | 2-1 |

Table continued

| Compound No. | $R^1$ | $R^2$ | $R^4$ |
|---|---|---|---|
| 1-362 | 4-F-Ph | 2-$NH_2$-4-Pym | 2,2-diF |
| 1-363 | 4-F-Ph | 2-$NH_2$-4-Pym | 2,2-diCl |
| 1-364 | 4-F-Ph | 2-$NH_2$-4-Pym | 2,2-diBr |
| 1-365 | 4-F-Ph | 2-$NH_2$-4-Pym | 3-Me |
| 1-366 | 4-F-Ph | 2-$NH_2$-4-Pym | 3-Et |
| 1-367 | 4-F-Ph | 2-$NH_2$-4-Pym | 3-Pr |
| 1-368 | 4-F-Ph | 2-$NH_2$-4-Pym | 3,3-diMe |
| 1-369 | 4-F-Ph | 2-$NH_2$-4-Pym | 5-Me |
| 1-370 | 4-F-Ph | 2-$NH_2$-4-Pym | 5-Et |
| 1-371 | 4-F-Ph | 2-$NH_2$-4-Pym | 5-Pr |
| 1-372 | 4-F-Ph | 2-$NH_2$-4-Pym | 5,5-diMe |
| 1-373 | 4-F-Ph | 2-$NH_2$-4-Pym | 6-Me |
| 1-374 | 4-F-Ph | 2-$NH_2$-4-Pym | 6-Et |
| 1-375 | 4-F-Ph | 2-$NH_2$-4-Pym | 6-Pr |
| 1-376 | 4-F-Ph | 2-$NH_2$-4-Pym | 6,6-diMe |
| 1-377 | 4-F-Ph | 2-$NH_2$-4-Pym | 6-Oxo |
| 1-378 | 4-F-Ph | 2-$NH_2$-4-Pym | 8-Me |
| 1-379 | 4-F-Ph | 2-$NH_2$-4-Pym | 8-Et |
| 1-380 | 4-F-Ph | 2-$NH_2$-4-Pym | 8-Pr |
| 1-381 | 4-F-Ph | 2-$NH_2$-4-Pym | 8-Ph |
| 1-382 | 4-F-Ph | 2-$NH_2$-4-Pym | 8a-Me |
| 1-383 | 4-F-Ph | 2-$NH_2$-4-Pym | 8a-Et |
| 1-384 | 4-F-Ph | 2-$NH_2$-4-Pym | 8a-Pr |
| 1-385 | 4-F-Ph | 2-MeNH-4-Pym | 1-Me |
| 1-386 | 4-F-Ph | 2-MeNH-4-Pym | 1-Et |
| 1-387 | 4-F-Ph | 2-MeNH-4-Pym | 1-Pr |
| 1-388 | 4-F-Ph | 2-MeNH-4-Pym | 1,1-diMe |
| 1-389 | 4-F-Ph | 2-MeNH-4-Pym | 2-Me |
| 1-390 | 4-F-Ph | 2-MeNH-4-Pym | 2-Et |
| 1-391 | 4-F-Ph | 2-MeNH-4-Pym | 2-Pr |
| 1-392 | 4-F-Ph | 2-MeNH-4-Pym | 2-Bu |
| 1-393 | 4-F-Ph | 2-MeNH-4-Pym | 2-Allyl |
| 1-394 | 4-F-Ph | 2-MeNH-4-Pym | 2-Ph |
| 1-395 | 4-F-Ph | 2-MeNH-4-Pym | 2-Bn |
| 1-396 | 4-F-Ph | 2-MeNH-4-Pym | 2-Phet |
| 1-397 | 4-F-Ph | 2-MeNH-4-Pym | 2,2-diMe |
| 1-398 | 4-F-Ph | 2-MeNH-4-Pym | 2-OH |
| 1-399 | 4-F-Ph | 2-MeNH-4-Pym | 2-MeO |

Table continued

| Compound No. | $R^1$ | $R^2$ | $R^4$ |
|---|---|---|---|
| 1-400 | 4-F-Ph | 2-MeNH-4-Pym | 2-EtO |
| 1-401 | 4-F-Ph | 2-MeNH-4-Pym | 2-PrO |
| 1-402 | 4-F-Ph | 2-MeNH-4-Pym | 2,2-di(MeO) |
| 1-403 | 4-F-Ph | 2-MeNH-4-Pym | 2,2-di(EtO) |
| 1-404 | 4-F-Ph | 2-MeNH-4-Pym | 2,2-$OCH_2CH_2O$- |
| 1-405 | 4-F-Ph | 2-MeNH-4-Pym | 2-Oxo |
| 1-406 | 4-F-Ph | 2-MeNH-4-Pym | 2-F |
| 1-407 | 4-F-Ph | 2-MeNH-4-Pym | 2-Cl |
| 1-408 | 4-F-Ph | 2-MeNH-4-Pym | 2-Br |
| 1-409 | 4-F-Ph | 2-MeNH-4-Pym | 2-I |
| 1-410 | 4-F-Ph | 2-MeNH-4-Pym | 2,2-diF |
| 1-411 | 4-F-Ph | 2-MeNH-4-Pym | 2,2-diCl |
| 1-412 | 4-F-Ph | 2-MeNH-4-Pym | 2,2-diBr |
| 1-413 | 4-F-Ph | 2-MeNH-4-Pym | 3-Me |
| 1-414 | 4-F-Ph | 2-MeNH-4-Pym | 3-Et |
| 1-415 | 4-F-Ph | 2-MeNH-4-Pym | 3-Pr |
| 1-416 | 4-F-Ph | 2-MeNH-4-Pym | 3,3-diMe |
| 1-417 | 4-F-Ph | 2-MeNH-4-Pym | 5-Me |
| 1-418 | 4-F-Ph | 2-MeNH-4-Pym | 5-Et |
| 1-419 | 4-F-Ph | 2-MeNH-4-Pym | 5-Pr |
| 1-420 | 4-F-Ph | 2-MeNH-4-Pym | 5,5-diMe |
| 1-421 | 4-F-Ph | 2-MeNH-4-Pym | 6-Me |
| 1-422 | 4-F-Ph | 2-MeNH-4-Pym | 6-Et |
| 1-423 | 4-F-Ph | 2-MeNH-4-Pym | 6-Pr |
| 1-424 | 4-F-Ph | 2-MeNH-4-Pym | 6,6-diMe |
| 1-425 | 4-F-Ph | 2-MeNH-4-Pym | 6-Oxo |
| 1-426 | 4-F-Ph | 2-MeNH-4-Pym | 8-Me |
| 1-427 | 4-F-Ph | 2-MeNH-4-Pym | 8-Et |
| 1-428 | 4-F-Ph | 2-MeNH-4-Pym | 8-Pr |
| 1-429 | 4-F-Ph | 2-MeNH-4-Pym | 8-Ph |
| 1-430 | 4-F-Ph | 2-MeNH-4-Pym | 8a-Me |
| 1-431 | 4-F-Ph | 2-MeNH-4-Pym | 8a-Et |
| 1-432 | 4-F-Ph | 2-MeNH-4-Pym | 8a-Pr |
| 1-433 | 3-Cl-Ph | 4-Pyr | 1-Me |
| 1-434 | 3-Cl-Ph | 4-Pyr | 1-Et |
| 1-435 | 3-Cl-Ph | 4-Pyr | 1-Pr |
| 1-436 | 3-Cl-Ph | 4-Pyr | 1,1-diMe |
| 1-437 | 3-Cl-Ph | 4-Pyr | 2-Me |

Table continued

| Compound No. | $R^1$ | $R^2$ | $R^4$ |
|---|---|---|---|
| 1-438 | 3-Cl-Ph | 4-Pyr | 2-Et |
| 1-439 | 3-Cl-Ph | 4-Pyr | 2-Pr |
| 1-440 | 3-Cl-Ph | 4-Pyr | 2-Bu |
| 1-441 | 3-Cl-Ph | 4-Pyr | 2-Allyl |
| 1-442 | 3-Cl-Ph | 4-Pyr | 2-Ph |
| 1-443 | 3-Cl-Ph | 4-Pyr | 2-Bn |
| 1-444 | 3-Cl-Ph | 4-Pyr | 2-Phet |
| 1-445 | 3-Cl-Ph | 4-Pyr | 2,2-diMe |
| 1-446 | 3-Cl-Ph | 4-Pyr | 2-OH |
| 1-447 | 3-Cl-Ph | 4-Pyr | 2-MeO |
| 1-448 | 3-Cl-Ph | 4-Pyr | 2-EtO |
| 1-449 | 3-Cl-Ph | 4-Pyr | 2-PrO |
| 1-450 | 3-Cl-Ph | 4-Pyr | 2,2-di(MeO) |
| 1-451 | 3-Cl-Ph | 4-Pyr | 2,2-di(EtO) |
| 1-452 | 3-Cl-Ph | 4-Pyr | 2,2-$OCH_2CH_2O$- |
| 1-453 | 3-Cl-Ph | 4-Pyr | 2-Oxo |
| 1-454 | 3-Cl-Ph | 4-Pyr | 2-F |
| 1-455 | 3-Cl-Ph | 4-Pyr | 2-Cl |
| 1-456 | 3-Cl-Ph | 4-Pyr | 2-Br |
| 1-457 | 3-Cl-Ph | 4-Pyr | 2-I |
| 1-458 | 3-Cl-Ph | 4-Pyr | 2,2-diF |
| 1-459 | 3-Cl-Ph | 4-Pyr | 2,2-diCl |
| 1-460 | 3-Cl-Ph | 4-Pyr | 2,2-diBr |
| 1-461 | 3-Cl-Ph | 4-Pyr | 3-Me |
| 1-462 | 3-Cl-Ph | 4-Pyr | 3-Et |
| 1-463 | 3-Cl-Ph | 4-Pyr | 3-Pr |
| 1-464 | 3-Cl-Ph | 4-Pyr | 3,3-diMe |
| 1-465 | 3-Cl-Ph | 4-Pyr | 5-Me |
| 1-466 | 3-Cl-Ph | 4-Pyr | 5-Et |
| 1-467 | 3-Cl-Ph | 4-Pyr | 5-Pr |
| 1-468 | 3-Cl-Ph | 4-Pyr | 5,5-diMe |
| 1-469 | 3-Cl-Ph | 4-Pyr | 6-Me |
| 1-470 | 3-Cl-Ph | 4-Pyr | 6-Et |
| 1-471 | 3-Cl-Ph | 4-Pyr | 6-Pr |
| 1-472 | 3-Cl-Ph | 4-Pyr | 6,6-diMe |
| 1-473 | 3-Cl-Ph | 4-Pyr | 6-Oxo |
| 1-474 | 3-Cl-Ph | 4-Pyr | 8-Me |
| 1-475 | 3-Cl-Ph | 4-Pyr | 8-Et |

Table continued

| Compound No. | $R^1$ | $R^2$ | $R^4$ |
|---|---|---|---|
| 1-476 | 3-Cl-Ph | 4-Pyr | 8-Pr |
| 1-477 | 3-Cl-Ph | 4-Pyr | 8-Ph |
| 1-478 | 3-Cl-Ph | 4-Pyr | 8a-Me |
| 1-479 | 3-Cl-Ph | 4-Pyr | 8a-Et |
| 1-480 | 3-Cl-Ph | 4-Pyr | 8a-Pr |
| 1-481 | 3-Cl-Ph | 2-$NH_2$-4-Pym | 1-Me |
| 1-482 | 3-Cl-Ph | 2-$NH_2$-4-Pym | 1-Et |
| 1-483 | 3-Cl-Ph | 2-$NH_2$-4-Pym | 1-Pr |
| 1-484 | 3-Cl-Ph | 2-$NH_2$-4-Pym | 1,1-diMe |
| 1-485 | 3-Cl-Ph | 2-$NH_2$-4-Pym | 2-Me |
| 1-486 | 3-Cl-Ph | 2-$NH_2$-4-Pym | 2-Et |
| 1-487 | 3-Cl-Ph | 2-$NH_2$-4-Pym | 2-Pr |
| 1-488 | 3-Cl-Ph | 2-$NH_2$-4-Pym | 2-Bu |
| 1-489 | 3-Cl-Ph | 2-$NH_2$-4-Pym | 2-Allyl |
| 1-490 | 3-Cl-Ph | 2-$NH_2$-4-Pym | 2-Ph |
| 1-491 | 3-Cl-Ph | 2-$NH_2$-4-Pym | 2-Bn |
| 1-492 | 3-Cl-Ph | 2-$NH_2$-4-Pym | 2-Phet |
| 1-493 | 3-Cl-Ph | 2-$NH_2$-4-Pym | 2,2-diMe |
| 1-494 | 3-Cl-Ph | 2-$NH_2$-4-Pym | 2-OH |
| 1-495 | 3-Cl-Ph | 2-$NH_2$-4-Pym | 2-MeO |
| 1-496 | 3-Cl-Ph | 2-$NH_2$-4-Pym | 2-EtO |
| 1-497 | 3-Cl-Ph | 2-$NH_2$-4-Pym | 2-PrO |
| 1-498 | 3-Cl-Ph | 2-$NH_2$-4-Pym | 2,2-di(MeO) |
| 1-499 | 3-Cl-Ph | 2-$NH_2$-4-Pym | 2,2-di(EtO) |
| 1-500 | 3-Cl-Ph | 2-$NH_2$-4-Pym | 2,2-$OCH_2CH_2O$- |
| 1-501 | 3-Cl-Ph | 2-$NH_2$-4-Pym | 2-Oxo |
| 1-502 | 3-Cl-Ph | 2-$NH_2$-4-Pym | 2-F |
| 1-503 | 3-Cl-Ph | 2-$NH_2$-4-Pym | 2-Cl |
| 1-504 | 3-Cl-Ph | 2-$NH_2$-4-Pym | 2-Br |
| 1-505 | 3-Cl-Ph | 2-$NH_2$-4-Pym | 2-1 |
| 1-506 | 3-Cl-Ph | 2-$NH_2$-4-Pym | 2,2-diF |
| 1-507 | 3-Cl-Ph | 2-$NH_2$-4-Pym | 2,2-diCl |
| 1-508 | 3-Cl-Ph | 2-$NH_2$-4-Pym | 2,2-diBr |
| 1-509 | 3-Cl-Ph | 2-$NH_2$-4-Pym | 3-Me |
| 1-510 | 3-Cl-Ph | 2-$NH_2$-4-Pym | 3-Et |
| 1-511 | 3-Cl-Ph | 2-$NH_2$-4-Pym | 3-Pr |
| 1-512 | 3-Cl-Ph | 2-$NH_2$-4-Pym | 3,3-diMe |
| 1-513 | 3-Cl-Ph | 2-$NH_2$-4-Pym | 5-Me |

Table continued

| Compound No. | $R^1$ | $R^2$ | $R^4$ |
|---|---|---|---|
| 1-514 | 3-Cl-Ph | 2-$NH_2$-4-Pym | 5-Et |
| 1-515 | 3-Cl-Ph | 2-$NH_2$-4-Pym | 5-Pr |
| 1-516 | 3-Cl-Ph | 2-$NH_2$-4-Pym | 5,5-diMe |
| 1-517 | 3-Cl-Ph | 2-$NH_2$-4-Pym | 6-Me |
| 1-518 | 3-Cl-Ph | 2-$NH_2$-4-Pym | 6-Et |
| 1-519 | 3-Cl-Ph | 2-$NH_2$-4-Pym | 6-Pr |
| 1-520 | 3-Cl-Ph | 2-$NH_2$-4-Pym | 6,6-diMe |
| 1-521 | 3-Cl-Ph | 2-$NH_2$-4-Pym | 6-Oxo |
| 1-522 | 3-Cl-Ph | 2-$NH_2$-4-Pym | 8-Me |
| 1-523 | 3-Cl-Ph | 2-$NH_2$-4-Pym | 8-Et |
| 1-524 | 3-Cl-Ph | 2-$NH_2$-4-Pym | 8-Pr |
| 1-525 | 3-Cl-Ph | 2-$NH_2$-4-Pym | 8-Ph |
| 1-526 | 3-Cl-Ph | 2-$NH_2$-4-Pym | 8a-Me |
| 1-527 | 3-Cl-Ph | 2-$NH_2$-4-Pym | 8a-Et |
| 1-528 | 3-Cl-Ph | 2-$NH_2$-4-Pym | 8a-Pr |
| 1-529 | 3-Cl-Ph | 2-MeNH-4-Pym | 1-Me |
| 1-530 | 3-Cl-Ph | 2-MeNH-4-Pym | 1-Et |
| 1-531 | 3-Cl-Ph | 2-MeNH-4-Pym | 1-Pr |
| 1-532 | 3-Cl-Ph | 2-MeNH-4-Pym | 1,1-diMe |
| 1-533 | 3-Cl-Ph | 2-MeNH-4-Pym | 2-Me |
| 1-534 | 3-Cl-Ph | 2-MeNH-4-Pym | 2-Et |
| 1-535 | 3-Cl-Ph | 2-MeNH-4-Pym | 2-Pr |
| 1-536 | 3-Cl-Ph | 2-MeNH-4-Pym | 2-Bu |
| 1-537 | 3-Cl-Ph | 2-MeNH-4-Pym | 2-Allyl |
| 1-538 | 3-Cl-Ph | 2-MeNH-4-Pym | 2-Ph |
| 1-539 | 3-Cl-Ph | 2-MeNH-4-Pym | 2-Bn |
| 1-540 | 3-Cl-Ph | 2-MeNH-4-Pym | 2-Phet |
| 1-541 | 3-Cl-Ph | 2-MeNH-4-Pym | 2,2-diMe |
| 1-542 | 3-Cl-Ph | 2-MeNH-4-Pym | 2-OH |
| 1-543 | 3-Cl-Ph | 2-MeNH-4-Pym | 2-MeO |
| 1-544 | 3-Cl-Ph | 2-MeNH-4-Pym | 2-EtO |
| 1-545 | 3-Cl-Ph | 2-MeNH-4-Pym | 2-PrO |
| 1-546 | 3-Cl-Ph | 2-MeNH-4-Pym | 2,2-di(MeO) |
| 1-547 | 3-Cl-Ph | 2-MeNH-4-Pym | 2,2-di(EtO) |
| 1-548 | 3-Cl-Ph | 2-MeNH-4-Pym | 2,2-$OCH_2CH_2O$- |
| 1-549 | 3-Cl-Ph | 2-MeNH-4-Pym | 2-Oxo |
| 1-550 | 3-Cl-Ph | 2-MeNH-4-Pym | 2-F |
| 1-551 | 3-Cl-Ph | 2-MeNH-4-Pym | 2-Cl |

Table continued

| Compound No. | $R^1$ | $R^2$ | $R^4$ |
|---|---|---|---|
| 1-552 | 3-Cl-Ph | 2-MeNH-4-Pym | 2-Br |
| 1-553 | 3-Cl-Ph | 2-MeNH-4-Pym | 2-I |
| 1-554 | 3-Cl-Ph | 2-MeNH-4-Pym | 2,2-diF |
| 1-555 | 3-Cl-Ph | 2-MeNH-4-Pym | 2,2-diCl |
| 1-556 | 3-Cl-Ph | 2-MeNH-4-Pym | 2,2-diBr |
| 1-557 | 3-Cl-Ph | 2-MeNH-4-Pym | 3-Me |
| 1-558 | 3-Cl-Ph | 2-MeNH-4-Pym | 3-Et |
| 1-559 | 3-Cl-Ph | 2-MeNH-4-Pym | 3-Pr |
| 1-560 | 3-Cl-Ph | 2-MeNH-4-Pym | 3,3-diMe |
| 1-561 | 3-Cl-Ph | 2-MeNH-4-Pym | 5-Me |
| 1-562 | 3-Cl-Ph | 2-MeNH-4-Pym | 5-Et |
| 1-563 | 3-Cl-Ph | 2-MeNH-4-Pym | 5-Pr |
| 1-564 | 3-Cl-Ph | 2-MeNH-4-Pym | 5,5-diMe |
| 1-565 | 3-Cl-Ph | 2-MeNH-4-Pym | 6-Me |
| 1-566 | 3-Cl-Ph | 2-MeNH-4-Pym | 6-Et |
| 1-567 | 3-Cl-Ph | 2-MeNH-4-Pym | 6-Pr |
| 1-568 | 3-Cl-Ph | 2-MeNH-4-Pym | 6,6-diMe |
| 1-569 | 3-Cl-Ph | 2-MeNH-4-Pym | 6-Oxo |
| 1-570 | 3-Cl-Ph | 2-MeNH-4-Pym | 8-Me |
| 1-571 | 3-Cl-Ph | 2-MeNH-4-Pym | 8-Et |
| 1-572 | 3-Cl-Ph | 2-MeNH-4-Pym | 8-Pr |
| 1-573 | 3-Cl-Ph | 2-MeNH-4-Pym | 8-Ph |
| 1-574 | 3-Cl-Ph | 2-MeNH-4-Pym | 8a-Me |
| 1-575 | 3-Cl-Ph | 2-MeNH-4-Pym | 8a-Et |
| 1-576 | 3-Cl-Ph | 2-MeNH-4-Pym | 8a-Pr |
| 1-577 | $3-CF_3-Ph$ | 4-Pyr | 1-Me |
| 1-578 | $3-CF_3-Ph$ | 4-Pyr | 1-Et |
| 1-579 | $3-CF_3-Ph$ | 4-Pyr | 1-Pr |
| 1-580 | $3-CF_3-Ph$ | 4-Pyr | 1,1-diMe |
| 1-581 | $3-CF_3-Ph$ | 4-Pyr | 2-Me |
| 1-582 | $3-CF_3-Ph$ | 4-Pyr | 2-Et |
| 1-583 | $3-CF_3-Ph$ | 4-Pyr | 2-Pr |
| 1-584 | $3-CF_3-Ph$ | 4-Pyr | 2-Bu |
| 1-585 | $3-CF_3-Ph$ | 4-Pyr | 2-Allyl |
| 1-586 | $3-CF_3-Ph$ | 4-Pyr | 2-Ph |
| 1-587 | $3-CF_3-Ph$ | 4-Pyr | 2-Bn |
| 1-588 | $3-CF_3-Ph$ | 4-Pyr | 2-Phet |
| 1-589 | $3-CF_3-Ph$ | 4-Pyr | 2,2-diMe |

Table continued

| Compound No. | $R^1$ | $R^2$ | $R^4$ |
|---|---|---|---|
| 1-590 | 3-$CF_3$-Ph | 4-Pyr | 2-OH |
| 1-591 | 3-$CF_3$-Ph | 4-Pyr | 2-MeO |
| 1-592 | 3-$CF_3$-Ph | 4-Pyr | 2-EtO |
| 1-593 | 3-$CF_3$-Ph | 4-Pyr | 2-PrO |
| 1-594 | 3-$CF_3$-Ph | 4-Pyr | 2,2-di(MeO) |
| 1-595 | 3-$CF_3$-Ph | 4-Pyr | 2,2-di(EtO) |
| 1-596 | 3-$CF_3$-Ph | 4-Pyr | 2,2-$OCH_2CH_2O$- |
| 1-597 | 3-$CF_3$-Ph | 4-Pyr | 2-Oxo |
| 1-598 | 3-$CF_3$-Ph | 4-Pyr | 2-F |
| 1-599 | 3-$CF_3$-Ph | 4-Pyr | 2-Cl |
| 1-600 | 3-$CF_3$-Ph | 4-Pyr | 2-Br |
| 1-601 | 3-$CF_3$-Ph | 4-Pyr | 2-I |
| 1-602 | 3-$CF_3$-Ph | 4-Pyr | 2,2-diF |
| 1-603 | 3-$CF_3$-Ph | 4-Pyr | 2,2-diCl |
| 1-604 | 3-$CF_3$-Ph | 4-Pyr | 2,2-diBr |
| 1-605 | 3-$CF_3$-Ph | 4-Pyr | 3-Me |
| 1-606 | 3-$CF_3$-Ph | 4-Pyr | 3-Et |
| 1-607 | 3-$CF_3$-Ph | 4-Pyr | 3-Pr |
| 1-608 | 3-$CF_3$-Ph | 4-Pyr | 3,3-diMe |
| 1-609 | 3-$CF_3$-Ph | 4-Pyr | 5-Me |
| 1-610 | 3-$CF_3$-Ph | 4-Pyr | 5-Et |
| 1-611 | 3-$CF_3$-Ph | 4-Pyr | 5-Pr |
| 1-612 | 3-$CF_3$-Ph | 4-Pyr | 5,5-diMe |
| 1-613 | 3-$CF_3$-Ph | 4-Pyr | 6-Me |
| 1-614 | 3-$CF_3$-Ph | 4-Pyr | 6-Et |
| 1-615 | 3-$CF_3$-Ph | 4-Pyr | 6-Pr |
| 1-616 | 3-$CF_3$-Ph | 4-Pyr | 6,6-diMe |
| 1-617 | 3-$CF_3$-Ph | 4-Pyr | 6-Oxo |
| 1-618 | 3-$CF_3$-Ph | 4-Pyr | 8-Me |
| 1-619 | 3-$CF_3$-Ph | 4-Pyr | 8-Et |
| 1-620 | 3-$CF_3$-Ph | 4-Pyr | 8-Pr |
| 1-621 | 3-$CF_3$-Ph | 4-Pyr | 8-Ph |
| 1-622 | 3-$CF_3$-Ph | 4-Pyr | 8a-Me |
| 1-623 | 3-$CF_3$-Ph | 4-Pyr | 8a-Et |
| 1-624 | 3-$CF_3$-Ph | 4-Pyr | 8a-Pr |
| 1-625 | 3-$CF_3$-Ph | 2-$NH_2$-4-Pym | 1-Me |
| 1-626 | 3-$CF_3$-Ph | 2-$NH_2$-4-Pym | 1-Et |
| 1-627 | 3-$CF_3$-Ph | 2-$NH_2$-4-Pym | 1-Pr |

Table continued

| Compound No. | $R^1$ | $R^2$ | $R^4$ |
|---|---|---|---|
| 1-628 | 3-$CF_3$-Ph | 2-$NH_2$-4-Pym | 1,1-diMe |
| 1-629 | 3-$CF_3$-Ph | 2-$NH_2$-4-Pym | 2-Me |
| 1-630 | 3-$CF_3$-Ph | 2-$NH_2$-4-Pym | 2-Et |
| 1-631 | 3-$CF_3$-Ph | 2-$NH_2$-4-Pym | 2-Pr |
| 1-632 | 3-$CF_3$-Ph | 2-$NH_2$-4-Pym | 2-Bu |
| 1-633 | 3-$CF_3$-Ph | 2-$NH_2$-4-Pym | 2-Allyl |
| 1-634 | 3-$CF_3$-Ph | 2-$NH_2$-4-Pym | 2-Ph |
| 1-635 | 3-$CF_3$-Ph | 2-$NH_2$-4-Pym | 2-Bn |
| 1-636 | 3-$CF_3$-Ph | 2-$NH_2$-4-Pym | 2-Phet |
| 1-637 | 3-$CF_3$-Ph | 2-$NH_2$-4-Pym | 2,2-diMe |
| 1-638 | 3-$CF_3$-Ph | 2-$NH_2$-4-Pym | 2-OH |
| 1-639 | 3-$CF_3$-Ph | 2-$NH_2$-4-Pym | 2-MeO |
| 1-640 | 3-$CF_3$-Ph | 2-$NH_2$-4-Pym | 2-EtO |
| 1-641 | 3-$CF_3$-Ph | 2-$NH_2$-4-Pym | 2-PrO |
| 1-642 | 3-$CF_3$-Ph | 2-$NH_2$-4-Pym | 2,2-di(MeO) |
| 1-643 | 3-$CF_3$-Ph | 2-$NH_2$-4-Pym | 2,2-di(EtO) |
| 1-644 | 3-$CF_3$-Ph | 2-$NH_2$-4-Pym | 2,2-$OCH_2CH_2O$- |
| 1-645 | 3-$CF_3$-Ph | 2-$NH_2$-4-Pym | 2-Oxo |
| 1-646 | 3-$CF_3$-Ph | 2-$NH_2$-4-Pym | 2-F |
| 1-647 | 3-$CF_3$-Ph | 2-$NH_2$-4-Pym | 2-Cl |
| 1-648 | 3-$CF_3$-Ph | 2-$NH_2$-4-Pym | 2-Br |
| 1-649 | 3-$CF_3$-Ph | 2-$NH_2$-4-Pym | 2-I |
| 1-650 | 3-$CF_3$-Ph | 2-$NH_2$-4-Pym | 2,2-diF |
| 1-651 | 3-$CF_3$-Ph | 2-$NH_2$-4-Pym | 2,2-diCl |
| 1-652 | 3-$CF_3$-Ph | 2-$NH_2$-4-Pym | 2,2-diBr |
| 1-653 | 3-$CF_3$-Ph | 2-$NH_2$-4-Pym | 3-Me |
| 1-654 | 3-$CF_3$-Ph | 2-$NH_2$-4-Pym | 3-Et |
| 1-655 | 3-$CF_3$-Ph | 2-$NH_2$-4-Pym | 3-Pr |
| 1-656 | 3-$CF_3$-Ph | 2-$NH_2$-4-Pym | 3,3-diMe |
| 1-657 | 3-$CF_3$-Ph | 2-$NH_2$-4-Pym | 5-Me |
| 1-658 | 3-$CF_3$-Ph | 2-$NH_2$-4-Pym | 5-Et |
| 1-659 | 3-$CF_3$-Ph | 2-$NH_2$-4-Pym | 5-Pr |
| 1-660 | 3-$CF_3$-Ph | 2-$NH_2$-4-Pym | 5,5-diMe |
| 1-661 | 3-$CF_3$-Ph | 2-$NH_2$-4-Pym | 6-Me |
| 1-662 | 3-$CF_3$-Ph | 2-$NH_2$-4-Pym | 6-Et |
| 1-663 | 3-$CF_3$-Ph | 2-$NH_2$-4-Pym | 6-Pr |
| 1-664 | 3-$CF_3$-Ph | 2-$NH_2$-4-Pym | 6,6-diMe |
| 1-665 | 3-$CF_3$-Ph | 2-$NH_2$-4-Pym | 6-Oxo |

Table continued

| Compound No. | $R^1$ | $R^2$ | $R^4$ |
|---|---|---|---|
| 1-666 | 3-$CF_3$-Ph | 2-$NH_2$-4-Pym | 8-Me |
| 1-667 | 3-$CF_3$-Ph | 2-$NH_2$-4-Pym | 8-Et |
| 1-668 | 3-$CF_3$-Ph | 2-$NH_2$-4-Pym | 8-Pr |
| 1-669 | 3-$CF_3$-Ph | 2-$NH_2$-4-Pym | 8-Ph |
| 1-670 | 3-$CF_3$-Ph | 2-$NH_2$-4-Pym | 8a-Me |
| 1-671 | 3-$CF_3$-Ph | 2-$NH_2$-4-Pym | 8a-Et |
| 1-672 | 3-$CF_3$-Ph | 2-$NH_2$-4-Pym | 8a-Pr |
| 1-673 | 3-$CF_3$-Ph | 2-MeNH-4-Pym | 1-Me |
| 1-674 | 3-$CF_3$-Ph | 2-MeNH-4-Pym | 1-Et |
| 1-675 | 3-$CF_3$-Ph | 2-MeNH-4-Pym | 1-Pr |
| 1-676 | 3-$CF_3$-Ph | 2-MeNH-4-Pym | 1,1-diMe |
| 1-677 | 3-$CF_3$-Ph | 2-MeNH-4-Pym | 2-Me |
| 1-678 | 3-$CF_3$-Ph | 2-MeNH-4-Pym | 2-Et |
| 1-679 | 3-$CF_3$-Ph | 2-MeNH-4-Pym | 2-Pr |
| 1-680 | 3-$CF_3$-Ph | 2-MeNH-4-Pym | 2-Bu |
| 1-681 | 3-$CF_3$-Ph | 2-MeNH-4-Pym | 2-Allyl |
| 1-682 | 3-$CF_3$-Ph | 2-MeNH-4-Pym | 2-Ph |
| 1-683 | 3-$CF_3$-Ph | 2-MeNH-4-Pym | 2-Bn |
| 1-684 | 3-$CF_3$-Ph | 2-MeNH-4-Pym | 2-Phet |
| 1-685 | 3-$CF_3$-Ph | 2-MeNH-4-Pym | 2,2-diMe |
| 1-686 | 3-$CF_3$-Ph | 2-MeNH-4-Pym | 2-OH |
| 1-687 | 3-$CF_3$-Ph | 2-MeNH-4-Pym | 2-MeO |
| 1-688 | 3-$CF_3$-Ph | 2-MeNH-4-Pym | 2-EtO |
| 1-689 | 3-$CF_3$-Ph | 2-MeNH-4-Pym | 2-PrO |
| 1-690 | 3-$CF_3$-Ph | 2-MeNH-4-Pym | 2,2-di(MeO) |
| 1-691 | 3-$CF_3$-Ph | 2-MeNH-4-Pym | 2,2-di(EtO) |
| 1-692 | 3-$CF_3$-Ph | 2-MeNH-4-Pym | 2,2-$OCH_2CH_2O$- |
| 1-693 | 3-$CF_3$-Ph | 2-MeNH-4-Pym | 2-Oxo |
| 1-694 | 3-$CF_3$-Ph | 2-MeNH-4-Pym | 2-F |
| 1-695 | 3-$CF_3$-Ph | 2-MeNH-4-Pym | 2-Cl |
| 1-696 | 3-$CF_3$-Ph | 2-MeNH-4-Pym | 2-Br |
| 1-697 | 3-$CF_3$-Ph | 2-MeNH-4-Pym | 2-I |
| I-698 | 3-$CF_3$-Ph | 2-MeNH-4-Pym | 2,2-diF |
| 1-699 | 3-$CF_3$-Ph | 2-MeNH-4-Pym | 2,2-diCl |
| 1-700 | 3-$CF_3$-Ph | 2-MeNH-4-Pym | 2,2-diBr |
| 1-701 | 3-$CF_3$-Ph | 2-MeNH-4-Pym | 3-Me |
| 1-702 | 3-$CF_3$-Ph | 2-MeNH-4-Pym | 3-Et |
| 1-703 | 3-$CF_3$-Ph | 2-MeNH-4-Pym | 3-Pr |

Table continued

| Compound No. | $R^1$ | $R^2$ | $R^4$ |
|---|---|---|---|
| 1-704 | $3-CF_3-Ph$ | 2-MeNH-4-Pym | 3,3-diMe |
| 1-705 | $3-CF_3-Ph$ | 2-MeNH-4-Pym | 5-Me |
| 1-706 | $3-CF_3-Ph$ | 2-MeNH-4-Pym | 5-Et |
| 1-707 | $3-CF_3-Ph$ | 2-MeNH-4-Pym | 5-Pr |
| 1-708 | $3-CF_3-Ph$ | 2-MeNH-4-Pym | 5,5-diMe |
| 1-709 | $3-CF_3-Ph$ | 2-MeNH-4-Pym | 6-Me |
| 1-710 | $3-CF_3-Ph$ | 2-MeNH-4-Pym | 6-Et |
| 1-711 | $3-CF_3-Ph$ | 2-MeNH-4-Pym | 6-Pr |
| 1-712 | $3-CF_3-Ph$ | 2-MeNH-4-Pym | 6,6-diMe |
| 1-713 | $3-CF_3-Ph$ | 2-MeNH-4-Pym | 6-Oxo |
| 1-714 | $3-CF_3-Ph$ | 2-MeNH-4-Pym | 8-Me |
| 1-715 | $3-CF_3-Ph$ | 2-MeNH-4-Pym | 8-Et |
| 1-716 | $3-CF_3-Ph$ | 2-MeNH-4-Pym | 8-Pr |
| 1-717 | $3-CF_3-Ph$ | 2-MeNH-4-Pym | 8-Ph |
| 1-718 | $3-CF_3-Ph$ | 2-MeNH-4-Pym | 8a-Me |
| 1-719 | $3-CF_3-Ph$ | 2-MeNH-4-Pym | 8a-Et |
| 1-720 | $3-CF_3-Ph$ | 2-MeNH-4-Pym | 8a-Pr |
| 1-721 | 3,4-diF-Ph | 4-Pyr | 1-Me |
| 1-722 | 3,4-diF-Ph | 4-Pyr | 1-Et |
| 1-723 | 3,4-diF-Ph | 4-Pyr | 1-Pr |
| 1-724 | 3,4-diF-Ph | 4-Pyr | 1,1-diMe |
| 1-725 | 3,4-diF-Ph | 4-Pyr | 2-Me |
| 1-726 | 3,4-diF-Ph | 4-Pyr | 2-Et |
| 1-727 | 3,4-diF-Ph | 4-Pyr | 2-Pr |
| 1-728 | 3,4-diF-Ph | 4-Pyr | 2-Bu |
| 1-729 | 3,4-diF-Ph | 4-Pyr | 2-Allyl |
| 1-730 | 3,4-diF-Ph | 4-Pyr | 2-Ph |
| 1-731 | 3,4-diF-Ph | 4-Pyr | 2-Bn |
| 1-732 | 3,4-diF-Ph | 4-Pyr | 2-Phet |
| 1-733 | 3,4-diF-Ph | 4-Pyr | 2,2-diMe |
| 1-734 | 3,4-diF-Ph | 4-Pyr | 2-OH |
| 1-735 | 3,4-diF-Ph | 4-Pyr | 2-MeO |
| 1-736 | 3,4-diF-Ph | 4-Pyr | 2-EtO |
| 1-737 | 3,4-diF-Ph | 4-Pyr | 2-PrO |
| 1-738 | 3,4-diF-Ph | 4-Pyr | 2,2-di(MeO) |
| 1-739 | 3,4-diF-Ph | 4-Pyr | 2,2-di(EtO) |
| 1-740 | 3,4-diF-Ph | 4-Pyr | $2,2-OCH_2CH_2O-$ |
| 1-741 | 3,4-diF-Ph | 4-Pyr | 2-Oxo |

Table continued

| Compound No. | $R^1$ | $R^2$ | $R^4$ |
|---|---|---|---|
| 1-742 | 3,4-diF-Ph | 4-Pyr | 2-F |
| 1-743 | 3,4-diF-Ph | 4-Pyr | 2-Cl |
| 1-744 | 3,4-diF-Ph | 4-Pyr | 2-Br |
| 1-745 | 3,4-diF-Ph | 4-Pyr | 2-I |
| 1-746 | 3,4-diF-Ph | 4-Pyr | 2,2-diF |
| 1-747 | 3,4-diF-Ph | 4-Pyr | 2,2-diCl |
| 1-748 | 3,4-diF-Ph | 4-Pyr | 2,2-diBr |
| 1-749 | 3,4-diF-Ph | 4-Pyr | 3-Me |
| 1-750 | 3,4-diF-Ph | 4-Pyr | 3-Et |
| 1-751 | 3,4-diF-Ph | 4-Pyr | 3-Pr |
| 1-752 | 3,4-diF-Ph | 4-Pyr | 3,3-diMe |
| 1-753 | 3,4-diF-Ph | 4-Pyr | 5-Me |
| 1-754 | 3,4-diF-Ph | 4-Pyr | 5-Et |
| 1-755 | 3,4-diF-Ph | 4-Pyr | 5-Pr |
| 1-756 | 3,4-diF-Ph | 4-Pyr | 5,5-diMe |
| 1-757 | 3,4-diF-Ph | 4-Pyr | 6-Me |
| 1-758 | 3,4-diF-Ph | 4-Pyr | 6-Et |
| 1-759 | 3,4-diF-Ph | 4-Pyr | 6-Pr |
| 1-760 | 3,4-diF-Ph | 4-Pyr | 6,6-diMe |
| 1-761 | 3,4-diF-Ph | 4-Pyr | 6-Oxo |
| 1-762 | 3,4-diF-Ph | 4-Pyr | 8-Me |
| 1-763 | 3,4-diF-Ph | 4-Pyr | 8-Et |
| 1-764 | 3,4-diF-Ph | 4-Pyr | 8-Pr |
| 1-765 | 3,4-diF-Ph | 4-Pyr | 8-Ph |
| 1-766 | 3,4-diF-Ph | 4-Pyr | 8a-Me |
| 1-767 | 3,4-diF-Ph | 4-Pyr | 8a-Et |
| 1-768 | 3,4-diF-Ph | 4-Pyr | 8a-Pr |
| 1-769 | 3,4-diF-Ph | $2-NH_2-4-Pym$ | 1-Me |
| 1-770 | 3,4-diF-Ph | $2-NH_2-4-Pym$ | 1-Et |
| 1-771 | 3,4-diF-Ph | $2-NH_2-4-Pym$ | 1-Pr |
| 1-772 | 3,4-diF-Ph | $2-NH_2-4-Pym$ | 1,1-diMe |
| 1-773 | 3,4-diF-Ph | $2-NH_2-4-Pym$ | 2-Me |
| 1-774 | 3,4-diF-Ph | $2-NH_2-4-Pym$ | 2-Et |
| 1-775 | 3,4-diF-Ph | $2-NH_2-4-Pym$ | 2-Pr |
| 1-776 | 3,4-diF-Ph | $2-NH_2-4-Pym$ | 2-Bu |
| 1-777 | 3,4-diF-Ph | $2-NH_2-4-Pym$ | 2-Allyl |
| 1-778 | 3,4-diF-Ph | $2-NH_2-4-Pym$ | 2-Ph |
| 1-779 | 3,4-diF-Ph | $2-NH_2-4-Pym$ | 2-Bn |

Table continued

| Compound No. | $R^1$ | $R^2$ | $R^4$ |
|---|---|---|---|
| 1-780 | 3,4-diF-Ph | $2-NH_2-4-Pym$ | 2-Phet |
| 1-781 | 3,4-diF-Ph | $2-NH_2-4-Pym$ | 2,2-diMe |
| 1-782 | 3,4-diF-Ph | $2-NH_2-4-Pym$ | 2-OH |
| 1-783 | 3,4-diF-Ph | $2-NH_2-4-Pym$ | 2-MeO |
| 1-784 | 3,4-diF-Ph | $2-NH_2-4-Pym$ | 2-EtO |
| 1-785 | 3,4-diF-Ph | $2-NH_2-4-Pym$ | 2-PrO |
| 1-786 | 3,4-diF-Ph | $2-NH_2-4-Pym$ | 2,2-di(MeO) |
| 1-787 | 3,4-diF-Ph | $2-NH_2-4-Pym$ | 2,2-di(EtO) |
| 1-788 | 3,4-diF-Ph | $2-NH_2-4-Pym$ | $2,2-OCH_2CH_2O-$ |
| 1-789 | 3,4-diF-Ph | $2-NH_2-4-Pym$ | 2-Oxo |
| 1-790 | 3,4-diF-Ph | $2-NH_2-4-Pym$ | 2-F |
| 1-791 | 3,4-diF-Ph | $2-NH_2-4-Pym$ | 2-Cl |
| 1-792 | 3,4-diF-Ph | $2-NH_2-4-Pym$ | 2-Br |
| 1-793 | 3,4-diF-Ph | $2-NH_2-4-Pym$ | 2-1 |
| 1-794 | 3,4-diF-Ph | $2-NH_2-4-Pym$ | 2,2-diF |
| 1-795 | 3,4-diF-Ph | $2-NH_2-4-Pym$ | 2,2-diCl |
| 1-796 | 3,4-diF-Ph | $2-NH_2-4-Pym$ | 2,2-diBr |
| 1-797 | 3,4-diF-Ph | $2-NH_2-4-Pym$ | 3-Me |
| 1-798 | 3,4-diF-Ph | $2-NH_2-4-Pym$ | 3-Et |
| 1-799 | 3,4-diF-Ph | $2-NH_2-4-Pym$ | 3-Pr |
| 1-800 | 3,4-diF-Ph | $2-NH_2-4-Pym$ | 3,3-diMe |
| 1-801 | 3,4-diF-Ph | $2-NH_2-4-Pym$ | 5-Me |
| 1-802 | 3,4-diF-Ph | $2-NH_2-4-Pym$ | 5-Et |
| 1-803 | 3,4-diF-Ph | $2-NH_2-4-Pym$ | 5-Pr |
| 1-804 | 3,4-diF-Ph | $2-NH_2-4-Pym$ | 5,5-diMe |
| 1-805 | 3,4-diF-Ph | $2-NH_2-4-Pym$ | 6-Me |
| 1-806 | 3,4-diF-Ph | $2-NH_2-4-Pym$ | 6-Et |
| 1-807 | 3,4-diF-Ph | $2-NH_2-4-Pym$ | 6-Pr |
| 1-808 | 3,4-diF-Ph | $2-NH_2-4-Pym$ | 6,6-diMe |
| 1-809 | 3,4-diF-Ph | $2-NH_2-4-Pym$ | 6-Oxo |
| 1-810 | 3,4-diF-Ph | $2-NH_2-4-Pym$ | 8-Me |
| 1-811 | 3,4-diF-Ph | $2-NH_2-4-Pym$ | 8-Et |
| 1-812 | 3,4-diF-Ph | $2-NH_2-4-Pym$ | 8-Pr |
| 1-813 | 3,4-diF-Ph | $2-NH_2-4-Pym$ | 8-Ph |
| 1-814 | 3,4-diF-Ph | $2-NH_2-4-Pym$ | 8a-Me |
| 1-815 | 3,4-diF-Ph | $2-NH_2-4-Pym$ | 8a-Et |
| 1-816 | 3,4-diF-Ph | $2-NH_2-4-Pym$ | 8a-Pr |
| 1-817 | 3,4-diF-Ph | 2-MeNH-4-Pym | 1-Me |

Table continued

| Compound No. | $R^1$ | $R^2$ | $R^4$ |
|---|---|---|---|
| 1-818 | 3,4-diF-Ph | 2-MeNH-4-Pym | 1-Et |
| 1-819 | 3,4-diF-Ph | 2-MeNH-4-Pym | 1-Pr |
| 1-820 | 3,4-diF-Ph | 2-MeNH-4-Pym | 1,1-diMe |
| 1-821 | 3,4-diF-Ph | 2-MeNH-4-Pym | 2-Me |
| 1-822 | 3,4-diF-Ph | 2-MeNH-4-Pym | 2-Et |
| 1-823 | 3,4-diF-Ph | 2-MeNH-4-Pym | 2-Pr |
| 1-824 | 3,4-diF-Ph | 2-MeNH-4-Pym | 2-Bu |
| 1-825 | 3,4-diF-Ph | 2-MeNH-4-Pym | 2-Allyl |
| 1-826 | 3,4-diF-Ph | 2-MeNH-4-Pym | 2-Ph |
| 1-827 | 3,4-diF-Ph | 2-MeNH-4-Pym | 2-Bn |
| 1-828 | 3,4-diF-Ph | 2-MeNH-4-Pym | 2-Phet |
| 1-829 | 3,4-diF-Ph | 2-MeNH-4-Pym | 2,2-diMe |
| 1-830 | 3,4-diF-Ph | 2-MeNH-4-Pym | 2-OH |
| 1-831 | 3,4-diF-Ph | 2-MeNH-4-Pym | 2-MeO |
| 1-832 | 3,4-diF-Ph | 2-MeNH-4-Pym | 2-EtO |
| 1-833 | 3,4-diF-Ph | 2-MeNH-4-Pym | 2-PrO |
| 1-834 | 3,4-diF-Ph | 2-MeNH-4-Pym | 2,2-di(MeO) |
| 1-835 | 3,4-diF-Ph | 2-MeNH-4-Pym | 2,2-di(EtO) |
| 1-836 | 3,4-diF-Ph | 2-MeNH-4-Pym | 2,2-$OCH_2CH_2O$- |
| 1-837 | 3,4-diF-Ph | 2-MeNH-4-Pym | 2-Oxo |
| 1-838 | 3,4-diF-Ph | 2-MeNH-4-Pym | 2-F |
| 1-839 | 3,4-diF-Ph | 2-MeNH-4-Pym | 2-Cl |
| 1-840 | 3,4-diF-Ph | 2-MeNH-4-Pym | 2-Br |
| 1-841 | 3,4-diF-Ph | 2-MeNH-4-Pym | 2-I |
| 1-842 | 3,4-diF-Ph | 2-MeNH-4-Pym | 2,2-diF |
| 1-843 | 3,4-diF-Ph | 2-MeNH-4-Pym | 2,2-diCl |
| 1-844 | 3,4-diF-Ph | 2-MeNH-4-Pym | 2,2-diBr |
| 1-845 | 3,4-diF-Ph | 2-MeNH-4-Pym | 3-Me |
| 1-846 | 3,4-diF-Ph | 2-MeNH-4-Pym | 3-Et |
| 1-847 | 3,4-diF-Ph | 2-MeNH-4-Pym | 3-Pr |
| 1-848 | 3,4-diF-Ph | 2-MeNH-4-Pym | 3,3-diMe |
| 1-849 | 3,4-diF-Ph | 2-MeNH-4-Pym | 5-Me |
| 1-850 | 3,4-diF-Ph | 2-MeNH-4-Pym | 5-Et |
| 1-851 | 3,4-diF-Ph | 2-MeNH-4-Pym | 5-Pr |
| 1-852 | 3,4-diF-Ph | 2-MeNH-4-Pym | 5,5-diMe |
| 1-853 | 3,4-diF-Ph | 2-MeNH-4-Pym | 6-Me |
| 1-854 | 3,4-diF-Ph | 2-MeNH-4-Pym | 6-Et |
| 1-855 | 3,4-diF-Ph | 2-MeNH-4-Pym | 6-Pr |

Table continued

| Compound No. | $R^1$ | $R^2$ | $R^4$ |
|---|---|---|---|
| 1-856 | 3,4-diF-Ph | 2-MeNH-4-Pym | 6,6-diMe |
| 1-857 | 3,4-diF-Ph | 2-MeNH-4-Pym | 6-Oxo |
| 1-858 | 3,4-diF-Ph | 2-MeNH-4-Pym | 8-Me |
| 1-859 | 3,4.diF-Ph | 2-MeNH-4-Pym | 8-Et |
| 1-860 | 3,4-diF-Ph | 2-MeNH-4-Pym | 8-Pr |
| 1-861 | 3,4-diF-Ph | 2-MeNH-4-Pym | 8-Ph |
| 1-862 | 3,4-diF-Ph | 2-MeNH-4-Pym | 8a-Me |
| 1-863 | 3,4-diF-Ph | 2-MeNH-4-Pym | 8a-Et |
| 1-864 | 3,4-diF-Ph | 2-MeNH-4-Pym | 8a-Pr |
| 1-865 | 4-F-Ph | 2-MeO-4-Pyr | 2-OH |
| 1-866 | 4-F-Ph | 2-MeO-4-Pyr | 2-MeO |
| 1-867 | 4-F-Ph | 2-MeO-4-Pyr | 2-Ph |
| 1-868 | 4-F-Ph | 2-MeO-4-Pyr | 8-Me |
| 1-869 | 4-F-Ph | 2-MeO-4-Pyr | 2-F |
| 1-870 | 4-F-Ph | 2-MeO-4-Pyr | 2-Cl |
| 1-871 | 4-F-Ph | 2-MeO-4-Pyr | 2-Br |
| 1-872 | 4-F-Ph | 2-MeO-4-Pyr | 2,2-diF |
| 1-873 | 4-F-Ph | 2-MeO-4-Pyr | 2,2-diCl |
| 1-874 | 4-F-Ph | 2-$NH_2$-4-Pyr | 2-OH |
| 1-875 | 4-F-Ph | 2-$NH_2$-4-Pyr | 2-MeO |
| 1-876 | 4-F-Ph | 2-$NH_2$-4-Pyr | 2-Ph |
| 1-877 | 4-F-Ph | 2-$NH_2$-4-Pyr | 8-Me |
| 1-878 | 4-F-Ph | 2-$NH_2$-4-Pyr | 2-F |
| 1-879 | 4-F-Ph | 2-$NH_2$-4-Pyr | 2-Cl |
| 1-880 | 4-F-Ph | 2-$NH_2$-4-Pyr | 2-Br |
| 1-881 | 4-F-Ph | 2-$NH_2$-4-Pyr | 2,2-diF |
| 1-882 | 4-F-Ph | 2-$NH_2$-4-Pyr | 2,2-diCl |
| 1-883 | 4-F-Ph | 2-MeNH-4-Pyr | 2-OH |
| 1-884 | 4-F-Ph | 2-MeNH-4-Pyr | 2-MeO |
| 1-885 | 4-F-Ph | 2-MeNH-4-Pyr | 2-Ph |
| 1-886 | 4-F-Ph | 2-MeNH-4-Pyr | 8-Me |
| 1-887 | 4-F-Ph | 2-MeNH-4-Pyr | 2-F |
| 1-888 | 4-F-Ph | 2-MeNH-4-pyr | 2-Cl |
| 1-889 | 4-F-Ph | 2-MeNH-4-Pyr | 2-Br |
| 1-890 | 4-F-Ph | 2-MeNH-4-Pyr | 2,2-diF |
| 1-891 | 4-F-Ph | 2-MeNH-4-Pyr | 2,2-diCl |
| 1-892 | 4-F-Ph | 2-(α-Me-BnNH)-4-Pyr | 2-OH |
| 1-893 | 4-F-Ph | 2-(α-Me-BnNH)-4-Pyr | 2-MeO |

Table continued

| Compound No. | $R^1$ | $R^2$ | $R^4$ |
|---|---|---|---|
| 1-894 | 4-F-Ph | 2-(α-Me-BnNH)-4-Pyr | 2-Ph |
| 1-895 | 4-F-Ph | 2-(α-Me-BnNH)-4-Pyr | 8-Me |
| 1-896 | 4-F-Ph | 2-(α-Me-BnNH)-4-Pyr | 2-F |
| 1-897 | 4-F-Ph | 2-(α-Me-BnNH)-4-Pyr | 2-Cl |
| 1-898 | 4-F-Ph | 2-(α-Me-BnNH)-4-Pyr | 2-Br |
| 1-899 | 4-F-Ph | 2-(α-Me-BnNH)-4-Pyr | 2,2-diF |
| 1-900 | 4-F-Ph | 2-(α-Me-BnNH)-4-Pyr | 2,2-diCl |
| 1-901 | 4-F-Ph | 2-BnNH-4-Pyr | 2-OH |
| 1-902 | 4-F-Ph | 2-BnNH-4-Pyr | 2-MeO |
| 1-903 | 4-F-Ph | 2-BnNH-4-Pyr | 2-Ph |
| 1-904 | 4-F-Ph | 2-BnNH-4-Pyr | 8-Me |
| 1-905 | 4-F-Ph | 2-BnNH-4-Pyr | 2-F |
| 1-906 | 4-F-Ph | 2-BnNH-4-Pyr | 2-Cl |
| 1-907 | 4-F-Ph | 2-BnNH-4-Pyr | 2-Br |
| 1-908 | 4-F-Ph | 2-BnNH-4-Pyr | 2,2-diF |
| 1-909 | 4-F-Ph | 2-BnNH-4-Pyr | 2,2-diCl |
| 1-910 | 4-F-Ph | 4-Pym | 2-OH |
| 1-911 | 4-F-Ph | 4-Pym | 2-MeO |
| 1-912 | 4-F-Ph | 4-Pym | 2-Ph |
| 1-913 | 4-F-Ph | 4-Pym | 8-Me |
| 1-914 | 4-F-Ph | 4-Pym | 2-F |
| 1-915 | 4-F-Ph | 4-Pym | 2-Cl |
| 1-916 | 4-F-Ph | 4-Pym | 2-Br |
| 1-917 | 4-F-Ph | 4-Pym | 2,2-diF |
| 1-918 | 4-F-Ph | 4-Pym | 2,2-diCl |
| 1-919 | 4-F-Ph | 2-MeO-4-Pym | 2-OH |
| 1-920 | 4-F-Ph | 2-MeO-4-Pym | 2-MeO |
| 1-921 | 4-F-Ph | 2-MeO-4-Pym | 2-Ph |
| 1-922 | 4-F-Ph | 2-MeO-4-Pym | 8-Me |
| 1-923 | 4-F-Ph | 2-MeO-4-Pym | 2-F |
| 1-924 | 4-F-Ph | 2-MeO-4-Pym | 2-Cl |
| 1-925 | 4-F-Ph | 2-MeO-4-Pym | 2-Br |
| 1-926 | 4-F-Ph | 2-MeO-4-Pym | 2,2-diF |
| 1-927 | 4-F-Ph | 2-MeO-4-Pym | 2,2-diCl |
| 1-928 | 4-F-Ph | 2-(α-Me-BnNH)-4-Pym | 2-OH |
| 1-929 | 4-F-Ph | 2-(α-Me-BnNH)-4-Pym | 2-MeO |
| 1-930 | 4-F-Ph | 2-(α-Me-BnNH)-4-Pym | 2-Ph |
| 1-931 | 4-F-Ph | 2-(α-Me-BnNH)-4-Pym | 8-Me |

Table continued

| Compound No. | $R^1$ | $R^2$ | $R^4$ |
|---|---|---|---|
| 1-932 | 4-F-Ph | 2-(α-Me-BnNH)-4-Pym | 2-F |
| 1-933 | 4-F-Ph | 2-(α-Me-BnNH)-4-Pym | 2-Cl |
| 1-934 | 4-F-Ph | 2-(α-Me-BnNH)-4-Pym | 2-Br |
| 1-935 | 4-F-Ph | 2-(α-Me-BnNH)-4-Pym | 2,2-diF |
| 1-936 | 4-F-Ph | 2-(α-Me-BnNH)-4-Pym | 2,2-diCl |
| 1-937 | 4-F-Ph | 2-BnNH-4-Pym | 2-OH |
| 1-938 | 4-F-Ph | 2-BnNH-4-Pym | 2-MeO |
| 1-939 | 4-F-Ph | 2-BnNH-4-Pym | 2-Ph |
| 1-940 | 4-F-Ph | 2-BnNH-4-Pym | 8-Me |
| 1-941 | 4-F-Ph | 2-BnNH-4-Pym | 2-F |
| 1-942 | 4-F-Ph | 2-BnNH-4-Pym | 2-Cl |
| 1-943 | 4-F-Ph | 2-BnNH-4-Pym | 2-Br |
| 1-944 | 4-F-Ph | 2-BnNH-4-Pym | 2,2-diF |
| 1-945 | 4-F-Ph | 2-BnNH-4-Pym | 2,2-diCl |
| 1-946 | Ph | 4-Pyr | 6,6-$(CH_2)_2$- |
| 1-947 | Ph | 2-$NH_2$-4-Pym | 6,6-$(CH_2)_2$- |
| 1-948 | Ph | 2-MeNH-4-Pym | 6,6-$(CH_2)_2$- |
| 1-949 | 3-F-Ph | 4-Pyr | 6,6-$(CH_2)_2$- |
| 1-950 | 3-F-Ph | 2-$NH_2$-4-Pym | 6,6-$(CH_2)_2$- |
| 1-951 | 3-F-Ph | 2-MeNH-4-Pym | 6,6-$(CH_2)_2$- |
| 1-952 | 4-F-Ph | 4-Pyr | 6,6-$(CH_2)_2$- |
| 1-953 | 4-F-Ph | 2-$NH_2$-4-Pym | 6,6-$(CH_2)_2$- |
| 1-954 | 4-F-Ph | 2-MeNH-4-Pym | 6,6-$(CH_2)_2$- |
| 1-955 | 3-Cl-Ph | 4-Pyr | 6,6-$(CH_2)_2$- |
| 1-956 | 3-Cl-Ph | 2-$NH_2$-4-Pym | 6,6-$(CH_2)_2$- |
| 1-957 | 3-Cl-Ph | 2-MeNH-4-Pym | 6,6-$(CH_2)_2$- |
| 1-958 | 3-$CF_3$-Ph | 4-Pyr | 6,6-$(CH_2)_2$- |
| 1-959 | 3-$CF_3$-Ph | 2-$NH_2$-4-Pym | 6,6-$(CH_2)_2$- |
| 1-960 | 3-$CF_3$-Ph | 2-MeNH-4-Pym | 6,6-$(CH_2)_2$- |
| 1-961 | 3,4-diF-Ph | 4-Pyr | 6,6-$(CH_2)_2$- |
| 1-962 | 3,4-diF-Ph | 2-$NH_2$-4-Pym | 6,6-$(CH_2)_2$- |
| 1-963 | 3,4-diF-Ph | 2-MeNH-4-Pym | 6,6-$(CH_2)_2$- |
| 1-964 | Ph | 4-Pyr | 6,6-diF |
| 1-965 | Ph | 2-$NH_2$-4-Pym | 6,6-diF |
| 1-966 | Ph | 2-MeNH-4-Pym | 6,6-diF |
| 1-967 | 3-F-Ph | 4-Pyr | 6,6-diF |
| 1-968 | 3-F-Ph | 2-$NH_2$-4-Pym | 6,6-diF |
| 1-969 | 3-F-Ph | 2-MeNH-4-Pym | 6,6-diF |

Table continued

| Compound No. | $R^1$ | $R^2$ | $R^4$ |
|---|---|---|---|
| 1-970 | 4-F-Ph | 4-Pyr | 6,6-diF |
| 1-971 | 4-F-Ph | $2-NH_2-4-Pym$ | 6,6-diF |
| 1-972 | 4-F-Ph | 2-MeNH-4-Pym | 6,6-diF |
| 1-973 | 3-Cl-Ph | 4-Pyr | 6,6-diF |
| 1-974 | 3-Cl-Ph | $2-NH_2-4-Pym$ | 6,6-diF |
| 1-975 | 3-Cl-Ph | 2-MeNH-4-Pym | 6,6-diF |
| 1-976 | $3-CF_3-Ph$ | 4-Pyr | 6,6-diF |
| 1-977 | $3-CF_3-Ph$ | $2-NH_2-4-Pym$ | 6,6-diF |
| 1-978 | $3-CF_3-Ph$ | 2-MeNH-4-Pym | 6,6-diF |
| 1-979 | 3,4-diF-Ph | 4-Pyr | 6,6-diF |
| 1-980 | 3,4-diF-Ph | $2-NH_2-4-Pym$ | 6,6-diF |
| 1-981 | 3,4-diF-Ph | 2-MeNH-4-Pym | 6,6-diF |
| 1-982 | 4-F-Ph | 4-Pyr | $2\text{->}CH_2$ |
| 1-983 | 4-F-Ph | 4-Pyr | 2->CHMe |
| 1-984 | 4-F-Ph | 4-Pyr | 2->CHEt |
| 1-985 | 4-F-Ph | 4-Pyr | 2->CHPr |
| 1-986 | 4-F-Ph | 4-Pyr | $2\text{->}C(Me)_2$ |
| 1-987 | 4-F-Ph | 4-Pyr | 2->CHPh |
| 1-988 | 4-F-Ph | 4-Pyr | 2,2-diPh |
| 1-989 | 4-F-Ph | 4-Pyr | $2,2-O(CH_2)_3O-$ |
| 1-990 | 4-F-Ph | 4-Pyr | $2,2-OCH_2C(Me)_2CH_2O-$ |
| 1-991 | 4-F-Ph | 4-Pyr | $2,2-(CH_2)_2-$ |
| 1-992 | 4-F-Ph | 4-Pyr | $2,2-(CH_2)_3-$ |
| 1-993 | 4-F-Ph | 4-Pyr | $2,2-(CH_2)_4-$ |
| 1-994 | 4-F-Ph | 4-Pyr | $2,2-(CH_2)_5-$ |
| 1-995 | 4-F-Ph | 4-Pyr | 2-MeS |
| 1-996 | 4-F-Ph | 4-Pyr | 2-EtS |
| 1-997 | 4-F-Ph | 4-Pyr | 2-PrS |
| 1-998 | 4-F-Ph | 4-Pyr | 2-BuS |
| 1-999 | 4-F-Ph | 4-Pyr | $2-MeSO_2$ |
| 1-1000 | 4-F-Ph | 4-Pyr | 2-PhO |
| 1-1001 | 4-Cl-Ph | 4-Pyr | 1-Me |
| 1-1002 | 4-Cl-Ph | 4-Pyr | 1-Et |
| 1-1003 | 4-Cl-Ph | 4-Pyr | 1-Pr |
| 1-1004 | 4-Cl-Ph | 4-Pyr | 1,1-diMe |
| 1-1005 | 4-Cl-Ph | 4-Pyr | 2-Me |
| 1-1006 | 4-Cl-Ph | 4-Pyr | 2-Et |
| 1-1007 | 4-Cl-Ph | 4-Pyr | 2-Pr |

Table continued

| Compound No. | $R^1$ | $R^2$ | $R^4$ |
|---|---|---|---|
| 1-1008 | 4-Cl-Ph | 4-Pyr | 2-Bu |
| 1-1009 | 4-Cl-Ph | 4-Pyr | 2-Allyl |
| 1-1010 | 4-Cl-Ph | 4-Pyr | 2-Ph |
| 1-1011 | 4-Cl-Ph | 4-Pyr | 2-Bn |
| 1-1012 | 4-Cl-Ph | 4-Pyr | 2-Phet |
| 1-1013 | 4-Cl-Ph | 4-Pyr | 2,2-diMe |
| 1-1014 | 4-Cl-Ph | 4-Pyr | 2-OH |
| 1-1015 | 4-Cl-Ph | 4-Pyr | 2-MeO |
| 1-1016 | 4-Cl-Ph | 4-Pyr | 2-EtO |
| 1-1017 | 4-Cl-Ph | 4-Pyr | 2-PrO |
| 1-1018 | 4-Cl-Ph | 4-Pyr | 2,2-di(MeO) |
| 1-1019 | 4-Cl-Ph | 4-Pyr | 2,2-di(EtO) |
| 1-1020 | 4-Cl-Ph | 4-Pyr | 2,2-$OCH_2CH_2O$- |
| 1-1021 | 4-Cl-Ph | 4-Pyr | 2-Oxo |
| 1-1022 | 4-Cl-Ph | 4-Pyr | 2-F |
| 1-1023 | 4-Cl-Ph | 4-Pyr | 2-Cl |
| 1-1024 | 4-Cl-Ph | 4-Pyr | 2-Br |
| 1-1025 | 4-Cl-Ph | 4-Pyr | 2-I |
| 1-1026 | 4-Cl-Ph | 4-Pyr | 2,2-diF |
| 1-1027 | 4-Cl-Ph | 4-Pyr | 2,2-diCl |
| 1-1028 | 4-Cl-Ph | 4-Pyr | 2,2-diBr |
| 1-1029 | 4-Cl-Ph | 4-Pyr | 3-Me |
| 1-1030 | 4-Cl-Ph | 4-Pyr | 3-Et |
| 1-1031 | 4-Cl-Ph | 4-Pyr | 3-Pr |
| 1-1032 | 4-Cl-Ph | 4-Pyr | 3,3-diMe |
| 1-1033 | 4-Cl-Ph | 4-Pyr | 5-Me |
| 1-1034 | 4-Cl-Ph | 4-Pyr | 5-Et |
| 1-1035 | 4-Cl-Ph | 4-Pyr | 5-Pr |
| 1-1036 | 4-Cl-Ph | 4-Pyr | 5,5-diMe |
| 1-1037 | 4-Cl-Ph | 4-Pyr | 6-Me |
| 1-1038 | 4-Cl-Ph | 4-Pyr | 6-Et |
| 1-1039 | 4-Cl-Ph | 4-Pyr | 6-Pr |
| 1-1040 | 4-Cl-Ph | 4-Pyr | 6,6-diMe |
| 1-1041 | 4-Cl-Ph | 4-Pyr | 6-Oxo |
| 1-1042 | 4-Cl-Ph | 4-Pyr | 8-Me |
| 1-1043 | 4-Cl-Ph | 4-Pyr | 8-Et |
| 1-1044 | 4-Cl-Ph | 4-Pyr | 8-Pr |
| 1-1045 | 4-Cl-Ph | 4-Pyr | 8-Ph |

Table continued

| Compound No. | $R^1$ | $R^2$ | $R^4$ |
|---|---|---|---|
| 1-1046 | 4-Cl-Ph | 4-Pyr | 8a-Me |
| 1-1047 | 4-Cl-Ph | 4-Pyr | 8a-Et |
| 1-1048 | 4-Cl-Ph | 4-Pyr | 8a+Pr |
| 1-1049 | 4-Cl-Ph | 4-Pyr | 6,6-$(CH_2)_2$- |
| 1-1050 | 4-Cl-Ph | 4-Pyr | 6,6-diF |
| 1-1051 | 4-Cl-Ph | 4-Pyr | 2->$CH_2$ |
| 1-1052 | 4-Cl-Ph | 4-Pyr | 2->CHMe |
| 1-1053 | 4-Cl-Ph | 4-Pyr | 2->CHEt |
| 1-1054 | 4-Cl-Ph | 4-Pyr | 2->CHPr |
| 1-1055 | 4-Cl-Ph | 4-Pyr | 2->$C(Me)_2$ |
| 1-1056 | 4-Cl-Ph | 4-Pyr | 2->CHPh |
| 1-1057 | 4-Cl-Ph | 4-Pyr | 2,2-diPh |
| 1-1058 | 4-Cl-Ph | 4-Pyr | 2,2-$O(CH_2)_3O$- |
| 1-1059 | 4-Cl-Ph | 4-Pyr | 2,2-$OCH_2C(Me)_2CH_2O$- |
| 1-1060 | 4-Cl-Ph | 4-Pyr | 2,2-$(CH_2)_2$- |
| 1-1061 | 4-Cl-Ph | 4-Pyr | 2,2-$(CH_2)_3$- |
| 1-1062 | 4-Cl-Ph | 4-Pyr | 2,2-$(CH_2)_4$- |
| 1-1063 | 4-Cl-Ph | 4-Pyr | 2,2-$(CH_2)_5$- |
| 1-1064 | 4-Cl-Ph | 4-Pyr | 2-MeS |
| 1-1065 | 4-Cl-Ph | 4-Pyr | 2-EtS |
| 1-1066 | 4-Cl-Ph | 4-Pyr | 2-PrS |
| 1-1067 | 4-Cl-Ph | 4-Pyr | 2-BuS |
| 1-1068 | 4-Cl-Ph | 4-Pyr | 2-$MeSO_2$ |
| 1-1069 | 4-Cl-Ph | 4-Pyr | 2-PhO |
| 1-1070 | 4-F-Ph | 4-Pyr | 2-(4-MeO-Ph) |
| 1-1071 | 4-F-Ph | 4-Pyr | 2-(4-Me-Ph) |
| 1-1072 | 4-F-Ph | 4-Pyr | 2-(4-F-Ph) |
| 1-1073 | 4-F-Ph | 4-Pyr | 2-(4-$CF_3$-Ph) |
| 1-1074 | 4-F-Ph | 4-Pyr | 2-(4-Cl-Ph) |
| 1-1075 | 4-F-Ph | 4-Pyr | 2-(2,4-diF-Ph) |
| 1-1076 | 3-$CF_3$-Ph | 4-Pyr | 2-(4-MeO-Ph) |
| 1-1077 | 3-$CF_3$-Ph | 4-Pyr | 2-(4-Me-Ph) |
| 1-1078 | 3-$CF_3$-Ph | 4-Pyr | 2-(4-F-Ph) |
| 1-1079 | 3-$CF_3$-Ph | 4-Pyr | 2-(4-$CF_3$-Ph) |
| 1-1080 | 3-$CF_3$-Ph | 4-Pyr | 2-(4-Cl-Ph) |
| 1-1081 | 3-$CF_3$-Ph | 4-Pyr | 2-(2,4-diF-Ph) |

Table 2

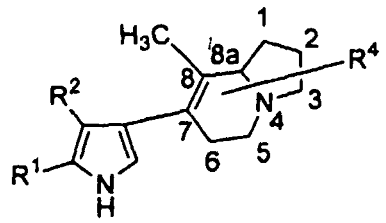

| Compound No. | $R^1$ | $R^2$ | $R^4$ |
|---|---|---|---|
| 2-1 | Ph | 4-Pyr | 1-Me |
| 2-2 | Ph | 4-Pyr | 1-Et |
| 2-3 | Ph | 4-Pyr | 1-Pr |
| 2-4 | Ph | 4-Pyr | 1,1-diMe |
| 2-5 | Ph | 4-Pyr | 2-Me |
| 2-6 | Ph | 4-Pyr | 2-Et |
| 2-7 | Ph | 4-Pyr | 2-Pr |
| 2-8 | Ph | 4-Pyr | 2-Bu |
| 2-9 | Ph | 4-Pyr | 2-Allyl |
| 2-10 | Ph | 4-Pyr | 2-Ph |
| 2-11 | Ph | 4-Pyr | 2-Bn |
| 2-12 | Ph | 4-Pyr | 2-Phet |
| 2-13 | Ph | 4-Pyr | 2,2-diMe |
| 2-14 | Ph | 4-Pyr | 2-OH |
| 2-15 | Ph | 4-Pyr | 2-MeO |
| 2-16 | Ph | 4-Pyr | 2-EtO |
| 2-17 | Ph | 4-Pyr | 2-PrO |
| 2-18 | Ph | 4-Pyr | 2,2-di(MeO) |
| 2-19 | Ph | 4-Pyr | 2,2-di(EtO) |
| 2-20 | Ph | 4-Pyr | 2,2-$OCH_2CH_2O$- |
| 2-21 | Ph | 4-Pyr | 2-Oxo |
| 2-22 | Ph | 4-Pyr | 2-F |
| 2-23 | Ph | 4-Pyr | 2-Cl |
| 2-24 | Ph | 4-Pyr | 2-Br |
| 2-25 | Ph | 4-Pyr | 2-I |
| 2-26 | Ph | 4-Pyr | 2,2-diF |
| 2-27 | Ph | 4-Pyr | 2,2-diCl |
| 2-28 | Ph | 4-Pyr | 2,2-diBr |
| 2-29 | Ph | 4-Pyr | 3-Me |
| 2-30 | Ph | 4-Pyr | 3-Et |
| 2-31 | Ph | 4-Pyr | 3-Pr |

Table continued

| Compound No. | $R^1$ | $R^2$ | $R^4$ |
|---|---|---|---|
| 2-32 | Ph | 4-Pyr | 3,3-diMe |
| 2-33 | Ph | 4-Pyr | 5-Me |
| 2-34 | Ph | 4-Pyr | 5-Et |
| 2-35 | Ph | 4-Pyr | 5-Pr |
| 2-36 | Ph | 4-Pyr | 5,5-diMe |
| 2-37 | Ph | 4-Pyr | 6-Me |
| 2-38 | Ph | 4-Pyr | 6-Et |
| 2-39 | Ph | 4-Pyr | 6-Pr |
| 2-40 | Ph | 4-Pyr | 6,6-diMe |
| 2-41 | Ph | 4-Pyr | 6-Oxo |
| 2-42 | Ph | 4-Pyr | 8a-Me |
| 2-43 | Ph | 4-Pyr | 8a-Et |
| 2-44 | Ph | 4-Pyr | 8a-Pr |
| 2-45 | 3-F-Ph | 4-Pyr | 1-Me |
| 2-46 | 3-F-Ph | 4-Pyr | 1-Et |
| 2-47 | 3-F-Ph | 4-Pyr | 1-Pr |
| 2-48 | 3-F-Ph | 4-Pyr | 1,1-diMe |
| 2-49 | 3-F-Ph | 4-Pyr | 2-Me |
| 2-50 | 3-F-Ph | 4-Pyr | 2-Et |
| 2-51 | 3-F-Ph | 4-Pyr | 2-Pr |
| 2-52 | 3-F-Ph | 4-Pyr | 2-Bu |
| 2-53 | 3-F-Ph | 4-Pyr | 2-Allyl |
| 2-54 | 3-F-Ph | 4-Pyr | 2-Ph |
| 2-55 | 3-F-Ph | 4-Pyr | 2-Bn |
| 2-56 | 3-F-Ph | 4-Pyr | 2-Phet |
| 2-57 | 3-F-Ph | 4-Pyr | 2,2-diMe |
| 2-58 | 3-F-Ph | 4-Pyr | 2-OH |
| 2-59 | 3-F-Ph | 4-Pyr | 2-MeO |
| 2-60 | 3-F-Ph | 4-Pyr | 2-EtO |
| 2-61 | 3-F-Ph | 4-Pyr | 2-PrO |
| 2-62 | 3-F-Ph | 4-Pyr | 2,2-di(MeO) |
| 2-63 | 3-F-Ph | 4-Pyr | 2,2-di(EtO) |
| 2-64 | 3-F-Ph | 4-Pyr | 2,2-$OCH_2CH_2O$- |
| 2-65 | 3-F-Ph | 4-Pyr | 2-Oxo |
| 2-66 | 3-F-Ph | 4-Pyr | 2-F |
| 2-67 | 3-F-Ph | 4-Pyr | 2-Cl |
| 2-68 | 3-F-Ph | 4-Pyr | 2-Br |
| 2-69 | 3-F-Ph | 4-Pyr | 2-I |

Table continued

| Compound No. | $R^1$ | $R^2$ | $R^4$ |
|---|---|---|---|
| 2-70 | 3-F-Ph | 4-Pyr | 2,2-diF |
| 2-71 | 3-F-Ph | 4-Pyr | 2,2-diCl |
| 2-72 | 3-F-Ph | 4-Pyr | 2,2-diBr |
| 2-73 | 3-F-Ph | 4-Pyr | 3-Me |
| 2-74 | 3-F-Ph | 4-Pyr | 3-Et |
| 2-75 | 3-F-Ph | 4-Pyr | 3-Pr |
| 2-76 | 3-F-Ph | 4-Pyr | 3,3-diMe |
| 2-77 | 3-F-Ph | 4-Pyr | 5-Me |
| 2-78 | 3-F-Ph | 4-Pyr | 5-Et |
| 2-79 | 3-F-Ph | 4-Pyr | 5-Pr |
| 2-80 | 3-F-Ph | 4-Pyr | 5,5-diMe |
| 2-81 | 3-F-Ph | 4-Pyr | 6-Me |
| 2-82 | 3-F-Ph | 4-Pyr | 6-Et |
| 2-83 | 3-F-Ph | 4-Pyr | 6-Pr |
| 2-84 | 3-F-Ph | 4-Pyr | 6,6-diMe |
| 2-85 | 3-F-Ph | 4-Pyr | 6-Oxo |
| 2-86 | 3-F-Ph | 4-Pyr | 8a-Me |
| 2-87 | 3-F-Ph | 4-Pyr | 8a-Et |
| 2-88 | 3-F-Ph | 4-Pyr | 8a-Pr |
| 2-89 | 4-F-Ph | 4-Pyr | 1-Me |
| 2-90 | 4-F-Ph | 4-Pyr | 1-Et |
| 2-91 | 4-F-Ph | 4-Pyr | 1-Pr |
| 2-92 | 4-F-Ph | 4-Pyr | 1,1-diMe |
| 2-93 | 4-F-Ph | 4-Pyr | 2-Me |
| 2-94 | 4-F-Ph | 4-Pyr | 2-Et |
| 2-95 | 4-F-Ph | 4-Pyr | 2-Pr |
| 2-96 | 4-F-Ph | 4-Pyr | 2-Bu |
| 2-97 | 4-F-Ph | 4-Pyr | 2-Allyl |
| 2-98 | 4-F-Ph | 4-Pyr | 2-Ph |
| 2-99 | 4-F-Ph | 4-Pyr | 2-Bn |
| 2-100 | 4-F-Ph | 4-Pyr | 2-Phet |
| 2-101 | 4-F-Ph | 4-Pyr | 2,2-diMe |
| 2-102 | 4-F-Ph | 4-Pyr | 2-OH |
| 2-103 | 4-F-Ph | 4-Pyr | 2-MeO |
| 2-104 | 4-F-Ph | 4-Pyr | 2-EtO |
| 2-105 | 4-F-Ph | 4-Pyr | 2-PrO |
| 2-106 | 4-F-Ph | 4-Pyr | 2,2-di(MeO) |
| 2-107 | 4-F-Ph | 4-Pyr | 2,2-di(EtO) |

Table continued

| Compound No. | $R^1$ | $R^2$ | $R^4$ |
|---|---|---|---|
| 2-108 | 4-F-Ph | 4-Pyr | 2,2-$OCH_2CH_2O$- |
| 2-109 | 4-F-Ph | 4-Pyr | 2-Oxo |
| 2-110 | 4-F-Ph | 4-Pyr | 2-F |
| 2-111 | 4-F-Ph | 4-Pyr | 2-Cl |
| 2-112 | 4-F-Ph | 4-Pyr | 2-Br |
| 2-113 | 4-F-Ph | 4-Pyr | 2-I |
| 2-114 | 4-F-Ph | 4-Pyr | 2,2-diF |
| 2-115 | 4-F-Ph | 4-Pyr | 2,2-diCl |
| 2-116 | 4-F-Ph | 4-Pyr | 2,2-diBr |
| 2-117 | 4-F-Ph | 4-Pyr | 3-Me |
| 2-118 | 4-F-Ph | 4-Pyr | 3-Et |
| 2-119 | 4-F-Ph | 4-Pyr | 3-Pr |
| 2-120 | 4-F-Ph | 4-Pyr | 3,3-diMe |
| 2-121 | 4-F-Ph | 4-Pyr | 5-Me |
| 2-122 | 4-F-Ph | 4-Pyr | 5-Et |
| 2-123 | 4-F-Ph | 4-Pyr | 5-Pr |
| 2-124 | 4-F-Ph | 4-Pyr | 5,5-diMe |
| 2-125 | 4-F-Ph | 4-Pyr | 6-Me |
| 2-126 | 4-F-Ph | 4-Pyr | 6-Et |
| 2-127 | 4-F-Ph | 4-Pyr | 6-Pr |
| 2-128 | 4-F-Ph | 4-Pyr | 6,6-diMe |
| 2-129 | 4-F-Ph | 4-Pyr | 6-Oxo |
| 2-130 | 4-F-Ph | 4-Pyr | 8a-Me |
| 2-131 | 4-F-Ph | 4-Pyr | 8a-Et |
| 2-132 | 4-F-Ph | 4-Pyr | 8a-Pr |
| 2-133 | 3-Cl-Ph | 4-Pyr | 1-Me |
| 2-134 | 3-Cl-Ph | 4-Pyr | 1-Et |
| 2-135 | 3-Cl-Ph | 4-Pyr | 1-Pr |
| 2-136 | 3-Cl-Ph | 4-Pyr | 1,1-diMe |
| 2-137 | 3-Cl-Ph | 4-Pyr | 2-Me |
| 2-138 | 3-Cl-Ph | 4-Pyr | 2-Et |
| 2-139 | 3-Cl-Ph | 4-Pyr | 2-Pr |
| 2-140 | 3-Cl-Ph | 4-Pyr | 2-Bu |
| 2-141 | 3-Cl-Ph | 4-Pyr | 2-Allyl |
| 2-142 | 3-Cl-Ph | 4-Pyr | 2-Ph |
| 2-143 | 3-Cl-Ph | 4-Pyr | 2-Bn |
| 2-144 | 3-Cl-Ph | 4-Pyr | 2-Phet |
| 2-145 | 3-Cl-Ph | 4-Pyr | 2,2-diMe |

Table continued

| Compound No. | $R^1$ | $R^2$ | $R^4$ |
|---|---|---|---|
| 2-146 | 3-Cl-Ph | 4-Pyr | 2-OH |
| 2-147 | 3-Cl-Ph | 4-Pyr | 2-MeO |
| 2-148 | 3-Cl-Ph | 4-Pyr | 2-EtO |
| 2-149 | 3-Cl-Ph | 4-Pyr | 2-PrO |
| 2-150 | 3-Cl-Ph | 4-Pyr | 2,2-di(MeO) |
| 2-151 | 3-Cl-Ph | 4-Pyr | 2,2-di(EtO) |
| 2-152 | 3-Cl-Ph | 4-Pyr | 2,2-$OCH_2CH_2O$- |
| 2-153 | 3-Cl-Ph | 4-Pyr | 2-Oxo |
| 2-154 | 3-Cl-Ph | 4-Pyr | 2-F |
| 2-155 | 3-Cl-Ph | 4-Pyr | 2-Cl |
| 2-156 | 3-Cl-Ph | 4-Pyr | 2-Br |
| 2-157 | 3-Cl-Ph | 4-Pyr | 2-I |
| 2-158 | 3-Cl-Ph | 4-Pyr | 2,2-diF |
| 2-159 | 3-Cl-Ph | 4-Pyr | 2,2-diCl |
| 2-160 | 3-Cl-Ph | 4-Pyr | 2,2-diBr |
| 2-161 | 3-Cl-Ph | 4-Pyr | 3-Me |
| 2-162 | 3-Cl-Ph | 4-Pyr | 3-Et |
| 2-163 | 3-Cl-Ph | 4-Pyr | 3-Pr |
| 2-164 | 3-Cl-Ph | 4-Pyr | 3,3-diMe |
| 2-165 | 3-Cl-Ph | 4-Pyr | 5-Me |
| 2-166 | 3-Cl-Ph | 4-Pyr | 5-Et |
| 2-167 | 3-Cl-Ph | 4-Pyr | 5-Pr |
| 2-168 | 3-Cl-Ph | -4-Pyr | 5,5-diMe |
| 2-169 | 3-Cl-Ph | 4-Pyr | 6-Me |
| 2-170 | 3-Cl-Ph | 4-Pyr | 6-Et |
| 2-171 | 3-Cl-Ph | 4-Pyr | 6-Pr |
| 2-172 | 3-Cl-Ph | 4-Pyr | 6,6-diMe |
| 2-173 | 3-Cl-Ph | 4-Pyr | 6-Oxo |
| 2-174 | 3-Cl-Ph | 4-Pyr | 8a-Me |
| 2-175 | 3-Cl-Ph | 4-Pyr | 8a-Et |
| 2-176 | 3-Cl-Ph | 4-Pyr | 8a-Pr |
| 2-177 | 3-$CF_3$-Ph | 4-Pyr | 1-Me |
| 2-178 | 3-$CF_3$-Ph | 4-Pyr | 1-Et |
| 2-179 | 3-$CF_3$-Ph | 4-Pyr | 1-Pr |
| 2-180 | 3-$CF_3$-Ph | 4-Pyr | 1,1-diMe |
| 2-181 | 3-$CF_3$-Ph | 4-Pyr | 2-Me |
| 2-182 | 3-$CF_3$-Ph | 4-Pyr | 2-Et |
| 2-183 | 3-$CF_3$-Ph | 4-Pyr | 2-Pr |

Table continued

| Compound No. | $R^1$ | $R^2$ | $R^4$ |
|---|---|---|---|
| 2-184 | 3-$CF_3$-Ph | 4-Pyr | 2-Bu |
| 2-185 | 3-$CF_3$-Ph | 4-Pyr | 2-Allyl |
| 2-186 | 3-$CF_3$-Ph | 4-Pyr | 2-Ph |
| 2-187 | 3-$CF_3$-Ph | 4-Pyr | 2-Bn |
| 2-188 | 3-$CF_3$-Ph | 4-Pyr | 2-Phet |
| 2-189 | 3-$CF_3$-Ph | 4-Pyr | 2,2-diMe |
| 2-190 | 3-$CF_3$-Ph | 4-Pyr | 2-OH |
| 2-191 | 3-$CF_3$-Ph | 4-Pyr | 2-MeO |
| 2-192 | 3-$CF_3$-Ph | 4-Pyr | 2-EtO |
| 2-193 | 3-$CF_3$-Ph | 4-Pyr | 2-PrO |
| 2-194 | 3-$CF_3$-Ph | 4-Pyr | 2,2-di(MeO) |
| 2-195 | 3-$CF_3$-Ph | 4-Pyr | 2,2-di(EtO) |
| 2-196 | 3-$CF_3$-Ph | 4-Pyr | 2,2-$OCH_2CH_2O$- |
| 2-197 | 3-$CF_3$-Ph | 4-Pyr | 2-Oxo |
| 2-198 | 3-$CF_3$-Ph | 4-Pyr | 2-F |
| 2-199 | 3-$CF_3$-Ph | 4-Pyr | 2-Cl |
| 2-200 | 3-$CF_3$-Ph | 4-Pyr | 2-Br |
| 2-201 | 3-$CF_3$-Ph | 4-Pyr | 2-I |
| 2-202 | 3-$CF_3$-Ph | 4-Pyr | 2,2-diF |
| 2-203 | 3-$CF_3$-Ph | 4-Pyr | 2,2-diCl |
| 2-204 | 3-$CF_3$-Ph | 4-Pyr | 2,2-diBr |
| 2-205 | 3-$CF_3$-Ph | 4-Pyr | 3-Me |
| 2-206 | 3-$CF_3$-Ph | 4-Pyr | 3-Et |
| 2-207 | 3-$CF_3$-Ph | 4-Pyr | 3-Pr |
| 2-208 | 3-$CF_3$-Ph | 4-Pyr | 3,3-diMe |
| 2-209 | 3-$CF_3$-Ph | 4-Pyr | 5-Me |
| 2-210 | 3-$CF_3$-Ph | 4-Pyr | 5-Et |
| 2-211 | 3-$CF_3$-Ph | 4-Pyr | 5-Pr |
| 2-212 | 3-$CF_3$-Ph | 4-Pyr | 5,5-diMe |
| 2-213 | 3-$CF_3$-Ph | 4-Pyr | 6-Me |
| 2-214 | 3-$CF_3$-Ph | 4-Pyr | 6-Et |
| 2-215 | 3-$CF_3$-Ph | 4-Pyr | 6-Pr |
| 2-216 | 3-$CF_3$-Ph | 4-Pyr | 6,6-diMe |
| 2-217 | 3-$CF_3$-Ph | 4-Pyr | 6-Oxo |
| 2-218 | 3-$CF_3$-Ph | 4-Pyr | 8a-Me |
| 2-219 | 3-$CF_3$-Ph | 4-Pyr | 8a-Et |
| 2-220 | 3-$CF_3$-Ph | 4-Pyr | 8a-Pr |
| 2-221 | 3,4-diF-Ph | 4-Pyr | 1-Me |

Table continued

| Compound No. | $R^1$ | $R^2$ | $R^4$ |
|---|---|---|---|
| 2-222 | 3,4-diF-Ph | 4-Pyr | 1-Et |
| 2-223 | 3,4-diF-Ph | 4-Pyr | 1-Pr |
| 2-224 | 3,4-diF-Ph | 4-Pyr | 1,1-diMe |
| 2-225 | 3,4-diF-Ph | 4-Pyr | 2-Me |
| 2-226 | 3,4-diF-Ph | 4-Pyr | 2-Et |
| 2-227 | 3,4-diF-Ph | 4-Pyr | 2-Pr |
| 2-228 | 3,4-diF-Ph | 4-Pyr | 2-Bu |
| 2-229 | 3,4-diF-Ph | 4-Pyr | 2-Allyl |
| 2-230 | 3,4-diF-Ph | 4-Pyr | 2-Ph |
| 2-231 | 3,4-diF-Ph | 4-Pyr | 2-Bn |
| 2-232 | 3,4-diF-Ph | 4-Pyr | 2-Phet |
| 2-233 | 3,4-diF-Ph | 4-Pyr | 2,2-diMe |
| 2-234 | 3,4-diF-Ph | 4-Pyr | 2-OH |
| 2-235 | 3,4-diF-Ph | 4-Pyr | 2-MeO |
| 2-236 | 3,4-diF-Ph | 4-Pyr | 2-EtO |
| 2-237 | 3,4-diF-Ph | 4-Pyr | 2-PrO |
| 2-238 | 3,4-diF-Ph | 4-Pyr | 2,2-di(MeO) |
| 2-239 | 3,4-diF-Ph | 4-Pyr | 2,2-di(EtO) |
| 2-240 | 3,4-diF-Ph | 4-Pyr | 2,2-$OCH_2CH_2O$- |
| 2-241 | 3,4-diF-Ph | 4-Pyr | 2-Oxo |
| 2-242 | 3,4-diF-Ph | 4-Pyr | 2-F |
| 2-243 | 3,4-diF-Ph | 4-Pyr | 2-Cl |
| 2-244 | 3,4-diF-Ph | 4-Pyr | 2-Br |
| 2-245 | 3,4-diF-Ph | 4-Pyr | 2-I |
| 2-246 | 3,4-diF-Ph | 4-Pyr | 2,2-diF |
| 2-247 | 3,4-diF-Ph | 4-Pyr | 2,2-diCl |
| 2-248 | 3,4-diF-Ph | 4-Pyr | 2,2-diBr |
| 2-249 | 3,4-diF-Ph | 4-Pyr | 3-Me |
| 2-250 | 3,4-diF-Ph | 4-Pyr | 3-Et |
| 2-251 | 3,4-diF-Ph | 4-Pyr | 3-Pr |
| 2-252 | 3,4-diF-Ph | 4-Pyr | 3,3-diMe |
| 2-253 | 3,4-diF-Ph | 4-Pyr | 5-Me |
| 2-254 | 3,4-diF-Ph | 4-Pyr | 5-Et |
| 2-255 | 3,4-diF-Ph | 4-Pyr | 5-Pr |
| 2-256 | 3,4-diF-Ph | 4-Pyr | 5,5-diMe |
| 2-257 | 3,4-diF-Ph | 4-Pyr | 6-Me |
| 2-258 | 3,4-diF-Ph | 4-Pyr | 6-Et |
| 2-259 | 3,4-diF-Ph | 4-Pyr | 6-Pr |

Table continued

| Compound No. | $R^1$ | $R^2$ | $R^4$ |
|---|---|---|---|
| 2-260 | 3,4-diF-Ph | 4-Pyr | 6,6-diMe |
| 2-261 | 3,4-diF-Ph | 4-Pyr | 6-Oxo |
| 2-262 | 3,4-diF-Ph | 4-Pyr | 8a-Me |
| 2-263 | 3,4-diF-Ph | 4-Pyr | 8a-Et |
| 2-264 | 3,4-diF-Ph | 4-Pyr | 8a-Pr |
| 2-265 | Ph | 4-Pyr | 6,6-$(CH_2)_2$- |
| 2-266 | 3-F-Ph | 4-Pyr | 6,6-$(CH_2)_2$- |
| 2-267 | 4-F-Ph | 4-Pyr | 6,6-$(CH_2)_2$- |
| 2-268 | 3-Cl-Ph | 4-Pyr | 6,6-$(CH_2)_2$- |
| 2-269 | 3-$CF_3$-Ph | 4-Pyr | 6,6-$(CH_2)_2$- |
| 2-270 | 3,4-diF-Ph | 4-Pyr | 6,6-$(CH_2)_2$- |
| 2-271 | Ph | 4-Pyr | 6,6-diF |
| 2-272 | 3-F-Ph | 4-Pyr | 6,6-diF |
| 2-273 | 4-F-Ph | 4-Pyr | 6,6-diF |
| 2-274 | 3-Cl-Ph | 4-Pyr | 6,6-diF |
| 2-275 | 3-$CF_3$-Ph | 4-Pyr | 6,6-diF |
| 2-276 | 3,4-diF-Ph | 4-Pyr | 6,6-diF |

Table 3

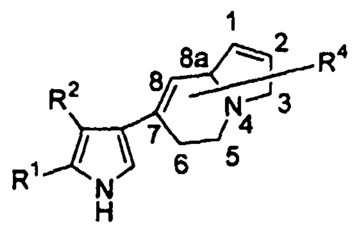

| Compound No. | $R^1$ | $R^2$ | $R^4$ |
|---|---|---|---|
| 3-1 | 4-F-Ph | 4-Pyr | 2-Me |
| 3-2 | 4-F-Ph | 4-Pyr | 2-Et |
| 3-3 | 4-F-Ph | 4-Pyr | 2-Pr |
| 3-4 | 4-F-Ph | 4-Pyr | 2-Bu |
| 3-5 | 4-F-Ph | 4-Pyr | 2-Allyl |
| 3-6 | 4-F-Ph | 4-Pyr | 2-Ph |
| 3-7 | 4-F-Ph | 4-Pyr | 2-Bn |
| 3-8 | 4-F-Ph | 4-Pyr | 2-Phet |
| 3-9 | 4-F-Ph | 4-Pyr | 3-Me |
| 3-10 | 4-F-Ph | 4-Pyr | 3-Et |
| 3-11 | 4-F-Ph | 4-Pyr | 3-Pr |

Table continued

| Compound No. | $R^1$ | $R^2$ | $R^4$ |
|---|---|---|---|
| 3-12 | 4-F-Ph | 4-Pyr | 3,3-diMe |
| 3-13 | 4-F-Ph | 4-Pyr | 5-Me |
| 3-14 | 4-F-Ph | 4-Pyr | 5-Et |
| 3-15 | 4-F-Ph | 4-Pyr | 5-Pr |
| 3-16 | 4-F-Ph | 4-Pyr | 5,5-diMe |
| 3-17 | 4-F-Ph | 4-Pyr | 6-Me |
| 3-18 | 4-F-Ph | 4-Pyr | 6-Et |
| 3-19 | 4-F-Ph | 4-Pyr | 6-Pr |
| 3-20 | 4-F-Ph | 4-Pyr | 6,6-diMe |
| 3-21 | 4-F-Ph | 4-Pyr | 6,6-$(CH_2)_2$- |
| 3-22 | 4-F-Ph | 4-Pyr | 6-Oxo |
| 3-23 | 4-F-Ph | 4-Pyr | 8-Me |
| 3-24 | 4-F-Ph | 4-Pyr | 8-Et |
| 3-25 | 4-F-Ph | 4-Pyr | 8-Pr |
| 3-26 | 4-F-Ph | 4-Pyr | 8-Ph |
| 3-27 | 4-F-Ph | 4-Pyr | 8a-Me |
| 3-28 | 4-F-Ph | 4-Pyr | 8a-Et |
| 3-29 | 4-F-Ph | 4-Pyr | 8a-Pr |

Table 4

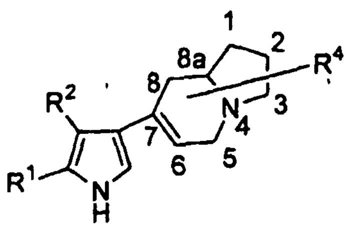

| Compound No. | $R^1$ | $R^2$ | $R^4$ |
|---|---|---|---|
| 4-1 | 4-F-Ph | 4-Pyr | 1-Me |
| 4-2 | 4-F-Ph | 4-Pyr | 1-Et |
| 4-3 | 4-F-Ph | 4-Pyr | 1-Pr |
| 4-4 | 4-F-Ph | 4-Pyr | 1,1-diMe |
| 4-5 | 4-F-Ph | 4-Pyr | 2-Me |
| 4-6 | 4-F-Ph | 4-Pyr | 2-Et |
| 4-7 | 4-F-Ph | 4-Pyr | 2-Pr |
| 4-8 | 4-F-Ph | 4-Pyr | 2-Bu |
| 4-9 | 4-F-Ph | 4-Pyr | 2-Allyl |
| 4-10 | 4-F-Ph | 4-Pyr | 2-Ph |

Table continued

| Compound No. | $R^1$ | $R^2$ | $R^4$ |
|---|---|---|---|
| 4-11 | 4-F-Ph | 4-Pyr | 2-Bn |
| 4-12 | 4-F-Ph | 4-Pyr | 2-Phet |
| 4-13 | 4-F-Ph | 4-Pyr | 2,2-diMe |
| 4-14 | 4-F-Ph | 4-Pyr | 2-OH |
| 4-15 | 4-F-Ph | 4-Pyr | 2-MeO |
| 4-16 | 4-F-Ph | 4-Pyr | 2-EtO |
| 4-17 | 4-F-Ph | 4-Pyr | 2-PrO |
| 4-18 | 4-F-Ph | 4-Pyr | 2,2-di(MeO) |
| 4-19 | 4-F-Ph | 4-Pyr | 2,2-di(EtO) |
| 4-20 | 4-F-Ph | 4-Pyr | 2,2-$OCH_2CH_2O$- |
| 4-21 | 4-F-Ph | 4-Pyr | 2-Oxo |
| 4-22 | 4-F-Ph | 4-Pyr | 2-F |
| 4-23 | 4-F-Ph | 4-Pyr | 2-Cl |
| 4-24 | 4-F-Ph | 4-Pyr | 2-Br |
| 4-25 | 4-F-Ph | 4-Pyr | 2-I |
| 4-26 | 4-F-Ph | 4-Pyr | 2,2-diF |
| 4-27 | 4-F-Ph | 4-Pyr | 2,2-diCl |
| 4-28 | 4-F-Ph | 4-Pyr | 2,2-diBr |
| 4-29 | 4-F-Ph | 4-Pyr | 3-Me |
| 4-30 | 4-F-Ph | 4-Pyr | 3-Et |
| 4-31 | 4-F-Ph | 4-Pyr | 3-Pr |
| 4-32 | 4-F-Ph | 4-Pyr | 3,3-diMe |
| 4-33 | 4-F-Ph | 4-Pyr | 5-Me |
| 4-34 | 4-F-Ph | 4-Pyr | 5-Et |
| 4-35 | 4-F-Ph | 4-Pyr | 5-Pr |
| 4-36 | 4-F-Ph | 4-Pyr | 5,5-diMe |
| 4-37 | 4-F-Ph | 4-Pyr | 6-Me |
| 4-38 | 4-F-Ph | 4-Pyr | 6-Et |
| 4-39 | 4-F-Ph | 4-Pyr | 6-Pr |
| 4-40 | 4-F-Ph | 4-Pyr | 6-Ph |
| 4-41 | 4-F-Ph | 4-Pyr | 8-Me |
| 4-42 | 4-F-Ph | 4-Pyr | 8-Et |
| 4-43 | 4-F-Ph | 4-Pyr | 8-Pr |
| 4-44 | 4-F-Ph | 4-Pyr | 8,8-diMe |
| 4-45 | 4-F-Ph | 4-Pyr | 8,8-$(CH_2)_2$- |
| 4-46 | 4-F-Ph | 4-Pyr | 8-Oxo |
| 4-47 | 4-F-Ph | 4-Pyr | 8a-Me |
| 4-48 | 4-F-Ph | 4-Pyr | 8a-Et |

Table continued

| Compound No. | $R^1$ | $R^2$ | $R^4$ |
|---|---|---|---|
| 4-49 | 4-F-Ph | 4-Pyr | 8a-Pr |
| 4-50 | 4-F-Ph | 4-Pyr | 2->$CH_2$ |
| 4-51 | 4-F-Ph | 4-Pyr | 2->CHMe |
| 4-52 | 4-F-Ph | 4-Pyr | 2->CHEt |
| 4-53 | 4-F-Ph | 4-Pyr | 2->CHPr |
| 4-54 | 4-F-Ph | 4-Pyr | 2->$C(Me)_2$ |
| 4-5 | 4-F-Ph | 4-Pyr | 2->CHPh |
| 4-56 | 4-F-Ph | 4-Pyr | 2,2-diPh |
| 4-57 | 4-F-Ph | 4-Pyr | 2,2-$O(CH_2)_3O$- |
| 4-58 | 4-F-Ph | 4-Pyr | 2,2-$OCH_2C(Me)_2CH_2O$- |
| 4-59 | 4-F-Ph | 4-Pyr | 2,2-$(CH_2)_2$- |
| 4-60 | 4-F-Ph | 4-Pyr | 2,2-$(CH_2)_3$- |
| 4-61 | 4-F-Ph | 4-Pyr | 2,2-$(CH_2)_4$- |
| 4-62 | 4-F-Ph | 4-Pyr | 2,2-$(CH_2)_5$- |
| 4-63 | 4-F-Ph | 4-Pyr | 2-MeS |
| 4-64 | 4-F-Ph | 4-Pyr | 2-EtS |
| 4-65 | 4-F-Ph | 4-Pyr | 2-PrS |
| 4-66 | 4-F-Ph | 4-Pyr | 2-BuS |
| 4-67 | 4-F-Ph | 4-Pyr | 2-$MeSO_2$ |
| 4-68 | 4-F-Ph | 4-Pyr | 2-PhO |

Table 5

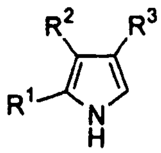

(I-1)

| Compound No. | $R^1$ | $R^2$ | $R^3$ |
|---|---|---|---|
| 5-1 | 4-F-Ph | 4-Pyr | 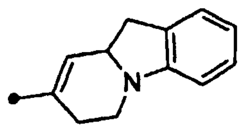 |
| 5-2 | 4-F-Ph | 4-Pyr | 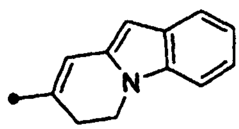 |

Table continued

| Compound No. | $R^1$ | $R^2$ | $R^3$ |
|---|---|---|---|
| 5-3 | 4-F-Ph | 4-Pyr | 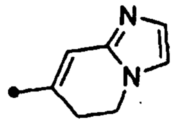 |
| 5-4 | 4-F-Ph | 4-Pyr | 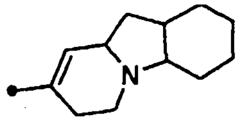 |
| 5-5 | 4-F-Ph | 4-Pyr | 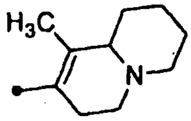 |
| 5-6 | 4-F-Ph | 4-Pyr | 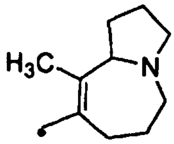 |
| 5-7 | 4-F-Ph | 4-Pyr | 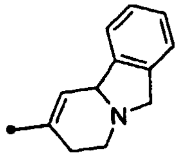 |
| 5-8 | 4-F-Ph | 4-Pyr | 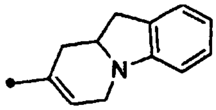 |

Table 6

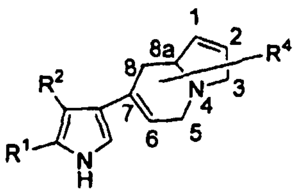

| Compound No. | $R^1$ | $R^2$ | $R^4$ |
|---|---|---|---|
| 6-1 | 4-F-Ph | 4-Pyr | 2-Me |
| 6-2 | 4-F-Ph | 4-Pyr | 2-Et |
| 6-3 | 4-F-Ph | 4-Pyr | 2-Pr |
| 6-4 | 4-F-Ph | 4-Pyr | 2-Bu |
| 6-5 | 4-F-Ph | 4-Pyr | 2-Allyl |
| 6-6 | 4-F-Ph | 4-Pyr | 2-Ph |
| 6-7 | 4-F-Ph | 4-Pyr | 2-Bn |
| 6-8 | 4-F-Ph | 4-Pyr | 2-Phet |

Table continued

| Compound No. | $R^1$ | $R^2$ | $R^4$ |
|---|---|---|---|
| 6-9 | 4-F-Ph | 4-Pyr | 3-Me |
| 6-10 | 4-F-Ph | 4-Pyr | 3-Et |
| 6-11 | 4-F-Ph | 4-Pyr | 3-Pr |
| 6-12 | 4-F-Ph | 4-Pyr | 3,3-diMe |
| 6-13 | 4-F-Ph | 4-Pyr | 5-Me |
| 6-14 | 4-F-Ph | 4-Pyr | 5-Et |
| 6-15 | 4-F-Ph | 4-Pyr | 5-Pr |
| 6-16 | 4-F-Ph | 4-Pyr | 5,5-diMe |
| 6-17 | 4-F-Ph | 4-Pyr | 6-Me |
| 6-18 | 4-F-Ph | 4-Pyr | 6-Et |
| 6-19 | 4-F-Ph | 4-Pyr | 6-Pr |
| 6-20 | 4-F-Ph | 4-Pyr | 6,6-diMe |
| 6-21 | 4-F-Ph | 4-Pyr | 6,6-$(CH_2)_2$- |
| 6-22 | 4-F-Ph | 4-Pyr | 6-Oxo |
| 6-23 | 4-F-Ph | 4-Pyr | 8-Me |
| 6-24 | 4-F-Ph | 4-Pyr | 8-Et |
| 6-25 | 4-F-Ph | 4-Pyr | 8-Pr |
| 6-26 | 4-F-Ph | 4-Pyr | 8-Ph |
| 6-27 | 4-F-Ph | 4-Pyr | 8a-Me |
| 6-28 | 4-F-Ph | 4-Pyr | 8a-Et |
| 6-29 | 4-F-Ph | 4-Pyr | 8a-Pr |

In the above tables, the following abbreviations are used:

Bn represents benzyl,
Bu represents butyl,
Et represents ethyl,
Me represents methyl,
Ph represents phenyl,
Phet represents phenethyl,
Pr represents propyl,
Pym represents pyrimidinyl
Pyr represents pyridyl,
$>CH_2$ represents methylidenyl,
>CHMe represents ethylidenyl,
>CHEt represents propylidenyl,
$>C(Me)_2$ represents isopropylidenyl,
>CHPr represents butylidenyl, and
>CHPh represents benzylidenyl.

In the above Tables 1 to 6, examples of preferred compounds include the compounds of Compound Nos. 1-5 to 1-7, 1-10, 1-14 to 1-23, 1-26, 1-27, 1-37 to 1-40, 1-42 to 1-44, 1-149 to 1-151, 1-154, 1-158 to 1-167, 1-170, 1-171, 1-181 to 1-184, 1-186 to 1-188, 1-197 to 1-199, 1-202, 1-206 to 1-215, 1-218, 1-219, 1-229 to 1-232, 1-234 to 1-236, 1-245 to 1-247, 1-250, 1-254 to 1-263, 1-266, 1-267, 1-277 to 1-280, 1-282 to 1-284, 1-293 to 1-296, 1-298, 1-301 to 1-311, 1-314, 1-315, 1-324 to 1-328, 1-330 to 1-332, 1-341 to 1-343, 1-346, 1-350 to 1-359, 1-362, 1-363, 1-373 to

1-376, 1-378 to 1-380, 1-389 to 1-391, 1-394, 1-398 to 1-407, 1-410, 1-411, 1-421 to 1-424, 1-426 to 1-428, 1-433 to 1-439, 1-442, 1-446 to 1-455, 1-458, 1-459, 1-469 to 1-472, 1-474 to 1-476, 1-485 to 1-487, 1-490, 1-494 to 1-503, 1-506, 1-507, 1-517 to 1-520, 1-522 to 1-524, 1-533 to 1-535, 1-538, 1-542 to 1-551, 1-554, 1-555, 1-565 to 1-568, 1-570 to 1-572, 1-581 to 1-583, 1-586, 1-590 to 1-599, 1-602, 1-603, 1-613 to 1-616, 1-618 to 1-620, 1-629 to 1-631, 1-634, 1-638 to 1-647, 1-650, 1-651, 1-661 to 1-664, 1-666 to 1-668, 1-677 to 1-679, 1-682, 1-686 to 1-695, 1-698, 1-699, 1-709 to 1-712, 1-714 to 1-716, 1-725 to 1-727, 1-730, 1-734 to 1-743, 1-746, 1-747, 1-757 to 1-760, 1-762 to 1-764, 1-946 to 1-987, 1-989 to 1-998, 1-1005 to 1-1008, 1-1010, 1-1014 to 1-1023, 1-1026, 1-1027, 1-1037 to 1-1040, 1-1042 to 1-1044, 1-1049 to 1-1056, 1-1058 to 1-1067, 1-1070 to 1-1081,
2-5 to 2-7, 2-10, 2-14 to 2-23, 2-26, 2-27, 2-37 to 2-40, 2-49 to 2-51, 2-54, 2-58 to 2-67, 2-70, 2-71, 2-81 to 2-84, 2-93 to 2-95, 2-98, 2-102 to 2-111, 2-114, 2-115, 2-125 to 2-128, 2-137 to 2-139, 2-142, 2-146 to 2-155, 2-158, 2-159, 2-169 to 2-172, 2-181 to 2-183, 2-186, 2-190 to 2-199, 2-202, 2-203, 2-213 to 2-216, 2-225 to 2-227, 2-230, 2-234 to 2-243, 2-246, 2-247, 2-257 to 2-260, 2-265 to 2-276,
3-1 to 3-4, 3-6, 3-17 to 3-21, 3-23 to 3-25,
4-5 to 4-7, 4-10, 4-14 to 4-23, 4-26, 4-27, 4-50 to 4-55, 4-57 to 4-66,
5-1, 5-3, 5-5 to 5-8,
6-1 to 6-3 and 6-6.

Examples of more preferred compounds include the compounds of Compound Nos. 1-5, 1-6, 1-10, 1-14 to 1-16, 1-18 to 1-23, 1-26, 1-27, 1-37, 1-38, 1-40, 1-42, 1-43, 1-149 to 1-151, 1-154, 1-158 to 1-160, 1-162 to 1-167, 1-170, 1-171, 1-181, 1-182, 1-184, 1-186, 1-187, 1-197, 1-198, 1-202, 1-206 to 1-208, 1-210 to 1-215, 1-218, 1-219, 1-229, 1-230, 1-232, 1-234, 1-235, 1-245, 1-246, 1-250, 1-254 to 1-256, 1-258 to 1-263, 1-266, 1-267, 1-277, 1-278, 1-280, 1-282, 1-283, 1-293 to 1-295, 1-298, 1-301 to 1-304, 1-306 to 1-311, 1-314, 1-315, 1-324 to 1-326, 1-328, 1-330, 1-331, 1-341, 1-342, 1-346, 1-350 to 1-352, 1-354 to 1-359, 1-362, 1-363, 1-373, 1-374, 1-376, 1-378, 1-379, 1-389, 1-390, 1-394, 1-398 to 1-400, 1-402 to 1-407, 1-410, 1-411, 1-421, 1-422, 1-424, 1-426, 1-427, 1-437 to 1-439, 1-442, 1-446 to 1-448, 1-450 to 1-455, 1-458, 1-459, 1-469, 1-470, 1-472, 1-474, 1-475, 1-485, 1-486, 1-490, 1-494 to 1-496, 1-498 to 1-503, 1-506, 1-507, 1-517, 1-518, 1-520, 1-522, 1-523, 1-533, 1-534, 1-538, 1-542 to 1-544, 1-546 to 1-551, 1-554, 1-555, 1-565, 1-566, 1-568, 1-570, 1-571, 1-581 to 1-583, 1-586, 1-590 to 1-592, 1-594 to 1-599, 1-602, 1-603, 1-613, 1-614, 1-616, 1-618, 1-619, 1-629, 1-630, 1-634, 1-638 to 1-640, 1-642 to 1-647, 1-650, 1-651, 1-661, 1-662, 1-664, 1-666, 1-667, 1-677, 1-678, 1-682, 1-686 to 1-688, 1-690 to 1-695, 1-698, 1-699, 1-709, 1-710, 1-712, 1-714, 1-715, 1-725, 1-726, 1-730, 1-734 to 1-736, 1-738 to 1-743, 1-746, 1-747, 1-757, 1-758, 1-760, 1-762, 1-763, 1-946 to 1-987, 1-989 to 1-998, 1-1005 to 1-1008, 1-1010, 1-1014 to 1-1023, 1-1051 to 1-1056, 1-1058 to 1-1067, 1-1070 to 1-1075,
2-93, 2-94, 2-98, 2-102 to 2-104, 2-106 to 2-111, 2-114, 2-115, 2-125, 2-126, 2-128, 2-137, 2-138, 2-142, 2-146, 2-147, 2-150 to 2-155, 2-158, 2-159, 2-169, 2-170, 2-172, 2-181, 2-182, 2-186, 2-190 to 2-192, 2-194 to 2-199, 2-202, 2-203, 2-213, 2-214, 2-216, 2-265 to 2-276,
3-1 to 3-4, 3-6, 3-17, 3-18, 3-20, 3-23, 3-24,
4-5, 4-10, 4-14 to 4-16, 4-20 to 4-23, 4-26, 4-27,
5-1, 5-3, and 5-5 to 5-8.

Examples of still preferred compounds include the compounds of Compound Nos. 1-5, 1-10, 1-14, 1-15, 1-20 to 1-23, 1-26, 1-37, 1-40, 1-42, 1-149 to 1-151, 1-154, 1-158, 1-159, 1-164 to 1-167, 1-170, 1-181, 1-184, 1-186, 1-197, 1-202, 1-206, 1-207, 1-212 to 1-215, 1-218, 1-229, 1-232, 1-234, 1-245, 1-250, 1-254, 1-255, 1-260 to 1-263, 1-266, 1-277, 1-280, 1-282, 1-293 to 1-295, 1-298, 1-301 to 1-304, 1-308 to 1-311, 1-314, 1-324, 1-325, 1-328, 1-330, 1-341, 1-346, 1-350, 1-351, 1-356 to 1-359, 1-362, 1-373, 1-376, 1-378, 1-389, 1-394, 1-398, 1-399, 1-404 to 1-407, 1-410, 1-421, 1-424, 1-426, 1-437 to 1-439, 1-442, 1-446, 1-447, 1-452 to 1-455, 1-458, 1-469, 1-472, 1-474, 1-485, 1-490, 1-494, 1-495, 1-500 to 1-503, 1-506, 1-517, 1-520, 1-522, 1-533, 1-538, 1-542, 1-543, 1-548 to 1-551, 1-554, 1-565, 1-568, 1-570, 1-581 to 1-583, 1-586, 1-590, 1-591, 1-596 to 1-599, 1-602, 1-613, 1-616, 1-618, 1-629, 1-634, 1-638, 1-639, 1-644 to 1-647, 1-650, 1-661, 1-664, 1-666, 1-677, 1-682, 1-686, 1-687, 1-692 to 1-695, 1-698, 1-709, 1-712, 1-714, 1-725, 1-730, 1-734, 1-735, 1-740 to 1-743, 1-746, 1-757, 1-760, 1-946, 1-949, 1-952 to 1-961, 1-964, 1-967, 1-970 to 1-979, 1-982 to 1-987, 1-991 to 1-996, 1-998, 1-1005 to 1-1008, 1-1010, 1-1014 to 1-1016, 1-1051 to 1-1056, 1-1070 to 1-1075,
2-93, 2-98, 2-102, 2-103, 2-108 to 2-111, 2-114, 2-125, 2-128, 2-137, 2-142, 2-146, 2-147, 2-152 to 2-155, 2-158, 2-169, 2-172, 2-181, 2-186, 2-190, 2-191, 2-196 to 2-199, 2-202,2-213,2-216,
3-1 to 3-4, 3-6, 3-17, 3-20, 3-23,
4-14, 4-15, 4-22, 4-23, and 4-26.

Examples of particularly preferred compounds include the following compounds:

2-(3-fluorophenyl)-4-[2-methyl-1,2,3,5,6,8a-hexahydroindolizin-7-yl]-3-(pyridin-4-yl)-1*H*-pyrrole,
2-(3-fluorophenyl)-4-[2-phenyl-1,2,3,5,6,8a-hexahydroindolizin-7-yl]-3-(pyridin-4-yl)-1*H*-pyrrole,
2-(3-fluorophenyl)-4-[2-hydroxy-1,2,3,5,6,8a-hexahydromdolizin-7-yl]-3-(pyridin-4-yl]-1*H*-pyrrole,
2-(3-fluorophenyl)-4-[2-methoxy-1,2,3,5,6,8a-hexahydroindolizin-7-yl]-3-(pyridin-4-yl)-1*H*-pyrrole,

4-[2-fluoro-1,2,3,5,6,8a-hexahydroindolizin-7-yl]-2-(3-fluorophenyl)-3-(pyridin-4-yl)-1*H*-pyrrole,
2-(4-fluorophenyl)-4-[2-methyl-1,2,3,5,6,8a-hexahydroindolizin-7-yl]-3-(pyridin-4-yl)-1*H*-pyrrole,
2-(4-fluorophenyl)-4-[2-phenyl-1,2,3,5,6,8a-hexahydroindolizin-7-yl]-3-(pyridin-4-yl)-1*H*-pyrrole,
2-(4-fluorophenyl)-4-[2-hydroxy-1,2,3,5,6,8a-hexahydroindolizin-7-yl]-3-(pyridin-4-yl)-1*H*-pyrrole,
2-(4-fluorophenyl)-4-[2-methoxy-1,2,3,5,6,8a-hexahydroindolizin-7-yl]-3-(pyridin-4-yl)-1*H*-pyrrole,
4-[2,2-ethylenedioxy-1,2,3,5,6,8a-hexahydroindolizin-7-yl]-2-(4-fluorophenyl)-3-(pyridin-4-yl)-1*H*-pyrrole,
2-(4-fluorophenyl)-4-[2-oxo-1,2,3,5,6,8a-hexahydroindolizin-7-yl]-3-(pyridin-4-yl)-1*H*-pyrrole,
4-[2-fluoro-1,2,3,5,6,8a-hexahydroindolizin-7-yl]-2-(4-fluorophenyl)-3-(pyridin-4-yl)-1*H*-pyrrole,
4-[2-chloro-1,2,3,5,6,8a-hexahydroindolizin-7-yl]-2-(4-fluorophenyl)-3-(pyridin-4-yl)-1*H*-pyrrole,
4-[2,2-difluoro-1,2,3,5,6,8a-hexahydroindolizin-7-yl]-2-(4-fluorophenyl)-3-(pyridin-4-yl)-1*H*-pyrrole,
2-(3-chlorophenyl)-4-[2-methyl-1,2,3,5,6,8a-hexahydroindolizin-7-yl]-3-(pyridin-4-yl)-1*H*-pyrrole,
2-(3-chlorophenyl)-4-[2-phenyl-1,2,3,5,6,8a-hexahydroindolizin-7-yl]-3-(pyridin-4-yl)-1*H*-pyrrole,
2-(3-chlorophenyl)-4-[2-hydroxy-1,2,3,5,6,8a-hexahydroindolizin-7-yl]-3-(pyridin-4-yl)-1*H*-pyrrole,
2-(3-chlorophenyl)-4-[2-methoxy-1,2,3,5,6,8a-hexahydroindolizin-7-yl]-3-(pyridin-4-yl)-1*H*-pyrrole,
2-(3-chlorophenyl)-4-[2-fluoro-1,2,3,5,6,8a-hexahydroindolizin-7-yl]-3-(pyridin-4-yl)-1*H*-pyrrole,
4-[2-chloro-1,2,3,5,6,8a-hexahydroindolizin-7-yl]-2-(3-chlorophenyl)-3-(pyridin-4-yl)-1*H*-pyrrole,
4-[2-methyl-1,2,3,5,6,8a-hexahydroindolizin-7-yl]-3-(pyridin-4-yl)-2-(3-trifluoromethylphenyl)-1*H*-pyrrole,
4-[2-phenyl-1,2,3,5,6,8a-hexahydroindolizin-7-yl]-3-(pyridin-4-yl)-2-(3-trifluoromethylphenyl)-1*H*-pyrrole,
4-[2-hydroxy-1,2,3,5,6,8a-hexahydroindolizin-7-yl]-3-(pyridin-4-yl)-2-(3-trifluoromethylphenyl)-1*H*-pyrrole,
4-[2-methoxy-1,2,3,5,6,8a-hexahydroindolizin-7-yl]-3-(pyridin-4-yl)-2-(3-trifluoromethylphenyl)-1*H*-pyrrole,
4-[2-fluoro-1,2,3,5,6,8a-hexahydroindolizin-7-yl]-3-(pyridin-4-yl)-2-(3-trifluoromethylphenyl)-1*H*-pyrrole,
4-[2-chloro-1,2,3,5,6,8a-hexahydroindolizin-7-yl]-3-(pyridin-4-yl)-2-(3-trifluoromethylphenyl)-1*H*-pyrrole,
4-[2,8-dimethyl-1,2,3,5,6,8a-hexahydroindolizin-7-yl]-2-(4-fluorophenyl)-3-(pyridin-4-yl)-1*H*-pyrrole,
2-(4-fluorophenyl)-4-[2-hydroxy-8-methyl-1,2,3,5,6,8a-hexahydroindolizin-7-yl]-3-(pyridin-4-yl)-1*H*-pyrrole,
2-(4-fluorophenyl)-4-[2-methoxy-8-methyl-1,2,3,5,6,8a-hexahydroindolizin-7-yl]-3-(pyridin-4-yl)-1*H*-pyrrole,
4-[2-fluoro-8-methyl-1,2,3,5,6,8a-hexahydroindolizin-7-yl]-2-(4-fluorophenyl)-3-(pyridin-4-yl)-1*H*-pyrrole,
4-[2-chloro-8-methyl-1,2,3,5,6,8a-hexahydroindolizin-7-yl]-2-(4-fluorophenyl)-3-(pyridin-4-yl)-1*H*-pyrrole,
2-(4-fluorophenyl)-4-[2-methyl-3,5,6,8a-tetrahydroindolizin-7-yl]-3-(pyridin-4-yl)-1*H*-pyrrole,
2-(4-fluorophenyl)-4-[2-hydroxy-1,2,3,5,8,8a-hexahydroindolizin-7-yl]-3-(pyridin-4-yl)-1*H*-pyrrole,
2-(4-fluorophenyl)-4-[2-methoxy-1,2,3,5,8,8a-hexahydroindolizin-7-yl]-3-(pyridin-4-yl)-1*H*-pyrrole,
4-[2-chloro-1,2,3,5,8,8a-hexahydroindolizin-7-yl]-2-(4-fluorophenyl)-3-(pyridin-4-yl)-1*H*-pyrrole,
4-[2,2-difluoro-1,2,3,5,8,8a-hexahydroindolizin-7-yl]-2-(4-fluorophenyl)-3-(pyridin-4-yl)-1*H*-pyrrole,
4-[cyclopropanespiro-6'-(1',2',3',5',6',8a'-hexahydroindolizin)-7'-yl]-2-(4-fluorophenyl)-3-(pyridin-4-yl)-1*H*-pyrrole,
2-(4-fluorophenyl)-4-[2-methylidene-1,2,3,5,6,8a-hexahydroindolizin-7-yl]-3-(pyridin-4-yl)-1*H*-pyrrole,
4-[2,2-dimethyl-1,2,3,5,6,8a-hexahydroindolizin-7-yl]-2-(4-fluorophenyl)-3-(pyridin-4-yl)-1*H*-pyrrole,
2-(4-fluorophenyl)-4-[2-methylthio-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-2-(4-fluorophenyl)-3-(pyridin-4-yl)-1*H*-pyrrole,
4-[2-ethyl-1,2,3,5,6,8a-hexahydroindolizin-7-yl]-2-(3-fluorophenyl)-3-(pyridin-4-yl)-1*H*-pyrrole,
2-(3-chlorophenyl)-4-[2-ethyl-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-3-(pyridin-4-yl)-2-(3-trifluoromethylphenyl)-1*H*-pyrrole,
2-(4-chlorophenyl)-4-[2-ethyl-1,2,3,5,6,8a-hexahydroindolizin-7-yl]-3-(pyridin-4-yl)-1*H*-pyrrole,
4-[2-butylthio-1,2,3,5,6,8a-hexahydroindolizin-7-yl]-2-(4-fluorophenyl)-3-(pyridin-4-yl)-1*H*-pyrrole,
4-[2-ethylthio-1,2,3,5,6,8a-hexahydroindolizin-7-yl]-2-(4-fluorophenyl)-3-(pyridin-4-yl)-1*H*-pyrrole,
4-[2-ethylidene-1,2,3,5,6,8a-hexahydroindolizin-7-yl]-2-(4-fluorophenyl)-3-(pyridin-4-yl)-1*H*-pyrrole,
2-(4-fluorophenyl)-4-[2-propyl-1,2,3,5,6,8a-hexahydroindolizin-7-yl]-3-(pyridin-4-yl)-1*H*-pyrrole,
2-(3-fluorophenyl)-4-[2-propyl-1,2,3,5,6,8a-hexahydroindolizin-7-yl]-3-(pyridin-4-yl)-1*H*-pyrrole,
2-(3-chlorophenyl)-4-[2-propyl-1,2,3,5,6,8a-hexahydroindolizin-7-yl]-3-(pyridin-4-yl)-1*H*-pyrrole,
4-[2-propyl-1,2,3,5,6,8a-hexahydroindolizin-7-yl]-3-(pyridin-4-yl)-2-(3-trifluoromethylphenyl)-1*H*-pyrrole,
2-(4-chlorophenyl)-4-[2-propyl-1,2,3,5,6,8a-hexahydroindolizin-7-yl]-3-(pyridin-4-yl)-1*H*-pyrrole,
4-[2-ethoxy-1,2,3,5,6,8a-hexahydroindolizin-7-yl]-2-(4-fluorophenyl)-3-(pyridin-4-yl)-1*H*-pyrrole,
4-[cyclopentanespiro-2'-(1',2',3',5',6',8a'-hexahydroindolizin)-7'-yl]-2-(4-fluorophenyl)-3-(pyridin-4-yl)-1*H*-pyrrole,
4-[2-benzylidene-1,2,3,5,6,8a-hexahydroindolizin-7-yl]-2-(4-fluorophenyl)-3-(pyridin-4-yl)-1*H*-pyrrole,
2-(4-fluorophenyl)-4-[2-propylidene-1,2,3,5,6,8a-hexahydroindolizin-7-yl]-3-(pyridin-4-yl)-1*H*-pyrrole,
4-[5,5-dimethyl-1,2,3,5,6,8a-hexahydroindolizin-7-yl]-2-(4-fluorophenyl)-3-(pyridin-4-yl)-1*H*-pyrrole,
4-[2-ethyl-3,5,6,8a-tetrahydroindolizin-7-yl]-2-(4-fluorophenyl)-3-(pyridin-4-yl)-1*H*-pyrrole,
2-(4-fluorophenyl)-4-[2-propyl-3,5,6,8a-tetrahydroindolizin-7-yl]-3-(pyridin-4-yl)-1*H*-pyrrole,
2-(4-fluorophenyl)-4-[2-phenyl-3,5,6,8a-tetrahydroindolizin-7-yl]-3-(pyridin-4-yl)-1*H*-pyrrole,
2-(4-chlorophenyl)-4-[2-methoxy-1,2,3,5,6,8a-hexahydroindolizin-7-yl]-3-(pyridin-4-yl)-1*H*-pyrrole,

2-(4-chlorophenyl)-4-[2-methyl-1,2,3,5,6,8a-hexahydroindolizin -7-yl]-3-(pyridin-4-yl)-1*H*-pyrrole, and
2-(4-chlorophenyl)-4-[2-phenyl-1,2,3,5,6,8a-hexahydroindolizin -7-yl]-3-(pyridin-4-yl)-1*H*-pyrrole.

Examples of especially preferred compounds include the following compounds:

2-(3-fluorophenyl)-4-[2-methyl-1,2,3,5,6,8a-hexahydroindolizin-7-yl]-3-(pyridin-4-yl)-1*H*-pyrrole,
2-(3-fluorophenyl)-4-[2-phenyl-1,2,3,5,6,8a-hexahydroindolizin-7-yl]-3-(pyridin-4-yl)-1*H*-pyrrole,
2-(3-fluorophenyl)-4-[2-methoxy-1,2,3,5,6,8a-hexahydroindolizin-7-yl]-3-(pyridin-4-yl)-1*H*-pyrrole,
2-(4-fluorophenyl)-4-[2-methyl-1,2,3,5,6,8a-hexahydroindolizin-7-yl]-3-(pyridin-4-yl)-1*H*-pyrrole,
2-(4-fluorophenyl)-4-[2-phenyl-1,2,3,5,6,8a-hexahydroindolizin-7-yl]-3-(pyridin-4-yl)-1*H*-pyrrole,
2-(4-fluorophenyl)-4-[2-methoxy-1,2,3,5,6,8a-hexahydroindolizin-7-yl]-3-(pyridin-4-yl)-1*H*-pyrrole,
2-(3-chlorophenyl)-4-[2-methyl-1,2,3,5,6,8a-hexahydroindolizin-7-yl]-3-(pyridin-4-yl)-1*H*-pyrrole,
2-(3-chlorophenyl)-4-[2-phenyl-1,2,3,5,6,8a-hexahydroindolizin-7-yl]-3-(pyridin-4-yl)-1*H*-pyrrole,
2-(3-chlorophenyl)-4-[2-methoxy-1,2,3,5,6,8a-hexahydroindolizin-7-yl]-3-(pyridin-4-yl)-1*H*-pyrrole,
4-[2-methyl-1,2,3,5,6,8a-hexahydroindolizin-7-yl]-3-(pyridin-4-yl)-2-(3-trifluoromethylphenyl)-1*H*-pyrrole,
4-[2-phenyl-1,2,3,5,6,8a-hexahydroindolizin-7-yl]-3-(pyridin-4-yl)-2-(3-trifluoromethylphenyl)-1*H*-pyrrole,
4-[2-methoxy-1,2,3,5,6,8a-hexahydroindolizin-7-yl]-3-(pyridin-4-yl)-2-(3-trifluoromethylphenyl)-1*H*-pyrrole,
2-(4-fluorophenyl)-4-[2-methyl-3,5,6,8a-tetrahydroindolizin-7-yl]-3-(pyridin-4-yl)-1H-pyrrole,
2-(4-fluorophenyl)-4-[2-methylidene-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-2-(4-fluorophenyl)-3-(pyridin-4-yl)-1*H*-pyrrole,
4-[2-ethyl-1,2,3,5,6,8a-hexahydroindolizin-7-yl]-2-(3-fluorophenyl)-3-(pyridin-4-yl)-1*H*-pyrrole,
2-(3-chlorophenyl)-4-[2-ethyl-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-3-(pyridin-4-yl)-2-(3-trifluoromethylphenyl)-1*H*-pyrrole,
2-(4-fluorophenyl)-4-[2-propyl-1,2,3,5,6,8a-hexahydroindolizin-7-yl]-3-(pyridin-4-yl)-1*H*-pyrrole,
2-(3-fluorophenyl)-4-[2-propyl-1,2,3,5,6,8a-hexahydroindolizin-7-yl]-3-(pyridin-4-yl)-1*H*-pyrrole,
2-(3-chlorophenyl)-4-[2-propyl-1,2,3,5,6,8a-hexahydroindolizin-7-yl]-3-(pyridin-4-yl)-1*H*-pyrrole,
4-[2-propyl-1,2,3,5,6,8a-hexahydroindolizin-7-yl]-3-(pyridin-4-yl)-2-(3-trifluoromethylphenyl)-1*H*-pyrrole,
4-[2-ethyl-3,5,6,8a-tetrahydroindolizin-7-yl]-2-(4-fluorophenyl)-3-(pyridin-4-yl)-1*H*-pyrrole,
2-(4-fluorophenyl)-4-[2-propyl-3,5,6,8a-tetrahydroindolizin-7-yl]-3-(pyridin-4-yl)-1*H*-pyrrole, and
2-(4-fluorophenyl)-4-[2-phenyl-3,5,6,8a-tetrahydroindolizin-7-yl]-3-(pyridin-4-yl)-1*H*-pyrrole.

Examples of the most preferred compounds include the following compounds:

2-(4-fluorophenyl)-4-[(2R,8aS)-2-phenyl-1,2,3,5,6,8a-hexahydroindolizin-7-yl]-3-(pyridin-4-yl)-1*H*-pyrrole,

2-(4-fluorophenyl)-4-[(8aS)-2-methyl-1,2,3,5,6,8a-hexahydroindolizin-7-yl]-3-(pyridin-4-yl)-1*H*-pyrrole,

2-(4-fluorophenyl)-4-[(8aS)-2-methylidene-1,2,3,5,6,8a-hexahydroindolizin-7-yl]-3-(pyridin-4-yl)-1*H*-pyrrole,

2-(4-fluorophenyl)-4-[(8aS)-2-methyl-3,5,6,8a-tetrahydroindolizin-7-yl]-3-(pyridin-4-yl)-1*H*-pyrrole,

4-[(2S,8aS)-2-ethyl-1,2,3,5,6,8a-hexahydroindolizin-7-yl)-2-(4-fluorophenyl)-3-(pyridin-4-yl)-1*H*-pyrrole, and

2-(4-fluorophenyl)-4-[(2S,8aS)-2-propyl-1,2,3,5,6,8a-hexahydroindolizin-7-yl]-3-(pyridin-4-yl)-1*H*-pyrrole.

Compounds of formula (I-1) of the present invention can be prepared by methods mentioned below.

**Method A**

Method A is a method for preparing a compound of general formula (I-1)

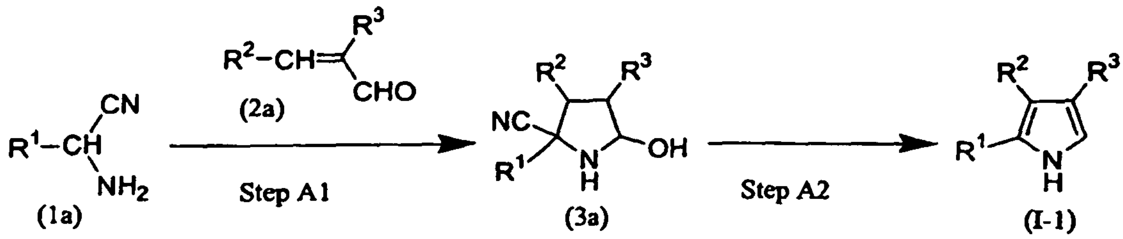


In the above formulae, $R^1$, $R^2$ and $R^3$ are as defined above.

## Step A1

In this Step, a pyrrolidine compound of formula (3a) is prepared by reacting an aminonitrile compound of formula (1a) with an α,β-unsaturated aldehyde compound of formula (2a). This reaction is well known in the field of synthetic organic chemistry and can be carried out using well known techniques, for example according to the method described in EP 0799823, the contents of which are incorporated herein by reference thereto.

In more detail, this Step is carried out in the presence of a base. There is no particular restriction on the nature of the bases used, and any base commonly used in reactions of this type may equally be used here. Examples of such bases include: alkali metal hydroxides, such as lithium hydroxide, sodium hydroxide and potassium hydroxide; alkali metal hydrides, such as lithium hydride, sodium hydride and potassium hydride; alkali metal amides, such as lithium amide, sodium amide, potassium amide and lithium bis(trimethylsilyl)amide; and alkali metal alkoxides, such as lithium ethoxide, sodium methoxide, sodium ethoxide and potassium t-butoxide. Of these, we prefer the lithium amides.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons, such as hexane and heptane; aromatic hydrocarbons, such as benzene, toluene and xylene; ethers, such as diethyl ether, diisopropyl ether, tetrahydrofuran and dioxane; and alcohols, such as methanol, ethanol, propanol, isopropanol and butanol. Of these, we prefer the ethers.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -78°C to 100°C, more preferably from -78°C to room temperature. The time required for the reaction may also vary widely, depending on many factors, notably the reaction temperature and the nature of the reagents and solvent employed. However, provided that the reaction is effected under the preferred conditions outlined above, a period of from 10 minutes to 30 hours, more preferably from 1 hour to 20 hours, will usually suffice.

## Step A2

In this Step, the desired pyrrole derivative of formula (I-1) of the present invention is prepared by the elimination of hydrogen cyanide and water from the compound of formula (3a) prepared in Step A1 above. These reactions are well known in the field of synthetic organic chemistry and can be carried out using well known techniques, for example according to the methods described in detail in EP 0799823.

In more detail, this may be achieved by heating the residue obtained by distilling off the solvent from the product of Step A1, or by heating the material obtained by extracting that residue, washing it with water and distilling off the solvent, preferably at a temperature not lower than 100°C, in the presence or absence of a solvent after completion of the reaction of Step A1. The reaction proceeds sufficiently in the absence of a solvent, but, when a solvent is used, the solvent is preferably inert and has a higher boiling point. Examples of suitable solvents include: toluene, xylene, dimethylformamide, dimethylacetamide, dimethyl sulfoxide, diglyme and diphenyl ether.

## Method F

In this method, compounds of formula (I-1) of the present invention [(I-1a), (I-1b) and (I-1c)] are prepared as shown in Reaction Scheme F.

## Reaction Scheme F

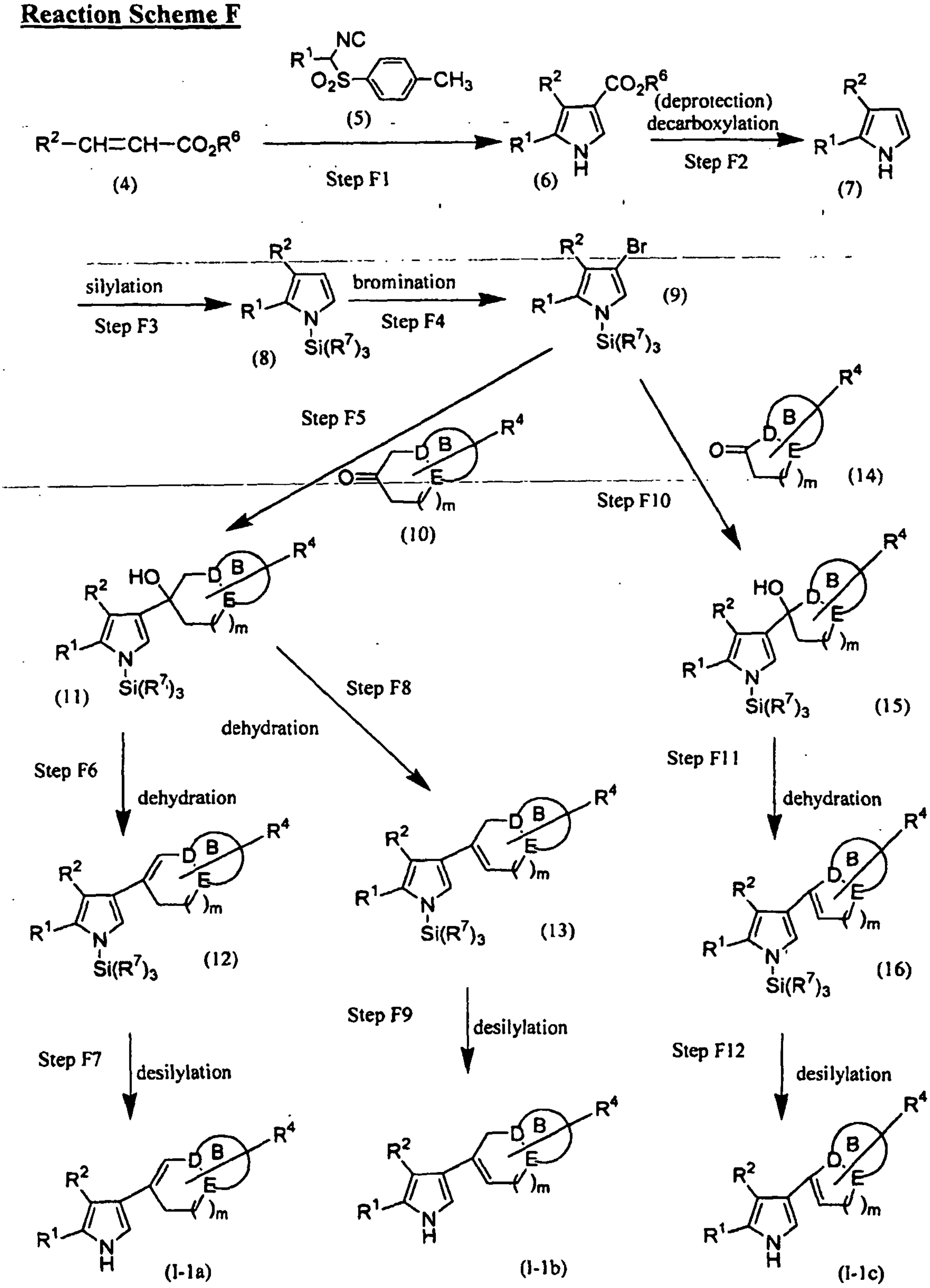


In the above formulae, B, D, E, $R^1$, $R^2$, $R^4$ and m have the same meanings as defined above, $R^6$ represents a hydrogen atom, a lower alkyl group as defined above or an aralkyl group as defined above, and each $R^7$ is the same or different, and each represents a hydrogen atom, a lower alkyl group as defined above, an aryl group as defined above or an aralkyl group as defined above.

**Step F1**

In this Step, a pyrrole carboxylic acid derivative of formula (6) is prepared by reacting an α,β-unsaturated compound of formula (4) with an isonitrile compound of formula (5). Reactions of this type are well known in the field of synthetic organic chemistry and can be carried out using well known techniques, for example according to the methods described in detail in R. Di Santo et al., Synthetic Communications, 25(6), pp. 795-802 (1995), the contents of which are incorporated herein by reference thereto.

**Step F2**

In this Step, a disubstituted pyrrole compound (7) is produced by first, where $R^6$ represents a lower alkyl group or an aralkyl group from the pyrrole-carboxylic acid ester (6), removing said protecting group $R^6$ to give the pyrrole-carboxylic acid compound (6) (wherein $R^6$ represents a hydrogen atom) and then performing a decarboxylation reaction on said compound. Decarboxylation reactions of this type are well known in the field of synthetic organic chemistry and can be carried out using well known techniques, for example according to the methods described in detail in N. Yoshida et al.: Yakugaku Zasshi, 93(5), 584-598 (1973) (the contents of which are incorporated herein by reference thereto) which employ heating under acidic, basic or neutral conditions; for example, it can be carried out using a solvent and an acid or base under conditions described below. When $R^6$ is a lower alkyl group or an aralkyl group, the deprotection reaction can be carried out using well known techniques according to the methods described in detail in T.W.Greene et al.: Protective Groups in Organic Synthesis, John Willey & Sons, Inc.

The decarboxylation reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include water and mixtures of water and an organic solvent, examples of which include aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; and esters such as methyl acetate and ethyl acetate, of which water, alcohols or a mixture thereof is preferred.

The acid to be used in the decarboxylation reaction is not particularly limited provided that it is one that is conventionally used as an acid in hydrolysis reactions, and examples thereof include mineral acids such as hydrochloric acid, sulfuric acid and phosphoric acid; carboxylic acids such as formic acid, acetic acid, propionic acid and trifluoroacetic acid; and sulfonic acids such as methanesulfonic acid and ethanesulfonic acid, of which hydrochloric acid, sulfuric acid or acetic acid is preferred.

The base to be used in the decarboxylation reaction is not particularly limited provided that it is one that is conventionally used as a base in hydrolysis reactions, and examples thereof include alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which sodium hydroxide or potassium hydroxide is preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient

**Steps F3 to F5**

In Step F3, a silylated compound (8) is produced by silylating a nitrogen atom at the 1-position of the disubstituted pyrrole compound (7) obtained according to the method of Step F2.

In Step F4, the silylated compound (8) obtained according to the method of Step F3 is converted into a brominated pyrrole compound (9) using a brominating agent (e.g. N-bromosuccinimide); and, in Step F5, the isolated compound (9) thus obtained is first lithiated and then reacted with a heterocyclyl ketone (10) to afford a hydroxyheterocyclyl compound (11).

The reactions of Steps F3, F4 and F5 can be carried out according to conventional techniques known in the

field of synthetic organic chemistry, such as the methods described in detail in Brian L. Bray et al., J. Org. Chem., 55, 6317-6318 (1990), the contents of which are incorporated herein by reference thereto.

**Step F6**

In this Step, an unsaturated heterocyclyl compound (12) is prepared by subjecting the hydroxyheterocyclyl compound (11) obtained in Step F5 to a dehydration reaction. The dehydration reaction is well known in the field of synthetic organic chemistry and can be carried out using well known techniques. For example, it can be carried out in the presence of an acid catalyst such as sulfuric acid, a solid catalyst such as alumina or a halogenation agent such as thionyl chloride [these reactions are described in detail, for example, in G.H.Coleman & H.F.Johnstone, Org. Synth., I, 183 (1941); R.L.Sawyer & D.W.Andrus, Org. Synth., III, 276 (1955); and J.S.Lamos et al., Tetrahedron Lett., 599 (1971), the contents of which are incorporated herein by reference thereto]. Alternatively, the dehydration reaction in this step can be performed by reaction of the hydroxyheterocyclyl compound (11) with a trialkylsilane, such as triethylsilane, tripmpylsilane or tributylsilane, and trifluoroacetic acid [see, for example, Francis A. Carey & Henry S. Tremper, J. Am. Chem. Soc., 91, 2967-2972 (1969), the contents of which are incorporated herein by reference thereto].

**Step F7**

In this Step, the desired compound of formula (I-1a) of the present invention is prepared by removing the protecting group (the silyl group) from the pyrrole nitrogen of the unsaturated heterocyclyl compound (12) prepared in Step F6. Desilylation reactions of this type are well known in the field of synthetic organic chemitry and can be carried out using well known techniques, for example using a desilylating reagent such as tetrabutylammonium fluoride (TBAF) according to the procedure described in detail in Brian L. Bray et al., J. Org. Chem., 55, 6317-6318 (1990), the contents of which are incorporated herein by reference thereto.

**Step F8**

In this Step, an unsaturated heterocyclyl compound (13) is prepared by subjecting the hydroxyheterocyclyl compound (11) obtained in Step F5 to a dehydration reaction. This dehydration reaction is carried out in a manner similar to that described for Step F6 above.

**Step F9**

In this Step, the desired compound of formula (I-1b) of the present invention is prepared by removing the protecting group (the silyl group) from the pyrrole nitrogen of the unsaturated heterocyclyl compound (13) prepared in Step F8. This Step can be performed in a manner similar to that described for Step F7 above.

**Step F10**

In this Step, a hydroxyhetemcyclyl compound (15) is prepared by first lithiating the compound (9) prepared in Step F4 and then reacting it with a heterocyclyl ketone (14). This Step can be carried out in a manner similar to that described for Step F5 above.

**Step F11**

In this Step, an unsaturated heterocyclyl compound (16) is prepared by subjecting the hydroxyheterocyclyl compound (15) obtained in Step F10 to a dehydration reaction. This dehydration reaction is carried out in a manner similar to that described for Step F6 above.

**Step F12**

In this Step, the desired compound of formula (I-1c) of the present invention is prepared by removing the protecting group (the silyl group) from the pyrrole nitrogen of the unsaturated heterocyclyl compound (16) prepared in Step F11. This Step can be performed in a manner similar to that described for Step F7 above.

**Method G**

Generally, the compounds of formula (I-1) of the present invention can be prepared by introducing the $R^3$ group

into a pyrrole compound already substituted on the pyrrole ring with the $R^1$ group and $R^2$ group. Compounds of formula (I-1) can be prepared, for example, according to the Method G, as shown in Reaction Scheme G below.

**Reaction Scheme G**

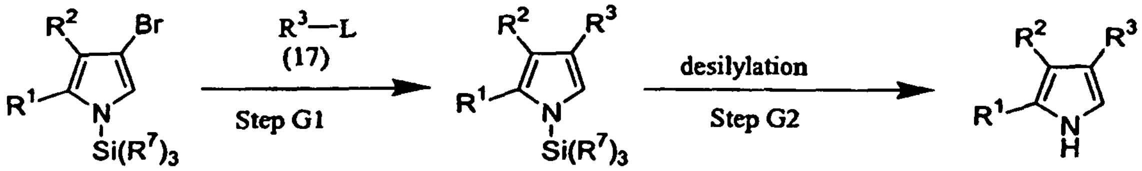


(9) (18) (I-1)

In the above formulae, $R^1$, $R^2$, $R^3$ and $R^7$ are as defined above, and L represents a leaving group.

The leaving group L is a group which is capable of leaving as a nucleophilic residue. Examples include halogen atoms such as fluorine, chlorine, bromine and iodine, trihalogenomethyloxy groups such as trichloromethoxy, lower alkanesulfonyloxy groups such as methanesulfonyloxy and ethanesulfonyloxy groups, lower halogeno alkane sulfonyloxy groups such as trifluoromethanesulfonyloxy and pentafluoroethanesulfonyloxy groups, and arylsulfonyloxy groups such as benzenesulfonyloxy, p-toluenesulfonyloxy and p-nitrobenzenesulfonyloxy groups. Of these, halogen atoms are preferred, and bromine atoms are particularly preferred.

<u>Step G1</u>

In this Step, a compound of formula (18) is prepared by first lithiating a bromopyrrole compound of formula (9) (prepared as described in Step F4 above) and then reacting the lithiated intermediate with a compound of formula (17) in a manner similar to Step F5 above. Substitution reactions of a lithiated pyrrole intermediate of this type are well known in the field of synthetic organic chemistry and can be carried out using well known techniques, for example according to the methods described in detail in WO 99/01449, the contents of which are incorporated herein by reference thereto.

**Step G2**

In this Step, a compound of formula (I-1) of the present invention is prepared by removing the protecting group (silyl group) of the compound of formula (18) obtained in Step G1 above according to a procedure similar that described in Step F7 above.

**Method H**

In this method, compounds of formula (I-1a) of the present invention wherein $R^2$ is a heteroaryl group which has at least one ring nitrogen atom and which is substituted with a group of formula $NR^aR^b$ can be prepared, as shown in Reaction Scheme H below.

**Reaction Scheme H**

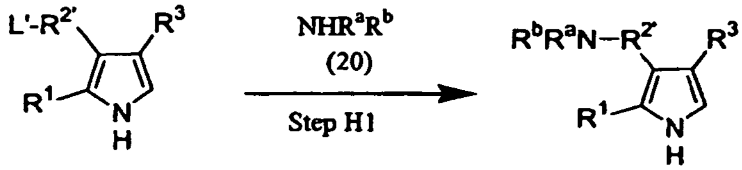


(19) (I-1a)

In the above formulae, $R^1$, $R^3$, $R^a$ and $R^b$ are defined above, $R^{2'}$ is a heteroaryl group which has at least one ring nitrogen atom and L' represents a leaving group, such that the group -$R^{2'}$-L' represents a heteroaryl group which has at least one ring nitrogen atom (as defined and exemplified above for the substituent $R^2$) which is substituted with a leaving group (e.g. 2-methanesulfonyl-pyrimidin-4-yl, 2-methanesulfonylpyridin-4-yl or the like).

The leaving group L' is a similar group to the leaving groups defined and exemplified above in the definition of L or it is a lower alkylsulfonyl group as defined and exemplified above, such as a methanesulfonyl, ethanesulfonyl, propanesulfonyl or butanesulfonyl group, or an arylsulfonyl group, such as a benzenesulfonyl, p-toluenesulfonyl or p-nitrobenzenesulfonyl group. The group L' is preferably a lower alkylsulfonyl group, and more preferably a methanesulfonyl group.

<u>**Step H1**</u>

In this Step, the desired compound of formula (I-1a) of the present invention is prepared by reacting a compound of formula (19) with an amine compound of formula (20) to replace the leaving group with a group of formula -$NR^aR^b$. This reaction is usually carried out in a solvent in the presence or absence of a base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols such as methanol, ethanol, propanol and isopropanol; ethers such as diethyl ether, diisopropyl ether, tetrahydrofuran and dioxane; aprotic polar solvents such as dimethylformamide, dimethylacetamide and dimethylsulfoxide; nitriles such as acetonitrile; esters such as methyl acetate and ethyl acetate; aromatic hydrocarbons such as benzene, toluene and xylene; and aliphatic hydrocarbons such as pentane, hexane and heptane, of which alcohols are preferred and methanol and ethanol are more preferred.

The base to be used in this step is not particularly limited, as long as it is effective in such reactions, and examples include: alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which amines are preferred, and triethylamine, pyridine and 1,8-diazabicyclo[5.4.0]undec-7-ene are more preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

Nearly all of the starting materials used in Methods A to H above, namely compounds (1a), (1b), (1c), (1d), (1e), (2a), (2b), (2c), (2d), (2e), (4), (5), (10), (14), (17) and (20), are either known compounds or they are compounds which can be prepared easily from known compounds by known methods (for example, the methods described in WO 97/5877), while the compound (19) can be synthesized from known compounds by performing reactions in a manner similar to that described in each of Methods A to E above.

Alternatively, starting compounds of general formula (10) defined above can also be prepared by Methods I to M described below.

**Method I**

This is a method for the preparation of compounds of formula (26) and (27) below which are compounds of formula (10) wherein D represents a group of formula >$CR^{4a}$- wherein $R^{4a}$ is as defined below and E represents a nitrogen atom, the method being carried out as shown in Reaction Scheme I below.

## Reaction Scheme I

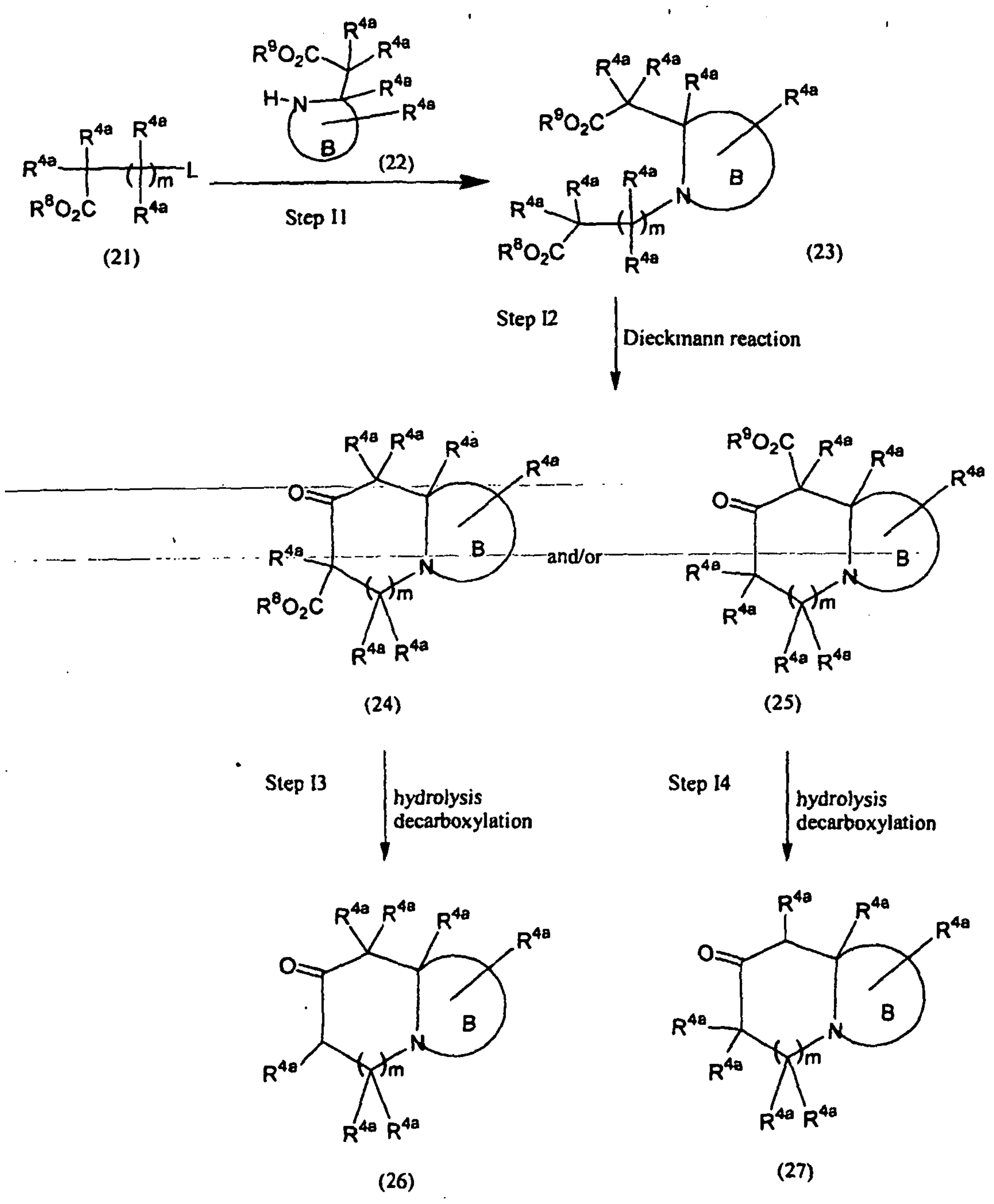


In the above formulae, B, L and m are as defined above, each of the groups $R^{4a}$ is the same or different and represents a hydrogen atom or a group of formula $R^4$ as defined above (provided that at least one of the $R^{4a}$ groups is the same group as that defined for $R^4$), and $R^8$ and $R^9$ are the same or different, and each represent a lower alkyl group as defined above or an aralkyl group as defined above.

**Step I1**

In this Step, a cyclic amine diester compound of formula (23) is prepared by the condensation of a cyclic amino acid ester compound of formula (22) with a carboxylic acid ester compound of formula (21) which has a leaving group (L).

This reaction is usually carried out in a solvent in the presence or absence of a base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols such as methanol, ethanol, propanol and isopropanol; ethers such as diethyl ether, diisopropyl ether, tetrahydrofuran and dioxane; aprotic polar solvents such as dimethylformamide, dimethylacetamide and dimethyl sulfoxide; nitriles such as acetonitrile; esters such as methyl acetate and ethyl acetate; aromatic hydrocarbons such as benzene, toluene and xylene; and aliphatic hydrocarbons such as pentane, hexane and heptane, of which alcohols, ethers, aprotic polar solvents and esters are preferred.

The base to be used in this step is not particularly limited, as long as it is effective in such reactions, and examples include: alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which sodium carbonate, potassium carbonate, triethylamine, pyridine and 1,8-diazabicyclo[5.4.0]undec-7-ene are preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

**Steps I2 to I4**

In Step I2, the cyclic amine diester compound of formula (23) prepared in Step I1 above is converted into a keto ester compound of formula (24) and/or a keto ester compound of formula (25) using a Dieckmann reaction. In Steps I3 and I4, the product of formula (24) and/or the product of formula (25) thus obtained is/are then hydrolyzed and decarboxylated successively to prepare the desired cyclic aminoketone compound of formula (26) and/or the desired cyclic aminoketone compound of formula (27).

The reactions in Steps 12 to I4 can be carried out according to the procedures described in J.R. Hatrison et al., J. Chem. Soc., Perkin Trans. 1, 1999, 3623-3631, the contents of which are incorporated herein by reference thereto. For example, Steps I3 and I4 can be carried out as follows.

The reactions of Steps 13 and 14 are usually carried out in a single step in the presence or absence of a solvent in the presence or absence of an acid or base.

The reactions are normally and preferably effected in the presence of a solvent. There is no particular restriction on the nature of the solvent to be employed, provided that it has no adverse effect on the reactions or on the reagents involved and that it can dissolve the reagents, at least to some extent. Examples of suitable solvents include: water, or a mixture of water and an organic solvent, (examples of which include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; and esters such as methyl acetate and ethyl acetate), of which water, a mixture of water and an alcohol and a mixture of water and an ether are preferred.

The acid to be used in the reactions is not particularly limited provided that it is one that is usually used as an acid in hydrolysis reactions, and examples thereof include: mineral acids such as hydrochloric acid, sulfuric acid and phosphoric acid; carboxylic acids such as formic acid, acetic acid, propionic acid or trifluoroacetic acid; and sulfonic acids such as methanesulfonic acid or ethanesulfonic acid. The two-step reaction is accelerated by the addition of an acid, of which mineral acids and carboxylic acids are preferred, and hydrochloric acid, sulfuric acid, formic acid and acetic acid are more preferred.

The base to be used in the reactions is not particularly limited provided that it is one that is usually used as a base in hydrolysis reactions, and examples thereof include: alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which alkali metal hydroxides are preferred, and sodium hydroxide and potassium hydroxide are more preferred.

The reactions can take place over a wide range of temperatures, and the precise reaction temperature is not critical to the invention. The preferred reaction temperature will depend upon such factors as the nature of the solvent,

and the starting material or reagent used. However, in general, we find it convenient to carry out the reactions at a temperature of from -20°C and 150°C, and preferably between 0°C and 100°C. The time required for the reactions may also vary widely, depending on many factors, notably the reaction temperature and the nature of the reagents and solvent employed. However, provided that the two-step reaction is effected under the preferred conditions outlined above, a period of from 10 minutes to 48 hours, and preferably from 30 minutes to 12 hours is usually sufficient.

**Method J**

This is a method for the preparation of a compound of formula (32) below which is a compound of formula (10) wherein E represents a nitrogen atom, D represents a group of formula >CH-, $R^{4a}$ is as defined above and W is as defined below, the method being carried out as shown in Reaction Scheme J below.

**Reaction Scheme J**

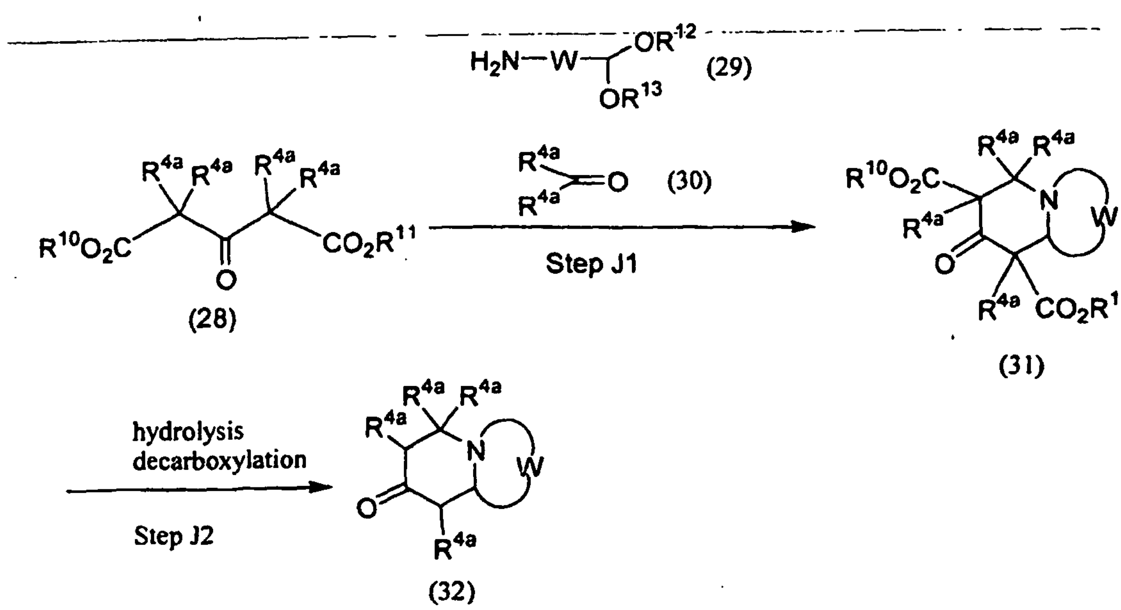

(29) (30) (28) (31) (32)

In the above formulae, $R^{4a}$ is as defined above,
$R^{10}$ and $R^{11}$ are the same or different, and each represents a lower alkyl group as defined above or an aralkyl group as defined above,
$R^{12}$ and $R^{13}$ are the same or different, and each represents a lower alkyl group as defined above or $R^{12}$ and $R^{13}$ together form a lower alkylene group as defined above,
W represents a lower alkylene group as defined above which is substituted with from one to three $R^4$ groups as defined above, said alkylene group optionally being interrupted by one or two atoms or groups selected from the group consisting of nitrogen atoms, oxygen atoms, sulfur atoms, >SO groups and >$SO_2$ groups, and
the cyclic group containing W shown in general formulae (31) and (32) is a group which corresponds to the cyclic group B as defined above which is unsubstitited or is substituted with from one to three $R^4$ groups.

Step J1 and Step J2 are both reactions which are well known in the field of organic chemistry and can be performed using any combination of such known methods; for example, they can be conducted in a manner similar to the reactions described in detail in O. Pollet et al., Heterocycles, 43, 1391 (1996) or Anet et al., Austral. J. Scient. Res., <A>3, 635-640 (1950), the contents of which are incorporated herein by reference thereto.

**Method K**

This is a method for the preparation of a compound of formula (37) below which is a compound of formula (10) wherein D represents a group of formula >$CR^{4a}$- wherein $R^{4a}$ is as defined above and E represents a nitrogen atom, the method being carried out as shown in Reaction Scheme K below.

## Reaction Scheme K

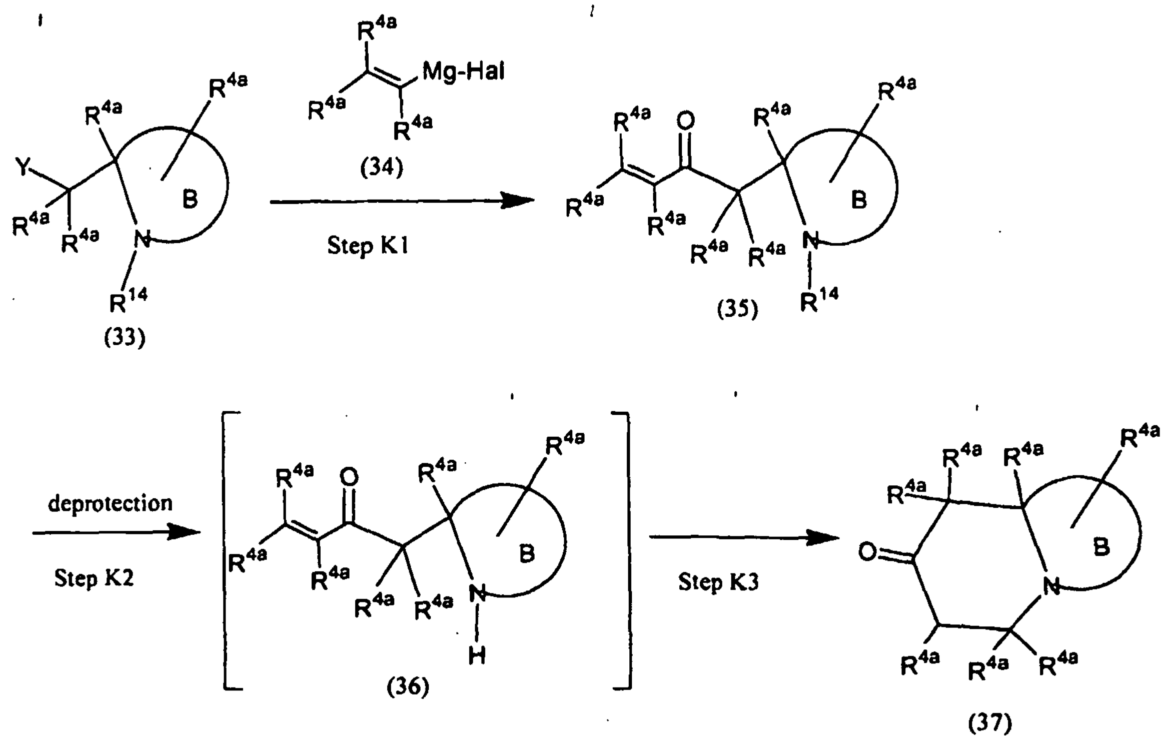


In the above formulae, B and $R^{4a}$ are as defined above,
$R^{14}$ represents an amino protecting group,
Hal represents a halogen atom (preferably, it is a chlorine atom, bromine atom or iodine atom), and
Y represents a halogenocarbonyl group (for example, -CO-Cl, -CO-Br or - CO-I), a N-(lower alkoxy)-N-(lower alkyl) carbamoyl group, wherein said lower alkoxy and lower alkyl moieties are as defined above (examples of such groups include N-methoxy-N-methylcarbamoyl, N-ethoxy-N-methylcarbamoyl and N-ethyl-N-methoxycarbamoyl groups) or a cyano group.

The amino protecting group in the definition of $R^{14}$ can be any protecting group for an amino group which is commonly used in organic synthesis, examples of which are found in T.W.Greene et al.: Protective Groups in Organic Synthesis, John Willey & Sons, Inc. Specific examples of suitable amino protecting groups include aliphatic acyl groups as defined and exemplified above, aromatic acyl groups as defined and exemplified above, silyl groups as defined and exemplified above, aralkyl groups as defined and exemplified above, alkoxycarbonyl groups as defined and exemplified above, alkenyloxycarbonyl groups as defined and exemplified above and aralkyloxycarbonyl groups as defined and exemplified above, of which alkoxycarbonyl groups are preferred, and t-butoxycarbonyl groups are more preferred.

### Step K1

In this Step, an α,β-unsaturated ketone derivative of formula (35) is prepared by reacting a cyclic amino acid derivative of formula (33) with a Grignard reagent of an olefin compound of formula (34). Reactions of this type are well known for the preparation of ketones from carboxylic acid derivatives and Grignard reagents, and any such reaction known in the field of organic synthesis can be employed; for example, it can be carried out using the procedures described in detail in H. R. Snyder et al., Org. Synth., III, 798 (1955); J. Cason et al., J. Org. Chem., 26, 1768 (1961); G. H. Posner et al., J. Am. Chem. Soc., 94, 5106 (1972); and G. H. Posner, Org. React., 19, 1 (1972), the contents of which are incorporated herein by reference thereto.

### Steps K2 and K3

In Step K2, the nitrogen protecting group ($R^{14}$) in the α,β-unsaturated ketone derivative of formula (35) prepared in Step K1 above is removed to afford a deprotected intermediate of formula (36) which is then cyclized in Step K3 to

give the desired cyclic aminoketone compound of formula (37). In Step K2, the deprotection reaction employed can be any which is conventionally used in organic synthesis (examples of which are described in T.W.Greene et al., Protective Groups in Organic Synthesis, John Willey & Sons, Inc.). Preferably, the deprotection reaction is conducted under neutral or acidic conditions. After the deprotection reaction, the resulting product of formula (36), which is not isolated, cyclizes immediately to give the desired aminoketone compound of formula (37). The deprotection reaction is more preferably conducted under acidic conditions, and the aminoketone compound of formula (37) is prepared without further reaction by neutralizing the reaction mixture.

**Method L**

This is a method for the preparation of a compound of formula (40) below which is a compound of formula (10) wherein D represents a group of formula >$CR^{4a}$- wherein $R^{4a}$ is as defined above and E represents a nitrogen atom, the method being carried out as shown in Reaction Scheme L below.

**Reaction Scheme L**

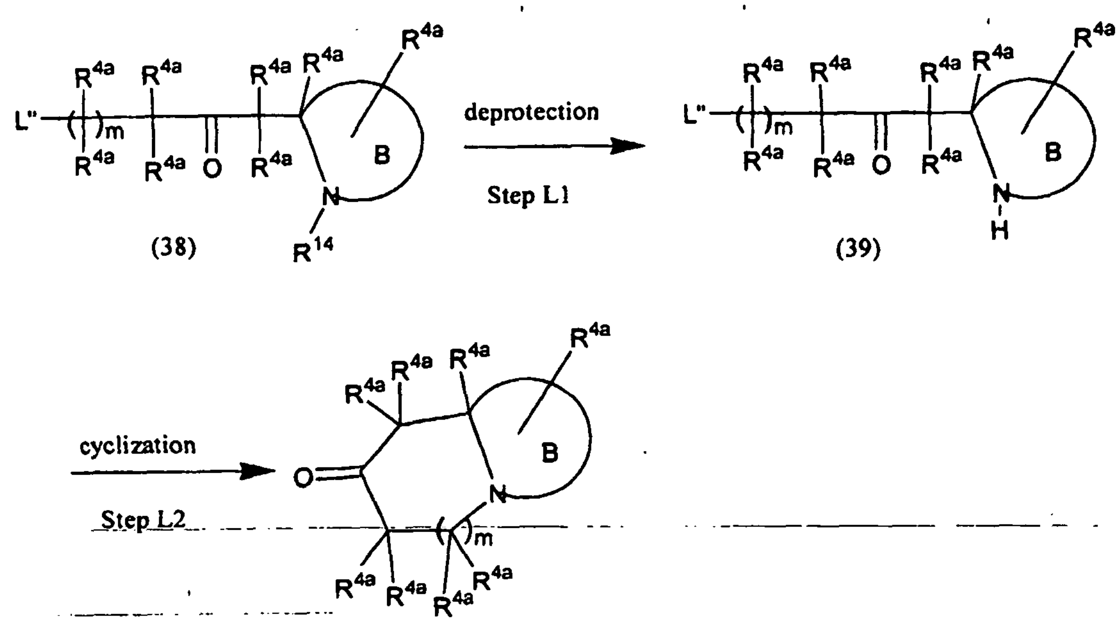


(38) (39)

(40)

In the above formulae, B, $R^{4a}$, $R^{14}$ and m are as defined above, and L" represents a leaving group as defined for the leaving group L above, a lower alkylsulfonyl group as defined above, an arylsulfonyl group as defined above or a halogeno lower alkylsulfonyl group wherein the halogeno lower alkyl moiety is as defined above (examples of said group including trifluoromethanesulfonyl and pentafluoroethanesulfonyl groups).

**Steps L1 and L2**

Steps L1 and L2 involve first removing the amino protecting group ($R^{14}$) from the ketone compound (38) having the leaving group L" to afford a deprotected intermediate of formula (39), and then cyclizing said intermediate to produce the desired aminoketone compound of formula (40). These steps can be carried out in a manner similar to the reactions described in Steps K2 and K3 above.

The starting compound of formula (38) used as the starting material in this method is either a known compound or it can be prepared from a known compound using known methods [for example, the methods described in S. W. Goldstein et al., J. Org. Chem., 57,1179-1190 (1992); and B. Achille et al., J. Comb. Chem., 2, 337-340 (2000), the contents of which are incorporated herein by reference thereto].

**Method M**

This is a method for the preparation of a compound of formula (47) below which is a compound of formula (10) wherein D represents a group of formula >$CR^{4a}$- wherein $R^{4a}$ is as defined above and E represents a nitrogen atom, the method being carried out as shown in Reaction Scheme M below.

**Reaction Scheme M**

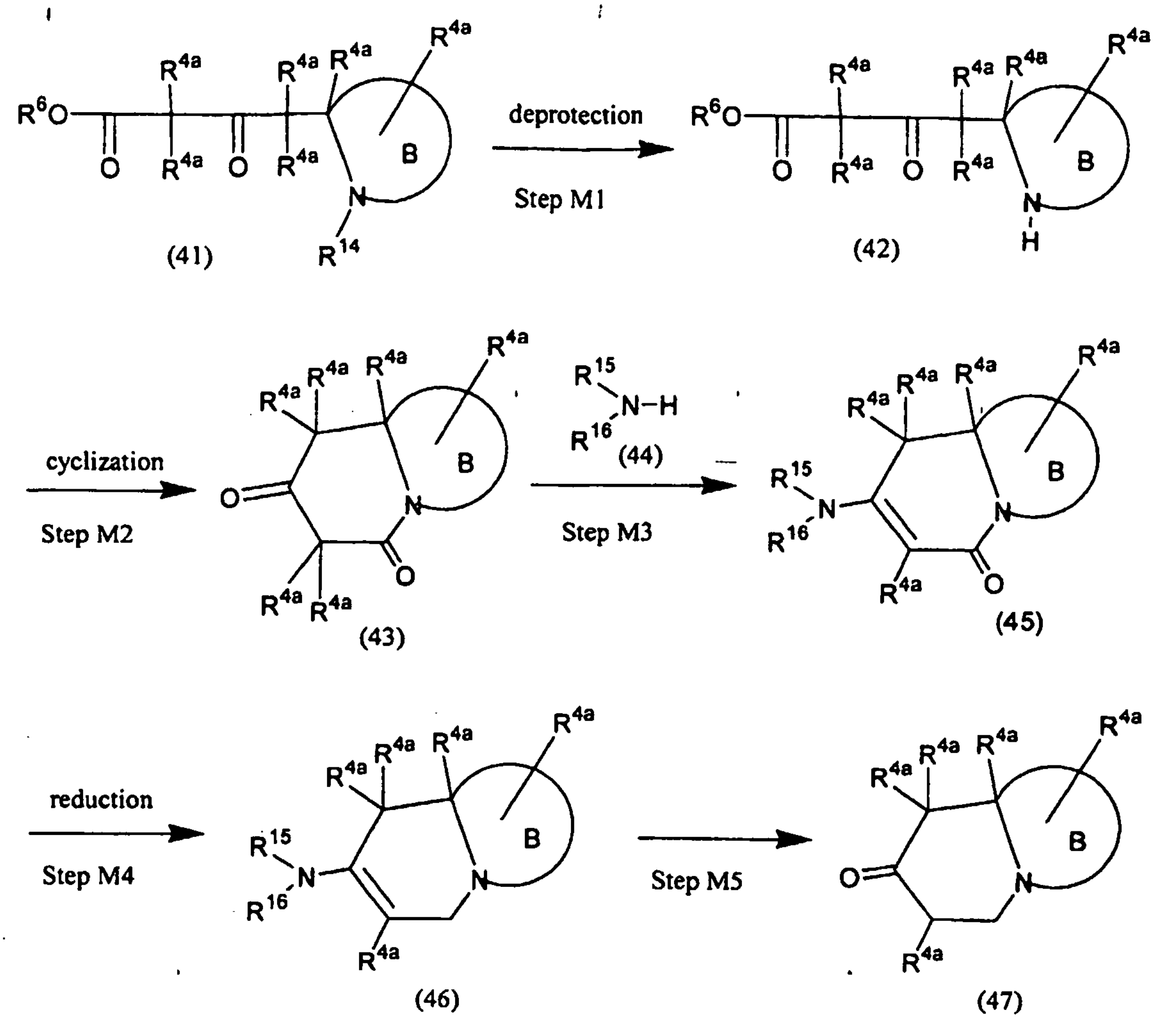


(41) (42) (43) (44) (45) (46) (47)

In the above formulae, $R^{4a}$, $R^6$, $R^{14}$ and B are as defined above, and $R^{15}$ and $R^{16}$ are the same or different, and each represents a hydrogen atom, a lower alkyl group as defined above or an aralkyl group as defined above, or $R^{15}$ and $R^{16}$, together with the nitrogen atom to which they are attached, form a 5- or 6-membered heterocyclic ring which includes one ring nitrogen atom and which may optionally include one further heteroatom selected from oxygen, sulfur and nitrogen atoms (examples of such groups include pyrrolidinyl, piperidyl, piperazinyl, morpholinyl and thiomorpholinyl groups).

**Steps M1 and M2**

In these Steps, a ketolactam compound of formula (43) is prepared by first removing the amino protecting group ($R^{14}$) from an α-ketoacid compound of formula (41) to afford a deprotected intermediate of formula (42), and then cyclizing said intermediate. These steps are conducted in a manner similar to that described in Steps K2 and K3 above.

**Step M3**

In this Step, a cyclic enaminolactam compound of formula (45) is prepared by reacting the ketolactam compound of formula (43) prepared in Step M2 above with a secondary amine compound of formula (44). Any of the techniques conventionally used in the field of organic synthetic chemistry for the preparation of enamine derivatives can be employed. For example, the step can be carried out according to the procedure described in G. Stork et al., J. Am, Chem. Soc., 85. 207 (1963) (the contents of which are incorporated herein by reference thereto) or as described below.

The reaction is usually carried out in a solvent in the presence or absence of an acid.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; and esters such as methyl acetate and ethyl acetate, of which ethers are preferred.

The acid to be used in the reaction is not particularly limited provided that it is one that is usually used in such reactions, and examples thereof include: inorganic acids such as hydrogen chloride, hydrogen bromide, sulfuric acid, perchloric acid and phosphoric acid; and organic acids such as acetic acid, formic acid, oxalic acid, methanesulfonic acid, p-toluenesulfonic acid, trifluoroacetic acid and trifluoromethanesulfonic acid. Of these, sulfuric acid, hydrochloric acid and p-toluensulfonic acid are preferred.

The reaction of this step can be carried out efficiently by removing water produced during the reaction by using molecular sieves or a water separator (for example, a Dean Stark Water Separator which can be obtained from Aldrich).

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

**Step M4**

In this Step, a cyclic enamine compound of formula (46) is produced by reducing the cyclic enaminolactam compound of formula (45) prepared in Step M3 above. Any of the techniques conventionally used in the field of organic synthetic chemistry for performing reduction reactions can be employed. For example, the reduction can be carried out according to the procedures described in S. Cortes et al., J. Org. Chem., 48, 2246 (1983); Y. Tsuda et al., Synthesis, 652 (1977); H. C. Brown et al., J. Am. Chem. Soc., 86, 3566 (1964) and R. J. Sundberg et al., J. Org. Chem., 46, 3730 (1981) (the contents of which are incoporated herein by reference thereto); or, alternatively, can be performed as described below.

This reaction is usually carried out in a solvent in the presence of a reducing reagent.

Examples of the reducing reagent to be employed include hydride reagents such as alkali metal borohydrides e.g. sodium borohydride and lithium borohydride, and aluminum hydrides e.g. lithium aluminum hydride and lithium triethoxyaluminohydride; a combination of a Lewis acid such as aluminum chloride, tin tetrachloride or titanium tetrachloride and a hydride reagent as defined above; and boron compounds such as diborane, of which lithium aluminium hydride is preferred.

In the reduction reaction, non-polar solvents can be used, preferred examples of which include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; and ethers such as diethyl ether, diisopropyl ether, tetrahydrofuran and dioxane, of which ethers are preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

**Step M5**

In this Step, the desired cyclic aminoketone compound of formula (47) is obtained by hydrolizing the cyclic enamine compound of formula (46) prepared in Step M4 above. This reaction is performed by bringing the cyclic enamine compound of formula (46) into contact with water in the presence or absence of a solvent with or without the addition of an acid or base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include water, or a mixture of water and an organic solvent (examples of which include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; and esters such as methyl acetate and ethyl acetate), of which water, a mixture of water and an alcohol and a mixture of water and an ether are preferred.

The acid to be used is not particularly limited provided that it is one that is usually used as an acid in hydrolysis reactions, and examples thereof include mineral acids such as hydrochloric acid, sulfuric acid and phosphoric acid; carboxylic acids such as formic acid, acetic acid, propionic acid and trifluoroacetic acid; and sulfonic acids such as methanesulfonic acid and ethanesulfonic acid, of which hydrochloric acid, sulfuric acid and acetic acid are preferred. The hydrolysis reaction is accelerated by the addition of an acid.

The base to be used is not particularly limited provided that it is one that is usually used as a base in hydrolysis reactions, and examples thereof include alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which sodium hydroxide and potassium hydroxide are preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

**Method N**

The compound of formula (45), which is an intermediate in the preparation of the cyclic aminoketone compound of formula (47) in Method M above, can also be produced by Method N according to Reaction Scheme N below.

**Reaction Scheme N**

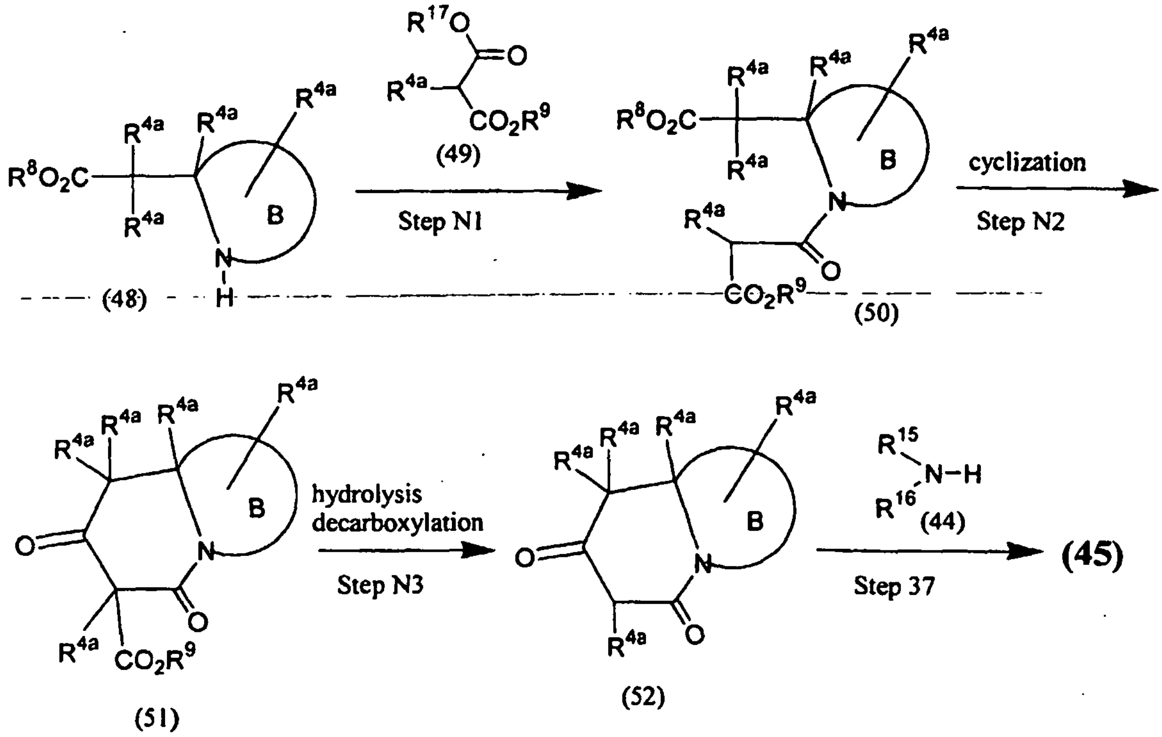


In the above formulae, B, $R^{4a}$, $R^8$, $R^9$, $R^{15}$ and $R^{16}$ are as defined above, and $R^{17}$ represents a hydrogen atom or a carboxyl protecting group.

The carboxyl protecting group in the definition of $R^{17}$ can be any such protecting group conventionally used in the organic chemistry; preferably, it is a lower alkyl group as defined above or an aralkyl group as defined above.

**Step N1**

In this Step, an aminodiester compound of formula (50) is produced by the reaction of a cyclic amino acid ester compound of formula (48) with a malonic acid derivative of formula (49) or a reactive derivative thereof. Any of the techniques conventionally used in the field of organic synthetic chemistry for amidation reactions can be employed, and this step can, for example, be carried out in the manner described in processes (a), (b) and (c) described below.

(a) When $R^{17}$ is a hydrogen atom, the reaction is conducted in a solvent in the presence of a condensing agent and in the presence or absence of a base.
There is no particular restriction on the nature of the solvent to be employed, provided that it has no adverse effect on the reaction or on the reagents involved and that it can dissolve the reagents, at least to some extent. Examples of the solvent to be employed include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; esters such as methyl acetate and ethyl acetate; water; or a mixture of these solvents described above, of which halogenated hydrocarbons, ethers and esters are preferred and dichloromethane, tetrahydrofuran and ethyl acetate are more preferred.
Any suitable condensing agent that is conventionally employed in such reactions can be employed, and examples include dicyclohexylcarbodiimide, 1-ethyl-3-(3-dimethylaminopropyl)carbodiimide, N,N'-carbonyldiimidazole and the like.
The base to be used is not particularly limited provided that it is one that is usually used as a base in such reactions, and examples thereof include alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which amines are preferred, and triethylamine, pyridine and 1,8-diazabicyclo-[5.4.0]undec-7-ene are more preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

Alternatively, where $R^{17}$ is a hydrogen atom, the reaction of Step N1 can also be carried out by converting the compound of formula (49) into a reactive derivative thereof followed by the procedure described in process (c) below.

(b) When $R^{17}$ is a carboxyl protecting group (preferably a lower alkyl group as defined above or an aralkyl group as defined above), the reaction is performed by heating in the presence or absence of a solvent.

When the reaction is conducted in a solvent, the same solvent as that described in process (a) can be used. The temperature for the reaction is between 30°C and 100°C, preferably between the range of ± 5°C of the boiling point of the solvent that is employed. Most preferably, the reaction is carried out by heating the reaction mixture under reflux.

When a solvent is not used in this reaction, the desired compound is prepared by heating a mixture of the compounds of formulae (48) and (49). The reaction can take place over a wide range of temperatures, and the precise reaction temperature is not critical to the invention. The preferred reaction temperature will depend upon such factors as the nature of the starting materials used. However, in general, we find it convenient to carry out the reaction at a temperature of from 30°C and 150°C, and preferably between 50°C and 120°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

(c) When a reactive derivative of a compound of formula (49) is used, the reactive derivative can be an acid halide, a mixed acid anhydride, an activated ester, an active amide or the like, and the reaction is conducted in a solvent in the presence of a condensing agent and in the presence or absence of a base.

The acid halide of the compound of formula (49) is prepared by the reaction of a compound of formula (49) wherein $R^{17}$ is a hydrogen atom with a halogenation reagent (for example, thionyl chloride, oxalyl chloride or the like); the mixed acid anhydride is prepared by the reaction of a compound of formula (49) wherein $R^{17}$ is a hydrogen atom with an acid halide (for example, methyl chlorocarbonate, ethyl chlorocarbonate or the like); the activated ester is prepared by the reaction of a compound of formula (49) wherein $R^{17}$ is a hydrogen atom with a compound containing a hydroxyl group (for example, N-hydroxysuccinimide, N-hydroxyphthalimide or the like) in the presence of a condensing agent such as those described in process (a) above; and the active amide (for example, a Weinreb amide) is prepared by the reaction of a compound of formula (49) wherein $R^{17}$ is a hydrogen atom with an N-(lower alkoxy)-N-(lower alkyl)hydroxylamine (for example, N-methoxy-N-methylhydroxylamine or the like) in the presence of a condensing agent such as those described in process (a) above. Each of these reactions described can be conducted under reaction conditions usually used in organic synthetic chemistry for such reactions.

With regard to the solvent, condensing agent and base, the solvents, condensing agents and bases described in process (a) above can be used.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

**Steps N2 and N3**

In Steps N2 and N3, a ketolactam compound of formula (52) is prepared by first executing a Dieckman reaction on the amido diester compound of formula (50) produced in Step N1 above to afford a ketolactam ester compound (51), followed by the performance of hydrolysis and decarboxylation reactions on the product thus obtained. These steps can be conducted in a manner similar to that described in Steps I2 and I3 above.

**Step N4**

In this step, the target cyclic enaminolactam compound of formula (45) is prepared by the reaction of the ketolactam compound of formula (52) obtained in Step N3 above with a secondary amine compound of formula (44), and the reaction is carried out in a manner similar to that described in Step M3 above.

**Method O**

The compound of formula (51), which is an intermediate in Method N described above, can also be synthesized by Method O according to Reaction Scheme O below.

**Reaction Scheme O**

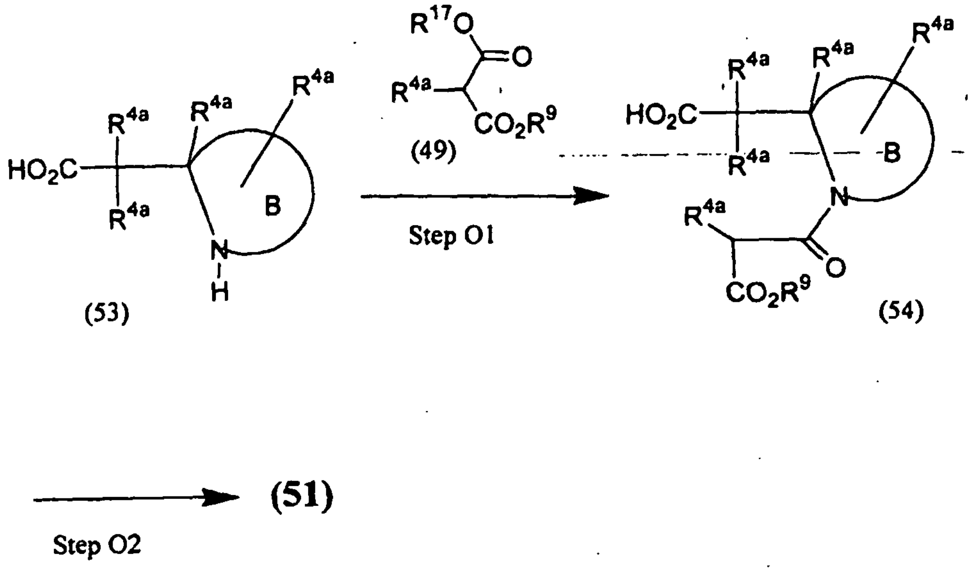


(53) (49) (54) (51)

In the above formulae, B, $R^{4a}$, $R^9$ and $R^{17}$ are as defined above.

**Step O1**

In this Step, an amido monoester compound of formula (54) is prepared by the reaction of a cyclic amino acid compound of formula (53) with a malonic acid derivative of formula (49) or a reactive derivative thereof. This step is carried out in a manner similar to that described in processes (a), (b) and (c) of Step N1 above.

**Step O2**

In this Step, the target ketolactam ester compound of formula (51) is prepared by the intramolecular condensation of a carboxyl group and an active methylene group of the amido monoester compound of formula (54) prepared in Step O1 above. In this step, the compound of formula (54) is either used in underivatised form or after first being converted into a reactive derivative thereof.

(a) When the compound of formula (54) is used in underivatised form, the reaction is conducted in a solvent in the presence of a condensing agent and with or without a base.

There is no particular restriction on the nature of the solvent to be employed, provided that it has no adverse effect on the reaction or on the reagents involved and that it can dissolve the reagents, at least to some extent. Examples of the solvent to be employed include: halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; and esters such as methyl acetate and ethyl acetate; water; or a mixture of these solvents described above,

of which halogentated hydrocarbons, ethers and esters are preferred, and dichloromethane, tetrahydrofuran and ethyl acetate are more preferred.

Any suitable condensing agent that is conventionally employed in such reactions can be employed, and examples include dicyclohexylcarbodiimide, 1-ethyl-3-(3-dimethylaminopropyl)carbodiimide, N,N'-carbonyldiimidazole or the like.

The base to be used is not particularly limited provided that it is one that is usually used as a base in such reactions, and examples thereof include alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene, of which amines are preferred, and triethylamine, pyridine and 1,8-diazabicyclo-[5.4.0]undec-7-ene are more preferred.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

(b) When the compound (54) is used after first being converted into a reactive derivative, examples of the reactive derivative include acid halides, mixed acid anhydrides, activated esters, active amides and the like.

The acid halides are prepared by the reaction of the compound of formula (54) with a halogenation reagent (for example, thionyl chloride, oxalyl chloride or the like); the mixed acid anhydride is prepared by the reaction of the compound of formula (54) with an acid halide (for example, methyl chlorocarbonate, ethyl chlorocarbonate or the like); the activated ester is prepared by the reaction of the compound of formula (54) with a compound containing a hydroxyl group (for example, N-hydroxysuccinimide, N-hydroxyphthalimide or the like) in the presence of a condensing agent such as those described in process (a) above; and the active amide (for example, Weinreb amide) is prepared by the reaction of the compound of formula (54) with a N-(lower alkoxy)-N-(lower alkyl)hydroxylamine (for example, N-methoxy-N-methylhydroxylamine or the like) in the presence of a condensing agent such as those described in process (a) above. Each of these reactions described above can be conducted employing reaction conditions conventionally employed in organic synthetic chemistry for such reactions.

The cyclisation of said reactive derivative is usually carried out in a solvent in the presence or absence of a base.

With regard to the solvent, condensing agent and base, the solvents, condensing agents and bases described in process (a) above can be used.

The reaction can take place over a wide range of temperatures, and the precise reaction temperature is not critical to the invention. The preferred reaction temperature will depend upon such factors as the nature of the solvent, and the starting material or reagent used. However, in general, we find it convenient to carry out the reaction at a temperature of from -20°C and 150°C, and preferably between 0°C and 100°C. The time required for the reaction may also vary widely, depending on many factors, notably the reaction temperature and the nature of the reagents and solvent employed. However, provided that the reaction is effected under the preferred conditions outlined above, a period of from 10 minutes to 48 hours, and preferably from 30 minutes to 12 hours is usually sufficient.

The substituent $R^3$, which is one of the components of the compound of general formula (I), can be substituted with various substituents ($R^4$). The substituents $R^4$ can be converted into other substituents falling within the scope of the definition of $R^4$ in each of the steps described above. The substituent $R^4$ can, for example, be converted as illustrated below employing conventional organic synthetic methods.

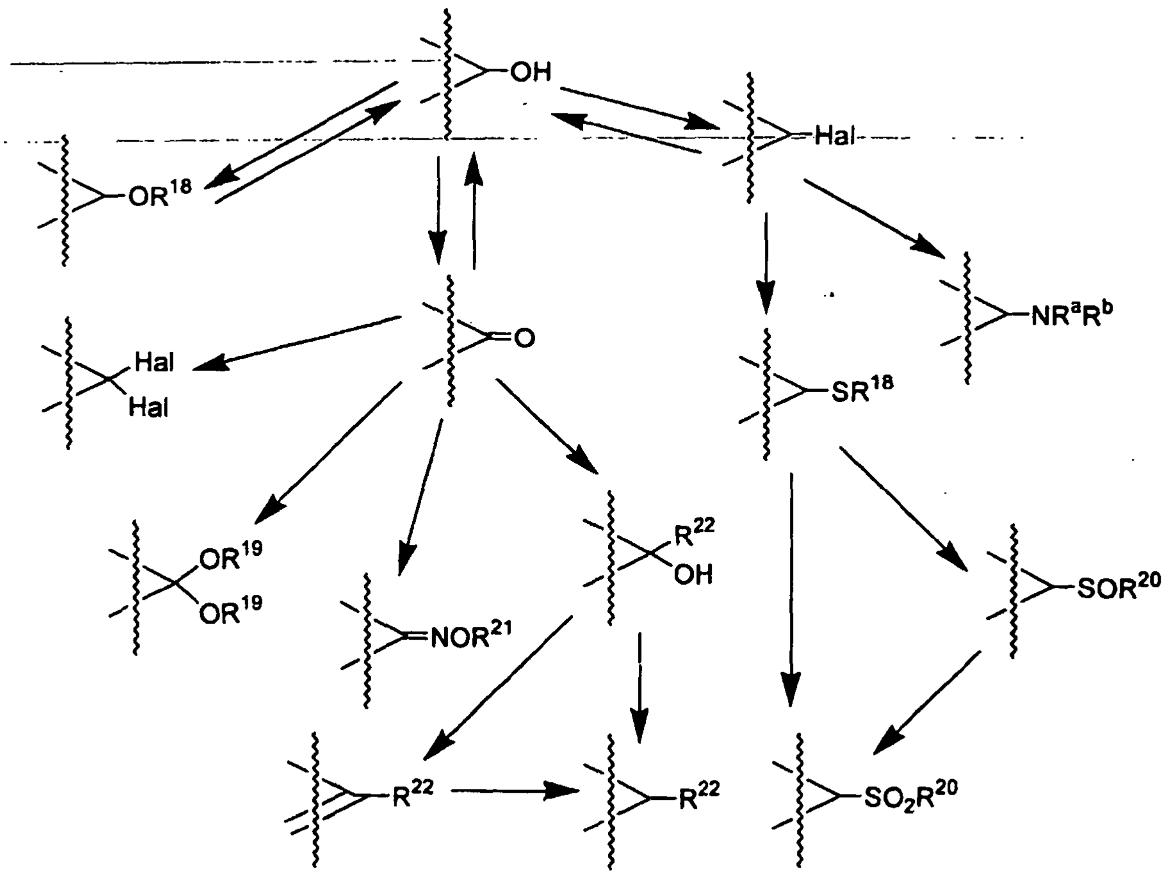

In the above formulae, $R^a$, $R^b$ and Hal have the same meanings as defined above,
$R^{18}$ represents a lower alkyl group as defined above, a halogenated lower alkyl group as defined above or an aryl group which may optionally be substituted with at least one substituent selected from Substituent group α and Substituent group β as defined above,
the groups $R^{19}$ are the same or different, and each represents a lower alkyl group as defined above or a halogenated lower alkyl group as defined above, or the two groups $R^{19}$ can together form a lower alkylene group as defined above,
$R^{20}$ represents a lower alkyl group as defined above,
$R^{21}$ represents a hydrogen atom or a lower alkyl group as defined above, and
$R^{22}$ represents
a lower alkyl group which may optionally be substituted with at least one substituent selected from Substituent group α, a lower alkenyl group which may optionally be substituted with at least one substituent selected from Substituent group α, a lower alkynyl group which may optionally be substituted with at least one substituent selected from Substituent group α, an aralkyl group or a cycloalkyl group as defined above in the definition of Substituent group β, or
an aryl group which may optionally be substituted with at least one substituent selected from Substituent group α and Substituent group β as defined above.

Furthermore, when $R^4$ is a halogen atom, a hydroxyl group, a cyano group or ' a lower alkylsulfonyl group, $R^4$ can be converted into a hydrogen atom by the formation of a double bond, followed by the reduction of said double bond using conventional methods as illustrated below.

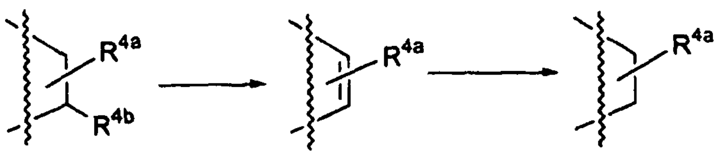

In the above formulae, $R^{4a}$ has the same meaning as defined above, and $R^{4b}$ represents a halogen atom, hydroxyl group, cyano group or lower alkylsulfonyl group.

Where $R^4$ represents a lower alkylidenyl group or an aralkylidenyl group, such a compound can be prepared from the corresponding oxo derivative as shown below. Subsequently, the alkylidenyl or aralkylidenyl compound can be converted to the corresponding alkyl or aralkyl derivative by reduction of the double bond.

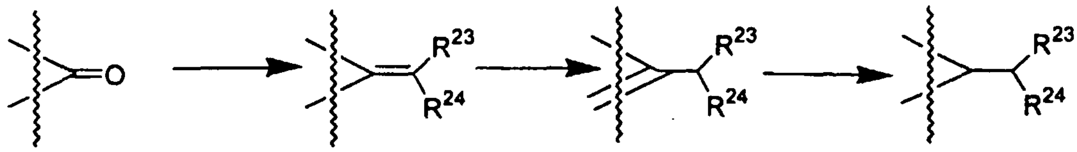

In the above formulae, $R^{23}$ and $R^{24}$ are the same or different, and each represents a hydrogen atom, a lower alkyl group as defined above, an aryl group as defined above or an aralkyl group as defined above.

After completion of each of the reactions described in the steps of Method A to Method O above, the desired compound may be isolated from the reaction mixture in a conventional manner. For example, it can be obtained by neutralizing the reaction mixture as needed, removing insoluble matters by filtration, if any are present, adding organic solvents which are not miscible with each other, such as water and ethyl acetate, washing with water or the like, separating the organic layer containing the desired compound, drying it over anhydrous magnesium sulfate or the like and then distilling off the solvent.

If necessary, the desired compound thus obtained can be isolated and purified by using a conventional method such as recrystallization or reprecipitation or by a chromatographic method. Examples of chromatography include adsorption column chromatography using a carrier such as silica gel, alumina or magnesium-silica gel type Florisil, chromatography using a synthetic adsorbent, for example, partition column chromatography using a carrier such as Sephadex LH-20 (product of Pharmacia), Amberlite XAD-1 (product of Rohm & Haas) or Diaion HP-20 (product of Mitsubishi Chemical), ion exchange chromatography and normal-phase-reverse-phase column chromatography (high-performance liquid chromatography) using a silica gel or alkylated silica gel. If necessary, two or more of these techniques can be used in combination to isolate and purify the desired compound.

The pyrrole derivatives of the present invention exhibit excellent inhibitory activity against the production of inflammatory cytokines. Consequently, they are effective as a medicament, particularly as an agent for the prophylaxis or treatment of diseases mediated by inflammatory cytokines. Examples of such a medicament include analgesics, anti-inflammatory drugs and virucides, and agents for the prophylaxis or treatment of chronic rheumatoid arthritis, osteoarthritis, allergic diseases, asthma, septicaemia, psoriasis, osteoporosis, autoimmune diseases (e.g. systemic lupus erythematosus, ulcerative colitis and Crohn's disease), diabetes, nephritis, hepatitis, cancer, ischemic heart disease, Alzheimer's disease and arteriosclerosis. Of these, the compounds of the present invention are particularly useful as analgesics and anti-inflammatory drugs and as agents for the prophylaxis or treatment of chronic rheumatism, osteoarthritis, allergic diseases, septicaemia, psoriasis, osteoporosis, ulcerative colitis, diabetes, hepatitis and arteriosclerosis.

The compounds of formula (I-1) and pharmacologically acceptable salts, esters and amides thereof according to the present invention can be administered by a number of different routes. Examples of these administration routes include oral administration in the form of tablets, capsules, granules, powders or syrups and parenteral administration in the form of injections or suppositories. Such formulations can be prepared in a known manner by using additives such as an excipients, lubricants, binders, disintegrators, stabilizers, corrigents and diluents.

Examples of suitable excipients include: organic excipients, examples of which include sugar derivatives such as lactose, sucrose, dextrose, mannitol and sorbitol, starch derivatives such as corn starch, potato starch, α-starch, dextrin and carboxymethyl starch, cellulose derivatives such as crystalline cellulose, low-substituted hydroxypropylcellulose, hydroxypropylmethylcellulose, carboxymethylcellulose, calcium carboxymethylcellulose and sodium internally-crosslinked carboxymethylcellulose, gum arabic, dextran and pullulan; and inorganic excipients, examples of which include silicate derivatives such as soft silicic acid anhydride, synthetic aluminum silicate and magnesium aluminometasilicate, phosphates such as calcium phosphate, carbonates such as calcium carbonate, and sulfates such as calcium sulfate.

Examples of suitable lubricants include: stearic acid; metal salts of stearic acid such as calcium stearate and magnesium stearate; talc; colloidal silica; waxes such as bee gum and spermaceti; boric acid; adipic acid; sulfates such as sodium sulfate; glycol; fumaric acid; sodium benzoate; DL-leucine; sodium salts of an aliphatic acid; lauryl sulfates such as sodium lauryl sulfate and magnesium lauryl sulfate; silicic acid derivatives such as silicic anhydride and silicic acid hydrate; and starch derivatives exemplified above as examples of suitable excipients.

Examples of suitable binders include polyvinylpyrrolidone, Macrogol™ and compounds similar to those exemplified above as suitable excipients.

Examples of suitable disintegrators include compounds similar to those exemplified above as suitable excipients and chemically modified starch or cellulose derivatives such as sodium cross carmellose, sodium carboxymethyl starch and crosslinked polyvinylpyrrolidone.

Examples of suitable stabilizers include: paraoxybenzoate esters such as methylparaben and propylparaben; alcohols such as chlorobutanol, benzyl alcohol and phenylethyl alcohol; benzalkonium chloride; phenol derivatives such as phenol and cresol; thimerosal; dehydroacetic acid; and sorbic acid. Examples of suitable corrigents include sweeteners, acidifiers and flavors commonly employed for this purpose.

The dose of the compound of formula (I-1) or a pharmacologically acceptable salt, ester or amide thereof according to the present invention will vary depending on a variety of factors including the condition to be treated, the age of the patient and the administration route. When administered orally, it is administered to an adult in an amount of 0.1 mg (preferably 0.5 mg) a day as a lower limit and 2000 mg (preferably 500 mg) a day as an upper limit. It can be administered in from one to several portions depending on the condition of the patient. When administered intravenously, it is administered to an adult in an amount of 0.01 mg (preferably 0.05 mg) a day as a lower limit and 200 mg (preferably 50 mg) a day as an upper limit. It can be administered in from one to several portions depending on the condition of the patient.

The following examples, preparative examples, formulation examples and test examples are intended to further illustrate the present invention and are not intended to limit the scope of this invention in any way.

Example 1

2-(4-Fluorophenyl)-4-[(2R,8aS)-2-methoxy-1,2,3,5,8,8a-hexahydroindolizin-7-yl]-3-(pyridin-4-yl)-1*H*-pyrrole (Compound No. 4-15)

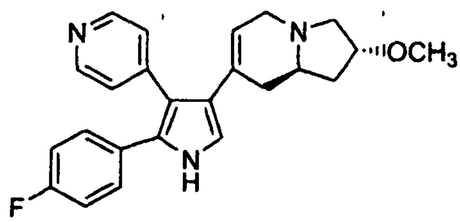

1 (i) 4-Ethoxycarbonyl-2-(4-fluorophenyl)-3-(pyridin-4-yl)-1H-pyrrole

36 ml (54.7 mmol) of a 1.53N solution of butyllithium in hexane were added to 240 ml of tetrahydrofuran. A solution of 15.90 g (54.7 mmol) of α-(p-toluenesulfonyl)-4-fluorobenzyl isocyanide in 120 ml of tetrahydrofuran was then added to the resulting solution at -45°C, followed by stirring of the resulting mixture for 10 minutes at the same temperature. At the end of this time, 25.00 g (273 mmol) of 95% lithium bromide were added, the resulting mixture was stirred for 30 minutes and then a solution of 8.73 g (49.2 mmol) of ethyl 3-(4-pyridyl)acrylate in 120 ml of tetrahydrofuran was added. The resulting mixture was stirred at the same temperature for 1 hour and then the cooling bath was removed and the mixture was stirred at room temperature for a further 1 hour. At the end of this time, 500 ml of water were added and the resulting mixture was extracted with ethyl acetate. The organic extract was washed with water and then dried over anhydrous sodium sulfate, after which it was concentrated by evaporation under reduced pressure to afford a solid. The solid was washed with diethyl ether to give 13.61 g (yield: 89%) of the title compound as a pale yellow powder.
$^1$H-NMR spectrum (500 MHz, $CDCl_3$) δ ppm:

8.84 (1H, broad singlet);
8.51 (2H, doublet, J=7 Hz);
7.58 (1H, doublet, J=3 Hz);
7.21 (2H, doublet, J=6 Hz);
7.11 (2H, doublet of doublets, J=9 Hz, 5 Hz);
6.97 (2H, triplet, J=9 Hz);
4.18 (2H, quartet, J=7 Hz);
1.20 (3H, triplet, J=7 Hz).

1(ii) 2-(4-Fluorophenyl)-3-(pyridin-4-yl)-1*H*-pyrrole

15.00 g (48.3 mmol) of 4-ethoxycarbonyl-2-(4-fluorophenyl)-3-(pyridin-4-yl)-1*H*-pyrrole [obtained as described in Example 1(i) above] were dissolved in a mixture of 90 ml of acetic acid, 30 ml of sulfuric acid and 60 ml of water, and the resulting solution was stirred at 100°C for 16 hours. After being cooled to room temperature, the reaction mixture was made basic by the addition of a 10% aqueous solution of sodium hydroxide before extracting with ethyl acetate. The organic extract thus obtained was washed with water, dried over anhydrous sodium sulfate and then concentrated by evaporation under reduced pressure to give 11.40 g (yield: 99%) of the title compound as a pale red powder.
$^1$H-NMR spectrum (500 MHz, $CDCl_3$) δ ppm:

9.78 (1H, broad singlet);
8.42 (2H, doublet, J=7 Hz);
7.37 (2H, doublet of doublets, J=9 Hz, 5 Hz);
7.22 (2H, doublet, J=6 Hz);
7.06 (2H, triplet, J=9 Hz);
6.90 (1H, triplet, J=3 Hz);
6.47 (1H, triplet, J=3 Hz).

1(iii) 2-(4-Fluorophenyl)-3-(pyridin-4-yl)-1-triisopropylsilyl-1*H*-pyrrole

11.30 g (47.4 mmol) of 2-(4-fluorophenyl)-3-(pyridin-4-yl)-1*H*-pyrrole [obtained as described in Example (ii) above] were dissolved in 300 ml of tetrahydrofuran. 31 ml (47.4 mmol) of a 1.57N solution of butyllithium in hexane were then added to the resulting solution at -78°C. After stirring the reaction mixture for 10 minutes, 13.4 ml (49.8 mmol) of triisopropylsilyl triflate were added at the same temperature. The resulting mixture was stirred at room temperature for 30 minutes. At the end of this time 200 ml of water and 300 ml of a saturated aqueous solution of sodium hydrogencarbonate were added, and the mixture was extracted with ethyl acetate. The organic extract was washed with water, dried over anhydrous sodium sulfate and then concentrated by evaporation under reduced pressure to afford 18.70 g (quantitative yield) of the title compound as a reddish purple oil.
$^1$H-NMR spectrum (500 MHz, DMSO-$d_6$) δ ppm:

8.25 (2H, doublet, J=6 Hz);
7.39 (2H, doublet of doublets, J=9 Hz, 6 Hz);
7.28 (2H, triplet, J=9 Hz);
7.00 (1H, doublet, J=3 Hz);
6.91(2H, doublet, J=7 Hz);
6.71 (1H, doublet, J=3 Hz);
1.15-1.05 (3H, multiplet);
0.98 (18H, doublet, J=8 Hz).

1(iv) 4-Bromo-2-(4-fluorophenyl)-3-(pyridin-4-yl)-1-triisopropylsilyl-1*H*-pyrrole

18.70 g (47.4 mmol) of 2-(4-fluorophenyl)-3-(pyridin-4-yl)-1-triisopropylsilyl-1*H*-pyrrole [obtained as described in Example 1(iii) above] were dissolved in 300 ml of tetrahydrofuran. A suspension of 8.61 g (47.4 mmol) of N-bromosuccinimide in 100 ml of tetrahydrofuran was then gradually added to the resulting mixture at -78°C. The resulting reaction mixture was then stirred at -78°C for 6 hours followed by a further 1 hour of stirring at room temperature, after which time 400 ml of hexane were added and any insoluble materials were filtered off. The filtrate was concentrated by evaporation under reduced pressure. The resulting residue was purified by chromatography on a silica gel column using a 2:1 by volume mixture of hexane and ethyl acetate as the eluant to afford 9.57 g (yield: 43%) of the title compound as pale yellow prisms.
$^1$H-NMR spectrum (500 MHz, DMSO-$d_6$) δ ppm:

8.36 (2H, doublet, J=6 Hz);
7.34 (2H, doublet of doublets, J=9 Hz, 6 Hz);
7.18 (2H, triplet, J=9 Hz);
7.12 (1H, singlet);
7.04 (2H, doublet, J=6 Hz);
1.16-1.08 (3H, multiplet);
0.99 (18H, doublet, J=8 Hz).

1(v) 2-(4-Fluorophenyl)-4-[(2R,8aS)-2-methoxy-1,2,3,5,8,8a-hexahydroindolizin-7-yl]-3-(pyridin-4-yl)-1*H*-pyrrole

3.00 g (6.34 mmol) of 4-bromo-2-(4-fluorophenyl)-3-(pyridin-4-yl)-1-triisopropylsilyl-1*H*-pyrrole [obtained as described in Example 1(iv) above] were dissolved in 60 ml of tetrahydrofuran. 4.36 ml (6.97 mmol) of a 1.6M solution of butyllithium in hexane were then added to the resulting solution at -78°C. After stirring the reaction mixture at -78°C for 10 minutes, 1.29 g (7.60 mmol) of (2R,8aS)-2-methoxy-1,2,3,5,6,7,8,8a-octahydroindolizin-7-one (prepared as described in Preparative Example 1 below) were added at the same temperature. The resulting mixture was stirred at -78°C for 2 hours and then at room temperature for 1 hour. At the end of this time a saturated aqueous solution of sodium hydrogencarbonate was added, and the reaction mixture was then extracted with ethyl acetate. The organic extract was washed with water, dried over anhydrous sodium sulfate and then concentrated by evaporation under reduced pressure.

The resulting residue was dissolved in 40 ml of dichloroethane, 1.95 ml (25.3 mmol) of trifluoroacetic acid were added to the solution thus obtained and the reaction mixture was then heated under reflux for 1 hour. After being cooled to room temperature, the reaction mixture was concentrated by evaporation under reduced pressure. The residue thus obtained was dissolved in 30 ml of tetrahydrofuran. 25.3 ml (25.3 mmol) of a 1M solution of tetrabutylammonium fluoride in tetrahydrofuran were added to the resulting solution and the mixture was then stirred for 10 minutes at room temperature. Water was added at the end of this time, and the resulting mixture was acidified with 1N hydrochloric acid and then extracted with ethyl acetate. The aqueous layer was made basic by the addition of sodium carbonate and then extracted with ethyl acetate. The organic extract was washed with water, dried over anhydrous sodium sulfate and then concentrated by evaporation under reduced pressure. The resulting residue was purified by chromatography on a silica gel column using a 100:10:1 by volume mixture of ethyl acetate, methanol and isopropylamine respectively as the eluant to afford 545 mg (yield: 22%) of the title compound (Rf value = 0.45) as a pale brown powder.
Melting point: 203 - 205°C (decomposition)
$^1$H-NMR spectrum (400 MHz, DMSO-$d_6$) $\delta$ ppm:

11.38 (1H, broad singlet);
8.44 (2H, doublet, J=6 Hz);
7.20-7.06 (6H, multiplet);
6.90 (1H, doublet, J=3 Hz);
5.27-5.22 (1H, multiplet);
3.92-3.85 (1H, multiplet);
3.40 (1H, doublet of doublets, J=9 Hz, 7 Hz);
3.29-3.19 (1H, multiplet);
3.16 (3H, singlet);
2.71-2.62 (1H, multiplet);
2.37-2.20 (2H, multiplet);
2.04-1.90 (2H, multiplet);
1.88-1.80 (1H, multiplet);
1.51-1.41 (1H, multiplet).

Example 2

2-(4-Fluorophenyl)-4-[(2R,8aS)-2-methoxy-1,2,3,5,6,8a-hexahydroindolizin-7-yl]-3-(pyridin-4-yl)-1*H*-pyrrole (Compound No. 1-303)

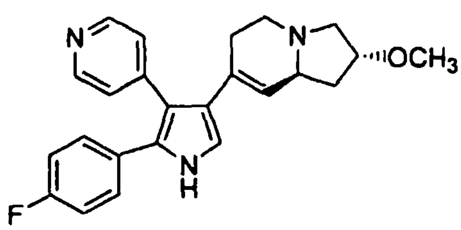

The silica gel column chromatography performed in Example 1 (v) above also provided 300 mg (yield: 12 %) of the title compound (Rf value = 0.40) as a pale brown powder.

Melting point: 198 - 200°C (decomposition)
$^1$H-NMR spectrum (400 MHz, DMSO-$d_6$) δ ppm:

11.39 (1H, broad singlet);
8.44 (2H, doublet, J=6 Hz);
7.21-7.05 (6H, multiplet);
6.92 (1H, doublet, J=3 Hz);
5.16-5.11 (1H, multiplet);
3.92-3.84 (1H, multiplet);
3.39-3.25 (1H, multiplet);
3.23-3.11 (1H, multiplet);
3.15 (3H, singlet);
3.05 (1H, doublet of doublets, J=10 Hz, 6 Hz);
2.86-2.77 (1H, multiplet);
2.64-2.54 (1H, multiplet);
2.30-2.19 (1H, multiplet);
2.10-2.00 (1H, multiplet);
1.76-1.67 (1H, multiplet);
1.48-1.38 (1H, multiplet).

Example 3

2-(4-Fluorophenyl)-4-[(2R,8aS)-2-hydroxy-1,2,3,5,8,8a-hexahydroindolizin-7-yl]-3-(pyridin-4-yl)-1*H*-pyrrole (Compound No. 4-14)

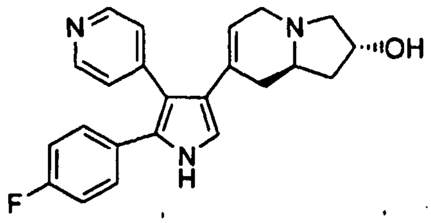

In a similar manner to the procedure described in Example 1 (v) above, a reaction and silica gel column chromatography (using a 100:10:2.5 by volume mixture of ethyl acetate, methanol and isopropylamine respectively as the eluant) were conducted, using (2R,8aS)-2-(t-butyldimethylsilyloxy)-1,2,3,5,6,7,8,8a-octahydroindolizin-7-one (prepared as described in Preparative Example 2 below) in place of (2R,8aS)-2-methoxy-1,2,3,5,6,7,8,8a-octahydroindolizin-7-one, to give 587 mg (yield: 25%) of the title compound (Rf value = 0.25) as a pale brown powder.
Melting point: 208 - 210°C (decomposition)
$^1$H-NMR spectrum (400 MHz, DMSO-$d_6$) δ ppm:

11.37 (1H, broad singlet);
8.44 (2H, doublet, J=6 Hz);
7.20-7.06 (6H, multiplet);
6.90 (1H, doublet, J=3 Hz);
5.26-5.22 (1H, multiplet);
4.72 (1H, doublet, J=4 Hz);
4.25-4.16 (1H, multiplet);
3.38-3.27 (1H, multiplet);
3.25-3.17 (1H, multiplet);
2.72-2.63 (1H, multiplet);
2.45-2.35 (1H, multiplet);
2.26-2.18 (1H, multiplet);
1.98-1.87 (2H, multiplet);

1.71-1.64 (1H, multiplet);
1.57-1.46 (1H, multiplet).

Example 4

2-(4-Fluorophenyl)-4-[(2R,8aS)-2-hydroxy-1,2,3,5,6,8a-hexahydroindolizin-7-yl]-3-(pyridin-4-yl)-1*H*-pyrrole (Compound No. 1-302)

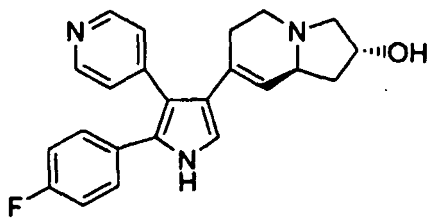

The silica gel column chromatography performed in Example 3 above also provided 213 mg (yield: 9 %) of the title compound (Rf value = 0.20) as a pale brown powder.
Melting point: 209 - 211°C (decomposition)
$^1$H-NMR spectrum (400 MHz, DMSO-$d_6$) δ ppm:

11.38 (1H, broad singlet);
8.44 (2H, doublet, J=5 Hz);
7.20-7.05 (6H, multiplet);
6.90 (1H, doublet, J=3 Hz);
5.16-5.12 (1H, multiplet);
4.63 (1H, doublet, J=5 Hz);
4.25-4.16 (1H, multiplet);
3.30-3.20 (1H, multiplet);
3.00 (1H, doublet of doublets, J=10 Hz, 6 Hz);
2.84-2.74 (1H, multiplet);
2.63-2.53 (1H, multiplet);
2.40 (1H, doublet of doublets, J=10 Hz, 4 Hz);
2.27-2.16 (1H, multiplet);
2.08-1.98 (1H, multiplet);
1.62-1.52 (1H, multiplet);
1.52-1.42 (1H, multiplet).

Example 5

4-[(2S,8aS)-2-Chloro-1,2,3,5,8,8a-hexahydroindolizin-7-yl]-2-(4-fluorophenyl)-3-(pyridin-4-yl)-1*H*-pyrrole (Compound No. 4-23)

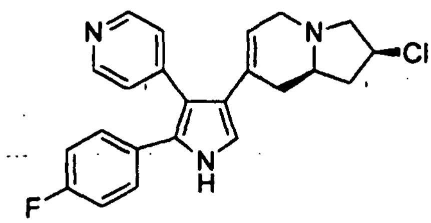

In a similar manner to the procedure described in Example 1(v) above, a reaction and silica gel column chromatography (using a 40:1:1 by volume mixture of ethyl acetate, methanol and isopropylamine respectively as the eluant) were conducted, using (2S,8aS)-2-chloro-1,2,3,5,6,7,8,8a-octahydroindolizin-7-one (prepared as described in Preparative Example 3 below) in place of (2R,8aS)-2-methoxy-1,2,3,5,6,7,8,8a-octahydroindolizin-7-one, to give 520 mg (yield: 21%) of the title compound (Rf value = 0.45) as a pale brown powder.
Melting point: 195 - 197°C (decomposition)
$^1$H-NMR spectrum (400 MHz, $CDCl_3$) δ ppm:

8.46 (2H, doublet, J=6 Hz);
8.38 (1H, broad singlet);
7.16 (2H, doublet, J=6 Hz);
7.13 (2H, doublet of doublets, J=9 Hz, 5 Hz);
6.97 (2H, triplet, J=9 Hz);
6.83 (1H, doublet, J=3 Hz);
5.54-5.53 (1H, multiplet);
4.42-4.36 (1H, multiplet);
3.54 (1H, doublet of doublets, J=16 Hz, 5 Hz);
3.39 (1H, doublet, J=11 Hz);
2.79 (1H, doublet, J=16 Hz);
2.68-2.60 (2H, multiplet);
2.30-2.16 (3H, multiplet);
1.85-1.76 (1H, multiplet).

Example 6

4-[(2S,8aS)-2-Chloro-1,2,3,5,6,8a-hexahydroindolizin-7-yl]-2-(4-fluorophenyl)-3-(pyridin-4-yl)-1*H*-pyrrole (Compound No. 1-311)

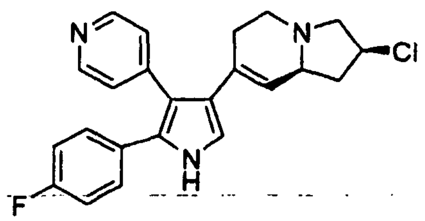

The silica gel column chromatography performed in Example 5 above also provided 400 mg (yield: 16 %) of the title compound (Rf value = 0.35) as a pale brown powder.
Melting point: 177 - 180°C (decomposition)
$^1$H-NMR spectrum (400 MHz, $CDCl_3$) δ ppm:

8.49 (2H, doublet, J=5 Hz);
8.37 (1H, broad singlet);
7.17 (2H, doublet, J=5 Hz);
7.13 (2H, doublet of doublets, J=8 Hz, 5 Hz);
6.98 (2H, triplet, J=8 Hz);
6.84 (1H, doublet, J=3 Hz);
5.40 (1H, singlet);
4.38-4.32 (1H, multiplet);
3.53-3.45 (1H, multiplet);
3.23 (1H, doublet of doublets, J=11 Hz, 7 Hz);
3.13-3.06 (2H, multiplet);
2.90-2.82 (1H, multiplet);
2.59 (1H, doublet of triplets, J=14 Hz, 8 Hz);

2.43-2.31 (1H, multiplet);
2.13-2.02 (1H, multiplet);
1.79-1.69 (1H, multiplet).

Example 7

4-[(8aS)-2,2-Difluoro-1,2,3,5,8,8a-hexahydroindolizin-7-yl]-2-(4-fluorophenyl)-3-(pyridin-4-yl)-1*H*-pyrrole (Compound No. 4-26)

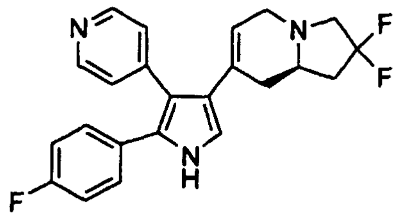

In a similar manner to the procedure described in Example I (v) above, a reaction and silica gel column chromatography (using a 49:1 by volume mixture of dichloromethane and methanol as the eluant) were conducted, using (8aS)-2,2-difluoro-1,2,3,5,6,7,8,8a-octahydroindolizin-7-one (prepared as described in Preparative Example 4 below) in place of (2R,8aS)-2-methoxy-1,2,3,5,6,7,8,8a-octahydroindolizin-7-one, to give 358 mg (yield: 28%) of the title compound (Rf value = 0.35) as a pale brown powder.
Melting point: 201 - 203°C (decomposition)
$^1$H-NMR spectrum (400 MHz, DMSO-$d_6$) δ ppm:

11.42 (1H, broad singlet);
8.45 (2H, doublet, J=6 Hz);
7.21-7.02 (6H, multiplet);
6.93 (1H, doublet, J=3 Hz);
5.27-5.22 (1H, multiplet);
3.48-3.37 (1H, multiplet);
3.33-3.22 (1H, multiplet);
2.77-2.68 (1H, multiplet);
2.59-2.36 (3H, multiplet);
2.34-2.26 (1H, multiplet);
2.16-2.06 (1H, multiplet);
1.96-1.78 (1H, multiplet).

Example 8

4-[(8aS)-2,2-Difluoro-1,2,3,5,6,8a-hexahydroindolizin-7-yl]-2-(4-fluorophenyl)-3-(pyridin-4-yl)-1*H*-pyrrole (Compound No. 1-314)

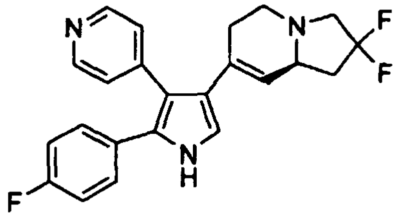

The silica gel column chromatography performed in Example 7 above also provided 290 mg (yield: 23 %) of the title compound (Rf value = 0.30) as a pale brown powder.
Melting point: 202 - 204°C (decomposition)
$^1$H-NMR spectrum (400 MHz, DMSO-$d_6$) δ ppm:

11.44 (1H, broad singlet);
8.43 (2H, doublet, J=6 Hz);
7.21-7.07 (6H, multiplet);
6.96 (1H, doublet, J=3 Hz);
5.15-5.11 (1H, multiplet);
3.46-3.39 (1H, multiplet);
3.26-3.15 (1H, multiplet);
2.98-2.85 (2H, multiplet);
2.71-2.62 (1H, multiplet);
2.39-2.25 (2H, multiplet);
2.12-2.04 (1H, multiplet);
1.83-1.67 (1H, multiplet).

Example 9

(±)-2-(4-Fluorophenyl)-3-(pyridin-4-yl)-4-(6,9,9a,10-tetrahydropyrido[1,2-a]indol-8-yl)-1*H*-pyrrole (Compound No. 5-8)

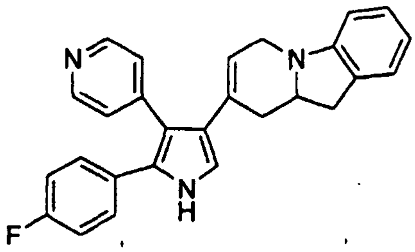

In a similar manner to the procedure described in Example 1 (v) above, a reaction and silica gel column chromatography (using a 1:1 by volume mixture of ethyl acetate and hexane as the eluant) were conducted, using (±)-6,7,8,9,9a,10-hexahydropyrido[1,2-a]indol-8-one (prepared as described in Preparative Example 5 below) in place of (2R,8aS)-2-methoxy-1,2,3,5,6,7,8,8a-octahydroindolizin-7-one, to give 70 mg (yield: 5%) of the title compound (Rf value = 0.40) as a pale yellow powder.
Melting point: 214 - 216°C (decomposition)
$^1$H-NMR spectrum (400 MHz, $CDCl_3$) δ ppm:

8.48 (2H, doublet, J=6 Hz);
8.29 (1H, broad singlet);
7.18 (2H, doublet, J=6 Hz);
7.14 (2H, doublet of doublets, J=9 Hz, 5 Hz);
7.11-7.06 (2H, multiplet);

6.98 (2H, triplet, J=9 Hz);
6.87 (1H, doublet, J=3 Hz);
6.69 (1H, triplet, J=8 Hz);
6.46 (1H, doublet, J=8 Hz);
5.62-5.60 (1H, multiplet);
4.00-3.90 (1H, multiplet);
3.47-3.34 (2H, multiplet);
3.03 (1H, doublet of doublets, J=15 Hz, 8 Hz);
2.61 (1H, doublet of doublets, J=15 Hz, 12 Hz);
2.51-2.33 (2H, multiplet).

Example 10

(±)-2-(4-Fluorophenyl)-3-(pyridin-4-yl)-4-(6,7,9a,10-tetrahydropyrido[1,2-a]indol-8-yl)-1*H*-pyrrole (Compound No. 5-1)

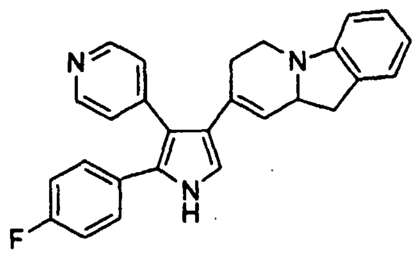

The silica gel column chromatography performed in Example 9 above also provided 230 mg (yield: 15 %) of the title compound (Rf value = 0.20) as a pale yellow powder.
Melting point: 205 - 207°C (decomposition)
$^1$H-NMR spectrum (400 MHz, $CDCl_3$) δ ppm:

8.32 (2H, doublet, J=6 Hz);
8.27 (1H, broad singlet);
7.15-7.05 (4H, multiplet);
6.95 (2H, triplet, J=9 Hz);
6.88 (2H, doublet, J=6 Hz);
6.77-6.72 (2H, multiplet);
6.60 (1H, doublet, J=8 Hz);
5.26 (1H, singlet);
4.3 5-4.26 (1H, multiplet);
3.77 (1H, doublet of doublets, J=14 Hz, 6 Hz);
3.35-3.27 (1H, multiplet);
3.13 (1H, doublet of doublets, J=15 Hz, 10 Hz);
2.55 (1H, doublet, J=15 Hz);
2.50-2.39 (1H, multiplet);
1.91-1.82 (1H, multiplet).

Example 11

2-(4-Fluorophenyl)-4-[(2R,8aS)-2-phenyl-1,2,3,5,8,8a-hexahydroindolizin-7-yl]-3-(pyridin-4-yl)-1*H*-pyrrole (Compound No. 4-10)

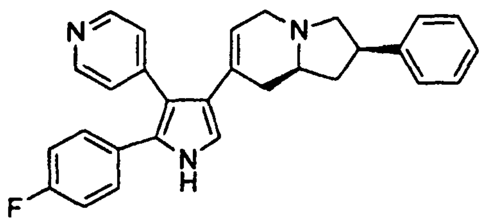

In a similar manner to the procedure described in Example 1(v) above, a reaction and silica gel column chromatography (using a 100:10:1 by volume mixture of ethyl acetate, methanol and isopropylamine respectively as the eluant) were conducted, using (2R,8aS)-2-phenyl-1,2,3,5,6,7,8,8a-octahydroindolizin-7-one (prepared as described in Preparative Example 6 below) in place of (2R,8aS)-2-methoxy-1,2,3,5,6,7,8,8a-octahydroindolizin-7-one, to give 399 mg (yield: 19%) of the title compound (Rf value = 0.45) as a pale brown powder.
Melting point: 191 - 193°C (decomposition)
$^1$H-NMR spectrum (400 MHz, DMSO-$d_6$) δ ppm:

11.39 (1H, broad singlet);
8.46 (2H, doublet, J=5 Hz);
7.3 8-7.06 (11H, multiplet);
6.94 (1H, doublet, J=2 Hz);
5.36-5.29 (1H, multiplet);
3.42-3.27 (2H, multiplet);
3.07-2.98 (1H, multiplet);
2.75-2.63 (1H, multiplet);
2.62-2.50 (1H, multiplet);
2.46-2.22 (3H, multiplet);
2.16-2.05 (1H, multiplet);
1.40-1.29 (1H, multiplet).

Example 12

2-(4-Fluorophenyl)-4-[(2R,8aS)-2-phenyl-1,2,3,5,6,8a-hexahydroindolizin-7-yl]-3-(pyridin-4-yl)-1*H*-pyrrole (Compound No. 1-298)

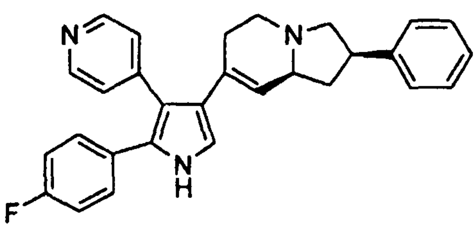

The silica gel column chromatography performed in Example 11 above also provided 369 mg (yield: 17 %) of the title compound (Rf value = 0.30) as a white powder.
Melting point: 208 - 210°C (decomposition)
$^1$H-NMR spectrum (400 MHz, DMSO-$d_6$) δ ppm:

11.40 (1H, broad singlet);
8.35 (2H, doublet, J=6 Hz);
7.38-7.07 (11H, multiplet);
6.95 (1H, doublet, J=3 Hz);
5.25-5.20 (1H, multiplet);
3.49-3.40 (1H, multiplet);

3.33-3.21 (1H, multiplet);
3.04-2.90 (2H, multiplet);
2.83-2.69 (2H, multiplet);
2.39-2.26 (2H, multiplet);
2.04-1.95 (1H, multiplet);
1.32-1.22 (1H, multiplet).

Example 13

4-[(8aS)-2,2-Ethylenedioxy-1,2,3,5,8,8a-hexahydroindolizin-7-yl]-2-(4-fluorophenyl)-3-(pyridin-4-yl)-1*H*-pyrrole (Compound No. 4-20)

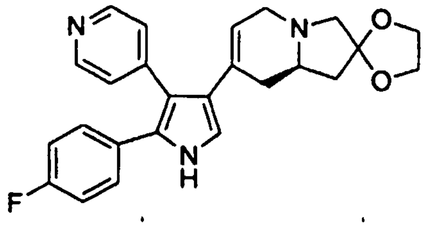

In a similar manner to the procedure described in Example 1(v) above, a reaction and silica gel column chromatography (using a 100:10:0.5 by volume mixture of ethyl acetate, methanol and isopropylamine as the eluant) were conducted, using (8aS)-2,2-ethylenedioxy-1,2,3,5,6,7,8,8a-octahydroindolizin-7-one (prepared as described in Preparative Example 7 below) in place of (2R,8aS)-2-methoxy-1,2,3,5,6,7,8,8a-octahydroindolizin-7-one, to give 694 mg (yield: 30%) of the title compound (Rf value = 0.55) as a white powder.
Melting point: 230 - 232°C (decomposition)
$^1$H-NMR spectrum (400 MHz, DMSO-$d_6$) δ ppm:

11.39 (1H, broad singlet);
8.44 (2H, doublet, J=6 Hz);
7.21-7.06 (6H, multiplet);
6.91 (1H, doublet, J=2 Hz);
5.27-5.21 (1H, multiplet);
3.91-3.71 (4H, multiplet);
3.27-3.18 (1H, multiplet);
3.12 (1H, doublet, J=10 Hz);
2.68-2.58 (1H, multiplet);
2.37-2.16 (3H, multiplet);
2.11-1.97 (2H, multiplet);
1.55 (1H, doublet of doublets, J=13 Hz, 10 Hz).

Example 14

4-[(8aS)-2,2-Ethylenedioxy-1,2,3,5,6,8a-hexahydroindolizin-7-yl]-2-(4-fluorophenyl)-3-(pyridin-4-yl)-1*H*-pyrrole (Compound No. 1-308)

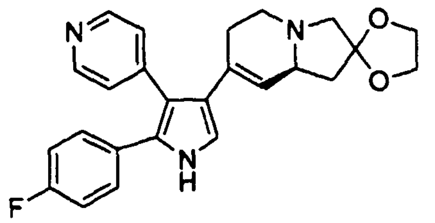

The silica gel column chromatography performed in Example 13 above also yielded 409 mg (yield: 8 %) of the title compound (Rf value = 0.40) as a pale brown powder.
Melting point: 196 - 198°C (decomposition)
$^1$H-NMR spectrum (400 MHz, DMSO-$d_6$) δ ppm:

11.40 (1H, broad singlet);
8.43 (2H, doublet, J=6 Hz);
7.21-7.05 (6H, multiplet);
6.93 (1H, doublet, J=3 Hz);
5.19-5.14 (1H, multiplet);
3.89-3.72 (4H, multiplet);
3.23-3.14 (1H, multiplet);
2.96-2.85 (2H, multiplet);
2.62-2.48 (2H, multiplet);
2.34-2.21 (1H, multiplet);
2.12-2.01(1H, multiplet);
1.93 (1H, doublet of doublets, J=13 Hz, 7 Hz);
1.51 (1H, doublet of doublets, J=13 Hz, 9 Hz).

Example 15

2-(4-Fluorophenyl)-4-[(8aS)-2-methyl-1,2,3,5,8,8a-hexahydroindolizin-7-yl]-3-(pyridin-4-yl)-1*H*-pyrrole (Compound No. 4-5)

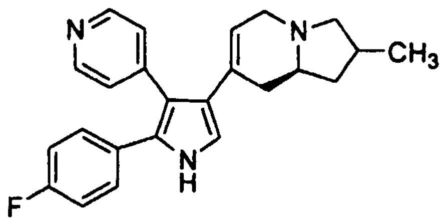

In a similar manner to the procedure described in Example 1(v) above, a reaction and silica gel column chromatography (using a 10:1:1 by volume mixture of ethyl acetate, methanol and isopropylamine respectively as the eluant) were conducted, using (8aS)-2-methyl-1,2,3,5,6,7,8,8a-octahydroindolizin-7-one (prepared as described in Preparative Example 8 below) in place of (2R,8aS)-2-methoxy-1,2,3,5,6,7,8,8a-octahydroindolizin-7-one, to give 253 mg (yield: 9%) of the title compound (Rf value = 0.65) as a pale brown powder.
Melting point: 190 - 193°C (decomposition)
$^1$H-NMR spectrum (400 MHz, $CDCl_3$) δ ppm:

8.46 (2H, doublet, J=6 Hz);
8.32 (1H, broad singlet);
7.17 (2H, doublet, J=6 Hz);

7.13 (2H, doublet of doublets, J=9 Hz, 5 Hz);
6.97 (2H, triplet, J=9 Hz);
6.82 (1H, doublet, J=3 Hz);
5.53-5.51 (1H, multiplet);
3.49-3.43 (1H, multiplet);
2.83 (1H, doublet of doublets, J=9 Hz, 3 Hz);
2.82-2.73 (1H, multiplet);
2.41 (1H, triplet, J=9 Hz);
2.30-2.05 (5H, multiplet);
1.09 (3H, doublet, J=7 Hz);

Example 16

2-(4-Fluorophenyl)-4-[(8aS)-2-methyl-1,2,3,5,6,8a-hexahydroindolizin-7-yl]-3-(pyridin-4-yl)-1*H*-pyrrole (Compound No. 1-293)

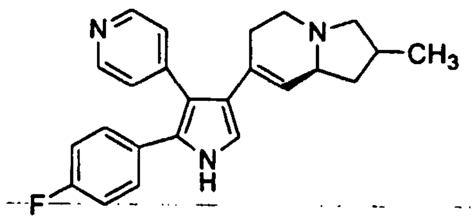

The silica gel column chromatography performed in Example 15 above also provided 280 mg (yield: 10 %) of the title compound (Rf value = 0.40) as a pale brown powder.
Melting point: 181 - 185°C (decomposition)
$^1$H-NMR spectrum (400 MHz, $CDCl_3$) δ ppm:

8.47 (2H, doublet, J=6 Hz);
8.30 (1H, broad singlet);
7.16 (2H, doublet, J=6 Hz);
7.13 (2H, doublet of doublets, J=9 Hz, 5 Hz);
6.97 (2H, triplet, J=9 Hz);
6.82 (1H, doublet, J=3 Hz);
5.41 (0.2H, singlet);
5.39 (0.8H, singlet);
3.53-3.43 (0.8H, multiplet);
3.41-3.22 (0.2H, multiplet);
3.11-3.05 (0.2H, multiplet);
3.04-2.90 (1H, multiplet);
2.89-2.77 (1.6H, multiplet);
2.73-2.64 (0.2H, multiplet);
2.48 (0.8H, triplet, J=9 Hz);
2.41-2.07 (3.2H, multiplet);
2.04-1.93 (1H, multiplet);
1.06 (2.4H, doublet, J=7 Hz);
1.02 (0.6H, doublet, J=7 Hz);
0.99-0.93 (1H, multiplet).

Example 17

2-(4-Fluorophenyl)-4-[(8aS)-8-methyl-1,2,3,5,8,8a-hexahydroindolizin-7-yl]-3-(pyridin-4-yl)-1*H*-pyrrole (Compound No. 4-41)

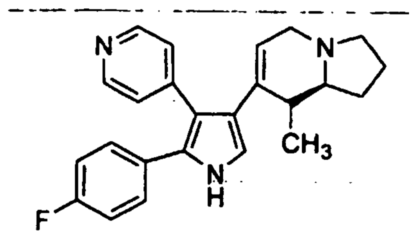

In a similar manner to the procedure described in Example 1(v) above, a reaction and silica gel column chromatography (using a 10:0.5:0.5 by volume mixture of ethyl acetate, methanol and isopropylamine respectively as the eluant) were conducted, using (8aS)-8-methyl-1,2,3,5,6,7,8,8a-octahydroindolizin-7-one (prepared as described in Preparative Example 9 below) in place of (2R,8aS)-2-methoxy-1,2,3,5,6,7,8,8a-octahydroindolizin-7-one, to give 57 mg (yield: 5%) of the title compound (Rf value = 0.45) as an orange powder.
Melting point: 205 - 207°C (decomposition)
$^1$H-NMR spectrum (500 MHz, $CDCl_3$) $\delta$ ppm:

8.46 (2H, doublet, J=8 Hz);
8.29-8.18 (1H, broad singlet);
7.18-7.13 (4H, multiplet);
6.97 (2H, triplet, J=9 Hz);
6.73 (1H, doublet, J=3 Hz);
5.62-5.55 (1H, multiplet);
3.58-3.50 (1H, multiplet);
3.24-3.17 (1H, multiplet);
2.77-2.68 (1H, multiplet);
2.23-2.08 (2H, multiplet);
2.04-1.95 (1H, multiplet);
1.90-1.78 (2H, multiplet);
1.77-1.68 (1H, multiplet);
1.43-1.33 (1H, multiplet);
0.76 (3H, doublet, J=7 Hz).

Example 18

2-(4-Fluorophenyl)-4-[(8aS)-8-methyl-1,2,3,5,6,8a-hexahydroindolizin-7-yl]-3-(pyridin-4-yl)-1*H*-pyrrole (Compound No. 1-330)

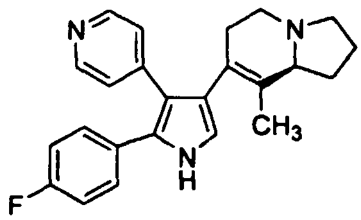

The silica gel column chromatography performed in Example 17 above also provided 708 mg (yield: 17%) of

the title compound (Rf value = 0.30) as a pale pink powder.
Melting point: 233 - 234°C
$^1$H-NMR spectrum (500 MHz, $CDCl_3$) δ ppm:

8.43 (2H, doublet, J=8 Hz);
8.36-8.25 (1H, broad singlet);
7.22 (2H, doublet of doublets, J=9 Hz, 5 Hz);
7.08 (2H, doublet, J=8 Hz);
7.00 (2H, triplet, J=9 Hz);
6.70 (1H, doublet, J=3 Hz);
3.07-3.01 (1H, multiplet);
2.97-2.92 (1H, multiplet);
2.91-2.84 (1H, multiplet);
2.70-2.62 (1H, multiplet);
2.57-2.49 (1H, multiplet);
2.34-2.24 (1H, multiplet);
2.12-2.03 (1H, multiplet);
2.02-1.94 (1H, multiplet);
1.92-1.84 (1H, multiplet);
1.81-1.70 (1H, multiplet);
1.55-1.45 (1H, multiplet);
1.46 (3H, singlet).

Example 19

4-[Cyclopropanespiro-6'-[(8a'S)-1',2',3',5',6',8a'-hexahydroindolizin]-7'-yl]-2-(4fluorophenyl)-3-(pyridin-4-yl)-1*H*-pyrrole (Compound No. 1-952)

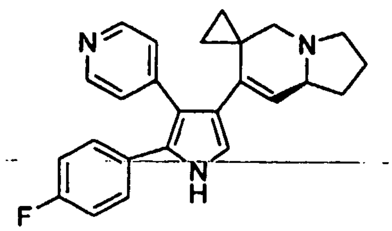

In a similar manner to the procedure described in Example 1(v) above, a reaction and silica gel column chromatography (using a 100:10:1 by volume mixture of ethyl acetate, methanol and isopropylamine respectively as the eluant) were conducted, using cydopropanespiro-6'-[(8a'S)-1',2',3',5',6',7',8',8a'-octahydroindolizin]-7'-one (prepared as described in Preparative Example 16 below) in place of (2R,8aS)-2-methoxy-1,2,3,5,6,7,8,8a-octahydroindolizin-7-one, to give 170 mg (yield: 11%) of the title compound (Rf value = 0.24) as a pale brown powder.
Melting point: 189 - 191 °C (decomposition)
$^1$H-NMR spectrum (400 MHz, DMSO-$d_6$) δ ppm:

11.35 (1H, broad singlet);
8.39 (2H, doublet, J=6 Hz);
7.23 (2H, doublet of doublets, J=9 Hz, 6 Hz);
7.14 (2H, triplet, J=9 Hz);
7.09 (2H, doublet, J=6 Hz);
6.69 (1H, doublet, J=2 Hz);
5.33-5.30 (1H, multiplet);
3.50-3.42 (1H, multiplet);
2.98-2.85 (2H, multiplet);
2.65-2.57 (1H, multiplet);

2.39 (1H, doublet, J=13 Hz);
1.91-1.80 (1H, multiplet);
1.76-1.53 (2H, multiplet);
1.31-1.20 (1H, multiplet);
0.56-0.42 (3H, multiplet);
0.22-0.15 (1H, multiplet).

Example 20

2-(4-Fluorophenyl)-4-[(2S,8aS)-2-methoxy-1,2,3,5,8,8a-hexahydroindolizin-7-yl]-3-(pyridin-4-yl)-1*H*-pyrrole (Compound No. 4-15)

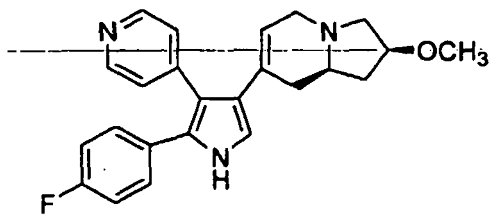

In a similar manner to the procedure described in Example 1(v) above, a reaction and silica gel column chromatography (using a 100:10:1 by volume mixture of ethyl acetate, methanol and isopropylamine respectively as the eluant) were conducted, using (2S,8aS)-2-methoxy-1,2,3,5,6,7,8,8a-octahydroindolizin-7-one (prepared as described in Preparative Example 10 below) in place of (2R,8aS)-2-methoxy-1,2,3,5,6,7,8,8a-octahydroindolizin-7-one, to give 228 mg (yield: 6%) of the title compound (Rf value = 0.50) as a white powder.
Melting point: 212 - 213°C (decomposition)
$^1$H-NMR spectrum (500 MHz, $CDCl_3$) δ ppm:

8.46 (2H, doublet, J=6 Hz);
8.38-8.27 (1H, broad singlet);
7.16 (2H, doublet, J=6 Hz);
7.12 (2H, doublet of doublets, J=9 Hz, 5 Hz);
6.97 (2H, triplet, J=9 Hz);
6.81 (1H, doublet, J=3 Hz);
5.52-5.47 (1H, multiplet);
3.91-3.84 (1H, multiplet);
3.54-3.47 (1H, multiplet);
3.30-3.24 (1H, multiplet);
3.27 (3H, singlet);
2.78-2.69 (1H, multiplet);
2.35 (1H, quintet, J=7 Hz);
2.27-2.10 (4H, multiplet);
1.48-1.39 (1H, multiplet).

Example 21

2-(4-Fluorophenyl)-4-[(2S,8aS)-2-methoxy-1,2,3,5,6,8a-hexahydroindolizin-7-yl)-3-(pyridin-4-yl)-1*H*-pyrrole (Compound No. 1-303)

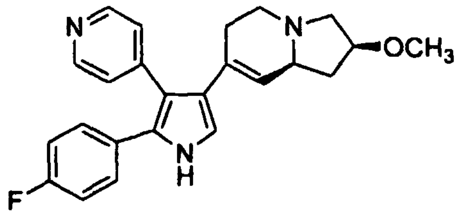

The silica gel column chromatography performed in Example 20 above also provided 184 mg (yield: 5%) of the title compound (Rf value = 0.30) as a pale brown powder.
Melting point: 219 - 220°C (decomposition)
$^1$H-NMR spectrum (500 MHz, $CDCl_3$) δ ppm:

8.47 (2H, doublet, J=6 Hz);
8.41-8.30 (1H, broad singlet);
7.17 (2H, doublet, J=6 Hz);
7.12 (2H, doublet of doublets, J=9 Hz, 5 Hz);
6.97 (2H, triplet, J=9 Hz);
6.82 (1H, doublet, J=3 Hz);
5.45-5.41 (1H, multiplet);
4.01-3.93 (1H, multiplet);
3.30 (3H, singlet);
3.28-3.17 (1H, broad singlet);
3.10-3.03 (1H, multiplet);
2.95 (1H, doublet of doublets, J=10 Hz, 4 Hz);
2.87-2.78 (1H, multiplet);
2.75-2.65 (1H, multiplet);
2.45-2.35 (1H, multiplet);
2.30-2.21 (1H, multiplet);
2.17-2.07 (1H, multiplet);
1.51-1.41 (1H, multiplet).

Example 22

2-(4-Fluorophenyl)-4-[(8aS)-2-methylidene-1,2,3,5,8,8a-hexahydroindolizin-7-yl]-3-(pyridin-4-yl)-1*H*-pyrrole (Compound No. 4-50)

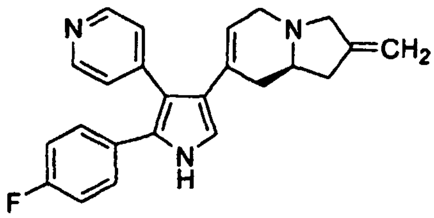

In a similar manner to the procedure described in Example 1(v) above, a reaction and silica gel column chromatography (using a 10:1:1 by volume mixture of ethyl acetate, methanol and isopropylamine respectively as the eluant) were conducted, using (8aS)-2-methylidene-1,2,3,5,6,7,8,8a-octahydroindolizin-7-one (prepared as described in Preparative Example 11 below) in place of (2R,8aS)-2-methoxy-1,2,3,5,6,7,8,8a-octahydroindolizin-7-one, to give 184 mg (yield: 10%) of the title compound (Rf value = 0.50) as a pale pink powder.
Melting point: 212 - 214°C (decomposition)

$^1$H-NMR spectrum (400 MHz, $CDCl_3$) δ ppm:

8.47 (2H, doublet, J=6 Hz);
8.29 (1H, broad singlet);
7.17 (2H, doublet, J=6 Hz);
7.13 (2H, doublet of doublets, J=9 Hz, 5 Hz);
6.97 (2H, triplet, J=9 Hz);
6.83 (1H, doublet, J=3 Hz);
5.52-5.48 (1H, multiplet);
4.92 (1H, broad singlet);
4.89 (1H, broad singlet);
3.79 (1H, doublet, J=13 Hz);
3.54-3.43 (1H, multiplet);
2.92-2.80 (2H, multiplet);
2.59 (1H, doublet of doublets, J=16 Hz, 6 Hz);
2.50-2.38 (1H, multiplet);
2.33-2.25 (1H, multiplet);
2.24-2.10 (2H, multiplet).

Example 23

2-(4-Fluorophenyl)-4-[(8aS)-2-methylidene-1,2,3,5,6,8a-hexahydroindolizin-7-yl]-3-(pyridin-4-yl)-1*H*-pyrrole Compound No. 1-982)

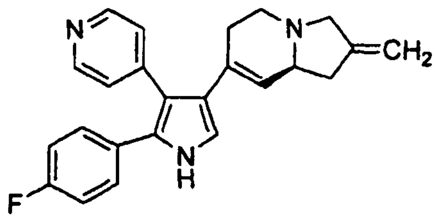

The silica gel column chromatography performed in Example 22 above also provided 195 mg (yield: 11 %) of the title compound (Rf value = 0.30) as a white powder.
Melting point: 217 - 218°C (decomposition)
$^1$H-NMR spectrum (400 MHz, $CDCl_3$) δ ppm:

8.46 (2H, doublet, J=6 Hz);
8.29 (1H, broad singlet);
7.20-7.09 (4H, multiplet);
6.97 (2H, triplet, J=9 Hz);
6.83 (1H, doublet, J=3 Hz);
5.42 (1H, doublet, J=2 Hz);
4.953 (1H, broad singlet);
4.949 (1H, broad singlet);
3.50-3.32 (3H, multiplet);
2.99-2.93 (1H, multiplet);
2.80-2.72 (1H, multiplet);
2.56 (1H, doublet of doublets, J=16 Hz, 7 Hz);
2.42-2.31 (1H, multiplet);
2.22-2.10 (2H, multiplet).

Example 24

(±)-4-(2,2-Diphenyl-1,2,3,5,8,8a-hexahydroindolizin-7-yl)-2-(4-fluorophenyl)-3-(pyridin-4-yl)-1*H*-pyrrole (Compound No. 4-56)

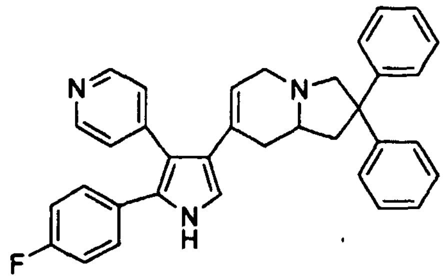

In a similar manner to the procedure described in Example 1(v) above, a reaction and silica gel column chromatography (using a 39:1 by volume mixture of methylene chloride and methanol as the eluant) were conducted, using (±)-2,2-diphenyl-1,2,3,5,6,7,8,8a-octahydroindolizin-7-one [prepared as described in J. Med. Chem., 31, 9, 1708-1712 (1988)] in place of (2R,8aS)-2-methoxy-1,2,3,5,6,7,8,8a-octahydro-indolizin-7-one, to give 363 mg (yield: 11%) of the title compound (Rf value = 0.50) as a pale brown powder.
Melting point: 224 - 227°C (decomposition)
$^1$H-NMR spectrum (400 MHz, DMSO-$d_6$) δ ppm:

11.39 (1H, broad singlet);
8.44 (2H, doublet, J=5 Hz);
7.33-7.07 (16H, multiplet);
6.93 (1H, doublet, J=3 Hz);
5.34-5.29 (1H, multiplet);
3.85 (1H, doublet, J=9 Hz);
3.42-3.32 (1H, multiplet);
2.86 (1H, doublet of doublets, J=13 Hz, 7 Hz);
2.75-2.65 (2H, multiplet);
2.49-2.39 (1H, multiplet);
2.33-2.24 (1H, multiplet);
2.16-2.03 (2H, multiplet).

Example 25

(±)-4-(2,2-Diphenyl-1,2,3,5,6,8a-hexahydroindolizin-7-yl)-2-(4-fluorophenyl)-3-(pyridin-4-yl)-1*H*-pyrrole (Compound No. 1-988)

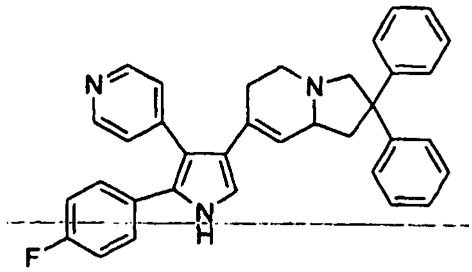

The silica gel column chromatography performed in Example 24 above also provided 0.50 g (yield: 15%) of

the title compound (Rf value = 0.30) as a pale brown powder.
Melting point: 241 - 244°C (decomposition)
$^1$H-NMR spectrum (400 MHz, DMSO-$d_6$) δ ppm:

11.38 (1H, broad singlet);
8.21 (2H, doublet, J=6 Hz);
7.36-7.28 (4H, multiplet);
7.25-7.08 (10H, multiplet);
7.02 (2H, doublet, J=6 Hz);
6.91 (1H, doublet, J=3 Hz);
5.24-5.21 (1H, multiplet);
3.62-3.55 (1H, multiplet);
3.53-3.47 (1H, multiplet);
3.17 (1H, doublet, J=6 Hz);
2.94-2.75 (3H, multiplet);
2.35-2.24 (1H, multiplet);
1.97-1.87 (1H, multiplet);
1.75 (1H, doublet of doublets, 13 Hz, 8 Hz).

Example 26

4-[(8aS)-2,2-Dimethyl-1,2,3,5,8,8a-hexahydroindolizin-7-yl]-2-(4-fluorophenyl)-3-(pyridin-4-yl)-1*H*-pyrrole (Compound No. 4-13)

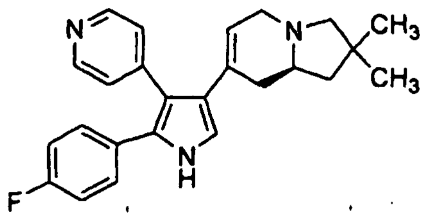

In a similar manner to the procedure described in Example 1(v) above, a reaction and silica gel column chromatography (using a 100:10:1 by volume mixture of ethyl acetate, methanol and isopropylamine respectively as the eluant) were conducted, using (8aS)-2,2-dimethyl-1,2,3,5,6,7,8,8a-octahydroindolizin-7-one (prepared as described in Preparative Example 17 below) in place of (2R,8aS)-2-methoxy-1,2,3,5,6,7,8,8a-octahydroindolizin-7-one, to give 0.85 g (yield: 32%) of the title compound (Rf value = 0.50) as a pale brown powder.
Melting point: 193 - 196°C (decomposition)
$^1$H-NMR spectrum (400 MHz, DMSO-$d_6$) δ ppm:

11.37 (1H, broad singlet);
8.44 (2H, doublet, J=6 Hz);
7.19-7.07 (6H, multiplet);
6.90 (1H, doublet, J=3 Hz);
5.27-5.22 (1H, multiplet);
3.27-3.18 (1H, multiplet);
2.78 (1H, doublet, J=9 Hz);
2.64-2.54 (1H, multiplet);
2.33-2.15 (2H, multiplet);
2.06-1.94 (1H, multiplet);
1.93-1.85 (1H, multiplet);
1.67 (1H, doublet of doublets, J=12 Hz, 7 Hz);
1.21-1.12 (1H, multiplet);
1.07 (3H, singlet);

1.02 (3H, singlet).

Example 27

4-[(8aS)-2,2-Dimethyl-1,2,3,5,6,8a-hexahydroindolizin-7-yl]-2-(4-fluorophenyl)-3-(pyridin-4-yl)-1*H*-pyrrole (Compound No. 1-301)

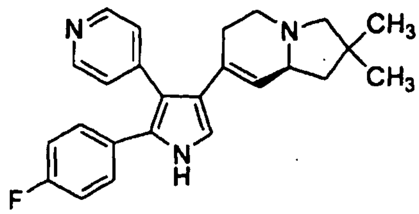

The silica gel column chromatography performed in Example 26 above also provided 0.47 g (yield: 18%) of the title compound (Rf value = 0.25) as a pale brown powder.
Melting point: 190 - 193°C (decomposition)
$^1$H-NMR spectrum (400 MHz, DMSO-$d_6$) δ ppm:

11.38 (1H, broad singlet);
8.43 (2H, doublet, J=6 Hz);
7.20-7.08 (6H, multiplet);
6.91 (1H, doublet, J=3 Hz);
5.15-5.11 (1H, multiplet);
3.28-3.19 (1H, multiplet);
2.95-2.86 (1H, multiplet);
2.66-2.55 (2H, multiplet);
2.36-2.21 (2H, multiplet);
2.00-1.92 (1H, multiplet);
1.56 (1H, doublet of doublets, J=12 Hz, 7 Hz);
1.10-0.98 (1H, multiplet);
1.05 (3H, singlet);
1.02 (3H, singlet).

Example 28

2-(4-Fluorophenyl)-4-[(2S,8aS)-2-methylthio-1,2,3,5,8,8a-hexahydroindolizin-7-yl]-3-(pyridin-4-yl)-1*H*-pyrrole (Compound No. 4-63)

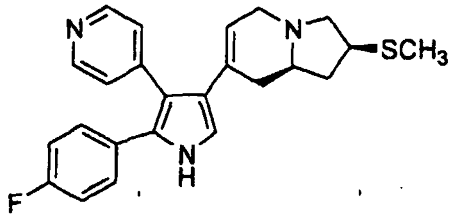

In a similar manner to the procedure described in Example 1 (v) above, a reaction and silica gel column chromatography (using a 100:1:1 by volume mixture of ethyl acetate, methanol and isopropylamine respectively as the

eluant) were conducted, using (2S,8aS)-2-methylthio-1,2,3,5,6,7,8,8a-octahydroindolizin-7-one (prepared as described in Preparative Example 12 below) in place of (2R,8aS)-2-methoxy-1,2,3,5,6,7,8,8a-octahydroindolizin-7-one, to give 1.51 g (yield: 17%) of the title compound (Rf value = 0.25) as a pale brown powder.
Melting point: 212 - 213°C (decomposition)
$^1$H-NMR spectrum (400 MHz, $CD_3OD$) δ ppm:

8.35 (2H, doublet of doublets, J=5 Hz, 1 Hz);
7.24 (2H, doublet of doublets, J=5 Hz, 1 Hz);
7.20-7.15 (2H, multiplet);
7.03-6.98 (2H, multiplet);
6.85 (1H, singlet);
5.38 (1H, triplet, J=2 Hz);
3.43-3.37 (1H, multiplet);
3.31-3.24 (1H, multiplet);
3.15 (1H, doublet of doublets, J=10 Hz, 3 Hz);
2.82-2.77 (1H, multiplet);
2.63 (1H, doublet of doublets, J=10 Hz, 9 Hz);
2.49-2.30 (3H, multiplet);
2.27-2.13 (1H, multiplet);
2.10 (3H, singlet);
1.37-1.29 (1H, multiplet).

Example 29

2-(4-Fluorophenyl)-4-[(2S,8aS)-2-methylthio-1,2,3,5,6,8a-hexahydroindolizin-7-yl]-3-(pyridin-4-yl)-1*H*-pyrrole (Compound No. 1-995)

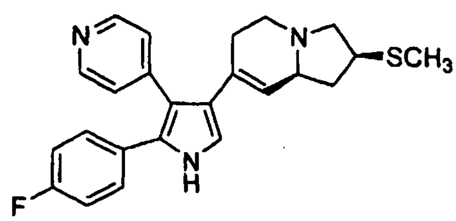

The silica gel column chromatography performed in Example 28 above also provided 1.03 g (yield: 12%) of the title compound (Rf value = 0.10) as a pale brown powder.
Melting point: 198 - 200°C (decomposition)
$^1$H-NMR spectrum (400 MHz, $CD_3OD$) δ ppm:

8.38 (2H, doublet of doublets, J=4 Hz, 1 Hz);
7.24 (2H, doublet of doublets, J=4 Hz, 1 Hz);
7.20-7.15 (2H, multiplet);
7.03-6.97 (2H, multiplet);
6.86 (1H, singlet);
5.28 (1H, doublet, J=2 Hz);
3.53-3.48 (1H, multiplet);
3.25 (1H, quintet, J=8 Hz);
3.14 (1H, doublet of doublets, J=10 Hz, 8 Hz);
3.08-3.03 (1H, multiplet);
2.87-2.78 (2H, multiplet);
2.40 (1H, doublet of double doublets, J=13 Hz, 8 Hz, 3 Hz);
2.36-2.30 (1H, multiplet);
2.17-2.16 (1H, multiplet);

2.14 (3H, singlet);
1.86 (1H, doublet of double doublets, J=13 Hz, 8 Hz, 3 Hz).

Example 30

2-(4-Fluorophenyl)-4-[(8aS)-2-methyl-3,5,8,8a-tetrahydroindolizin-7-yl]-3-(pyridin-4-yl)-1*H*-pyrrole (Compound No. 6-1)

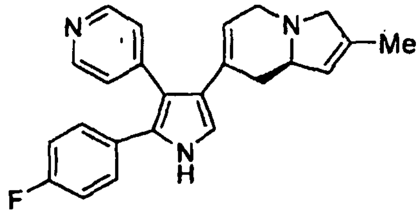

In a similar manner to the procedure described in Example 1(v) above, a reaction and silica gel column chromatography (using a 10:1:1 by volume mixture of ethyl acetate, methanol and isopropylamine respectively as the eluant) were conducted, using (8aS)-2-methyl-3,5,6,7,8,8a-hexahydroindolizin-7-one (prepared as described in Preparative Example 19 below) in place of (2R,8aS)-2-methoxy-1,2,3,5,6,7,8,8a-octahydroindolizin-7-one, to give 130 mg (yield: 3%) of the title compound (Rf value = 0.50) as a pale brown powder.
Melting point: 183 - 185°C (decomposition)
$^1$H-NMR spectrum (400 MHz, $CDCl_3$) δ ppm:

8.48 (2H, doublet, J=6 Hz);
8.37 (1H, broad singlet);
7.20-7.09 (4H, multiplet);
6.97 (2H, triplet, J=9 Hz);
6.83 (1H, doublet, J=3 Hz);
5.58-5.50 (1H, multiplet);
5.38-5.32 (1H, multiplet);
3.71-3.32 (4H, multiplet);
3.30-3.20 (1H, multiplet);
2.50-2.28 (2H, multiplet);
1.79 (3H, multiplet).

Example 31

2-(4-Fluorophenyl)-4-[(8aS)-2-methyl-3,5,6,8a-tetrahydroindolizin-7-yl]-3-(pyridin-4-yl)-1*H*-pyrrole (Compound No. 3-1)

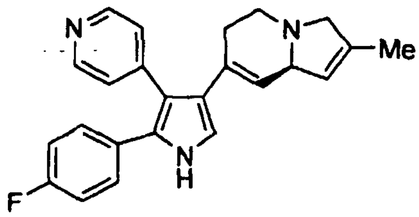

The silica gel column chromatography performed in Example 30 above also provided 190 mg (yield: 5%) of

the title compound (Rf value = 0.30) as a pale brown powder.
Melting point: 181 - 183°C (decomposition)
$^1$H-NMR spectrum (400 MHz, $CDCl_3$) δ ppm:

8.45 (2H, doublet, J=6 Hz);
8.37 (1H, broad singlet);
7.20-7.09 (4H, multiplet);
6.97 (2H, triplet, J=9 Hz);
6.81 (1H, doublet, J=3 Hz);
5.44 (1H, broad singlet);
5.24 (1H, broad singlet);
4.42-4.38 (1H, multiplet);
3.60-3.44 (2H, multiplet);
3.04-2.92 (2H, multiplet);
2.40-2.28 (1H, multiplet);
1.97-1.85 (1H, multiplet);
1.75 (3H, singlet).

Example 32

4-[(2S,8aS)-2-Ethyl-1,2,3,5,6,8a-hexahydroindolizin-7-yl)-2-(4-fluorophenyl)-3-(pyridin-4-yl)-1*H*-pyrrole (Compound No. 1-294)

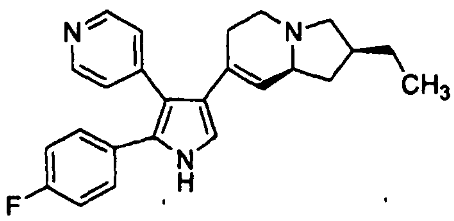

In a similar manner to the procedure described in Example 1(v) above, a reaction and silica gel column chromatography (using a 100:10:5 by volume mixture of ethyl acetate, methanol and isopropylamine respectively as the eluant) were conducted, using (2S,8aS)-2-ethyl-1,2,3,5,6,7,8,8a-octahydroindolizin-7-one (prepared as described in Preparative Example 27 below) in place of (2R,8aS)-2-methoxy-1,2,3,5,6,7,8,8a-octahydroindolizin-7-one, to give 1.12 g (yield: 21%) of the title compound (Rf value = 0.50) as a pale brown powder.
Melting point: 203 - 205°C (decomposition)
$^1$H-NMR spectrum (400 MHz, $CDCl_3$) δ ppm:

11.39-11.38 (1H, broad singlet);
8.43 (2H, doublet, J=6 Hz);
7.19-7.16 (2H, multiplet);
7.15-7.10 (4H, multiplet);
6.91 (1H, doublet, J=3 Hz);
5.13-5.12 (1H, broad singlet);
3.32-3.26 (2H, multiplet);
2.94-2.90 (1H, multiplet);
2.70-2.64 (2H, multiplet);
2.50-2.41 (1H, multiplet);
2.31-2.25 (1H, multiplet);
2.02-1.88 (3H, multiplet);
1.38-1.28 (2H, multiplet);

Example 33

4-[(2S,8aS)-2-Butylthio-1,2,3,5,8,8a-hexahydroindolizin-7-yl)-2-(4-fluorophenyl)-3-(pyridin-4-yl)-1*H*-pyrrole (Compound No. 4-66)

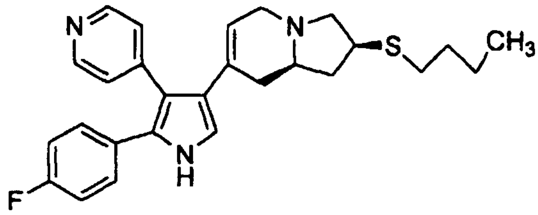

In a similar manner to the procedure described in Example 1(v) above, a reaction and silica gel column chromatography (using a 100:1:1 by volume mixture of ethyl acetate, methanol and isopropylamine respectively as the eluant) were conducted, using (2S,8aS)-2-butylthio-1,2,3,5,6,7,8,8a-octahydroindolizin-7-one (prepared as described in Preparative Example 14 below) in place of (2R,8aS)-2-methoxy-1,2,3,5,6,7,8,8a-octahydroindolizin-7-one, to give 426 mg (yield: 8%) of the title compound (Rf value = 0.25) as a pale pink powder.
Melting point: 189 - 190°C (decomposition)
$^1$H-NMR spectrum (400 MHz, $CD_3OD$) δ ppm:

8.35 (2H, doublet of doublets, J=5 Hz, 2 Hz);
7.24 (2H, doublet of doublets, J=5 Hz, 2 Hz);
7.15 (2H, doublet of doublets, J=5 Hz, 3 Hz);
7.03.6.97 (2H, multiplet);
6.85 (1H, singlet);
5.3 7 (1H, triplet, J=2 Hz);
3.42-3.33 (2H, multiplet);
3.13 (1H, doublet of doublets, J=10 Hz, 3 Hz);
2.83-2.77 (1H, multiplet);
2.66 (1H, triplet, J=10 Hz);
2.56 (2H, triplet, J=7 Hz);
2.53-2.30 (3H, multiplet);
2.21-2.13 (1H, multiplet);
1.57 (2H, quintet, J=8 Hz);
1.42 (2H, sextet, J=7 Hz);
1.36-1.29 (1H, multiplet);
0.92 (3H, triplet, J=7 Hz).

Example 34

4-[(2S,8aS)-2-Butylthio-1,2,3,5,6,8a-hexahydroindolizin-7-yl)-2-(4-fluorophenyl)-3-(pyridin-4-yl)-1*H*-pyrrole Compound No. 1-998)

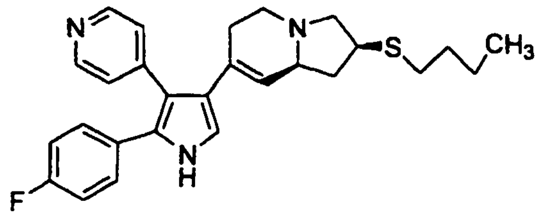

The silica gel column chromatography performed in Example 33 above also provided 612 mg (yield: 13%) of the title compound (Rf value = 0.10) as a pale brown powder.
Melting point: 199 - 200°C (decomposition)
$^1$H-NMR spectrum (400 MHz, $CD_3OD$) $\delta$ ppm:

8.37 (2H, doublet of doublets, J=5 Hz, 2 Hz);
7.23 (2H, doublet of doublets, J=5 Hz, 2 Hz);
7.17 (2H, doublet of doublets, J=6 Hz, 3 Hz);
7.03-6.97 (2H, multiplet);
6.86 (1H, singlet);
5.26 (1H, doublet, J=1 Hz);
3.53-3.49 (1H, multiplet);
3.14 (1H, doublet of doublets, J=10 Hz, 8 Hz);
3.08-3.03 (1H, multiplet);
2.88-2.76 (2H, multiplet);
2.59 (2H, triplet, J=7 Hz);
2.44-2.29 (2H, multiplet);
2.16-2.11 (1H, multiplet);
1.59 (2H, triplet of triplets, J=16 Hz, 7 Hz);
1.44 (2H, sextet, J=7 Hz);
1.35 (2H, triplet of triplets, J=13 Hz, 8 Hz);
1.24 (3H, triplet, J=7 Hz).

Example 35

4-[(2S,8aS)-2-Ethylthio-1,2,3,5,8,8a-hexahydroindolizin-7-yl]-2-(4-fluorophenyl)-3-(pyridin-4-yl)-1*H*-pyrrole (Compound No. 4-64)

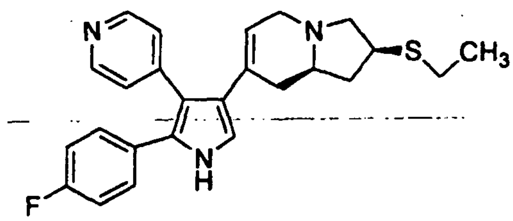

In a similar manner to the procedure described in Example 1(v) above, a reaction and silica gel column chromatography (using a 100:1:1 by volume mixture of ethyl acetate, methanol and isopropylamine respectively as the eluant) were conducted, using (2S,8aS)-2-ethylthio-1,2,3,5,6,7,8,8a-octahydroindolizin-7-one (prepared as described in Preparative Example 13 below) in place of (2R,8aS)-2-methoxy-1,2,3,5,6,7,8,8a-octahydroindolizin-7-one, to give 672 mg (yield: 24%) of the title compound (Rf value = 0.25) as a pale brown powder.
Melting point: 205 - 207°C (decomposition)
$^1$H-NMR spectrum (400 MHz, $CD_3OD$) $\delta$ ppm:

8.35 (2H, doublet of doublets, J=4 Hz, 1 Hz);
7.23 (2H, doublet of doublets, J=4 Hz, 1 Hz);
7.19-7.15 (2H, multiplet);
7.03-6.97 (2H, multiplet);
6.85 (1H, singlet);
5.38 (1H, triplet, J=2 Hz);
3.42-3.33 (2H, multiplet);
3.13 (1H, doublet of doublets, J=10 Hz, 3 Hz);
2.83-2.77 (1H, multiplet);
2.66 (1H, doublet of doublets, J=10 Hz, 8 Hz);
2.57 (2H, quartet, J=7 Hz);
2.50-2.33 (2H, multiplet);
2.31-2.30 (1H, multiplet);
2.21-2.13 (1H, multiplet);,
1.38-1.30 (1H, multiplet);
1.25 (3H, triplet, J=7 Hz).

Example 36

4-[(2S,8aS)-2-Ethylthio-1,2,3,5,6,8a-hexahydroindolizin-7-yl]-2-(4-fluorophenyl)-3-(pyridin-4-yl)-1*H*-pyrrole (Compound No. 1-996)

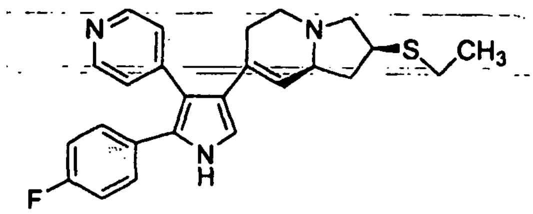

The silica gel column chromatography performed in Example 35 above also provided 563 mg (yield: 20%) of the title compound (Rf value = 0.10) as a pale pink powder.
Melting point: 193 - 196°C (decomposition)
$^1$H-NMR spectrum (400 MHz, $CD_3OD$) δ ppm:

8.37 (2H, doublet of doublets, J=4 Hz, 2 Hz);
7.23 (2H, doublet of doublets, J=4 Hz, 2 Hz);
7.21-7.15 (2H, multiplet);
7.02-6.98 (2H, multiplet);
6.86 (1H, singlet);
5.27 (1H, broad singlet);
3.54-3.49 (1H, multiplet);
3.32 (1H, quintet, J=8 Hz);
3.14 (1H, doublet of doublets, J=10 Hz, 8 Hz);
3.08-3.02 (1H, doublet of triplets, J=12 Hz, 5 Hz);
2.85 (1H, triplet of doublets, J=12 Hz, 5 Hz);
2.78 (1H, doublet of triplets, J=10 Hz, 8 Hz);
2.60 (2H, quartet, J=8 Hz);
2.41 (1H, doublet of triplets, J=12 Hz, 9 Hz);
2.36-2.29 (1H, multiplet);
2.17-2.11 (1H, multiplet);
1.35 (1H, doublet of triplets, J=13 Hz, 8 Hz);
1.27 (3H, triplet, J=8 Hz).

Example 37

4-[(8aS)-2-Ethylidene-1,2,3,5,6,8a-hexahydroindolizin-7-yl]- 2-(4-fluorophenyl)-3-(pyridin-4-yl)-1*H*-pyrrole (Compound No. 1-983)

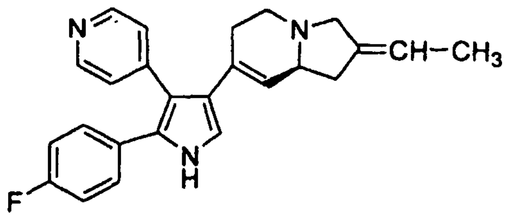

In a similar manner to the procedure described in Example 1(v) above, a reaction and silica gel column chromatography (using a 100:5:3 by volume mixture of ethyl acetate, methanol and isopropylamine respectively as the eluant) were conducted, using (8aS)-2-ethylidene-1,2,3,5,6,7,8,8a-octahydroindolizin-7-one (prepared as described in Preparative Example 24 below) in place of (2R,8aS)-2-methoxy-1,2,3,5,6,7,8,8a-octahydroindolizin-7-one, to give 474 mg (yield: 4%) of the title compound (Rf value = 0.50) as a white powder.
Melting point: 244 - 246°C (decomposition)
$^1$H-NMR spectrum (400 MHz, $CDCl_3$) δ ppm:

8.42 (2H, doublet, J=6 Hz);
7.18-7.12 (4H, multiplet);
7.10-7.07 (2H, multiplet);
6.93 (1H, doublet, J=3 Hz);
5.31-5.29 (1H, broad singlet);
5.18-5.16 (1H, broad singlet);
3.30-3.29 (1H, multiplet);
3.24-3.23 (1H, multiplet);
3.19-3.10 (1H, multiplet);
2.84-2.80 (1H, multiplet);
2.64-2.55 (1H, multiplet);
2.35-2.26 (2H, multiplet);
2.10-2.07 (1H, multiplet);
1.82-1.78 (1H, multiplet);
1.53 (3H, doublet, J=6 Hz).

Example 38

2-(4-Fluorophenyl)-4-[(8aS)-2,2-pronylenedioxy-1,2,3,5,8,8a-hexahydroindolizin-7-yl]-3-(pyridin-4-yl)-1*H*-pyrrole (Compound No. 4-57)

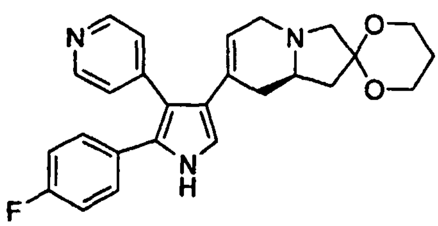

In a similar manner to the procedure described in Example 1(v) above, a reaction and silica gel column chromatography (using a 100:10:0.25 by volume mixture of ethyl acetate, methanol and isopropylamine respectively as the eluant) were conducted, using (8aS)-2,2-propylenedioxy-1,2,3,5,6,7,8,8a-octahydroindolizin-7-one (prepared as described in Preparative Example 21 below) in place of (2R,8aS)-2-methoxy-1,2,3,5,6,7,8,8a-octahydroindolizin-7-one, to give 2.11 g (yield: 29%) of the title compound (Rf value = 0.48) as a pale brown powder.
Melting point: 164 - 166°C (decomposition)
$^1$H-NMR spectrum (400 MHz, DMSO-$d_6$) δ ppm:

11.39 (1H, broad singlet);
8.45 (2H, doublet, J=6 Hz);
7.20-7.08 (6H, multiplet);
6.91 (1H, doublet, J=3 Hz);
5.27-5.22 (1H, multiplet);
3.86-3.69 (4H, multiplet);
3.39 (1H, doublet, J=10 Hz);
3.27-3.19 (1H, multiplet);
2.66-2.57 (1H, multiplet);
2.35-2.19 (3H, multiplet);
2.15 (1H, doublet, J=10 Hz);
2.06-1.96 (1H, multiplet);
1.62-1.54 (2H, multiplet);
1.50-1.42 (1H, multiplet).

Example 39

2-(4-Fluorophenyl)-4-[(8aS)-2,2-propylenedioxy-1,2,3,5,6,8a-hexahydroindolizin-7-yl]-3-(pyridin-4-yl)-1*H*-pyrrole (Compound No. 1-989)

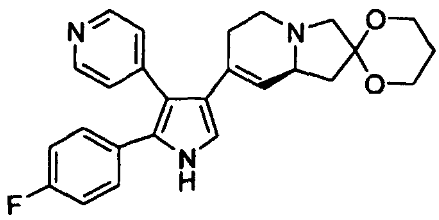

The silica gel column chromatography performed in Example 38 above also provided 1.38 g (yield: 19%) of the title compound (Rf value = 0.22) as a pale brown powder.
Melting point: 214 - 216°C (decomposition)
$^1$H-NMR spectrum (400 MHz, DMSO-$d_6$) δ ppm:

11.40 (1H, broad singlet);
8.43 (2H, doublet, J=6 Hz);
7.22-7.07 (6H, multiplet);
6.93 (1H, doublet, J=3 Hz);
5.20-5.16 (1H, multiplet);
3.85-3.70 (4H, multiplet);
3.14-3.04 (1H, multiplet);
3.07 (1H, doublet, J=10 Hz);
2.93-2.85 (1H, multiplet);
2.62 (1H, doublet, J=10 Hz);
2.54-2.44 (1H, multiplet);
2.33-2.21 (1H, multiplet);

2.16-2.04 (2H, multiplet);
1.68-1.44 (2H, multiplet);
1.47 (1H, doublet of doublets, J=13 Hz, 9 Hz).

Example 40

4-[(8aS)-2,2-(2',2'-Dimethylpropylenedioxy)-1,2,3,5,8,8a-hexahydroindolizin-7-yl]-2-(4-fluorophehyl)-3-(pyridin-4-yl)-1*H*-pyrrole (Compound No. 4-58)

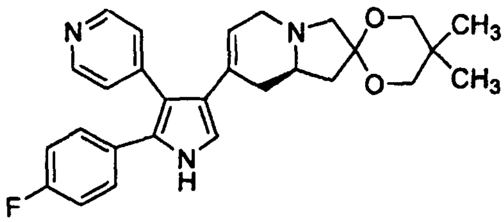

In a similar manner to the procedure described in Example 1(v) above, a reaction and silica gel column chromatography (using a 100:10:0.25 by volume mixture of ethyl acetate, methanol and isopropylamine respectively as the eluant) were conducted, using (8aS)-2,2-(2',2'-dimethylpropylenedioxy)-1,2,3,5,6,7,8,8a-octahydroindolizin-7-one (prepared as described in Preparative Example 22 below) in place of (2R,8aS)-2-methoxy-1,2,3,5,6,7,8,8a-octahydroindolizin-7-one, to give 1.85 g (yield: 26%) of the title compound (Rf value = 0.58) as a pale brown powder.
Melting point: 235 - 237°C (decomposition)
$^1$H-NMR spectrum (400 MHz, DMSO-$d_6$) δ ppm:

11.39 (1H, broad singlet);
8.45 (2H, doublet, J=6 Hz);
7.20-7.08 (6H, multiplet);
6.91 (1H, doublet, J=3 Hz);
5.27-5.22 (1H, multiplet);
3.47-3.30 (5H, multiplet);
3.26-3.18 (1H, multiplet);
2.66-2.58 (1H, multiplet);
2.36-2.19 (3H, multiplet);
2.16 (1H, doublet, J=10 Hz);
2.07-1.96 (1H, multiplet);
1.47 (1H, doublet of doublets, J=12 Hz, 10 Hz);
0.88 (6H, singlet).

Example 41

4-[(8aS)-2,2-(2',2'-Dimethylpropylenedioxy)-1,2,3,5,6,8a-hexahydroindolizin-7-yl]-2-(4-fluorophenyl)-3-(pyridin-4-yl)-1*H*-pyrrole (Compound No. 1-990)

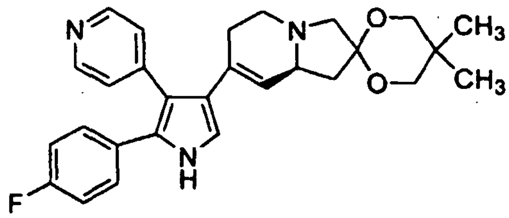

The silica gel column chromatography performed in Example 40 above also provided 1.37 g (yield: 19%) of the title compound (Rf value = 0.20) as a white powder.
Melting point: 235 - 237°C (decomposition)
$^1$H-NMR spectrum (400 MHz, DMSO-$d_6$) δ ppm:

11.40 (1H, broad singlet);
8.43 (2H, doublet, J=6 Hz);
7.19-7.08 (6H, multiplet);
6.93 (1H, doublet, J=3 Hz);
5.19-5.15 (1H, multiplet);
3.47-3.29 (4H, multiplet);
3.14-3.05 (2H, multiplet);
2.93-2.85 (1H, multiplet);
2.63 (1H, doublet, J= 10 Hz);
2.54-2.45 (1H, multiplet);
2.33-2.22 (1H, multiplet);
2.14-2.04 (2H, multiplet);
1.46 (1H, doublet of doublets, J=13 Hz, 9 Hz);
0.91 (3H, singlet);
0.85 (3H, singlet).

Example 42

2-(4-Fluorophenyl)-4-[(2S,8aS)-2-propyl-1,2,3,5,8,8a-hexahydroindolizin-7-yl]-3-(pyridin-4-yl)-1*H*-pyrrole (Compound No. 4-7)

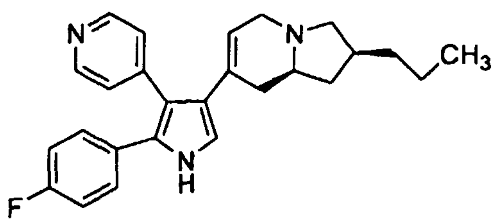

In a similar manner to the procedure described in Example 1(v) above, a reaction and silica gel column chromatography (using a 100:5:1 by volume mixture of ethyl acetate, methanol and isopropylamine respectively as the eluant) were conducted, using (2S,8aS)-2-propyl-1,2,3,5,6,7,8,8a-octahydroindolizin-7-one (prepared as described in Preparative Example 28 below) in place of (2R,8aS)-2-methoxy-1,2,3,5,6,7,8,8a-octahydroindolizin-7-one, to give 684 mg (yield: 5%) of the title compound (Rf value = 0.60) as a pale yellow powder.
Melting point: 205 - 206°C (decomposition)
$^1$H-NMR spectrum (400 MHz, $CDCl_3$) δ ppm:

11.36-11.35 (1H, broad singlet);
8.44 (2H, doublet, J=6 Hz);

7.17-7.14 (4H, multiplet);
7.13-7.09 (2H, multiplet);
6.90 (1H, doublet, J=3 Hz);
5.25-5.24 (1H, broad singlet);
3.36-3.30 (1H, multiplet);
3.27-3.22 (1H, multiplet);
2.74-2.72 (1H, multiplet);
2.61-2.51 (1H, multiplet);
2.23-2.10 (3H, multiplet);
2.07-1.97 (3H, multiplet);
1.38-1.20 (4H, multiplet);
0.86 (3H, triplet, J=7 Hz).

Example 43

2-(4-Fluorophenyl)-4-[(2S,8aS)-2-propyl-1,2,3,5,6,8a-hexahydroindolizin-7-yl]-3-(pyridin-4-yl)-1*H*-pyrrole Compound No. 1-295)

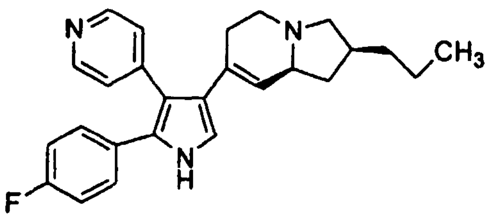

The silica gel column chromatography performed in Example 42 above also provided 359 mg (yield: 3%) of the title compound (Rf value = 0.50) as a pale yellow powder.
Melting point: 202 - 203°C (decomposition)
$^1$H-NMR spectrum (400 MHz, $CDCl_3$) $\delta$ ppm:

11,38-11.37 (1H, broad singlet);
8.42 (2H, doublet, J=6 Hz);
7.18-7.13 (4H, multiplet);
7.12-7.09 (2H, multiplet);
6.91 (1H, doublet, J=3 Hz);
5.12-5.11 (1H, broad singlet);
3.31-3.23 (1H, multiplet);
2.92-2.88 (1H, multiplet);
2.67-2.61 (2H, multiplet);
2.51-2.49 (1H, multiplet);
2.42-2.3 8 (1H, multiplet);
2.30-2.25 (1H, multiplet);
2.02-1.89 (3H, multiplet);
1.36-1.22 (4H, multiplet);
0.88 (3H, doublet, J=7 Hz).

Example 44

4-[(2R,8aS)-2-Ethoxy-1,2,3,5,8,8a-hexahydroindolizin-7-yl]-2-(4-fluorophenyl)-3-(pyridin-4-yl)-1*H*-pyrrole (Compound No. 4-16)

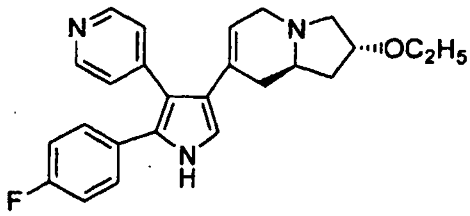

In a similar manner to the procedure described in Example 1(v) above, a reaction and silica gel column chromatography (using a 10:1:1 by volume mixture of ethyl acetate, methanol and isopropylamine respectively as the eluant) were conducted, using (2R,8aS)-2-ethoxy-1,2,3,5,6,7,8,8a-octahydroindolizin-7-one (prepared as described in Preparative Example 20 below) in place of (2R,8aS)-2-methoxy-1,2,3,5,6,7,8,8a-octahydroindolizin-7-one, to give 285 mg (yield: 9%) of the title compound (Rf value = 0.65) as a pale brown powder.
Melting point: 194 - 196°C (decomposition)
$^1$H-NMR spectrum (400 MHz, $CDCl_3$) δ ppm:

8.46 (2H, doublet, J=6 Hz);
8.23 (1H, broad singlet);
7.16 (2H, doublet, J=6 Hz);
7.12 (2H, doublet of doublets, J=9 Hz, 5 Hz);
6.97 (2H, triplet, J=9 Hz);
6.82 (1H, doublet, J=3 Hz);
5.51-5.47 (1H, multiplet);
4.14-4.08 (1H, multiplet);
3.62-3.53 (1H, multiplet);
3.50-3.36 (3H, multiplet);
2.93-2.82 (1H, multiplet);
2.59-2.46 (1H, multiplet);
2.32-2.15 (2H, multiplet);
2.14-2.01 (1H, multiplet);
2.00-1.92 (1H, multiplet);
1.70-1.60 (1H, multiplet);
1.19 (3H, triplet, J=7 Hz).

Example 45

4-[(2R,8aS)-2-Ethoxy-1,2,3,5,6,8a-hexahydroindolizin-7-yl]-2-(4-fluorophenyl)-3-(pyridin-4-yl)-1*H*-pyrrole (Compound No. 1-304)

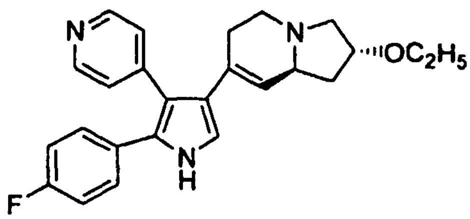

The silica gel column chromatography performed in Example 44 above also provided 231 mg (yield: 7%) of the title compound (Rf value = 0.60) as a pale brown powder.
Melting point: 192 - 195°C (decomposition)
$^1$H-NMR spectrum (400 MHz, $CDCl_3$) δ ppm:

8.47 (2H, doublet, J=6 Hz);

8.25 (1H, broad singlet);
7.20-7.09 (4H, multiplet);
6.97 (2H, triplet, J=9 Hz);
6.82 (1H, doublet, J=3 Hz);
5.40 (1H, doublet, J=2 Hz);
4.10-4.02 (1H, multiplet);
3.50-3.34 (3H, multiplet);
3.16 (1H, doublet of doublets, J=11 Hz, 6 Hz);
2.99-2.90 (1H, multiplet);
2.80-2.65 (2H, multiplet);
2.46-2.30 (1H, multiplet);
2.18-2.04 (1H, multiplet);
1.96-1.88 (1H, multiplet);
1.69-1.60 (1H, multiplet);
1.19 (3H, triplet, J=7 Hz).

Example 46

(±)-4-[Cyclopentanespiro-2'-(1',2',3',5',8',8a'-hexahydroindolizin)-7'-yl]-2-(4-fluorophenyl)-3-(pyridin-4-yl)-1*H*-pyrrole (Compound No. 4-61)

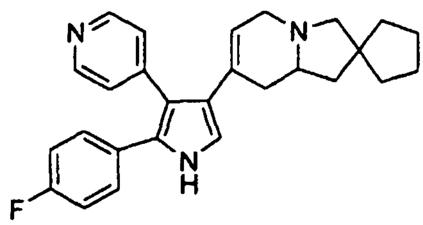

In a similar manner to the procedure described in Example 1(v) above, a reaction and silica gel column chromatography (using a 100:10:0.25 by volume mixture of ethyl acetate, methanol and isopropylamine respectively as the eluant) were conducted, using (±)-cyclopentanespiro-2'-(1',2',3',5',6',7',8',8a'-octahydroindolizin)-7'-one (prepared as described in Preparative Example 18 below) in place of (2R,8aS)-2-methoxy-1,2,3,5,6,7,8,8a-octahydroindolizin-7-one, to give 2.05 g (yield: 29%) of the title compound (Rf value = 0.53) as a pale brown powder.
Melting point: 206 - 208°C (decomposition)
$^1$H-NMR spectrum (400 MHz, DMSO-$d_6$) δ ppm:

11.37 (1H, broad singlet);
8.45 (2H, doublet, J=5 Hz);
7.20-7.07 (6H, multiplet);
6.90 (1H, doublet, J=3 Hz);
5.27-5.22 (1H, multiplet);
3.28-3.20 (1H, multiplet);
2.90 (1H, doublet, J=9 Hz);
2.64-2.55 (1H, multiplet);
2.29-2.17 (2H, multiplet);
2.05-1.94 (2H, multiplet);
1.80 (1H, doublet of doublets, J=12 Hz, 6 Hz);
1.64-1.42 (8H, multiplet);
1.26 (1H, doublet of doublets, J=12 Hz, 10 Hz).

Example 47

(±)-4-[Cyclopentanespiro-2'-(1',2',3',5',6',8a'-hexahydroindolizin)-7'-yl]-2-(4-fluorophenyl)-3-(pyridin-4-yl)-1*H*-pyrrole (Compound No. 1-993)

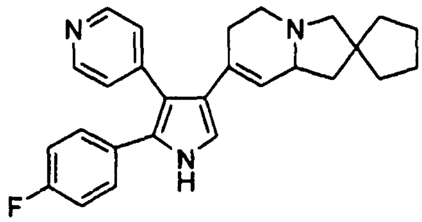

The silica gel column chromatography performed in Example 46 above also provided 1.31 g (yield: 19%) of the title compound (Rf value = 0.19) as a pale brown powder.
Melting point: 202 - 204°C (decomposition)
$^1$H-NMR spectrum (400 MHz, DMSO-$d_6$) δ ppm:

11.38 (1H, broad singlet);
8.43 (2H, doublet, J=6 Hz);
7.20-7.08 (6H, multiplet);
6.91 (1H, doublet, J=3 Hz);
5.17-5.13 (1H, multiplet);
3.25-3.18 (1H, multiplet);
2.92-2.85 (1H, multiplet);
2.70 (1H, doublet, J=9 Hz);
2.65-2.56 (1H, multiplet);
2.37 (1H, doublet, J=9 Hz);
2.34-2.23 (1H, multiplet);
2.01-1.92 (1H, multiplet);
1.69 (1H, doublet of doublets, J=12 Hz, 7 Hz);
1.61-1.39 (8H, multiplet);
1.15 (1H, doublet of doublets, J=12 Hz, 8 Hz).

Example 48

4-[(2S,8aS)-2-Benzyl-1,2,3,5,6,8a-hexahydroindolizin-7-yl]-2-(4-fluorophenyl)-3-(pyridin-4-yl)-1*H*-pyrrole (Compound No. 1-299)

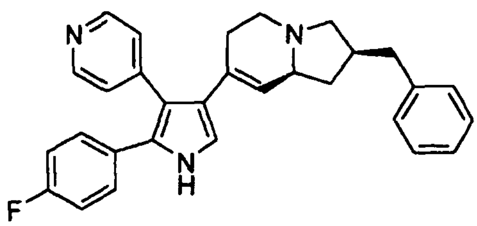

In a similar manner to the procedure described in-Example 1(v) above, a reaction and silica gel column chromatography (using a 100:10:2 by volume mixture of ethyl acetate, methanol and isopropylamine respectively as the eluant) were conducted, using (2S,8aS)-2-benzyl-1,2,3,5,6,7,8,8a-octahydroindolizin-7-one (prepared as described in

Preparative Example 29 below) in place of (2R,8aS)-2-methoxy-1,2,3,5,6,7,8,8a-octahydroindolizin-7-one, to give 252 mg (yield: 11%) of the title compound (Rf value = 0.50) as a pale brown powder.
Melting point: 207 - 209°C (decomposition)
$^1$H-NMR spectrum (400 MHz, $CDCl_3$) δ ppm:

11.39-11.38 (1H, broad singlet);
8.46 (2H, doublet, J=6 Hz);
7.28 (2H, triplet, J=8 Hz);
7.20-7.11 (9H, multiplet);
6.92 (1H, doublet, J=3 Hz);
5.14-5.13 (1H, broad singlet);
3.39-3.21 (2H, multiplet);
2.91-2.83 (1H, multiplet);
2.65-2.49 (5H, multiplet);
2.32-2.29 (2H, multiplet);
1.99-1.87 (2H, multiplet).

Example 49

4-[(8aS)-2-Benzylidene-1,2,3,5,6,8a-hexahydroindolizin-7-yl]-2-(4-fluorophenyl)-3-(pyridin-4-yl)-1*H*-pyrrole (Compound No. 1-987)

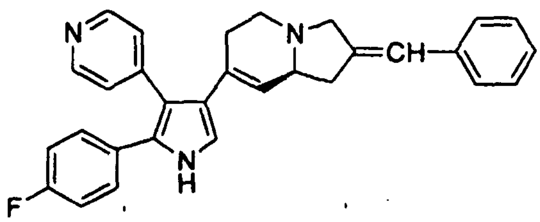

In a similar manner to the procedure described in Example 1(v) above, a reaction and silica gel column chromatography (using a 100:10:2 by volume mixture of ethyl acetate, methanol and isopropylamine respectively as the eluant) were conducted, using (8aS)-2-benzylidene-1,2,3,5,6,7,8,8a-octahydroindolizin-7-one (prepared as described in Preparative Example 26 below) in place of (2R,8aS)-2-methoxy-1,2,3,5,6,7,8,8a-octahydroindolizin-7-one, to give 17 mg (yield: 3%) of the title compound (Rf value = 0.50) as a pale brown powder.
Melting point: 243 - 245°C (decomposition)
$^1$H-NMR spectrum (400 MHz, $CDCl_3$) δ ppm:

8.41 (2H, doublet, J=6 Hz);
8.31-8.29 (1H, broad singlet);
7.38-7.27 (4H, multiplet);
7.21(1H, triplet, J=7 Hz);
7.15-7.10 (4H, multiplet);
6.97 (2H, triplet, J=9 Hz);
6.84 (1H, doublet, J=3 Hz);
6.40 (1H, singlet);
5.50-5.49 (1H, broad singlet);
3.65-3.58 (3H, multiplet);
2.96-2.93 (1H, multiplet);
2.85-2.78 (2H, multiplet);
2.47-2.42 (1H, multiplet);

Example 50

2-(4-Fluorophenyl)-4-[(2S,8aS)-2-phenoxy-1,2,3,5,8,8a-hexahydroindolizin-7-yl]-3-(pyridin-4-yl)-1*H*-pyrrole (Compound No. 4-68)

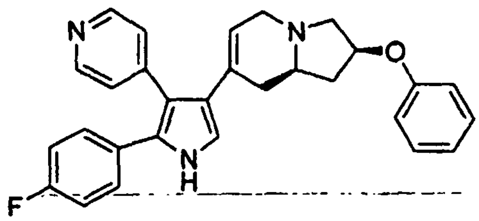

In a similar manner to the procedure described in Example 1(v) above, a reaction and silica gel column chromatography (using a 19:1 by volume mixture of ethyl acetate and methanol as the eluant) were conducted, using (2S, 8aS)-2-phenoxy-1,2,3,5,6,7,8,8a-octahydroindolizin-7-one (prepared as described in Preparative Example 23 below) in place of (2R,8aS)-2-methoxy-1,2,3,5,6,7,8,8a-octahydroindolizin-7-one, to give 2.00 g (yield: 33%) of the title compound (Rf value = 0.63) as a white powder.
Melting point: 212 - 214°C (decomposition)
$^1$H-NMR spectrum (400 MHz, DMSO-$d_6$) δ ppm:

11.40 (1H, broad singlet);
8.45 (2H, doublet, J=6 Hz);
7.27 (2H, triplet, J=8 Hz);
7.21-7.08 (6H, multiplet);
6.96-6.82 (4H, multiplet);
5.28-5.24 (1H, multiplet);
4.88-4.80 (1H, multiplet);
3.34-3.27 (1H, multiplet);
3.19-3.11 (1H, multiplet);
2.70-2.50 (2H, multiplet);
2.46-2.05 (4H, multiplet);

Example 51

2-(4-Fluorophenyl)-4-[(2S,8aS)-2-phenoxy-1,2,3,5,6,8a-hexahydroindolizin-7-yl]-3-(pyridin-4-yl)-1*H*-pyrrole (Compound No. 1-1000)

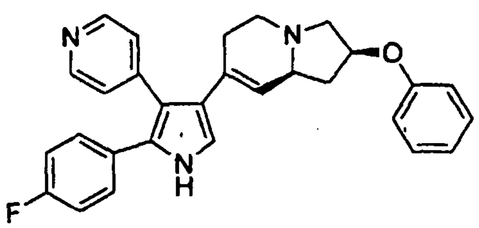

The silica gel column chromatography performed in Example 50 above also provided 0.90 g (yield: 15%) of the title compound (Rf value = 0.10) as a pale brown powder.
Melting point: 199 - 201 °C (decomposition)
$^1$H-NMR spectrum (400 MHz, DMSO-$d_6$) δ ppm:

11.41 (1H, broad singlet);
8.33 (2H, doublet, J=6 Hz);
7.29 (2H, triplet, J=8 Hz);
7.21-7.08 (6H, multiplet);
6.97-6.86 (4H, multiplet);
5.28-5.24 (1H, multiplet);
4.88-4.82 (1H, multiplet);
3.21-3.14 (1H, multiplet);
3.04-2.91 (3H, multiplet);
2.67-2.57 (1H, multiplet);
2.44-2.35 (1H, multiplet);
2.33-2.23 (1H, multiplet);
2.11-2.02 (1H, multiplet);
1.44-1.36 (1H, multiplet).

Example 52

2-(4-Fluoronhenyl)-4-[(2S,8aS)-2-methylsulfonyl-1,2,3,5,8,8a-hexahydroindolizin-7-yl]-3-(pyridin-4-yl)-1*H*-pyrrole (Compound No. 4-67)

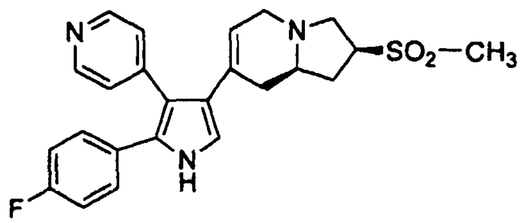

In a similar manner to the procedure described in Example 1(v) above, a reactiori and silica gel column chromatography (using a 100:1:1 by volume mixture of ethyl acetate, methanol and isopropylamine respectively as the eluant) were conducted, using (2S,8aS)-2-methylsulfonyl-1,2,3,5,6,7,8,8a-octahydroindolizin-7-one (prepared as described in Preparative Example 15 below) in place of (2R,8aS)-2-methoxy-1,2,3,5,6,7,8,8a-octahydroindolizin-7-one, to give 30 mg (yield: 2%) of the title compound (Rf value = 0.2) as a brown powder.
Melting point: >250°C (decomposition)
$^1$H-NMR spectrum (400 MHz, $CD_3OD$) δ ppm:

8.40-8.36 (2H, multiplet);
7.25 (2H, doublet, J=5 Hz);
7.22-7.15 (2H, multiplet);
7.05-6.98 (2H, multiplet);
6.87 (1H, singlet);
5.39 (1H, triplet, J=2 Hz);
3.81-3.74 (1H, multiplet);
3.59 (1H, doublet of doublets, J=11 Hz, 3 Hz);
3.49-3.44 (1H, multiplet);
2.93 (3H, singlet);
2.90-2.83 (1H, multiplet);
2.67 (1H, triplet, J=11 Hz);
2.57-2.27 (3H, multiplet);
2.27-2.20 (1H, multiplet);
1.88-1.80 (1H, multiplet).

Example 53

2-(4-Fluorophenyl)-4-[(2S,8aS)-2-methylsulfonyl-1,2,3,5,6,8a-hexahydroindolizin-7-yl]-3-(pyridin-4-yl)-1*H*-pyrrole (Compound No. 1-999)

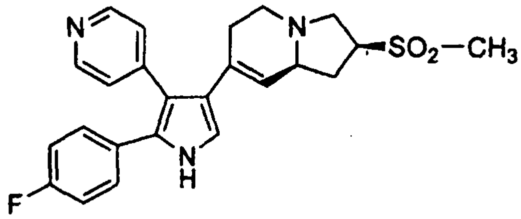

The silica gel column chromatography performed in Example 52 above also provided 46 mg (yield: 2%) of the title compound (Rf value = 0.05) as a brown powder.
Melting point: 147 - 150°C (decomposition)
$^1$H-NMR spectrum (400 MHz, $CD_3OD$) δ ppm:

8.39-8.35 (2H, multiplet);
7.24 (2H, doublet, J=5 Hz);
7.19-7.16 (2H, multiplet);
7.03-6.87 (2H, multiplet);
6.86 (1H, singlet);
5.38 (1H, triplet, J=2 Hz);
3.69-3.65 (1H, multiplet);
3.62-3.56 (1H, multiplet);
3.44-3.38 (1H, multiplet);
2.92 (3H, singlet);
2.82-2.78 (1H, multiplet);
2.63-2.53 (1H, multiplet);
2.50-2.39 (1H, multiplet);
2.36-2.15 (3H, multiplet);
1.92-1.76 (1H, multiplet).

Example 54

2-(4-Fluorophenyl)-4-[(8aS)-2-propylidene-1,2,3,5,6,8a-hexahydroindolizin-7-yl]-3-(pyridin-4-yl)-1*H*-pyrrole (Compound No. 1-984)

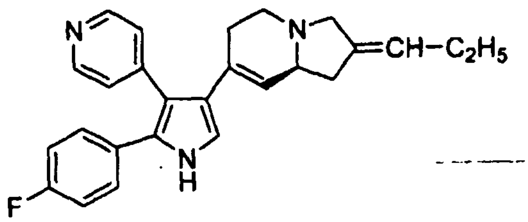

In a similar manner to the procedure described in Example 1(v) above, a reaction and silica gel column chromatography (using a 100:5:1 by volume mixture of ethyl acetate, methanol and isopropylamine respectively as the eluant) were conducted, using (8aS)-2-propylidene-1,2,3,5,6,7,8,8a-octahydroindolizin-7-one (prepared as described in Preparative Example 25 below) in place of (2R,8aS)-2-methoxy-1,2,3,5,6,7,8,8a-octahydroindolizin-7-one, to give the title compound as two gemoetric isomers, the E-form and Z-form:

Isomer A
Rf value = 0.50, 0.56 g (yield: 4%) of an orange powder
Melting point: 185 - 187°C (decomposition)
$^1$H-NMR spectrum (400MHz, $CDCl_3$) δ ppm:

11.41-11.40 (1H, broad singlet);
8.41 (2H, doublet, J=6 Hz);
7.18-7.1 (4H, multiplet);
7.10-7.07 (2H, multiplet);
6.93 (1H, doublet, J=3 Hz);
5.23-5.18 (1H, multiplet);
5.14-5.13 (1H, broad singlet);
3.41-3.36 (1H, multiplet);
3.22-3.13 (2H, multiplet);
2.93-2.89 (1H, multiplet);
2.68-2.66 (1H, multiplet);
2.40-2.29 (2H, multiplet);
2.10-2.06 (1H, multiplet);
1.97-1.87 (2H, multiplet);
1.57-1.55 (1H, multiplet);
0.94 (3H, doublet, J=7 Hz).

Isomer B
Rf value = 0.45, 1.58 g of a white powder (yield: 11%)
Melting point: 249 - 251 °C (decomposition)
$^1$H-NMR spectrum (400MHz, $CDCl_3$) δ ppm:

11.47-11.46 (1H, broad singlet);
8.42 (2H, doublet, J=6 Hz);
7.19-7.14 (4H, multiplet);
7.13-7.07 (2H, multiplet);
6.92 (1H, doublet, J=3 Hz);
5.27-5.23 (1H, multiplet);
5.18-5.17 (1H, broad singlet);
4.14 (1H, quartet, J=5 Hz);
3.24-3.21 (1H, multiplet);
3.13-3.10 (1H, multiplet);
2.85-2.80 (1H, multiplet);
2.63-2.58 (1H, multiplet);
2.37-2.31 (2H, multiplet);
2.11-2.07 (1H, multiplet);
1.95-1.90 (2H, multiplet);
1.88-1.78 (1H, multiplet);
0.92 (3H, doublet, J=8 Hz).

Preparative Examples

Preparative Example 1

(2R,8aS)-2-Methoxy-1,2,3,5,6,7,8,8a-octahydroindolizin-7-one

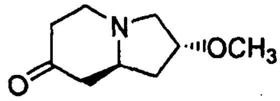

1(i) (2S,4R)-1-Benzyloxycarbonyl-2-cyanomethyl-4-methoxypyrrolidine

17.9 ml (17.9 mmol) of a 1M solution of borane-tetrahydrofuran complex in tetrahydrofuran were added at 0°C with stirring to a solution of 2.00g (7.16 mmol) of (2S,4R)-1-benzyloxycarbonyl-4-methoxyproline in 20 ml of tetrahydrofuran. The resulting mixture was stirred at 0°C for 1 hour and then at room temperature for 3 hours, at the end of which time the reaction mixture was cooled to 0°C again. Methanol was then added carefully to the cooled mixture, and the mixture was then concentrated by evaporation under reduced pressure. A saturated aqueous solution of sodium hydrogencarbonate was added to the residue thus obtained, and the mixture was extracted with ethyl acetate. The organic extract was washed with water, dried over anhydrous magnesium sulfate and then concentrated by evaporation under reduced pressure to afford 1.82 g of the reduced product, an alcohol derivative, as a brown oil.

1.13 ml (8.14 mmol) of triethylamine were added to a solution of the oil obtained above in 25 ml of dichloromethane, and then 0.58 ml (7.46 mmol) of methanesulfonyl chloride were added to the ice-cooled mixture with stirring. After stirring at the same temperature for 30 minutes, a saturated aqueous solution of sodium hydrogencarbonate was added, and the mixture was extracted with dichloromethane. The organic extract was washed with water, dried over anhydrous magnesium sulfate and then concentrated by evaporation under reduced pressure to afford 2.19 g of the mesylated derivative as a brown oil.

0.31 g (6.32 mmol) of sodium cyanide were added to a solution of the mesylated derivative obtained above in 22 ml of dimethyl sulfoxide, and the resulting mixture was stirred at 100°C for 30 minutes. At the end of this time, a saturated aqueous solution of sodium hydrogencarbonate was added to the reaction mixture, and the mixture was extracted with ethyl acetate. The organic extract was washed with water, dried over anhydrous sodium sulfate and then concentrated under reduced pressure to afford 1.70 g (yield: 88%) of the title compound as a pale brown oil.
$^1$H-NMR spectrum (400 MHz, $CDCl_3$) δ ppm:

7.43-7.28 (5H, multiplet);
5.26-5.08 (2H, multiplet);
4.20-4.10 (1H, multiplet);
4.03-3.93 (1H, multiplet);
3.90 (0.4H, doublet, J=12 Hz);
3.74 (0.6H, doublet, J=12 Hz);
3.56-3.44 (1H, multiplet);
3.31 (1.2H, singlet);
3.30 (1.8H, singlet);
3.16 (0.6H, doublet of doublets, J=17 Hz, 6 Hz);
2.80 (0.4H, doublet of doublets, J=17 Hz, 6 Hz);
2.76-2.58 (1H, multiplet);
2.39-2.30 (1H, multiplet);
2.08-1.97 (1H, multiplet).

1(ii) (2S,4R)-1-Benzyloxycarbonyl-2-(3-ethoxycarbonyl-2-oxopronyl)-4-methoxypyrrolidine

0.5 ml (4.51 mmol) of ethyl bromoacetate were added at 90°C to a suspension of 57.19 g (875 mmol) of zinc powder in 600 ml of tetrahydrofuran and the resulting reaction mixture was heated under reflux for 1 hour. A solution of 30.00 g (109 mmol) of (2S,4R)-1-benzyloxycarbonyl-2-cyanomethyl-4-methoxypyrrolidine [prepared as described in Preparative Example 1(i) above] in 30 ml of tetrahydrofuran and 84.9 ml (766 mmol) of ethyl bromoacetate were added successively to this reaction mixture, and the resulting mixture was then heated under reflux for a further 1.5 hours. After being cooled to room temperature, the reaction mixture was filtrated and the filtrate was concentrated by evaporation under reduced pressure. The residue thus obtained was dissolved in ethyl acetate and washed with a saturated aqueous solution of sodium hydrogencarbonate. The organic extract was washed with water, dried over anhydrous magnesium sulfate and then concentrated by evaporation under reduced pressure. The residue thus obtained was dissolved in a mixture of 200 ml of dioxane and 100 ml of a 1N aqueous solution of hydrochloric acid and then allowed to stand at room temperature for 3 hours. At the end of this time, water was added to the reaction mixture which was then extracted with ethyl acetate. The organic extract was washed with water and then concentrated by evaporation under reduced

pressure. The resulting residue was purified by chromatography on a silica gel column using a 2:3 by volume mixture of ethyl acetate and hexane as the eluant to afford 28.23 g (yield: 71%) of the title compound as a pale yellow oil.
$^1$H-NMR spectrum (400 MHz, $CDCl_3$) δ ppm:

7.43-7.27 (5H, multiplet);
5.24-5.06 (2H, multiplet);
4.33-4.10 (3H, multiplet);
3.93-3.86 (1H, multiplet);
3.78 (0.4H, doublet, J=12 Hz);
3.65 (0.6H, doublet, J=12 Hz);
3.52-3.24 (3.6H, multiplet);
3.29 (3H, singlet);
3.14-3.05 (0.4H, multiplet);
2.80-2.62 (1H, multiplet);
2.42-2.32 (1H, multiplet);
1.84-1.73 (1H, multiplet);
1.34-1.21 (3H, multiplet).

1(iii) (2R,8aS)-2-Methoxy-1,2,3,5,6,7,8,8a-octahydroindolizin-7-one

3.79 ml (45.4 mmol) of pyrrolidine, 1.50 g of molecular sieves (MS4A) and 3.75 g of 20% palladium hydroxide on carbon were added to a solution of 15.00 g (41.3 mmol) of (2S,4R)-1-benzyloxycarbonyl-2-(3-ethoxycarbonyl-2-oxopropyl)-4-methoxypyrrolidine [prepared as described in Preparative Example 1(ii) above] in 150 ml of ethyl acetate and the mixture was then stirred for 2 hours at room temperature under a hydrogen atmosphere. At the end of this time, the reaction mixture was filtered and the filtrate was concentrated by evaporation under reduced pressure. The resulting residue was dissolved in 150 ml of tetrahydrofuran, and the solution thus obtained was added dropwise with stirring to an ice-cooled suspension of 4.70 g (124 mmol) of lithium aluminum hydride in 100 ml of tetrahydrofuran, and the reaction mixture was then stirred for a further 18 hours at room temperature. At the end of this time, 19 ml of a 4% aqueous solution of sodium hydroxide were added carefully to the reaction mixture at 0°C, and after the addition of 250 ml of ethanol, the resulting mixture was filtered. The filtrate thus obtained was concentrated by evaporation under reduced pressure and the residue was purified by chromatography on an alumina column using ethyl acetate as the eluant to afford 4.13 g (yield: 59%) of the title compound as a pale brown powder.
$^1$H-NMR spectrum (400 MHz, $CDCl_3$) δ ppm:

4.12-4.04 (1H, multiplet);
3.54 (1H, doublet of doublets, J=10 Hz, 7 Hz);
3.34-3.24 (1H, multiplet);
3.29 (3H, singlet);
2.63-2.30 (5H, multiplet);
2.29-2.19 (2H, multiplet);
2.00 (1H, doublet of double doublets, J=13 Hz, 6 Hz, 1 Hz);
1.79-1.67 (1H, multiplet).

Preparative Example 2

(2R,8aS)-2-(t-Butyldimethylsilyloxy)-1,2,3,5,6,7,8,8a-octahydroindolizin-7-one

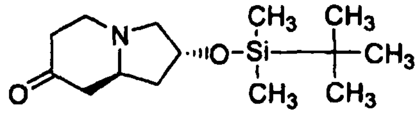

2(i) (2R,8aS)-2-Hydroxy-1,2,3,5,6,7,8,8a-octahydroindolizin-7-one

2.63 g (15.5 mmol) of (2R,8aS)-2-methoxy-1,2,3,5,6,7,8,8a-octahydroindolizin-7-one [prepared as described in the Preparative Example 1 above] were dissolved in 26 ml of a 47% aqueous solution of hydrobromic acid and the resulting mixture was stirred for 8 hours at 100°C. After being cooled to 0°C, the reaction mixture was neutralized by the addition of sodium carbonate and then concentrated by evaporation under reduced pressure. Ethanol was added to the residue thus obtained and all insoluble materials were filtered off. The filtrate thus obtained was concentrated by evaporation under reduced pressure, and the resulting residue was purified by chromatography on an alumina column using a 39:1 by volume mixture of ethyl acetate and methanol as the eluant to afford 1.30 g (yield: 52%) of the title compound as a pale yellow oil.
$^1$H-NMR spectrum (400 MHz, $CDCl_3$) δ ppm:

4.63-4.56 (1H, multiplet);
3.58 (1H, doublet of doublets, J=10 Hz, 7 Hz);
3.31-3.24 (1H, multiplet);
2.72-2.44 (4H, multiplet);
2.39-2.32 (1H, multiplet);
2.29-2.20 (2H, multiplet);
1.97-1.70 (3H, multiplet).

2(ii) (2R,8aS)-2-(t-Butyldimethylsilyloxy)-1,2,3,5,6,7,8,8a-octahydroindolizin-7-one

1.70 g (24.9 mmol) of imidazole and 1.88 g (12.5 mmol) oft-butyldimethylsilyl chloride were added to a solution of 1.30 g (8.1 mmol) of (2R,8aS)-2-hydroxy-1,2,3,5,6,7,8,8a-octahydroindolizin-7-one [obtained as described in Preparative Example 2(i) above] in 30 ml of dichloromethane and the resulting mixture was stirred for 20 hours at room temperature. At the end of this time, water was added and the reaction mixture was extracted with dichloromethane. The organic extract was washed with water and concentrated by evaporation under reduced pressure, and the resulting residue was then purified by chromatography on an alumina column using a 9:1 by volume mixture of hexane and ethyl acetate as the elaunt to afford 1.98 g (yield: 88%) of the title compound as a colorless oil.
$^1$H-NMR spectrum (400 MHz, $CDCl_3$) δ ppm:

4.56-4.48 (1H, multiplet);
3.50-3.42 (1H, multiplet);
3.30-3.23 (1H, multiplet);
2.69-2.32 (5H, multiplet);
2.30-2.17 (2H, multiplet);
1.90-1.78 (2H, multiplet);
0.88 (9H, singlet);
0.06 (3H, singlet);
0.05 (3H, singlet).

Preparative Example 3

(2S,8aS)-2-Chloro-1,2,3,5,6,7,8,8a-octahydroindolizin-7-one

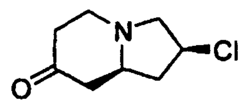

3.93 g (15.0 mmol) of triphenylphosphine were added to a solution of 1.55 g (10.0 mmol) of (2R,8aS)-2-hydroxy-1,2,3,5,6,7,8,8a-octahydroindolizin-7-one [obtained as described in Preparative Example 2(i) above] in 45 ml of carbon tetrachloride and the resulting mixture was heated under reflux for 5 hours. After removal of the solvent by evaporation under reduced pressure, the residue was purified by chromatography on an alumina column using a 1:1 by volume mixture of ethyl acetate and hexane as the eluant to afford 1.52 g (yield: 88%) of the title compound as a pale yellow oil.

$^1$H-NMR spectrum (400 MHz, $CDCl_3$) δ ppm:

4.47-4.40 (1H, multiplet);
3.39-3.31 (2H, multiplet);
2.78-2.63 (3H, multiplet);
2.57-2.42 (2H, multiplet);
2.42-2.26 (3H, multiplet);
1.94 (1H, doublet of double doublets, J=14 Hz, 10 Hz, 5 Hz).

Preparative Example 4

(8aS)-2,2-Difluoro-1,2,3,5,6,7,8,8a-octahydroindolizin-7-one

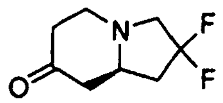

In a similar manner to the procedures described in Preparative Examples 1(i), (ii) and (iii) above, reactions were carried out successively, using (2S)-1-benzyloxycarbonyl-4,4-difluoroproline as a starting material instead of (2S, 4R)-1-benzyloxycarbonyl-4-methoxyproline, to give the title compound as a pale yellow oil (total yield for the 3 steps: 14%).
$^1$H-NMR spectrum (400 MHz, $CDCl_3$) δ ppm:

3.55-3.45 (1H, multiplet);
3.33-3.24 (1H, multiplet);
2.72-2.33 (8H, multiplet);
2.17-2.00 (1H, multiplet).

Preparative Example 5

(±)-6,7,8,9,9a,10-Hexahydropyrido[1,2-a]indol-8-one

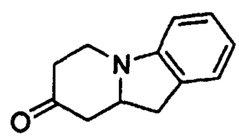

5(i) 1-Benzyloxycarbonylindoline-2-methanol

4.6 g (212 mmol) of lithium borohydride were added in three portions to a solution of 33.0 g (106 mmol) of methyl 1-benzyloxycarbonylindoline-2-carboxylate in 450 ml of tetrahydrofuran, and the resulting mixture was stirred for 5 hours at room temperature. At the end of this time, ice was added and the mixture was stirred for a further 1 hour before extracting with ethyl acetate. The organic extract was washed with water, dried over anhydrous magnesium sulfate and then concentrated by evaporation under reduced pressure. The resulting residue was purified by chromatography on a silica gel column using a 2:5 by volume mixture of ethyl acetate and hexane as the eluant to afford 25.0 g (yield: 83%) of the title compound as a colorless oil.
$^1$H-NMR spectrum (400 MHz, $CDCl_3$) δ ppm:

7.47-7.28 (6H, multiplet);
7.19-7.10 (2H, multiplet);

6.97 (1H, triplet, J=7 Hz);
5.30 (2H, singlet);
4.72-4.53 (2H, multiplet);
3.82-3.63 (2H, multiplet);
3.33 (1H, doublet of doublets, J=16 Hz, 10 Hz);
3.00-2.77 (1H, multiplet).

5(ii) 1-Benzyloxycarbonyl-2-cyanomethylindoline

In a similar manner to the procedures described in Preparative Example 1 (i) above, methanesulfonylation and cyanogenation were carried out, using 1-benzyloxycarbonylindoline-2-methanol [obtained as described in Preparative Example 5(i) above], to give the title compound as an orange oil (yield: 65%).
$^1$H-NMR spectrum (400 MHz, $CDCl_3$) $\delta$ ppm:

7.48-7.32 (6H, multiplet);
7.23-7.16 (2H, multiplet);
7.02 (1H, triplet, J=7 Hz);
5.31 (2H, singlet);
4.81-4.68 (1H, multiplet);
3.50 (1H, doublet of doublets, J=16 Hz, 10 Hz);
3.01 (1H, doublet, J=16 Hz);
2.99-2.50 (2H, multiplet).

5(iii) 1-Benzyloxycarbonyl-2-(3-ethoxycarbonyl-2-oxopropyl)indoline

In a similar manner to that described in Preparative Example 1(ii) above, a reaction was carried out, using 1-benzyloxycarbonyl-2-cyanomethylindoline [obtained as described in Preparative Example 5(ii) above] instead of (2S, 4R)-1-benzyloxycarbonyl-2-cyanomethyl-4-methoxypyrrolidine, to give the title compound as a yellow oil (yield: 47%).
$^1$H-NMR spectrum (400 MHz, $CDCl_3$) $\delta$ ppm:

7.45-7.32 (6H, multiplet);
7.22-7.13 (2H, multiplet);
6.97 (1H, triplet, J=7 Hz);
5.28 (2H, singlet);
4.92-4.84 (1H, multiplet);
4.22-4.12 (2H, multiplet);
3.46 (1H, doublet of doublets, J=16 Hz, 9 Hz);
3.43-3.31 (2H, multiplet);
2.84 (1H, doublet of doublets, J=6 Hz, 4 Hz);
2.80 (1H, doublet of doublets, J=6 Hz, 4 Hz),
2.74 (1H, doublet of doublets, J=16 Hz, 2 Hz);
1.29 (3H, triplet, J=7 Hz).

5(iv) 2-(3-Ethoxycarbonyl-2-oxopropyl)indoline

In a similar manner to that described in Preparative Example 1(iii) above, a debenzylation reaction using hydrogen gas and palladium hydroxide on carbon was performed, using 1-benzyloxycarbonyl-2-(3-ethoxycarbonyl-2-oxopropyl)indoline [obtained as described in Preparative Example 5(iii) above] instead of (2S, 4R)-1-benzyloxycarbonyl-2-(3-ethoxycarbonyl-2-oxopropyl)-4-methoxypyrrolidine, to give the title compound as an orange oil (yield: quantitative).
$^1$H-NMR spectrum (400 MHz, $CDCl_3$) $\delta$ ppm:

7.08 (1H, doublet, J=7 Hz);
6.99 (1H, triplet, J=7 Hz);
6.68 (1H, triplet, J=7 Hz);
6.57 (1H, doublet, J=7 Hz);
4.28-4.16 (1H, multiplet);
4.12-4.01 (2H, multiplet);
3.39-3.18 (4H, multiplet);

3.03-2.81 (1H, multiplet);
2.81-2.71 (1H, multiplet);
1.32-1.21 (3H, multiplet).

5(v) (±)-6,7,8,9,9a,10-Hexahydropyrido[1,2-a]indol-8-one

14.1 ml (56.6 mmol) of a 4N solution of hydrogen chloride in dioxane were added to a solution of 7.0 g (28.3 mmol) of 2-(3-ethoxycarbonyl-2-oxopropyl)indoline [obtained as described in Preparative Example 5(iv) above] in 140 ml dichloromethane and the resulting mixture was stirred for 2 hours at room temperature. At the end of this time, the reaction mixture was concentrated by evaporation under reduced pressure. 2.6 ml (31.1 mmol) of pyrrolidine were added to a solution of the residue in 140 ml of ethanol, and the mixture was then stirred for 5 hours at room temperature before concentrating by evaporation under reduced pressure. 3.18 g (84.9 mmol) of lithium aluminum hydride were then added to a solution of the resulting residue in 100 ml of tetrahydrofuran with ice-cooling and the resulting mixture was stirred for 18 hours at room temperature.

At the end of this time, 13 ml of a 4% aqueous solution of sodium hydroxide were added carefully at 0°C to the reaction mixture, and after the addition of 150 ml of ethanol, the resulting mixture was filtered. The filtrate thus obtained was concentrated by evaporation under reduced pressure and the residue was purified by chromatography on an alumina column using a 1:5 by volume mixture of ethyl acetate and hexane as the eluant to afford 720 mg (yield: 14%) of the title compound as a pale yellow oil.
$^1$H-NMR spectrum (400 MHz, $CDCl_3$) δ ppm:

7.20-7.10 (2H, multiplet);
6.61-6.52 (2H, multiplet);
3.20-3.09 (2H, multiplet);
2.74-2.33 (7H, multiplet).

Preparative Example 6

(2R,8aS)-2-Phenyl-1,2,3,5,6,7,8,8a-octahydroindolizin-7-one

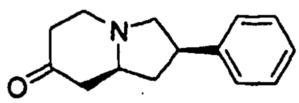

6(i) (S)-1-Benzyloxycarbonyl-2-hydroxymethyl-4-phenyl-3-pyrroline

Methyl (S)-1-benzyloxycarbonyl-4-phenyl-3-pyrroline-2-carboxylate was reduced with using lithium borohydride, in a similar manner to that described in Preparative Example 5(i) above, to afford the title compound as a pale yellow powder (yield: 78%).
$^1$H-NMR spectrum (400 MHz, $CDCl_3$) δ ppm:

7.47-7.27 (10H, multiplet);
6.11-6.07 (0.2H, multiplet);
6.03-5.99 (0.8H, multiplet);
5.23 (2H, doublet of doublets, J=16 Hz, 12 Hz);
5.00-4.94 (0.8H, multiplet);
4.85-4.80 (0.2H, multiplet);
4.77-4.70 (0.2H, multiplet);
4.65 (0.8H, triplet of doublets, J=15 Hz, 2 Hz);
4.56 (1H, doublet of double doublets, J=15 Hz, 5 Hz, 2 Hz);
4.26 (1H, broad singlet);
3.94-3.83 (1H, multiplet);
3.80-3.74 (0.2H, multiplet);
3.72 (0.8H, doublet of doublets, J=12 Hz, 7 Hz).

6(ii) (S)-1-Benzyloxycarbonyl-2-cyanomethyl-4-phenyl-3-pyrroline

In a similar manner to the procedures described in Preparative Example 1(i) above, methanesulfonylation and cyanogenation were performed, using (S)-1-benzyloxycarbonyl-2-hydroxymethyl-4-phenyl-3-pyrroline [obtained as described in Preparative Example 6(i) above], to give the title compound as a pale yellow powder (yield: 80%).
$^1$H-NMR spectrum (400 MHz, $CDCl_3$) δ ppm:

7.47-7.29(10H, multiplet);
6.17-6.11(1H, multiplet);
5.25 (1H, doublet, J=12 Hz)
5.18 (1H, doublet, J=12 Hz);
5.01-4.90 (1H, multiplet);
4.77-4.60 (2H, multiplet);
3.10 (0.7H, doublet of doublets, J=17 Hz, 6 Hz);
2.90 (0.7H, doublet of doublets, J=17 Hz, 3 Hz);
2.86-2.73 (0.6H, multiplet).

6(iii) (2S,4R)-1-Benzyloxycarbonyl-2-cyanomethyl-4-phenylpyrrolidine

2.32 g of 20% palladium hydroxide on carbon were added to a solution of 11.60 g (36.4 mmol) of (S)-1-benzyloxycarbonyl-2-cyanomethyl-4-phenyl-3-pyrroline [obtained as described in Preparative Example 6(ii) above] in 150 ml of ethyl acetate, and the resulting mixture was stirred for 7 hours at room temperature under a hydrogen atmosphere. At the end of this time, the reaction mixture was filtered and the filtrate was concentrated by evaporation under reduced pressure. The resulting residue was purified by chromatography on a silica gel column using a 4:1 by volume mixture of hexane and ethyl acetate as the eluant to afford 8.18 g (yield: 70%) of the title compound as a pale brown oil.
$^1$H-NMR spectrum (400 MHz, $CDCl_3$) δ ppm:

7.44-7.17 (10H, multiplet);
5.26-5.09 (2H, multiplet);
4.32-4.06 (2H, multiplet);
3.50-3.41 (1H, multiplet);
3.37-3.26 (1H, multiplet);
3.21 (0.7H, doublet of doublets, J=17 Hz, 6 Hz);
2.93 (0.3 Hz, doublet of doublets, J=17 Hz, 6 Hz);
2.88-2.59 (2H, multiplet);
2.19-2.07 (1H, multiplet).

6(iv) (2S,4R)-1-Benzyloxycarbonyl-2-(3-ethoxycarbonyl-2-oxopropyl)-4-phenylpyrrolidine

In a similar manner to that described in Preparative Example 1(ii) above, a reaction was performed, using (2S,4R)-1-benzyloxycarbonyl-2-cyanomethyl-4-phenylpyrrolidine [obtained as described in Preparative Example 6(iii) above] instead of (2S,4R)-1-benzyloxycarbonyl-2-cyanomethyl-4-methoxypyrrolidine, to give the title compound as a pale yellow oil (yield: 72%).
$^1$H-NMR spectrum (400 MHz, $CDCl_3$) δ ppm:

7.43-7.17 (10H, multiplet);
5.23-5.07 (2H, multiplet);
4.31-4.10 (4H, multiplet);
3.55-3.20 (5H, multiplet);
2.87-2.67 (2H, multiplet);
1.85-1.74 (1H, multiplet);
1.32-1.21 (3H, multiplet).

6(v) (2R,8aS)-2-Phenyl-1,2,3,5,6,7,8,8a-octahydroindolizin-7-one

In a similar manner to that described in Preparative Example 1(iii) above, a reaction was conducted, using (2S,4R)-1-benzyloxycarbonyl-2-(3-ethoxycarbonyl-2-oxopropyl)-4-phenylpyrrolidine [obtained as described in Preparative Example 6(iv) above] instead of (2S,4R)-1-benzyloxycarbonyl-2-(3-ethoxycarbonyl-2-oxopropyl)-4-methoxypyrrolidine,

to give the title compound as a pale yellow oil (yield: 27%).
$^1$H-NMR spectrum (400 MHz, $CDCl_3$) δ ppm:

7.42-7.14 (5H, multiplet);
3.42-3.28 (2H, multiplet);
3.18 (1H, doublet of doublets, J=9 Hz, 3 Hz);
2.77-2.63 (2H, multiplet);
2.58-2.30 (6H, multiplet);
1.70-1.58 (1H, multiplet).

Preparative Example 7

(8aS)-2,2-Ethylenedioxy-1,2,3,5,6,7,8,8a-octahydroindolizin-7-one

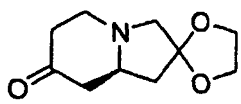

7(i) (S)-1-Benzyloxycarbonyl-4,4-ethylenedioxy-2-hydroxymethylpyrrolidine

(S)-1-Benzyloxycarbonyl-4,4-ethylenedioxyproline methyl ester was reduced using lithium borohydride, in a similar manner to that described in Preparative Example 5(i) above, to afford the title compound as a colorless oil (yield: 85%).
$^1$H-NMR spectrum (400 MHz, $CDCl_3$) δ ppm:

7.42-7.29 (5H, multiplet);
5.14 (2H, singlet);
4.22-4.08 (1H, multiplet);
4.02-3.88 (4H, multiplet);
3.82-3.62 (2H, multiplet);
3.59 (1H, doublet, J=12 Hz);
3.47 (1H, doublet, J=12 Hz);
2.27-2.18 (1H, multiplet);
1.90-1.82 (1H, multiplet).

7(ii) (S)-1-Benzyloxycarbonyl-2-cyanomethyl-4,4-ethylenedioxypyrrolidine

In a similar manner to the procedures described in Preparative Example 1(i) above, methanesulfonylation and cyanogenation were performed, using (S)-1-benzyloxycarbonyl-4,4-ethylenedioxy-2-hydroxymethylpyrrolidine [obtained as described in Preparative Example 7(i) above], to give the title compound as a colorless oil (yield: 88%).
$^1$H-NMR spectrum (400 MHz, $CDCl_3$) δ ppm:

7.43-7.28 (5H, multiplet);
5.22-5.08 (2H, multiplet);
4.30-4.21 (1H, multiplet);
4.06-3.88 (4H, multiplet);
3.62-3.44 (2H, multiplet);
3.00-2.72 (2H, multiplet);
2.41-2.29 (1H, multiplet);
2.17-2.10 (1H, multiplet).

7(iii) (8aS)-2,2-Ethylenedioxy-1,2,3,5,6,7,8,8a-octahydroindolizin-7-one

In a similar manner to the procedures described in Preparative Examples 1 (ii) and 1(iii) above, reactions were

carried out successively, using (S)-1-benzyloxycarbonyl-2-cyanomethyl-4,4-ethylenedioxypyrrolidine [obtained as described in Preparative Example 7(ii) above] as the starting material instead of (2S,4R)-1-benzyloxycarbonyl-2-cyanomethyl-4-methoxypyrrolidine, to give the title compound as a white powder (total yield for the two steps: 17%).
$^1$H-NMR spectrum (400 MHz, $CDCl_3$) δ ppm:

4.06-3.84 (4H, multiplet);
3.32-3.24 (1H, multiplet);
3.22 (1H, doublet, J=10 Hz);
2.73-2.61 (1H, multiplet);
2.58-2.32 (6H, multiplet);
2.23 (1H, doublet of doublets, J=13 Hz, 6 Hz);
1.89 (1H, doublet of doublets, J=13 Hz, 10 Hz).

Preparative Example 8

(8aS)-2-Methyl-1,2,3,5,6,7,8,8a-octahydroindolizin-7-one

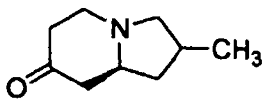

8(i) (S)-1-Benzyloxycarbonyl-4-methylideneproline methyl ester

4.80 g (13.4 mmol) of methyltriphenylphosphonium bromide were added to a suspension of 1.41 g (12.6 mmol) of potassium t-butoxide in 100 ml of diethyl ether, and the resulting mixture was stirred for 15 minutes at 5°C. At the end of this time, a solution of 2.50 g (9.0 mmol) of (S)-1-benzyloxycarbonyl-4-oxoproline methyl ester in 30 ml of diethyl ether was added to the mixture thus obtained, and the mixture was stirred for an additional 3 hours at 35°C. At the end of this time, 50 ml of a saturated aqueous solution of ammonium chloride were added to the reaction mixture with ice-cooling, and the resulting mixture was partitioned. The organic extract thus obtained was washed with water, dried over anhydrous magnesium sulfate and then concentrated by evaporation under reduced pressure. The residue was purified by chromatography on a silica gel column using a 1:3 by volume mixture of ethyl acetate and hexane as the eluant to afford 1.80 g (yield: 72%) of the title compound as a pale yellow oil.
$^1$H-NMR spectrum (400 MHz, $CDCl_3$) δ ppm:

7.40-7.23 (5H, multiplet);
5.21-4.95 (4H, multiplet);
4.60-4.50 (1H, multiplet);
4.20-4.09 (2H, multiplet);
3.74 (1.5H, singlet);
3.60 (1.5H, singlet);
3.07-2.91 (1H, multiplet);
2.65 (1H, doublet, J=16 Hz).

8(ii) (2S)-4-Methylproline methyl ester

180 mg of 10% palladium on carbon were added to a solution of 1.80 g (6.5 mmol) of (S)-1-benzyloxycarbonyl-4-methylideneproline methyl ester [obtained as described in Preparative Example 8(i) above] in 50 ml of methanol, and the resulting mixture was stirred for 2 hours at room temperature under a hydrogen atmosphere. At the end of this time, the reaction mixture was filtered and the filtrate was concentrated by evaporation under reduced pressure to afford 0.93 g (quantitative yield) of the title compound as a pale yellow oil.
$^1$H-NMR spectrum (400 MHz, $CDCl_3$) δ ppm:

3.82 (1H, triplet, J=8 Hz);

3.74 (3H, singlet);
3.08 (1H, doublet of doublets, J=10 Hz, 7 Hz);
2.60 (1H, doublet of doublets, J=10 Hz, 8 Hz);
2.33 (1H, doublet of triplets, J=12 Hz, 8 Hz);
2.28-2.15 (1H, multiplet);
1.44-1.37 (1H, multiplet);
1.27 (1H, doublet of doublets, J=14 Hz, 7 Hz);
1.02 (3H, doublet, J=7 Hz).

8(iii) (2S)-1-Benzyloxycarbonyl-2-hydroxymethyl-4-methylpyrrolidine

20 ml of an aqueous solution containing 1.89 g (22.5 mmol) of sodium hydrogencarbonate and 1.54 ml (10.8 mmol) of benzyl chloroformate were added to a solution of 0.93 g (6.5 mmol) of (2S)-4-methylproline methyl ester [obtained as described in Preparative Example 8(ii) above] in 20 ml of toluene, and the resulting mixture was stirred at room temperature over night. At the end of this time, the reaction mixture was extracted with ethyl acetate. The organic extract was washed with water and concentrated under reduced pressure to afford 1.78 g (yield: 99%) of (2S)-1-benzyloxycarbonyl-4-methylproline methyl ester as a pale yellow oil. Subsequently, all of the compound thus obtained was reduced using lithium borohydride, in a similar manner to that described in Preparative Example 5(i) above, to afford 1.07 g (yield: 66%) of the title compound as a pale yellow oil.
$^1$H-NMR spectrum (400 MHz, $CDCl_3$) δ ppm:

7.42-7.29 (5H, multiplet);
5.14 (2H, broad singlet);
5.07-4.90 (1H, multiplet);
4.08-3.87 (1H, multiplet);
3.86-3.40 (4H, multiplet);
2.90-2.65 (1H, multiplet);
2.40-2.05 (2H, multiplet);
1.02 (3H, doublet, J=6 Hz).

8(iv) (2S)-1-Benzyloxycarbonyl-2-cyanomethyl-4-methylpyrrolidine

In a similar manner to the procedures described in Preparative Example 1(i) above, methanesulfonylation and cyanogenation were conducted, using (2S)-1-benzyloxycarbonyl-2-hydroxymethyl-4-methylpyrrolidine [obtained as described in Preparative Example 8(iii) above], to give the title compound as a colorless oil (yield: 70%).
$^1$H-NMR spectrum (400 MHz, $CDCl_3$) δ ppm:

7.50-7.30 (5H, multiplet);
5.25-5.05 (2H, multiplet);
4.20-3.78 (1.8H, multiplet);
3.70-3.62 (0.2H, multiplet);
3.15-2.88 (1.4H, multiplet);
2.84-2.67 (1.2H, multiplet);
2.62-2.50 (0.4H, multiplet);
2.45-2.30 (0.8H, multiplet);
2.23-2.00 (1H, multiplet);
1.89-1.77 (0.2H, multiplet);
1.60-1.49 (1H, multiplet);
1.10-1.03 (3H, multiplet).

8(v) (2S)-1-Benzyloxycarbonyl-2-(3-ethoxycarbonyl-2-oxopropyl)-4-methylpyrrolidine

In a similar manner to that described in Preparative Example 1 (ii) above, a reaction was performed, using (2S)-1-benzyloxycarbonyl-2-cyanornethyl-4-methylpyrrolidine [obtained as described in Preparative Example 8(iv) above] instead of (2S,4R)-1-benzyloxycarbonyl-2-cyanomethyl-4-methoxypyrrolidine, to give the title compound as a pale yellow oil (yield: 66%).
$^1$H-NMR spectrum (400 MHz, $CDCl_3$) δ ppm:

7.43-7.28 (5H, multiplet);
5.20-4.99 (2H, multiplet);
4.27-4.03 (3H, multiplet);
3.56-3.40 (1.6H, multiplet);
3.37-3.25 (0.4H, multiplet);
3.00-2.89 (0.2H, multiplet);
2.89-2.75 (0.8H, multiplet);
2.75-2.56 (1H, multiplet);
2.50-2.22 (1H, multiplet);
2.20-2.05 (1H, multiplet);
1.32-1.15 (4H, multiplet);
1.08 (0.6H, doublet, J=6 Hz);
1.02 (2.4H, doublet, J=6 Hz).

8(vi) (8aS)-2-Methyl-1,2,3,5,6,7,8,8a-octahydroindolizin-7-one

In a similar manner to that described in Preparative Example 1 (iii) above, a reaction was performed, using (2S)-1-benzyloxycarbonyl-2-(3-ethoxycarbonyl-2-oxopropyl)-4-methylpyrrolidine [obtained as described in Preparative Example 8(v) above] instead of (2S,4R)-1-benzyloxycarbonyl-2-(3-ethoxycarbonyl-2-oxopropyl)-4-methoxypyrrolidine, to give the title compound as a colorless oil (yield: 34%).
$^1$H-NMR spectrum (400 MHz, $CDCl_3$) δ ppm:

3.34-3.22 (1.2H, multiplet);
2.77 (0.8H, doublet of doublets, J=9 Hz, 3 Hz);
2.68-2.55 (1H, multiplet);
2.51-2.43 (2H, multiplet);
2.39-2.24 (5H, multiplet);
2.20-2.10 (1H, multiplet);
1.87-1.75 (0.8H, multiplet);
1.57-1.51 (0.2H, multiplet);
1.14 (2.4H, doublet, J=7 Hz);
1.04 (0.6H, doublet, J=7 Hz).

Preparative example 9

(8aS)-8-Methyl-1,2,3,5,6,7,8,8a-octahydroindolizin-7-one

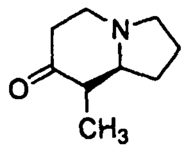

9(i) (2S)-1-Methylmalonyl-2-(1-carboxyethyl)pyrrolidine

2.01 ml (18.7 mmol) of methyl malonyl chloride were added dropwise to a stirred, ice-cooled mixture of a solution of 2.43g (17.0 mmol) of (2S)-2-(1-carboxyethyl)pyrrolidine [prepared as described in Tetrahedron Lett., 40, 2891-2894 (1999)] in 60 ml of dichloromethane and 2.61 ml (18.7 mmol) of triethylamine. After stirring the resulting mixture at the same temperature for 15 minutes and then at room temperature for 30 minutes, a saturated aqueous solution of sodium hydrogencarbonate was added. Subsequently, the resulting mixture was adjusted to pH 2 with concentrated hydrochloric acid and then extracted with a 4:1 by volume mixture of dichloromethane and isopropanol. The organic extract was washed with water, dried over anhydrous magnesium sulfate and then concentrated by evaporation under reduced pressure to afford 4.47 g (yield: quantitative) of the title compound as a pale brown oil.
$^1$H-NMR spectrum (500 MHz, $CDCl_3$) δ ppm:

4.55-4.47 (0.4H, multiplet);
4.34-4.25 (0.6H, multiplet);
3.76 (2.4H, singlet);
3.59-3.42 (4H, multiplet);
3.25-3.16 (0.6H, multiplet);
2.14-1.80 (5H, multiplet);
1.18 (1.8H, doublet, J=7 Hz);
1.08 (1.2H, doublet, J=7 Hz).

9(ii) (8aS)-6-Methoxycarbonyl-8-methyl-1,2,3,5,6,7,8,8a-octahydroindolizine-5,7-dione

4.13 g (25.5 mmol) of carbonyldiimidazole were added to a solution of 4.47 g (17.0 mmol) of (2S)-1-methylmalonyl-2-(1-carboxyethyl)pyrrolidine [obtained as described in Preparative Example 9(i) above] in 120 ml of tetrahydrofuran at room temperature, and the resulting mixture was stirred for 30 minutes at room temperature. At the end of this time, 3.81 ml (25.5 mmol) of 1,8-diazabicyclo[5.4.0]undec-7-ene were added and the resulting mixture was stirred for an additional 1 hour. The reaction mixture was then concentrated by evaporation under reduced pressure, and the residue was partitioned between dichloromethane and a 1N aqueous solution of hydrochloric acid. The separated organic extract was washed with water, dried over anhydrous magnesium sulfate and then concentrated by evaporation under reduced pressure to afford 4.16 g (yield: quantitative) of the title compound as a brown oil.
$^1$H-NMR spectrum (500 MHz, $CDCl_3$) δ ppm:

3.90 (3H, singlet);
3.89-3.82 (1H, multiplet);
3.73-3.64 (1H, multiplet);
3.53-3.32 (2H, multiplet);
2.67-2.52 (2H, multiplet);
2.33-2.25 (0.5H, multiplet);
2.07-1.96 (1.5H, multiplet);
1.87-1.74 (1H, multiplet);
1.68-1.55 (1H, multiplet);
1.43 (1.5H, doublet, J=7 Hz);
1.12 (1.5H, doublet, J=7 Hz).

9(iii) (8aS)-8-Methyl-1,2,3,5,6,7,8,8a-octahydroindolizine-5,7-dione

4.16 g (17.0 mmol) of (8aS)-6-methoxycarbonyl-8-methyl-1,2,3,5,6,7,8,8a-octahydroindolizine-5,7-dione [obtained as described in Preparative Example 9(ii) above] were dissolved in 40 ml of a 10% aqueous solution of acetic acid and the resulting solution was stirred at 110°C for 30 minutes. After being cooled to room temperature, the reaction mixture was made basic using a saturated aqueous solution of sodium hydrogencarbonate and then extracted with a 4:1 by volume mixture of dichloromethane and isopropanol. The organic extract was washed with water, dried over anhydrous magnesium sulfate and then concentrated by evaporation under reduced pressure to afford 2.62 g (yield: 92%) of the title compound as a brown oil.
$^1$H-NMR spectrum (500 MHz, $CDCl_3$) δ ppm:

4.03-3.97 (0.3H, multiplet);
3.74-3.41 (2.7H, multiplet);
3.27 (1.4H, singlet);
3.26 (0.6H, singlet);
2.73-2.67 (0.3H, multiplet);
2.38-2.32 (0.7H, multiplet);
2.30-2.23 (0.7H, multiplet);
2.14-2.01 (1.3H, multiplet);
1.96-1.86 (1H, multiplet);
1.73-1.63 (1H, multiplet);
1.77 (2.1H, doublet, J=7 Hz);
1.08 (0.9H, doublet, J=7 Hz).

9(iv) (8aS)-8-Methyl-7-(1-pyrrolidinyl)-1,2,3,5,8,8a-hexahydroindolizin-5-one

2.62 ml (31.4 mmol) of pyrrolidine were added to a solution of 2.62 g (15.7 mmol) of (8aS)-8-methyl-1,2,3,5,6,7,8,8a-octahydroindolizine-5,7-dione [obtained as described in Preparative Example 9(iii) above] in 30 ml of ethanol and the resulting mixture was stirred for 30 minutes at 80°C. After the reaction was completed, the solvent and excess pyrrolidine were removed from the reaction mixture by evaporation under reduced pressure to afford 3.67 g (yield: quantitative) of the title compound as a brown oil.
$^1$H-NMR spectrum (500 MHz, $CDCl_3$) δ ppm:

4.52 (1H, singlet);
3.80-3.65 (2H, multiplet);
3.44-3.34 (1H, multiplet);
3.33-3.17 (4H, multiplet);
2.61-2.43 (1H, multiplet);
2.02-1.89 (6H, multiplet);
1.88-1.72 (4H, multiplet);
1.01 (3H, doublet, J=7 Hz).

9(v) (8aS)-8-Methyl-1,2,3,5,6,7,8,8a-octahydroindolizin-7-one

1.79 g (47.1 mmol) of lithium aluminum hydride were added in several portions to a stirred, ice-cooled solution of 3.67 g (15.7 mmol) of (8aS)-8-methyl-7-(1-pyrrolidinyl)-1,2,3,5,8,8a-hexahydroindolizin-5-one [obtained as described in Preparative Example 9(iv) above] in 50 ml of tetrahydrofuran and the resulting mixture was stirred at room temperature overnight. At the end of this time, 7.22 ml of a 1N aqueous solution of sodium hydroxide were added followed by the addition of ethanol, and any insoluble material was then filtered off. The filtrate thus obtained was concentrated by evaporation under reduced pressure, and the resulting residue was purified by chromatography on an alumina column using ethyl acetate as the eluant to afford 1.69 g (yield: 70%) of the title compound as a pale yellow oil.
$^1$H-NMR spectrum (500 MHz, $CDCl_3$) δ ppm:

3.36-3.29 (1H, multiplet);
3.18-3.13 (1H, multiplet);
2.72-2.63 (1H, multiplet);
2.40-2.30 (3H, multiplet);
2.26-2.17 (1H, multiplet);
2.05-1.91 (3H, multiplet);
1.87-1.78 (1H, multiplet);
1.64-1.55 (2H, multiplet);
1.01 (3H, doublet, J=7 Hz).

Preparative Example 10

(2S,8aS)-2-Methoxy-1,2,3,5,6,7,8,8a-octahydroindolizin-7-one

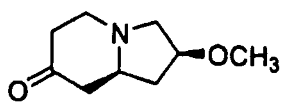

10(i) (2S,4S)-4-Methoxy-1-methylmalonylhomoproline

7.52 ml (54 mmol) of triethylamine were added dropwise with ice-cooling and stirring to a suspension of 4.40 g (22.5, mmol) of (2S,4S)-4-methoxyhomoproline hydrochloride in 100 ml of methylene chloride, followed by the further dropwise addition with ice-cooling of 2.66 ml (24.8 mmol) of methyl malonyl chloride, and the resulting mixture was then stirred for 15 minutes at the same temperature. After the reaction mixture was stirred for a further 2 hours at room temperature, a saturated aqueous solution of sodium hydrogencarbonate was added, the pH value of the separated aqueous layer was adjusted to 2 by the addition of concentrated hydrochloric acid, and then the mixture was extracted

with a 4:1 by volume mixture of methylene chloride and isopropanol. The organic extract was washed with water, dried over anhydrous magnesium sulfate and then concentrated by evaporation under reduced pressure to afford 5.83 g (yield: quantitative) of the title compound as a brown oil.
$^1$H-NMR spectrum (500 MHz, $CDCl_3$) δ ppm:

4.51-4.44 (0.8H, multiplet);
4.36-4.30 (0.2H, multiplet);
4.05-3.97 (1H, multiplet);
3.78-3.73 (2.6H, multiplet);
3.67-3.52 (2.4H, multiplet);
3.48-3.38 (2H, multiplet);
3.35-3.30 (3H, multiplet);
3.14-3.08 (0.8H, multiplet);
3.03 (0.2H, doublet of doublets, J=16 Hz, 9 Hz);
2.83-2.77 (0.2H, multiplet);
2.69 (0.8H, doublet of doublets, J=16 Hz, 9 Hz);
2.21-2.12 (3H, multiplet).

10(ii) (2S,8aS)-2-Methoxy-6-methoxycarbonyl-1,2,3,5,6,7,8,8a-octahydroindolizine-5,7-dione

4.02 g (24.8 mmol) of carbonyldiimidazole were added to a suspension of 5.83 g (22.5 mmol) of (2S,4S)-4-methoxy-1-methylmalonylhomoproline [obtained as described in Preparative Example 10(i) above] in 90 ml of tetrahydrofuran and the resulting mixture was then stirred for 30 minutes at room temperature. At the end of this time, 3.71 ml (24.8 mmol) of 1,8-diazabicyclo[5.4.0]undec-7-ene were added and the resulting reaction mixture was stirred for 1 hour at room temperature. The reaction mixture was then concentrated by evaporation under reduced pressure and the resulting residue was partitioned between methylene chloride and a 1N aqueous hydrochloric acid solution. The organic extract thus obtained was washed with water, dried over anhydrous magnesium sulfate and then concentrated by evaporation under reduced pressure to afford 5.21 g (yield: quantitative) of the title compound as a brown oil.
$^1$H-NMR spectrum (500 MHz, $CDCl_3$) δ ppm:

4.02-3.96 (1H, multiplet);
3.90 (3H, singlet);
3.87-3.79 (1H, multiplet);
3.78-3.72 (1H, multiplet);
3.55 (1H, doublet of doublets, J=13 Hz, 6 Hz);
3.39-3.29 (4H, multiplet);
2.74 (1H, doublet of doublets, J=17 Hz, 13 Hz);
2.59 (1H, doublet of doublets, J=13 Hz, 4 Hz);
2.49-2.42 (1H, multiplet);
1.84-1.77 (1H, multiplet).

10(iii) (2S,8aS)-2-Methoxy-1,2,3,5,6,7,8,8a-octahydroindolizine-5,7-dione

5.21 g (22.5 mmol) of (2S,8aS)-2-methoxy-6-methoxycarbonyl-1,2,3,5,6,7,8,8a-octahydroindolizine-5,7-dione [obtained as described in Preparative Example 10(ii) above] were dissolved in 50 ml of a 10% aqueous solution of acetic acid and the resulting mixture was stirred for 1 hour at 110°C. After it was cooled to room temperature, the reaction mixture was made alkaline by the addition of a saturated aqueous solution of sodium hydrogencarbonate, and it was then extracted with a 4:1 by volume mixture of methylene chloride and isopropanol. The organic extract was washed with water, dried over anhydrous magnesium sulfate and then concentrated by evaporation under reduced pressure to afford 3.38 g (yield: 82 %) of the title compound as a pale brown oil.
$^1$H-NMR spectrum (500 MHz, $CDCl_3$) δ ppm:

4.06-3.90 (3H, multiplet);
3.52 (1H, doublet of doublets, J=12 Hz, 5 Hz);
3.37-3.28 (5H, multiplet);
2.73 (1H, doublet of doublets, J=17 Hz, 3 Hz);
2.55-2.43 (2H, multiplet);
1.95-1.87 (1H, multiplet).

10(iv) (2S,8aS)-2-Methoxy-7-(1-pyrrolidinyl)-1,2,3,5,8,8a-hexahydroindolizin-5-one

3.07 ml (36.8 mmol) of pyrrolidine were added to a solution of 3.38 g (18.4 mmol) of (2S,8aS)-2-methoxy-1,2,3,5,6,7,8,8a-octahydroindolizine-5,7-dione [obtained as described in Preparative Example 10(iii) above] in 34 ml of ethanol, and the resulting mixture was left to stand for 30 minutes at room temperature. At the end of this time, the solvent and any excess pyrrolidine were distilled off under reduced pressure to afford 4.26 g (yield: 98 %) of the title compound as a brown oil.
$^1$H-NMR spectrum (500 MHz, $CDCl_3$) δ ppm:

4.60 (1H, singlet);
4.05-3.96 (1H, multiplet);
3.77-3.65 (2H, multiplet);
3.57 (1H, doublet of doublets, J=12 Hz, 5 Hz);
3.34 (3H, singlet);
3.33-3.16 (4H, multiplet);
2.87 (1H, doublet of doublets, J= 16 Hz, 5 Hz);
2.50-2.37 (2H, multiplet);
2.02-1.83 (4H, multiplet);
1.80-1.70 (1H, multiplet).

10(v) (2S,8aS)-2-Methoxy-1,2,3,5,6,7,8,8a-octahydroindolizin-7-one

2.10 g (55 mmol) of lithium aluminum hydride were added, with stirring and ice-cooling, to a solution of 4.26 g (18.1 mmol) of (2S,8aS)-2-methoxy-7-(1-pyrrolidinyl)-1,2,3,5,8,8a-hexahydroindolizin-5-one [obtained as described in Preparative Example 10(iv) above] in 50 ml of tetrahydrofuran, and the resulting mixture was then stirred for a further 3 hours at room temperature. At the end of this time, 8.40 ml of a 1N aqueous solution of sodium hydroxide were carefully added to the reaction mixture, followed by the addition of ethanol, and then any insoluble materials were removed by filtration. The filtrate thus obtained was concentrated by evaporation under reduced pressure and the resulting residue was purified by chromatography on an alumina column using ethyl acetate as the eluant afford to 1.60 g (yield: 51 %) of the title compound as a pale brown oil.
$^1$H-NMR spectrum (500 MHz, $CDCl_3$) δ ppm:

3.96-3.90 (1H, multiplet);
3.35-3.21 (2H, multiplet);
3.32 (3H, singlet);
2.75-2.65 (1H, multiplet);
2.55-2.17 (5H, multiplet);
1.63-1.53 (2H, multiplet);
1.32-1.20 (1H, multiplet).

Preparative Example 11

(8aS)-2-Methylidene-1,2,3,5,6,7,8,8a-octahydroindolizin-7-one

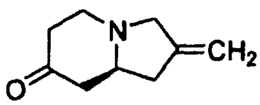

11(i) (S)-1-Benzyloxycarbonyl-2-cyanomethyl-4-methylidenepyrrolidine

In a similar manner to the procedures described in Preparative Examples 5(i) and 5(ii) above, reduction, methanesulfonylation and cyanogenation were conducted, using (S)-1-benzyloxycarbonyl-4-methylideneproline methyl ester [obtained as described in Preparative Example 8(i) above], instead of methyl 1-benzyloxycarbonylindoline-2-carboxylate, to give the title compound as a colorless oil (yield: 61%).

$^1$H-NMR spectrum (400 MHz, $CDCl_3$) δ ppm:

7.42-7.30 (5H, multiplet);
5.20-5.02 (4H, multiplet);
4.36-4.27 (1H, multiplet);
4.22-3.93 (2H, multiplet);
2.92 (1H, doublet of doublets, J=16 Hz, 9 Hz);
2.80-2.38 (3H, multiplet).

11(ii) (S)-1-(t-Butoxycarbonyl)-4-methylidenehomoproline ethyl ester

7.00 g (27.3 mmol) of (S)-1-benzyloxycarbonyl-2-cyanomethyl-4-methylidenepyrrolidine [obtained as described in Preparative Example 11(i) above] were dissolved in 100 ml of ethanol, and the resulting solution was stirred for I hour at room temperature while bubbling hydrogen chloride gas through it, before raising the temperature to 80°C and continuing to bubble hydrogen chloride gas through with stirring for a further 2 hours. The ethanol was then distilled off and the resulting residue was dissolved in 100 ml of water. The aqueous layer was washed with ethyl acetate and 50 ml of dioxane were then added to the aqueous layer. The aqueous dioxane solution thus obtained was neutralized by the addition of triethylamine. A further 3.80 ml (27.3 mmol) of triethylamine were then added, followed by the addition of 8.95 g (41.0 mmol) of di-t-butyl dicarbonate. The resulting mixture was stirred for 2 hours at room temperature, at the end of which time the reaction mixture was extracted with ethyl acetate. The organic extract was washed with water, dried over anhydrous magnesium sulfate and then concentrated by evaporation under reduced pressure. The resulting residue was purified by chromatography on a silica gel column using a 1:3 by volume mixture of ethyl acetate and hexane as the eluant to afford 3.40 g (yield: 46 %) of the title compound as a colorless oil.
$^1$H-NMR spectrum (400 MHz, $CDCl_3$) δ ppm:

5.01 (2H, broad singlet);
4.43-4.21 (1H, multiplet);
4.12 (2H, doublet of doublets, J=14 Hz, 7 Hz);
4.08-3.90 (1H, multiplet);
3.84 (1H, doublet of doublets, J=15 Hz, 2 Hz);
2.88-2.55 (2H, multiplet);
2.41-2.29 (2H, multiplet);
1.49 (9H, singlet);
1.30-1.18 (3H, multiplet).

11(iii) (S)-4-Methylidenehomoproline trifluoroacetate

18.9 ml (18.9 mmol) of a 1N aqueous solution of sodium hydroxide were added to a solution of 3.40 g (12.6 mmol) of (S)-1-(t-butoxycarbonyl)-4-methylidenehomoproline ethyl ester [obtained as described in Preparative Example 11(ii) above] in 35 ml of ethanol, and the resulting mixture was stirred for 2 hours at room temperature. At the end of this time, the reaction mixture was concentrated by evaporation under reduced pressure and then partitioned between ethyl acetate and water. The organic extract was washed with water, dried over anhydrous magnesium sulfate and then concentrated by evaporation under reduced pressure. The resulting residue was dissolved in 30 ml of methylene chloride and 9.7 ml (126 mmol) of trifluoroacetic acid were added to the solution thus obtained, which was then stirred for 2 hours at room temperature. At the end of this time, the reaction mixture was concentrated by evaporation under reduced pressure to afford 3.08 g (yield: 96 %) of the title compound as a white powder.
$^1$H-NMR spectrum (400 MHz, $CDCl_3$) δ ppm:

5.22-5.12 (2H, multiplet);
4.03-3.88 (2H, multiplet);
3.80-3.30 (3H, multiplet);
2.91-2.79 (2H, multiplet).

11(iv) (8aS)-2-Methylidene-1,2,3,5,6,7,8,8a-octahydroindolizin-7-one

In a similar manner to the procedures described in Preparative Examples 10(i), 10(ii), 10(iii), 10(iv) and 10(v) above, reactions were conducted in turn, using (S)-4-methylidenehomoproline trifluoroacetate [obtained as described in Preparative Example 11 (iii) above] in place of (2S,4S)-4-methoxyhomoproline hydrochloride, to give the title compound

as a yellow oil (yield: 39 %).
$^1$H-NMR spectrum (400 MHz, $CDCl_3$) δ ppm:

4.97 (1H, broad singlet);
4.94 (1H, broad singlet);
3.72 (1H, doublet, J=13 Hz);
3.34-3.25 (1H, multiplet);
3.00-2.91 (1H, multiplet);
2.70-2.20 (8H, multiplet).

Preparative Example 12

(2S,8aS)-2-Methylthio-1,2,3,5,6,7,8,8a-octahydroindolizin-7-one

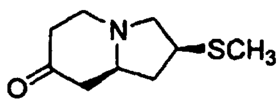

12(i) (2S,4R)-1-Benzyloxycarbonyl-2-hydroxymethyl-4-(p-toluenesulfonyloxy)-pyrrolidine

a) 37.5 ml (270 mmol) of triethylamine, 49.04 g (257 mmol) of p-toluenesulfonyl chloride and 2.99 g (24.5 mmol) of 4-dimethylaminopyridine were added in turn to a solution of 77.70 g (245mmol) of (2S,4R)-1-benzyloxycarbonyl-4-hydroxyproline methyl ester in 600 ml of methylene chloride, and the resulting mixture was then stirred at room temperature overnight. At the end of this time, the reaction mixture was concentrated by evaporation under reduced pressure and the residue was partitioned between ethyl acetate and water. The organic extract was washed with water, dried over anhydrous magnesium sulfate and then concentrated by evaporation under reduced pressure. The resulting residue was purified by chromatography on a silica gel column using a 1:1 by volume mixture of hexane and ethyl acetate as the eluant to afford 106.18 g (quantitative yield) of (2S,4R)-1-benzyloxycarbonyl-4-(p-toluenesulfonyloxy)proline methyl ester as a yellow oil.
b) In a similar manner to the procedures described in Preparative Example 5(i) above, 106.18 g (245 mmol) of (2S, 4R)-1-benzyloxycarbonyl-4-(p-toluenesulfonyloxy)proline methyl ester [prepared as described in step (a) above] were reduced to give the title compound 104.98 g as a yellow oil (yield: quantitative).

12(ii) (2S,4S)-1-Benzyloxycarbonyl-2-(t-butyldimethylsilyloxy)methyl-4-acetylthiopyrrolidine

a) 37.4 ml (269 mmol) of triethylamine, 38.76 g (257 mmol) of t-butyldimethylsilyl chloride and 2.99 g (24.5 mmol) of 4-dimethylaminopyridine were added, in turn, to a solution of 104.98 g (245 mmol) of (2S,4R)-1-benzyloxycarbonyl-2-hydroxymethyl-4-(p-toluenesulfonyloxy)pyrrolidine [obtained as described in Preparative Example 12(i) above] in 610 ml of methylene chloride, and the resulting mixture was then stirred at room temperature overnight. At the end of this time, the reaction mixture was concentrated by evaporation under reduced pressure, and the resulting residue was partitioned between ethyl acetate and water. The organic extract was washed with water, dried over anhydrous magnesium sulfate and then concentrated by evaporation under reduced pressure. The resulting residue was purified by chromatography on a silica gel column using a 1:1 by volume mixture of hexane and ethyl acetate as the eluant to afford 119.34 g (yield: 94 %) of (2S,4R)-1-benzyloxycarbonyl-2-(t-butyldimethylsilyloxyloxy)methyl-4(p-toluenesulfonyloxy)-pyrrolidine as a yellow oil.
b) 29.34 g (257 mmol) of sodium thioacetate were added to a solution of 127.17 g (245 mmol) of (2S,4R)-1-benzyloxycarbonyl-2-(t-butyldimethylsilyloxy)-methyl-4-(p-toluenesulfonyloxy)pyrrolidine [obtained as described in step (a) above] in 245 ml of dimethylformamide, and the resulting mixture was stirred for 1.5 hours at 60°C. After it was cooled to room temperature, water was added and it was extracted with ethyl acetate. The organic extract was washed with water, dried over anhydrous magnesium sulfate and then concentrated by evaporation under reduced

pressure. The resulting residue was purified by chromatography on a silica gel using an 8:1 by volume mixture of hexane and ethyl acetate as the eluant to afford 91.67 g (yield: 88 %) of the title compound as an orange oil.

12(iii) (2S,4S)-1-Benzyloxycarbonyl-2-(t-butyldimethylsilyloxy)methyl-4-methylthiopyrrolidine

6.22 ml (99.9 mmol) of methyl iodide were added at room temperature to a solution of 35.26 g (83.2 mmol) of (2S,4S)-1-benzyloxycarbonyl-2-(t-butyldimethylsilyloxy)-methyl-4-acetylthiopyrrolidine [obtained as described in Preparative Example 12(ii) above] in 166 ml methanol, 149 ml (41.6 mmol) of a 28% solution of sodium methoxide in methanol were further added at 0°C, and then the resulting mixture was stirred for 1 hour at the same temperature. At the end of this time, the reaction mixture was concentrated by evaporation under reduced pressure, and the residue was partitioned between ethyl acetate and water. The organic extract was washed with water, dried over anhydrous magnesium sulfate and then concentrated by evaporation under reduced pressure. The residue was purified by chromatography on a silica gel column using a 9:1 by volume mixture of hexane and ethyl acetate as the eluant to afford 30.74 g (yield: 93 %) of the title compound as a yellow oil.

12(iv) (2S,4S)-1-Benzyloxycarbonyl-2-hydroxymethyl-4-methylthiopyrrolidine

85.5 ml (85.5 mmol) of a 1M solution oftetrabutylammonium fluoride in tetrahydrofuran were added to a solution of 30.74 g (77.7 mmol) of (2S,4S)-1-benzyloxycarbonyl-2-(t-butyldimethylsilyloxy)methyl-4-methylthiopyrrolidine [obtained as described in Preparative Example in 12(iii) above] in 155 ml of tetrahydrofuran, and the resulting mixture was stirred at room temperature overnight. At the end of this time, the reaction mixture was concentrated by evaporation under reduced pressure and the resulting residue was partitioned between ethyl acetate and water. The organic extract was washed with water, dried over anhydrous magnesium sulfate and then concentrated by evaporation under reduced pressure. The resulting residue was purified by chromatography on a silica gel column using a 1:1 by volume mixture of hexane and ethyl acetate as the eluant to afford 21.87 g (yield: quantitative) of the title compound as a yellow oil.

12(v) (2S,4S)-1-Benzyloxycarbonyl-2-cyanomethyl-4-methylthiopyrrolidine

In a similar manner to the procedures described in Preparative Example 1(i) above, methanesulfonylation and cyanogenation were conducted, using (2S,4S)-1-benzyloxycarbonyl-2-hydroxymethyl-4-methylthiopyrrolidine [obtained as described in Preparative Example 12(iv) above], to give the title compound (yield: 52 %) as a yellow oil.

12(vi) (2S,4S)-4-Methylthiohomoproline hydrochloride

37 ml of a 35% aqueous hydrochloric acid solution were added to 7.43 g (25.6 mmol) of (2S,4S)-1-benzyloxycarbonyl-2-cyanomethyl-4-methylthiopyrrolidine [obtained as described in Preparative Example 12(v) above], and the resulting solution was stirred at 80°C overnight. At the end of this time, the reaction mixture was concentrated by evaporation under reduced pressure and the resulting residue was washed with ethyl acetate followed by the addition of ethanol. The insoluble materials were removed by filtration and the filtrate was concentrated by evaporation under reduced pressure to afford 5.39 g (yield: quantitative) of the title compound as a brown oil.

12(vii) (2S,8aS)-2-Methylthio-1,2,3,5,6,7,8,8a-octahydroindolizin-7-one

In a similar manner to the procedures described in Preparative Examples 10(i), 10(ii), 10(iii), 10(iv) and 10(v) above, reactions were conducted in turn, using (2S,4S)-4-methylthiohomoproline hydrochloride [obtained as described in Preparative Example 12(vi) above] in place of (2S,4S)-4-methoxyhomoproline hydrochloride, to give (yield: 37 %) of the title compound as yellow oil.
$^1$H-NMR spectrum (400 MHz, $CDCl_3$) δ ppm:

3.32-3.23 (2H, multiplet);
3.17 (1H, doublet of doublets, J=10 Hz, 2 Hz);
2.72-2.59 (2H, multiplet);
2.51-2.40 (2H, multiplet);
2.37-2.31 (4H, multiplet);
2.15 (3H, singlet);
1.52-1.45 (1H, multiplet).

Preparative Example 13

(2S,8aS)-2-Ethylthio-1,2,3,5,6,7,8,8a-octahydroindolizin-7-one

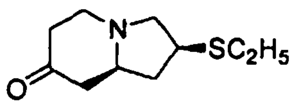

In a similar manner to the procedures described in Preparative Examples 12(iii), 12(iv), 12(v), 12(vi) and 12 (vii) above, reactions were conducted in turn, using ethyl iodide in place of methyl iodide, to give the title compound as a yellow oil (yield: 8 %).
$^1$H-NMR spectrum (400 MHz, $CDCl_3$) $\delta$ ppm:

3.36-3.28 (2H, multiplet);
3.16 (1H, doublet of doublets, J=10 Hz, 2 Hz);
2.74-2.57 (4H, multiplet);
2.52-2.41 (2H, multiplet);
2.38-2.27 (4H, multiplet);
1.53-1.46 (1H, multiplet);
1.28 (3H, triplet, J=7 Hz).

Preparative Example 14

(2S,8aS)-2-Butylthio-1,2,3,5,6,7,8,8a-octahydroindolizin-7-one

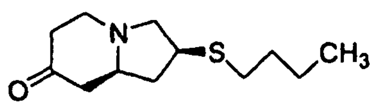

In a similar manner to the procedures described in Preparative Examples 12(iii), 12(iv), 12(v), 12(vi) and 12 (vii) above, reactions were conducted in turn, using butyl bromide in place of methyl iodide, to give the title compound as a brown oil (yield: 13 %).

Preparative Example 15

(2S,8aS)-2-Methylsulfonyl-1,2,3,5,6,7,8,8a-octahydroindolizin-7-one

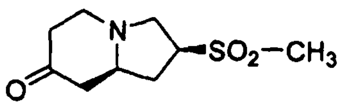

4.6 ml (9.23 mmol) of a 2N aqueous sulfuric acid solution and 3 ml of an aqueous solution of sodium tungstate dihydrate (101 mg, 0.31 mmol) were added to a solution of 1.14 g (6.15 mmol) of (2S,8aS)-2-methylthio-1,2,3,5,6,7,8,8a-octahydroindolizin-7-one [obtained as described in Preparative Example 12 above] in 15 ml of methanol, and 1.35 ml (12.3 mmol) of a 30% aqueous solution of hydrogen peroxide were then added dropwise at 55°C to the resulting mixture. The mixture was then stirred for 1 hour at the same temperature, at the end of which time 30 ml of a saturated aqueous

solution of sodium hydrogencarbonate were added to it before extracting with methylene chloride. The organic extract was washed with water, dried over anhydrous magnesium sulfate and then concentrated by evaporation under reduced pressure. The resulting residue was purified by chromatography on an alumina column using ethyl acetate as the eluant to afford 945 mg (yield: 71 %) of the title compound as a yellow oil.
$^1$H-NMR spectrum (400 MHz, $CDCl_3$) δ ppm:

3.62-3.55 (2H, multiplet);
3.63-3.32 (1H, multiplet);
2.93 (3H, singlet);
2.69-2.56 (3H, multiplet);
2.52-2.43 (2H, multiplet);
2.41-2.33 (3H, multiplet);
2.02-1.94 (1H, multiplet).

Preparative Example 16

Cyclopropanespiro-6'-[(8a'S)-1',2',3',5',6',7',8',8a'-octahydroindolizin]-7'-one

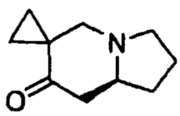

16(i) 1-Ethoxycarbonyl-1-[(S)-2-ethoxycarbonylmethylpyrrolidin-1-yl]methylcyclopropane

4.25 g of 10% palladium on carbon were added to a solution of 12.74 g (43.7 mmol) of (S)-1-benzyloxycarbonylhomoproline ethyl ester in 200 ml of ethanol, and the resulting mixture was stirred at room temperature for 2 hours under a hydrogen atmosphere. At the end of this time, the reaction mixture was filtered and the filtrate was concentrated by evaporation under reduced pressure. The resulting residue was dissolved in 150 ml of ethanol and 6.84 g (48.1 mmol) of 1-ethoxycarbonyl-1-formylcyclopropane were added to the solution thus obtained with ice-cooling. After the resulting mixture was stirred for 1 hour at 0°C, 1.92 g (30.6 mmol) of sodium cyanotrihydroborate were added and the mixture was then stirred for 2 hours at room temperature. At the end of this time, 300 ml of water were added to the reaction mixture, and it was then extracted with ethyl acetate. The organic extract was washed with water, dried over anhydrous magnesium sulfate and then concentrated by evaporation under reduced pressure. The resulting residue was purified by chromatography on a silica gel column using a 7:3 by volume mixture of hexane and ethyl acetate as the eluant to afford 3.07 g (yield: 25 %) of the title compound as a pale brown oil.
$^1$H-NMR spectrum (400 MHz, $CDCl_3$) δ ppm:

4.21-4.07 (4H, multiplet);
3.24 (1H, doublet, J=13 Hz);
3.22-3.12 (1H, multiplet);
2.82-2.68 (2H, multiplet);
2.28-2.13 (3H, multiplet);
2.03-1.90 (1H, multiplet);
1.82-1.64 (2H, multiplet);
1.56-1.45 (1H, multiplet);
1.36-1.21 (7H, multiplet);
1.12-1.03 (1H, multiplet);
0.84-0.70 (2H, multiplet).

16(ii) Cyclopropanespiro-6'-[(8a'S)-8'-ethoxycarbonyl-1',2',3',5',6',7',8',8a'-octahydroindolizin]-7'-one

518 mg (11.9 mmol) of 55% sodium hydride were suspended in 20 ml of toluene, 3 drops of methanol were added using a Pasteur pipette and the resulting mixture was heated to 130°C. 3.06 g (10.8 mmol) of 1-ethoxycarbonyl-1-[(S)-2-ethoxycarbonylmethylpyrrolidin-1-yl]methylcyclopropane [obtained as described in Preparative Example 16(i)

above] were then added and the resulting mixture was heated for 10 minutes under reflux. After the reaction mixture was cooled to 0°C, it was partitioned between a saturated aqueous solution of sodium chloride and ethyl acetate. The organic extract was washed with water, dried over anhydrous magnesium sulfate and then concentrated by evaporation under reduced pressure. The resulting residue was purified by chromatography on a silica gel column using a 9:1 by volume mixture of ethyl acetate and methanol as the eluant to afford 2.02 g (yield: 79 %) of the title compound as a colorless oil.
$^1$H-NMR spectrum (400 MHz, $CDCl_3$) δ ppm:

4.31-4.15 (2H, multiplet);
3.32 (1H, doublet, J=11 Hz);
3.15 (1H, triplet of doublets, J=9 Hz, 3 Hz);
2.92-2.82 (2H, multiplet);
2.67 (1H, doublet, J=11 Hz);
2.29 (1H, quartet, J=9 Hz);
2.12-1.80 (3H, multiplet);
1.71-1.55 (2H, multiplet);
1.29 (3H, triplet, J=7 Hz);
1.09-1.02 (1H, multiplet);
0.99-0.92 (1H, multiplet);
0.69-0.62 (1H, multiplet).

16(iii) Cyclopropanespiro-6'-[(8a'S)-1',2',3',5',6',7',8',8a'-octahydroindolizin]-7'-one

1 ml of a 1N aqueous solution of sodium hydroxide was added to a solution of 50 mg (0.21 mmol) of cyclopropanespiro-6'-[(8a'S)-8'-ethoxycarbonyl-1',2',3',5',6',7',8',8a'-octahydroindolizin]-7'-one [obtained as described in Preparative Example 16(ii) above] in 1 ml of ethanol, and the mixture thus obtained was heated for 1 hour under reflux. At the end of this time, the reaction mixture was concentrated by evaporation under reduced pressure, and the resulting residue was purified by chromatography on an alumina column using ethyl acetate as the eluant to afford 10 mg (yield: 29 %) of the title compound as a colorless oil.
$^1$H-NMR spectrum (400 MHz, $CDCl_3$) δ ppm:

3.17 (1H, triplet of doublets, J=9 Hz, 3 Hz);
2.76 (1H, doublet, J=12 Hz);
2.69 (1H, doublet, J=12 Hz);
2.67 (1H, doublet of doublets, J=16 Hz, 3 Hz);
2.56-2.44 (1H, multiplet);
2.34 (1H, doublet of doublets, J=16 Hz, 12 Hz);
2.30-2.18 (1H, multiplet);
2.12-1.93 (2H, multiplet);
1.90-1.78 (1H, multiplet);
1.64-1.50 (2H, multiplet);
1.08-1.00 (1H, multiplet);
0.96-0.88 (1H, multiplet);
0.67-0.58 (1H, multiplet).

Preparative Example 17

(8aS)-2,2-Dimethyl-1,2,3,5,6,7,8,8a-octahydroindolizin-7-one

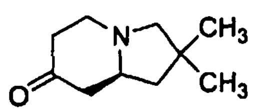

17(i) (S)-1-Benzyloxycarbonyl-4,4-diemthyl-2-hydroxymethylpyrrolidine

A solution of 32.35 g (226 mmol) of (S)-4,4-dimethyl-2-hydroxymethyl-5-oxopyrrolidine in 300 ml of tetrahydrofuran was added dropwise over a period of 20 minutes with ice-cooling and stirring, to keep the temperature between 8 and 17°C, to a suspension of 25.72 g (678 mmol) of lithium aluminum hydride in 500 ml of tetrahydrofuran. The resulting mixture was then heated under reflux for 7 hours, at the end of which time it was cooled to 0°C and 103 ml of a 4% aqueous solution of sodium hydroxide were added carefully thereto. Any insoluble material was removed by filtration and the filtrate was concentrated by evaporation under reduced pressure. The residue thus obtained was dissolved in 500 ml of methylene chloride and then 40.94 ml (294 mmol) of triethylamine were added, followed by the further addition of 38.71 ml (271 mmol) of benzyloxycarbonyl chloride with ice-cooling and stirring. The resulting mixtue was stirred for 1 hour at the same temperature and then stirred for a further 1 hour at room temperature. At the end of this time, a saturated aqueous solution of sodium hydrogencarbonate was added to the reaction mixture, and it was then extracted with methylene chloride. The organic extract was washed with water, dried over anhydrous magnesium sulfate and then concentrated by evaporation under reduced pressure. The resulting residue was purified by chromatography on a silica gel column using a 1:1 by volume mixture of hexane and ethyl acetate as the eluant to afford 43.97 g (yield: 74 %) of the title compound as a colorless oil.
$^1$H-NMR spectrum (400 MHz, $CDCl_3$) δ ppm:

7.42-7.29 (5H, multiplet);
5.20-5.10 (2H, multiplet);
4.89 (1H, doublet of doublets, J=9 Hz, 3 Hz);
4.14-4.05 (1H, multiplet);
3.73-3.59 (2H, multiplet);
3.41 (1H, doublet, J=11 Hz);
3.06 (1H, doublet, J=11 Hz);
1.80 (1H, doublet of double doublets, J=13 Hz, 7 Hz, 1 Hz);
1.33 (1H, doublet of doublets, J=12 Hz, 10 Hz);
1.08 (3H, singlet);
1.02 (3H, singlet).

17(ii) (8aS)-2,2-Dimethyl-1,2,3,5,6,7,8,8a-octahydroindolizin-7-one

In a similar manner to the procedures described in Preparative Examples 12(v), 12(vi) and 12(vii) above, reactions were conducted in turn, using (S)-1-benzyloxycarbonyl-4,4-dimethyl-2-hydroxymethylpyrrolidine [obtained as described in Preparative Example 17(i) above] instead of (2S,4S)-1-benzyloxycarbonyl-2-hydroxymethyl-4-methylthiopyrrolidine, to give the title compound as a brown oil (yield: 36 %).
$^1$H-NMR spectrum (400 MHz, $CDCl_3$) δ ppm:

3.26-3.19 (1H, multiplet);
2.89 (1H, doublet, J=9 Hz);
2.68-2.57 (1H, multiplet);
2.49-2.25 (5H, multiplet);
2.07 (1H, doublet, J=9 Hz);
1.73 (1H, doublet of doublets, J=12 Hz, 6 Hz);
1.40 (1H, doublet of doublets, J=12 Hz, 10 Hz);
1.20 (3H, singlet);
1.07 (3H, singlet).

Preparative Example 18

Cyclopentanespiro-2'-(1',2',3',5',6',7',8,8a'-octahydroindolizin)-7'-one

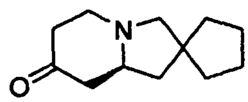

18(i) 1-Cyano-1-formylmethylcyclopentane diethyl acetal

100 ml (157 mmol) of a 1.57M solution of n-butyllithium in hexane were added dropwise with ice-cooling to a solution of 22.00 ml (157 mmol) of diisopropylamine in 500 ml of tetrahydrofuran, and the resulting mixture was then stirred for 30 minutes at the same temperature. At the end of this time, 14.89 ml (143 mmol) of cyclopentanecarbonitrile were added dropwise to the reaction mixture on a dry ice-acetone bath. The resulting mixture was stirred for 15 minutes at -78°C, at the end of which time a solution of 27.31 ml (157 mmol) of hexamethylphosphoramide in 50 ml of tetrahydrofuran were added dropwise. After stirring the resulting mixture for 30 minutes at -78°C, 23.62 ml (157 mmol) of bromoacetaldehyde diethyl acetal were added dropwise and the resulting mixture was then stirred for 2 hours at -78°C and for a further 20 hours at room temperature. At the end of this time, ice-water was added to the reaction mixture, which was then extracted with ethyl acetate. The organic extract was washed with water, dried over anhydrous magnesium sulfate and then concentrated by evaporation under reduced pressure. The resulting residue was purified by chromatography on a silica gel column using a 19:1 by volume mixture of hexane and ethyl acetate as the eluant to afford 23.92 g (yield: 79 %) of the title compound as a colorless oil.
$^1$H-NMR spectrum (400 MHz, $CDCl_3$) δ ppm:

4.75 (1H, triplet, J=5 Hz);
3.76-3.66 (2H, multiplet);
3.61-3.52 (2H, multiplet);
2.22-2.13 (2H, multiplet);
1.93 (2H, doublet, J=5 Hz);
1.91-1.63 (6H, multiplet);
1.23 (6H, triplet, J=7 Hz).

18(ii) 1-Aminomethyl-1-formylmethylcyclopentane diethyl acetal

8.66 ml (162 mmol) of concentrated sulfuric acid were added dropwise over a period of 10 minutes with ice-cooling and stirring to a suspension of 12.33 g (325 mmol) of lithium aluminum hydride in 460 ml of tetrahydrofuran. The resulting mixture was stirred for 1 hour at 0°C, at the end of which time 22.88 g (108 mmol) of 1-cyano-1-formylmethylcyclopentane diethylacetal [obtained as described in Preparative Example 18(i) above] were added to the reaction mixture in small portions before stirring the reaction mixture for 2 hours at room temperature. At the end of this time, the reaction mixture was cooled to 0°C again and 49.3 ml of a 4% aqueous solution of sodium hydroxide were added carefully. The insoluble materials were removed from the mixture by filtration and the filtrate was concentrated by evaporation under reduced pressure to afford 18.69 g (yield: 80 %) of the title compound as a pale yellow oil.
$^1$H-NMR spectrum (400 MHz, $CDCl_3$) δ ppm:

4.51 (1H, triplet, J=5 Hz);
3.70-3.60 (2H, multiplet);
3.54-3.41 (2H, multiplet);
2.50 (2H, singlet);
1.70 (2H, doublet, J=5 Hz);
1.69-1.34 (10H, multiplet);
1.21 (6H, triplet, J=7 Hz).

18(iii) Cyclopentanespiro-2'-(1',2',3',5',6',7',8',8a'-octahydroindolizin)-7'-one

8.67 ml (104 mmol) of methylvinylketone were added with ice-cooling and stirring to a solution of 18.68 g (86.7 mmol) of 1-aminomethyl-1-formylmethylcyclopentane diethyl acetal [obtained as described in Preparative Example 18 (ii) above] in 400 ml of diethyl ether, and then the resulting mixture was stirred for 24 hours at room temperature. At the end of this time, the reaction mixture was extracted with 200 ml of a 3N aqueous hydrochloric acid solution and the aqueous layer was stirred for 3 hours at 100°C. The reaction mixture was then cooled to room temperature, made alkaline by the addition of sodium hydrogencarbonate and then extracted with ethyl acetate. The organic extract was washed

with water, dried over anhydrous magnesium sulfate and then concentrated by evaporation under reduced pressure. The resulting residue was purified by chromatography on an alumina column using a 9:1 by volume mixture of hexane and ethyl acetate as the eluant to afford 5.75 g (yield: 34 %) of the title compound as a colorless oil.
$^1$H-NMR spectrum (400 MHz, $CDCl_3$) δ ppm:

3.27-3.21 (1H, multiplet);
3.03 (1H, doublet, J=9 Hz);
2.69-2.59 (1H, multiplet);
2.47 (1H, doublet of triplets, J=13 Hz, 2 Hz);
2.42-2.26 (4H, multiplet);
2.19 (1H, doublet, J=9 Hz);
1.86 (1H, doublet of doublets, J=12 Hz, 5 Hz);
1.80-1.46 (9H, multiplet).

Preparative Example 19

(8aS)-2-Methyl-3,5,6,7,8,8a-hexahydroindolizin-7-one

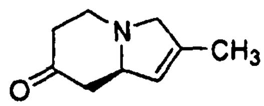

19(i) (S)-2-Carboxymethyl-4-methyl-3-pyrroline hydrochloride

150 ml of concentrated hydrochloric acid were added to 24.00 g (93.6 mmol) of (S)-1-benzyloxycarbonyl-2-cyanomethyl-4-methylidenepyrrolidine [obtained as described in Preparative Example 11(i) above], and the solution was stirred at 80°C overnight. At the end of this time, the reaction solution was concentrated by evaporation under reduced pressure and ethyl acetate was added to the residue thus obtained. The aqueous layer was then concentrated by evaporation under reduced pressure to afford 16.60 g (yield: quantitative) of the title compound as a white powder.
$^1$H-NMR spectrum (400 MHz, DMSO-$d_6$) δ ppm:

5.47-5.40 (1H, multiplet);
4.60-4.47 (1H, multiplet);
4.15-3.75 (2H, multiplet);
3.60-3.35 (2H, multiplet);
1.74 (3H, multiplet).

19(ii) (8aS)-2-Methyl-3,5,6,7,8,8a-hexahydroindolizin-7-one

In a similar manner to the procedures described in Preparative Examples 10(i), 10(ii), 10(iii), 10(iv) and 10(v) above, reactions were conducted in turn, using (S)-2-carboxymethyl-4-methyl-3-pyrroline hydrochloride [obtained as described in Preparative Example 19(i) above] in place of (2S,4S)-4-methoxyhomoproline hydrochloride, to give the title compound as an orange oil (yield: 10 %).
$^1$H-NMR spectrum (400 MHz, $CDCl_3$) δ ppm:

5.44-5.38 (1H, multiplet);
3.75-3.67 (1H, multiplet);
3.58 (1H, doublet of doublets, J=13 Hz, 3 Hz);
3.48-3.39 (1H, multiplet);
3.30-3.21 (1H, multiplet);
3.01-2.92 (1H, multiplet);
2.63-2.49 (2H, multiplet);
2.45-2.35 (2H, multiplet);
1.79 (3H, singlet).

Preparative Example 20.

(2R,8aS)-2-Ethoxy-1,2,3,5,6,7,8,8a-octahydroindolizin-7-one

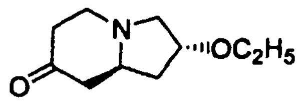

In a similar manner to the procedures described in Preparative Examples 1(i), 1(ii) and 1(iii) above, reactions were conducted in turn, using (2S,4R)-1-benzyloxycarbonyl-4-ethoxyproline instead of (2S,4R)-1-benzyloxycarbonyl-4-methoxyproline, to give the title compound as an orange oil (yield: 5 %).
$^1$H-NMR spectrum (400 MHz, $CDCl_3$) δ ppm:

4.21-4.13 (1H, multiplet);
3.60-3.38 (3H, multiplet);
3.28 (1H, doublet of doublets, J=11 Hz, 7 Hz);
2.66-2.20 (7H, multiplet);
1.99 (1H, doublet of doublets, J=13 Hz, 6 Hz);
1.82-1.70 (1H, multiplet);
1.20 (3H, triplet, J=7 Hz).

Preparative Example 21

(8aS)-2,2-Propylenedioxy-1,2,3,5,6,7,8,8a-octahydroindolizin-7-one

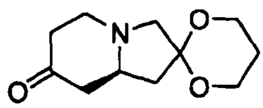

In a similar manner to the procedures described in Preparative Examples 7(i), 7(ii) and 7(iii) above, reactions were conducted in turn, using (S)-1-benzyloxycarbonyl-4,4-propylenedioxyproline methyl ester in place of (S)-1-benzyloxycarbonyl-4,4-ethylenedioxyproline methyl ester, to give the title compound as a pale yellow powder (yield: 16 %).
$^1$H-NMR spectrum (400 MHz, $CDCl_3$) δ ppm:

4.00-3.82 (4H, multiplet);
3.51 (1H, doublet, J=10 Hz);
3.33-3.25 (1H, multiplet);
2.74-2.62 (1H, multiplet);
2.57-2.48 (2H, multiplet);
2.43-2.31 (5H, multiplet);
1.86-1.62 (2H, multiplet);
1.82 (1H, doublet of doublets, J=13 Hz, 10 Hz).

Preparative Example 22

(8aS)-2,2-(2',2'-Dimethylpropylenedioxy)-1,2,3,5,6,7,8,8a-octahydroindolizin-7-one

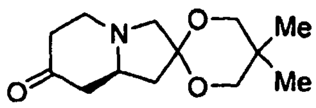

In a similar manner to the procedures described in Preparative Examples 7(i), 7(ii) and 7(iii) above, reactions were conducted in turn, using (S)-1-benzyloxycarbonyl-4,4-(2',2'-dimethylpropylenedioxy)proline methyl ester in place of (S)-1-benzyloxycarbonyl-4,4-ethylenedioxyproline methyl ester, to give the title compound as a pale yellow powder (yield: 19 %).
$^1$H-NMR spectrum (400 MHz, $GDCl_3$) δ ppm:

3.57-3.41 (5H, multiplet);
3.31-3.24 (1H, multiplet);
2.73-2.62 (1H, multiplet);
2.57-2.46 (2H, multiplet);
2.42-2.31 (5H, multiplet);
1.82 (1H, doublet of doublets, J=13 Hz, 10 Hz);
1.00 (3H, singlet);
0.96 (3H, singlet).

Preparative Example 23

(2S,8aS)-2-Phenoxy-1,2,3,5,6,7,8,8a-octahydroindolizin-7-one

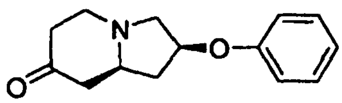

23(i) (2S,4S)-1-Benzyloxycarbonyl-4-phenoxyhomoproline benzyl ester

6.53 ml (74.3 mmol) of phenol and 19.48 g (74.3 mmol) of triphenylphosphine were added to a solution of 18.29 g (49.5 mmol) of (2S,4R)-1-benzyloxycarbonyl-4-hydroxyhomoproline benzyl ester in 300 ml of tetrahydrofuran, and the resulting mixture was cooled to 0°C. 11.69 ml (74.3 mmol) of diethylazodicarboxylate (DEAD) were added dropwise to the mixture at the same temperaure, and then the resulting mixture was stirred at room temperature overnight. At the end of this time, the reaction mixture was concentrated by evaporation under reduced pressure, diethyl ether was added to the residue, and then the insoluble materials were removed by filtration. The resulting filtrate was washed with a saturated aqueous solution of sodium hydrogencarbonate and with water in turn, dried over anhydrous magnesium sulfate and then concentrated by evaporation under reduced pressure. The resulting residue was purified by chromatography on a silica gel column using a 3:1 by volume mixture of hexane and ethyl acetate as the elaunt to afford 11.14 g (yield: 51 %) of the title compound as a colorless oil.
$^1$H-NMR spectrum (400 MHz, $CDCl_3$) δ ppm:

7.40-7.21 (12H, multiplet);
6.97 (1H, triplet, J=7 Hz);
6.84 (2H, doublet, J=8 Hz);
5.21-4.99 (4H, multiplet);
4.93-4.83 (1H, multiplet);
4.48-4.38 (1H, multiplet);
3.84-3.68 (2H, multiplet);
3.24-3.15 (0.6H, multiplet);
3.04-2.95 (0.4H, multiplet);
2.92-2.76 (1H, multiplet);

2.39-2.30 (1H, multiplet);
2.26-2.17 (1H, multiplet).

23(ii) (2S,4S)-4-Phenoxyhomoproline hydrochloride

2.22 g of 20% palladium hydroxide on carbon were added to a solution of 11.09 g (24.9 mmol) of (2S,4S)-1-benzyloxycarbonyl-4-phenoxyhomoproline benzyl ester [obtained as described in Preparative Example 23(i) above] in 220 ml of tetrahydrofuran, and then the mixture was stirred for 6 hours at room temperature under a hydrogen atmosphere. At the end of this time, 6.85 ml (27.4 mmol) of a 4N solution of hydrogen chloride in dioxane were added to the reaction mixture, and the mixture was filtered. The filtrate thus obtained was concentrated by evaporation under reduced pressure to afford 6.39 g (yield: quantitative) of the title compound as a pale brown powder.
$^1$H-NMR spectrum (400 MHz, DMSO-$d_6$) $\delta$ ppm:

12.70 (1H, broad singlet);
7.33 (2H, doublet of doublets, J=8 Hz, 7 Hz);
6.99 (1H, triplet, J=7 Hz);
6.96 (2H, doublet, J=8 Hz);
5.16-5.09 (1H, multiplet);
3.98-3.87 (1H, multiplet);
3.50 (1H, doublet of doublets, J=13 Hz, 5 Hz);
3.42-3.28 (2H, multiplet);
2.89 (1H, doublet of doublets, J=18 Hz, 8 Hz);
2.80 (1H, doublet of doublets, J=18 Hz, 6 Hz);
2.64-2.54 (1H, multiplet);
1.91-1.81 (1H, multiplet).

23(iii) (2S,8aS)-2-Phenoxy-1,2,3,5,6,7,8,8a-octahydroindolizin-7-one

In a similar manner to the procedures described in Preparative Examples 10(i), 10(ii), 10(iii), 10(iv) and 10(v) above, reactions were conducted in turn, using (2S,4S)-4-phenoxyhomoproline hydrochloride [obtained as described in Preparative Example 23(ii) above] in place of (2S,4S)-4-methoxyhomoproline hydrochloride, to give the title compound as a pale brown powder (yield: 25 %).
$^1$H-NMR spectrum (400 MHz, $CDCl_3$) $\delta$ ppm:

7.28 (2H, doublet of doublets, J=8 Hz, 7 Hz);
6.95 (1H, triplet, J=7 Hz);
6.87 (2H, doublet, J=8 Hz);
4.90-4.82 (1H, multiplet);
3.40 (1H, doublet, J=11 Hz);
3.39-3.32 (1H, multiplet);
2.80-2.68 (1H, multiplet);
2.67-2.27 (7H, multiplet);
1.86-1.74 (1H, multiplet).

Preparative Example 24

(8aS)-2-Ethylidene-1,2,3,5,6,7,8,8a-octahydroindolizin-7-one

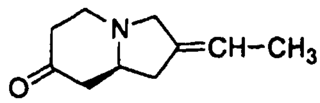

In a similar manner to the procedures described in Preparative Example 8(i) above, reactions were conducted in turn, using ethyltriphenylphosphonium bromide in place of methyltriphenylphosphonium bromide, and then the resulting

(S)-1-benzyloxycarbonyl-4-ethylideneproline methyl ester was subjected to reactions in turn in a similar manner to the procedures described in Preparative Examples 11(i), 11(ii), 11(iii) and 11(iv) above to give the title compound as a brown oil (yield: 9 %).
$^1$H-NMR spectrum (400 MHz, $CDCl_3$) δ ppm:

5.45-5.30 (1H, multiplet);
3.78 (0.5H, doublet, J=3 Hz);
3.64 (0.5H, doublet, J=3 Hz);
3.37-3.28 (1H, multiplet);
2.94-2.85 (1H, multiplet);
2.69-2.09 (8H, multiplet);
1.68-1.61 (3H, multiplet).

Preparative Example 25

(8aS)-2-Propylidene-1,2,3,5,6,7,8,8a-octahydroindolizin-7-one

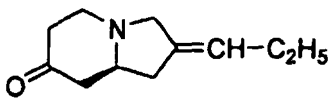

In a similar manner to the procedures described in Preparative Example 8(i) above, reactions were conducted in turn, using propyltriphenylphosphonium bromide in place of methyltriphenylphosphonium bromide, and then the resulting (S)-1-benzyloxycarbonyl-4-propylideneproline methyl ester was subjected to reactions in turn in a similar manner to the procedures described in Preparative Examples 1 1(i), 11 (ii), 11(iii) and 11(iv) above to give the title compound as a brown oil (yield: 10 %).
$^1$H-NMR spectrum (400 MHz, $CDCl_3$) δ ppm:

5.37-5.25 (1H, multiplet);
3.78 (0.5H, doublet, J=3 Hz);
3.62 (0.5H, doublet, J=3 Hz);
3.36-3.28 (1H, multiplet);
2.94-2.85 (1H, multiplet);
2.69-2.10 (8H, multiplet);
2.04-1.91 (2H, multiplet);
1.01-0.92 (3H, multiplet).

Preparative Example 26

(8aS)-2-Benzylidene-1,2,3,5,6,7,8,8a-octahydroindolizin-7-one

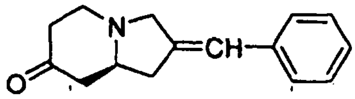

In a similar manner to the procedures described in Preparative Example 8(i) above, reactions were conducted in turn, using benzyltriphenylphosphonium bromide in place of methyltriphenylphosphonium bromide, and then the resulting (S)-1-benzyloxycarbonyl-4-benzylideneproline methyl ester was subjected to reactions in turn in a similar manner to the procedures described in Preparative Examples 11(i), I 1(ii), 11(iii) and 11(iv) above to give the title compound as a brown oil (yield: 0.4 %).

$^1$H-NMR spectrum (400 MHz, $CDCl_3$) δ ppm:

7.41-7.26 (4H, multiplet);
7.21-7.15 (1H, multiplet);
6.39-6.32 (1H, multiplet);
4.70-4.02 (1H, multiplet);
3.89-3.72 (1H, multiplet);
3.40-3.35 (1H, multiplet);
3.26-3.10 (1H, multiplet);
2.98-2.78 (1H, multiplet);
2.75-2.38 (5H, multiplet);
2.11-1.84 (1H, multiplet).

Preparative Example 27

(2S,8aS)-2-Ethyl-1,2,3,5,6,7,8,8a-octahydroindolizin-7-one

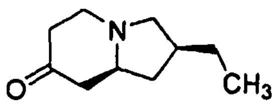

In a similar manner to the procedures described in Preparative Examples 8(ii), 8(iii), 8(iv), 8(v) and 8(vi) above, reactions were conducted in turn, using (S)-1-benzyloxycarbonyl-4-ethylideneproline methyl ester [prepared as described in Preparative Example 24 above] instead of (S)-1-benzyloxycarbonyl-4-methylideneproline methyl ester, to give the title compound as a brown oil (yield: 10 %).
$^1$H-NMR spectrum (400 MHz, $CDCl_3$) δ ppm:

3.28-3.23 (1H, multiplet);
2.87 (1H, doublet, J=9 Hz);
2.67-2.58 (1H, multiplet);
2.56-2.40 (2H, multiplet);
2.32-2.26 (4H, multiplet);
2.13-2.05 (2H, multiplet);
1.54-1.45 (2H, multiplet);
1.18 (1H, doublet, J=6 Hz);
0.90 (3H, triplet, J=7 Hz).

Preparative Example 28

(2S,8aS)-2-Propyl-1,2,3,5,6,7,8,8a-octahydroindolizin-7-one

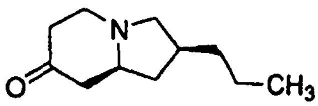

In a similar manner to the procedures described in Preparative Examples 8(ii), 8(iii), 8(iv), 8(v) and 8(vi) above, reactions were conducted in turn, using (S)-1-benzyloxycarbonyl-4-propylideneproline methyl ester [prepared as described in Preparative Example 25 above] instead of (S)-1-benzyloxycarbonyl-4-methylideneproline methyl ester, to give the title compound as a brown oil (yield: 24 %).
$^1$H-NMR spectrum (400 MHz, $CDCl_3$) δ ppm:

3.29-3.21 (1H, multiplet);
2.87-2.73 (1H, multiplet);
2.67-2.57 (1H, multiplet);
2.53-2.41 (1H, multiplet);
2.40-2.24 (3H, multiplet);
2.22-2.09 (2H, multiplet);
1.99-1.85 (1H, multiplet);
1.83-1.56 (1H, multiplet);
1.53-1.39 (2H, multiplet);
1.37-1.22 (2H, multiplet);
1.21-1.14 (1H, multiplet);
0.93-0.86 (3H, multiplet).

Preparative Example 29

(2S,8aS)-2-Benzyl-1,2,3,5,6,7,8,8a-octahydroindolizin-7-one

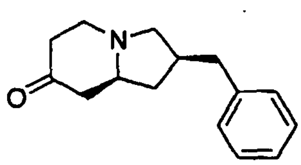

In a similar manner to the procedures described in Preparative Examples 8(ii), 8(iii), 8(iv), 8(v) and 8(vi) above, reactions were conducted in turn, using (S)-1-benzyloxycarbonyl-4-benzylideneproline methyl ester [prepared as described in Preparative Example 26 above] instead of (S)-1-benzyloxycarbonyl-4-methylideneproline methyl ester, to give the title compound as a brown oil (yield: 4 %).
$^1$H-NMR spectrum (400 MHz, $CDCl_3$) δ ppm:

7.38-7.28 (2H, multiplet);
7.27-7.11 (3H, multiplet);
3.36-3.23 (1H, multiplet);
3.04-2.88 (1H, multiplet);
2.86-2.21 (8H, multiplet);
2.11-2.05 (1H, multiplet);
2.02-1.72 (2H, multiplet);
1.34-1.25 (2H, multiplet).

Formulation Examples

The formulations containing a compound represented by the general formula (I-1) defined above, or a pharmaceutically acceptable salt, ester or amide thereof of the present invention can be prepared by methods such as the followings.

Formulation Example 1

Powder

5 g of the compound of Example 2, 895 g of lactose and 100 g of corn starch are mixed in a blender to provide the desired powder.

Formulation Example 2

Granules

5 g of the compound of Example 4, 865 g of lactose and 100 g of low-substituted hydroxypropylcellulose are mixed, 300 g of a 10% aqueous hydroxypropyl cellulose solution are added to the resulting mixture, and this is then kneaded. The product thus obtained is then granulated using an extrusion granulating machine and dried to provide the desired granules.

Formulation Example 3

Capsules

5 g of the compound of Example 6, 115 g of lactose, 58 g of corn starch and 2 g of magnesium stearate are mixed using a V-shaped mixer, No. 3 capsules are chosen and then each of said No. 3 capsules,is filled with 180 mg of the resulting mixture to provide the desired capsules.

Formulation example 4

Tablets

5 g of the compound of Example 8, 90 g of lactose, 34 g of corn starch, 20 g of crystalline cellulose and 1 g of magnesium stearate are mixed in a blender, and the resulting mixture is then formed into tablets with a tablet machine to provide the desired tablets.

Test Examples

Test Example 1

Inhibition of the Production of the Cytokines IL-1β and TNFα *in vitro* in Human Whole Blood

This test was performed according to the method of Hartman, et al. [D.A. Hartman, S.J. Ochalski and R.P. Carlson; The effects of anti-inflammatory and antiallergic drugs on cytokine release after stimulation of human whole blood by lipopolysaccharide and zymosan A: Inflamm. Res., 44, 269 (1995)].

Peripheral blood samples were collected in the presence of heparin from healthy adult volunteers. 1000 μl of whole blood were added to an Eppendorf tube to which 2 μl of a dimethyl sulfoxide solution of the test compound had been added in advance, after which 10 μl of lipopolysaccharide (E. coli O26: B6 origin, Difco) were added as a stimulant (final concentration of said lipopolysaccharide: 10 μg/ml). This was mixed well and then incubated for 6 hours at 37°C in the presence of 5% $CO_2$. At the end of the incubation, the mixture was cooled to 4°C to stop the reaction, followed immediately by centrifuging for 5 minutes at 14,000 rpm to separate and collect the supernatant plasma. The IL-1β and TNFα produced and released into the plasma were measured using a commercially available enzyme immunoassay (ELISA) kits [Cayman (IL-1β) and Genzyme (TNFα)]. The procedure was also repeated in the absence of test compound. The inhibitory effect [$IC_{50}$ (μM)] on the production of IL-1β and TNFα was determined by the method of least squares from the amounts of the cytokines produced in the presence and absence of the test compound. The results for the inhibitory effect on TNFα production are as shown in Table 7 below.

**Table 7 Inhibitory Effect on TNFα Production *(in vitro)***

| Test compound | $IC_{50}$ [μM] |
|---|---|
| Compound of Example 2 | 0.062 |
| Compound of Example 4 | 0.054 |
| Compound of Example 6 | 0.027 |
| Compound of Example 12 | 0.0025 |
| Compound of Example 14 | 0.0040 |
| Compound of Example 16 | 0.0022 |

Table continued

| Test compound | $IC_{50}$ [μM] |
|---|---|
| Compound of Example 19 | 0.044 |
| Compound of Example 21 | 0.046 |
| Compound of Example 23 | 0.0037 |
| Compound of Example 27 | 0.0038 |
| Compound of Example 29 | 0.0036 |
| Compound of Example 31 | 0.0024 |
| Compound of Example 32 | 0.0045 |
| Compound of Example 34 | 0.046 |
| Compound of Example 36 | 0.018 |
| Compound of Example 37 | 0.010 |
| Compound of Example 43 | 0.006 |
| Compound of Example 47 | 0.010 |
| Compound A | 1.90 |
| Compound B | 1.73 |

In Table 7 above, Compounds A and B are the following prior art compounds:

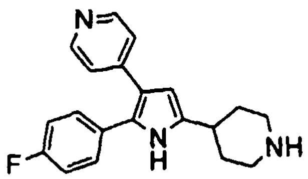

**Compound A (Compound of Example 4 of WO97/5877)**

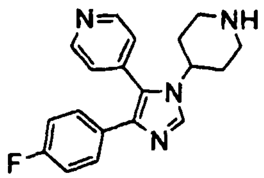

**Compound B (Compound of Example 23 of WO96/21452)**

The results for the inhibitory effect on IL-1β production are as shown in Table 8 below.

**Table 8 Inhibitory Effect on IL-1β Production *(in vitro)***

| Test compound | $IC_{50}$ [μM] |
|---|---|
| Compound of Example 2 | 0.031 |
| Compound of Example 4 | 0.041 |
| Compound of Example 12 | 0.0026 |
| Compound of Example 14 | 0.0092 |
| Compound of Example 16 | 0.0017 |
| Compound of Example 19 | 0.0083 |
| Compound of Example 21 | 0.018 |
| Compound of Example 23 | 0.0038 |
| Compound of Example 27 | 0.0014 |

Table continued

| Test compound | $IC_{50}$ [μM] |
|---|---|
| Compound of Example 29 | 0.0010 |
| Compound of Example 31 | 0.0046 |
| Compound of Example 32 | 0.0027 |
| Compound of Example 34 | 0.049 |
| Compound of Example 36 | 0.026 |
| Compound of Example 37 | 0.058 |
| Compound of Example 43 | 0.008 |
| Compound of Example 47 | 0.033 |

As shown in Tables 7 and 8 above, in this test, the compounds of the present invention showed excellent inhibitory activity against the production of TNFα and IL-1β *in vitro*.

Test Example 2

Inhibition of the Production of TNFα *in vivo*

This test was performed according to the method of Ochalski, et al. [S.J. Ochalski, D.A. Hartman, M.T. Belfast, T.L. Walter, K.B. Glaser and R.P. Carlson; Inhibition of endotoxin-induced hypothermia and serum TNF-α levels in CD-1 mice by various pharmacological agents: Agents Actions 39, C52-C54 (1993)].

The production of TNFα was induced in mice by the intravenous injection of lipopolysaccharide (E. coli O26: B6 origin, Difco) which was prepared to a concentration of 0.045 mg/ml using physiological saline. The saline preparation of lipopolysaccharide was administered at the rate of 10 ml/l kg of body weight into the caudal vein of Balb/c mice (males, age 5-7 weeks, body weight: approx. 22 g, Japan Charles River) which had been fasted overnight starting on the day before the experiment. One hour after administration, the mice were laparotomized under ether anaesthesia and blood was collected from the abdominal vena cava. Blood collection was performed using a 1 ml volume disposable syringe equipped with a 23G needle which had been moistened with heparin on the inside wall. Following blood collection, the blood was immediately transferred to a 1.5 ml volume Eppendorf tube and centrifuged at 4°C and 14,000 rpm to separate the plasma. This plasma was then stored at -20°C until measurement of TNFα. The measurement of the amount of TNFα was performed with a commercially available enzyme immunoassay (ELISA) kit (Mouse TNFα ELISA KIT, Genzyme).

To determine the inhibitory activity of the test compounds, each test compound was suspended in a 0.5% tragacanth solution and then administered orally to the Balb/c micc at the rate of 10 ml/1 kg of body weight 30 minutes before injection of lipopolysaccharide. The level of TNFα production was then determined as described above. In the control group, 0.5% tragacanth solution was administered at the rate of 10 ml/1 kg of body weight to the test mice instead of the solutions of the test compounds. A minimum of 3 dose levels of the test compound was administered to groups of 5 test mice for each test compound. The inhibitory rate relative to the control group was calculated for each dose level. From the inhibitory rates and the dosages, $ID_{50}$ values were calculated by the least squares method, the results being shown in Table 9 below.

**Table 9 Inhibitory Effect on TNFα Production *(in vivo)***

| Test compound | $ID_{50}$ [mg/kg] |
|---|---|
| Compound of Example 16 | 0.71 |
| Compound of Example 31 | 0.36 |
| Compound of Example 32 | 0.61 |
| Compound of Example 43 | 0.40 |

As can be seen from Table 9 above, the compounds of the present invention were found to show excellent inhibitory activity against the production of TNFα *in vivo*.

Test Example 3

Inhibition of the Production of IL-1β *in vivo*

This test was performed according to the method of Griffiths, et al. [Richard J. Griffiths, Ethan J. Stam, James T. Downs and Ivan G. Otterness; ATP Induces the Release of IL-1 from LPS-Primed Cells In Vivo: J. Immunol., 154, 2821-2828 (1995)].

The production of IL-1β was induced in mice by the intraperitoneal injection of lipopolysaccharide followed by the intraperitoneal injection of adenosine triphosphate (ATP). This was achieved by first administering a solution of lipopolysaccharide (E. coli 026: B6 origin, Difco), which had been prepared to a concentration of 0.0045 mg/ml using physiological saline, at the rate of 10 ml of said saline solution/1 kg of body weight into the peritoneal cavity of Balb/c mice (males, age 5-7 weeks, body weight: approx. 22 g, Japan Charles River) which had been fasted overnight starting on the day before the experiment. Two hours later, 0.5 ml of ATP, which had been prepared to a concentration of 6.03 mg/ml using physiological saline, were administered into the peritoneal cavity. 0.5 hours after the administration of ATP, the mice were sacrificed by suffocation using dry ice followed immediately by intraperitoneal injection of 3 ml of washing phosphate buffer solution [containing heparin (10 U/ml), p-toluenesulfonyl fluoride (0.25 mM), leupepsin (1 μg/ml), pepstatin (1 μg/ml) and EDTA (1 mM)] to wash the peritoneal cavity. A 1 ml volume disposable syringe equipped with a 21 G needle was then used to recover the washing liquid. After the recovery, the washing liquid from the peritoneal cavity was immediately transferred to a 1.5 ml volume Eppendorf tube and centrifuged at 4°C and 7,500 rpm to separate the supernatant. This supernatant was then stored at -20°C until measurement of IL-1β.

The measurement of the amount of IL-1β was performed with an enzyme immunoassay (ELISA) kit (Mouse IL-1β ELISA KIT, Genzyme).

To determine the inhibitory activity of the test compounds, each test compound was suspended in a 0.5% tragacanth solution and then administered orally to the Balb/c mice at the rate of 10 ml/l kg of body weight 30 minutes before injection of lipopolysaccharide. The level of TNFα production was then determined as described above. In the control group, 0.5% tragacanth solution was administered to the test mice at the rate of 10 ml/l kg of body weight instead of the solutions of the test compounds. A minimum of 3 dose levels of the test compound was administered to groups of 5 test mice for each test compound. The mean inhibitory rate relative to the control group was calculated for each dose level.

In this test, the compounds of the present invention demonstrated an excellent inhibitory effect against the production of IL-1β *in vivo.*

Test Example 4

Activity in Preventing the Development of Adjuvant-Induced Arthritis *in vivo*

The test was performed according to the method described by Winder et al. (Arthritis Rheum., 12, 472-482, 1969).

Heat-killed dried Mycobacterium butyricum (Difco Laboratories, Lot 679123) was ground on an agate mortar, and was then suspended in dry-sterilised liquid paraffin (first grade, Wako Pure Chemical Industries, Ltd.) to make a 2 mg/ml suspension. The resulting suspension was then sonicated and used as an adjuvant. Arthritis was induced by the intradermal injection of the adjuvant (100 μg of heat killed dried bacterium/0.05 ml of paraffin/paw) into the heel of the right hind paw of a Lewis rat (male, age 9 weeks, 190 g, Japan Charles River). The test compounds, which had been suspended in a 0.5% aqueous sodium carboxymethyl cellulose solution (CMC, Daiichi Pure Chemicals, Co., Ltd.), were administered orally at the rate of 5 ml/kg once a day from the day of injection of the adjuvant (day 0) to day 20.

The volumes of the right hind paw (adjuvant-injected paw) and left hind paw (non-injected paw) were measured on days 3, 5, 7, 10, 13, 15, 18 and 21 using a Plethysmometer™ (Ugo Basile), the hind paws being soaked from the toe to the hairline in the bath of the Plethysmometer™. The volumes of the swollen feet (adjuvant-injected right hind foot volume - non-injected left hind foot volume) were calculated. The percent inhibition of swelling of the injected foot of the treated animals as compared to that of the control animals on day 21 was calculated as follows.

$$\text{Inhibition (\%)} = \{1-(\text{swollen foot volume of compound-treated animals})/(\text{swollen foot volume of control animals})\} \times 100$$

A linear regression curve was obtained from the percent inhibition and the logarithmic value of the dosage by

the least squares method. $ID_{50}$ values were calculated using this curve, the results being shown in Table 10 below.

**Table 10 Activity in Preventing the Development of Adjuvant-Induced Arthritis *in vivo***

| Test compound | $ID_{50}$ [mg/kg] |
|---|---|
| Compound of Example 12 | 2.1 |
| Compound of Example 16 | 1.2 |

As can be seen from Table 10 above, in this test, the compounds of the present invention showed excellent activity in preventing the development of adjuvant-induced arthritis.

Test Example 5

Activity in Preventing the Development of Arthritis Induced by Anti-Collagen Antibody *in vivo*

In this test, an anti-collagen antibody-induced mouse arthritis model was employed.

0.5 ml (2 mg of antibody) of an anti-collagen antibody solution (4 mg/ml, Arthritogenic mAb Cocktail: product of Immuno-Biological Laboratories Co., Ltd) were injected into the caudal vein of Balb/c mice (males, age 5-6 weeks old, Japan Charles River). Three days after injection, 0.1 ml [0.05 mg of lipopolysaccharide] of a lipopolysaccharide solution (0.5 mg/ml, Arthritogenic mAb Cocktail: product of Immuno-Biological Laboratories Co., Ltd) were administered to the mice by intraperitoneal injection.

The test compounds, which had been suspended in 0.5% tragacanth were administered orally to the test animals at the rate of 10 ml/l kg of body weight once per day for 7 days from the day when the anti-collagen antibody was administered. To the mice of the control group, 0.5% tragacanth solution was administered at the rate of 10 ml/kg of body weight once per day for 7 days from the day when the anti-collagen antibody was administered, instead of solutions of the test compounds.

After the administration of the test compounds (or 0.5% tragacanth solution), the degree of edema in the 4 paws of each test mouse was scored according to the following basis:

0: normal (edema is not observed);
1: edema is observed in one of the five toes;
2: edema is observed in two or more of the five toes;
3: the whole of the paw is swollen.

The degree of arthritis in the test mouse was evaluated by the total of the edema scores in the 4 paws. The rate of suppression was calculated from the degrees of arthritis of the control animals and of the animals treated with the test compounds. From the rates of suppression and the dosages, $ID_{50}$ values were calculated by the least squares method.

In this test, the compounds of the present invention showed excellent activity in preventing the development of arthritis induced by anti-collagen antibody.

Test Example 6

Activity in Treating Arthritis Induced by Anti-Collagen Antibody *in vivo*

In this test, an anti-collagen antibody-induced mouse arthritis model was employed.

0.5 ml (2 mg of antibody) of an anti-collagen antibody solution (4 mg/ml, Arthritogenic mAb Cocktail: product of Immuno-Biological Laboratories Co., Ltd) were injected into the caudal vein of Balb/c mice (males, age 5-6 weeks old, Japan Charles River). Three days after injection, 0.1 ml [0.05 mg of lipopolysaccharide] of a lipopolysaccharide solution (0.5 mg/ml, Arthritogenic mAb Cocktail: product of Immuno-Biological Laboratories Co., Ltd) were administered to the mice by intraperitoneal injection.

7 days after the administration of the anti-collagen antibody solution, the degree of edema in the 4 paws of each test mouse was scored according to the basis as shown in Test Example 5 above.

Those mice in which edema in both the hind paws had been scored as "3" were selected. Test compounds, which had been suspended in 0.5% tragacanth solution, were administered orally to the selected mice at the rate of 10 ml/kg of body weight once per day for 3 days. To the mice of the control group, 0.5% tragacanth solution was administered at the rate of 10 ml/kg of body weight once per day for 3 days instead of solutions of the test compounds.

After the administration of the test compounds (or 0.5% tragacanth solution), the degree of arthritis in each test mouse was evaluated in the same manner as described in Test Example 5. The rates of treatment of arthritis induced by anti-collagen antibody were calculated from the degrees of arthritis of the control animals and of the compound-treated animals.

From the rates of treatment and the dosages, $ID_{50}$ values were calculated by the least squares method.

In this test, the compounds of the present invention showed excellent activity in treating arthritis induced by anti-collagen antibody.

Effect of the invention

As illustrated above, the compounds of the present invention exhibit excellent activity in inhibiting the production of inflammatory cytokines, particularly in inhibiting the production of IL-1β and TNFα. Furthermore, the compounds of the present invention have satisfactory oral absorptivity and a low level of toxicity. Consequently, the compounds of the present invention are useful as pharmaceuticals, suitable for the prohylaxis and treatment of both humans and animals. They can, for example, be used as an analgesic, an anti-inflammatory agent and an antiviral agent as well as an agent for use in the prophylaxis and treatment of chronic rheumatoid arthritis, degenerative arthritis, allergic diseases, asthma, septicaemia, psoriasis, osteoporosis, autoimmune diseases (e.g., systemic lupus erythematosus, ulcerative colitis, Crohn's disease and the like), diabetes, glomerular nephritis, hepatitis, cancer, ischemic heart disease, Alzheimer's disease and arteriosclerosis. Of these applications, the compounds of the present invention are particularly useful as an analgesic and an anti-inflammatory agent and as an agent for the prophlaxis and treatment of chronic rheumatoid arthritis, degenerative arthritis, allergic diseases, septicaemia, psoriasis, osteoporosis, ulcerative colitis, diabetes, hepatitis and arteriosclerosis.

## Claims

**1.** A compound of formula (I-1), or a pharmacologically acceptable salt, ester or amide thereof:

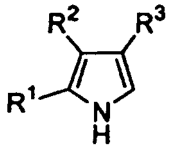

(I-1)

wherein:

$R^1$ is selected from the group consisting of aryl groups defined below which may optionally be substituted with at least one substituent selected from Substituent group a defined below and Substituent group β defined below, and heteroaryl groups defined below which may optionally be substituted with at least one substituent selected from Substituent group α defined below and Substituent group β defined below;

$R^2$ represents a heteroaryl group defined below which has at least one ring nitrogen atom, said heteroaryl group optionally being substituted with at least one substituent selected from Substituent group α defined below and Substituent group β defined below; and

$R^3$ represents a group of general formula (IIa), (IIb) or (IIc) shown below:

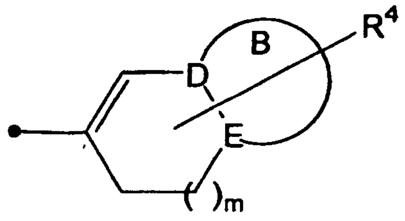

(IIa)

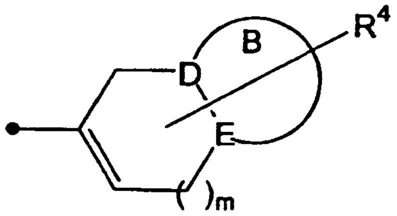

(IIb)

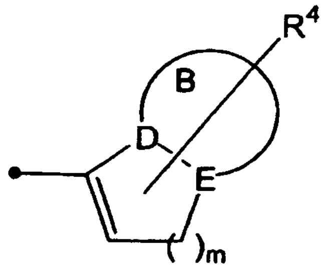

(IIc)

wherein

m represents 1 or 2,

one of D and E represents a nitrogen atom and the other represents a group of formula >C($R^5$)- (wherein $R^5$ is selected from the group consisting of hydrogen atoms, Substituent group α defined below and Substituent group β defined below),

B represents a 4- to 7-membered heterocyclic ring which has at least one ring nitrogen atom (said heterocyclic ring may be saturated or unsaturated, and may optionally be fused with a group selected from aryl groups defined below, heteroaryl groups defined below, cycloalkyl groups defined below and heterocyclyl groups defined below), and

$R^4$ represents from 1 to 3 substituents which are independently selected from the group consisting of Substituent group α defined below, Substituent group β defined below and Substituent group γ defined below, or

where B is a heterocyclic ring which is fused to an aryl group, a heteroaryl group, a cycloalkyl group or a heterocyclyl group, $R^4$ may be a hydrogen atom;

Substituent group α consists of hydroxyl groups, nitro groups, cyano groups, halogen atoms, lower alkoxy groups defined below, halogeno lower alkoxy groups defined below, lower alkylthio groups defined below, halogeno lower alkylthio groups defined below and groups of formula $-NR^aR^b$ (wherein $R^a$ and $R^b$ are the same or different from each other and each is independently selected from the group consisting of hydrogen atoms, lower alkyl groups defined below, lower alkenyl groups defined below, lower alkynyl groups defined below, aralkyl groups defined below and lower alkylsulfonyl groups defined below, or $R^a$ and $R^b$, taken together with the nitrogen atom to which they are attached, form a heterocyclyl group);

Substituent group β consists of lower alkyl groups defined below which may optionally be substituted with at least one substituent selected from Substituent group α defined above, lower alkenyl groups defined below which may optionally be substituted with at least one substituent selected from Substituent group α defined above, lower alkynyl groups defined below which may optionally be substituted with at least one substituent selected from Substituent group α defined above, aralkyl groups defined below and cycloalkyl groups defined below;

Substituents group γ consists of oxo groups, hydroxyimino groups, lower alkoxyimino groups defined below, lower alkylene groups defined below, lower alkylenedioxy groups defined below, lower alkylsulfinyl groups defined below, lower alkylsulfonyl groups defined below, aryl groups defined below which may optionally be substituted with at least one substituent selected from Substituent group α defined above and Substituent group β defined above, aryloxy groups defined below which may optionally be substituted with at least one substituent selected from Substituent group α defined above and Substituent group β defined above, lower alkylidenyl groups and aralkylidenyl groups;

said optionally substituted aryl groups in the definitions of $R^1$, ring B and Substituent group γ above are aromatic hydrocarbon groups having from 6 to 14 carbon atoms in one or more rings, said aryl groups optionally being fused with a cycloalkyl group having from 3 to 10 carbon atoms;

said optionally subsituted heteroaryl groups in the definition of $R^1$ and ring B above are 5- to 7-membered aromatic heterocyclic groups containing from 1 to 3 heteroatoms selected from the group consisting of sulfur atoms, oxygen atoms and nitrogen atoms, said heteroaryl groups optionally being fused with another cyclic group selected from the group consisting of aryl groups defined above and cycloalkyl groups having from 3 to 10 carbon atoms;

said optionally substituted heteroaryl groups having at least one ring nitrogen atom in the definition of $R^2$ above are 5- to 7-membered aromatic heterocyclic groups containing at least one nitrogen atom and optionally containing one or two further heteroatoms selected from the group consisting of sulfur atoms, oxygen atoms and nitrogen atoms;

said lower alkyl groups in the definitions of $R^a$, $R^b$ and Substituent group β above, and the lower alkyl moiety of said lower alkyl groups which may optionally be substituted with at least one substituent selected from Substituent group α in the definition of Substituent group β above are straight or branched alkyl groups having from 1 to 6 carbon atoms;
said lower alkenyl groups in the definitions of $R^a$, $R^b$ and Substituent group β above, and the lower alkenyl moiety of said lower alkenyl groups which may optionally be substituted with at least one substituent selected from Substituent group α in the definition of Substituent group β above are straight or branched alkenyl groups having from 2 to 6 carbon atoms;
said lower alkynyl groups in the definitions of $R^a$, $R^b$ and Substituent group β above, and the lower alkynyl moiety of said lower alkynyl groups which may optionally be substituted with at least one substituent selected from Substituent group α in the definition of Substituent group β above are straight or branched alkynyl groups having from 2 to 6 carbon atoms;
said aralkyl groups in the definitions of $R^a$, $R^b$ and Substituent group β above are lower alkyl groups as defined above which are substituted with at least one aryl group as defined above which may optionally be substituted with from I to 3 substituents selected from Substituent group α defined above and Substituent group β defined above;
said lower alkylsulfonyl groups in the definitions of $R^a$, $R^b$ and Substituent γ above are lower alkyl groups as defined above which are bonded to a sulfonyl group;
where ring B is fused with a heterocyclyl group, said heterocyclyl groups are 4- to 7-membered heterocyclyl groups which contain from 1 to 3 ring heteroatoms selected from the group consisting of oxygen atoms, sulfur atoms and nitrogen atoms;
where $R^a$ and $R^b$ together with the nitrogen atom to which they are attached represent a heterocyclyl group, said heterocyclyl groups are 4- to 7-membered heterocyclyl groups which contain one nitrogen atom and which optionally contain one further heteroatom selected from the group consisting of oxygen atoms, sulfur atoms and nitrogen atoms, said heterocyclyl groups optionally being fused with another cyclic group selected from the group consisting of aryl groups defined above and heteroaryl groups defined above;
said lower alkoxy groups in the definition of Substituent group α above are groups in which an oxygen atom is bonded to a lower alkyl group as defined above;
said halogeno lower alkoxy groups in the definition of Substituent group α above are groups in which a lower alkoxy group as defined above is substituted with at least one halogen atom;
said lower alkylthio groups in the definition of Substituent group α above are groups in which a sulfur atom is bonded to a lower alkyl group as defined above;
said halogeno lower alkylthio groups in the definition of Substituent group α above are groups in which a lower alkylthio group as defined above is substituted with at least one halogen atom;
said cycloalkyl groups in the definition of Substituent group β and ring B above are cycloalkyl groups having from 3 to 7 carbon atoms;
said lower alkoxyimino groups in the definition of Substituent group γ above are groups wherein the hydrogen atom of a hydroxyimino group is replaced by a lower alkyl group as defined above;
said lower alkylene groups in the definition of Substituent group γ above are alkylene groups having from 2 to 6 carbon atoms;
said lower alkylenedioxy groups in the definition of Substituent group γ above are groups wherein an alkylene moiety, which is a straight or branched chain alkylene group having from 1 to 6 carbon atoms, is subsitituted with 2 oxy groups; said lower alkylsulfinyl groups in the definition of Substituent group γ above are groups in which a lower alkyl group as defined above is bonded to a sulfinyl group;
said lower alkylidenyl groups in the definition of Substituent group γ above are straight or branched alkylidenyl groups having from 1 to 6 carbon atoms;
said aralkylidenyl groups in the definition of Substituent group γ above are lower alkylidenyl groups as defined above which are substituted with 1 or more aryl groups as defined above;
said optionally substituted aryloxy groups in the definition of Substituent group γ above are groups in which an oxygen atom is attached to an aryl group as defined above.

**2.** A compound according to claim 1 or a pharmacologically acceptable salt, ester or amide thereof, wherein $R^1$ is an aryl group which may optionally be substituted with at least one substituent selected from Substituent group a and Substituent group β as defined in claim 1.

**3.** A compound according to claim 1 or a pharmacologically acceptable salt, ester or amide thereof, wherein $R^1$ is a phenyl or naphthyl group, said groups optionally being substituted with at least one substituent selected from Substituent group a and Substituent group β as defined in claim 1.

**4.** A compound according to claim 1 or a pharmacologically acceptable salt, ester or amide thereof, wherein $R^1$ is a phenyl group which may optionally be substituted with at least one substituent selected from Substituent group $\alpha^1$ defined below and Substituent group $\beta^1$ defined below;
said Substituent group $\alpha^1$ consists of halogen atoms, lower alkoxy groups, halogeno lower alkoxy groups and groups of formula $-NR^aR^b$ (wherein one of $R^a$ and $R^b$ represents a hydrogen atom or a lower alkyl group, and the other represents a hydrogen atom, a lower alkyl group or an aralkyl group), said lower alkyl groups, lower alkoxy groups, halogeno lower alkoxy groups and aralkyl groups being as defined in claim 1;
said Substituent group $\beta^1$ consists of lower alkyl groups, halogeno lower alkyl groups, hydroxyl lower alkyl groups, nitro lower alkyl groups, amino lower alkyl groups, lower alkylamino lower alkyl groups, di(lower alkyl)amino lower alkyl groups and aralkylamino lower alkyl groups, said lower alkyl groups, halogeno lower alkyl groups, alkyl moieties of said hydroxyl lower alkyl groups, nitro lower alkyl groups, amino lower alkyl groups, lower alkylamino lower alkyl groups and di(lower alkyl)amino lower alkyl groups and alkyl and aralkyl moieties of said aralkylamino lower alkyl groups being as defined in claim 1.

**5.** A compound according to claim 1 or a pharmacologically acceptable salt, ester or amide thereof, wherein $R^1$ is a phenyl group which may optionally be substituted with at least one substituent selected from the group consisting of halogen atoms, halogeno lower alkyl groups as defined in claim 1 and halogeno lower alkoxy groups as defined in claim 1.

**6.** A compound according to claim 1 or a pharmacologically acceptable salt, ester or amide thereof, wherein $R^1$ is a substituent selected from the group consisting of phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl and 3-trifluoromethylphenyl groups.

**7.** A compound according to any one of claims 1 to 6 or a pharmacologically acceptable salt, ester or amide thereof, wherein $R^2$ is a 5- or 6-membered aromatic heterocyclic group which has one or two nitrogen atoms, said group optionally being substituted with at least one substituent selected from Substituent group $\alpha$ and Substituent group $\beta$ as defined in claim 1.

**8.** A compound according to any one of claims 1 to 6 or a pharmacologically acceptable salt, ester or amide thereof, wherein $R^2$ is a pyridyl or pyrimidinyl group, said groups optionally being substituted with at least one substituent selected from the group consisting of Substituent group $\alpha$ and Substituent group $\beta$ as defined in claim 1.

**9.** A compound according to any one of claims 1 to 6 or a pharmacologically acceptable salt, ester or amide thereof, wherein $R^2$ is a 4-pyridyl or 4-pyrimidinyl group, said groups optionally being substituted with at least one substituent selected from the group consisting of Substituent group $\alpha$ and Substituent group $\beta$ as defined in claim 1.

**10.** A compound according to any one of claims 1 to 6 or a pharmacologically acceptable salt, ester or amide thereof, wherein $R^2$ is a 4-pyridyl or 4-pyrimidinyl group, said groups optionally being substituted at the 2-position thereof with a substituent selected from the group consisting of Substituent group $\alpha$ and Substituent group $\beta$ as defined in claim 1.

**11.** A compound according to any one of claims 1 to 6 or a pharmacologically acceptable salt, ester or amide thereof, wherein $R^2$ is a 4-pyridyl or 4-pyrimidinyl group, said groups optionally being substituted at the 2-position thereof with a substituent selected from the group consisting of methoxy, amino, methylamino, benzylamino, and $\alpha$-methylbenzylamino groups.

**12.** A compound according to any one of claims 1 to 11 or a pharmacologically acceptable salt, ester or amide thereof, wherein B is a 5- or 6-membered heterocyclic ring which has one ring nitrogen atom and optionally has one further ring heteroatom or ring group selected from a nitrogen atom, oxygen atom, sulfur atom, >SO and $>SO_2$ (said ring may be saturated or unsaturated and may optionally be fused with an aryl group, a heteroaryl group, a cycloalkyl group or a heterocyclyl group as defined in claim 1).

**13.** A compound according to any one of claims 1 to 11 or a pharmacologically acceptable salt, ester or amide thereof, wherein B is a 5- or 6-membered heterocyclic ring which consists of the group D, the group E and three or four carbon atoms (said ring may be saturated or unsaturated and may optionally be fused with an aryl group, a heteroaryl group, a cycloalkyl group or a heterocyclyl group as defined in claim 1).

**14.** A compound according to any one of claims 1 to 11 or a pharmacologically acceptable salt, ester or amide thereof, wherein B is a pyrrolidinyl ring or a pyrrolinyl ring.

**15.** A compound according to any one of claims 1 to 14 or a pharmacologically acceptable salt, ester or amide thereof, wherein $R^3$ is a group of general formula (IIa) or general formula (IIb).

**16.** A compound according to any one of claims 1 to 14 or a pharmacologically acceptable salt, ester or amide thereof, wherein $R^3$ is a group of general formula (IIa).

**17.** A compound according to any one of claims I to 16 or a pharmacologically acceptable salt, ester or amide thereof, wherein m is 1.

**18.** A compound according to any one of claims 1 to 17 or a pharmacologically acceptable salt, ester or amide thereof, wherein $R^4$ is 1 or 2 substituents which are independently selected from the group consisting of Substituent group $\alpha$ as defmed in claim 1, Substituent group $\beta$ as defined in claim 1 and Substituent group $\gamma^1$, wherein said Substituent group $\gamma^1$ consists of oxo groups, hydroxyimino groups, lower alkoxyimino groups, lower alkylene groups, lower alkylenedioxy groups, lower alkylsulfinyl groups, lower alkylsulfonyl groups and aryl groups which may optionally be substituted with at least one substituent selected from Substituent group $\alpha$ and Substituent group $\beta$ as defined in claim 1.

**19.** A compound according to any one of claims 1 to 17 or a pharmacologically acceptable salt, ester or amide thereof, wherein $R^4$ is a substituent selected from the group consisting of hydroxy groups, halogen atoms, lower alkoxy groups, lower alkylthio groups, halogeno lower alkoxy groups, lower alkyl groups, halogeno lower alkyl groups, oxo groups, aryl groups optionally substituted with at least one substituent selected from Substituent group $\alpha$ and Substituent group $\beta$, lower alkylenedioxy groups, lower alkylene groups and lower alkylsulfonyl groups as defined in claim 1.

**20.** A compound according to any one of claims I to 17 or a pharmacologically acceptable salt, ester or amide thereof, wherein $R^4$ is a substituent selected from the group consisting of hydroxy groups, fluorine atoms, chlorine atoms, methoxy groups, ethoxy groups, propoxy groups, methyl groups, ethyl groups, propyl groups and phenyl groups which may optionally be substituted with at least one substituent selected from Substituent group $\alpha$ and Substituent group $\beta$ as defined in claim 1.

**21.** A compound according to any one of claims 1 to 17 or a pharmacologically acceptable salt, ester or amide thereof, wherein $R^4$ is a substituent selected from the group consisting of methoxy groups, methyl groups, ethyl groups, propyl groups and phenyl groups.

**22.** A compound according to any one of claims 1 to 17 or a pharmacologically acceptable salt, ester or amide thereof, wherein $R^4$ is a substituent selected from the group consisting of aryloxy groups which may optionally be substituted with at least one substituent selected from Substituent group $\alpha$ and Substituent group $\beta$, alkylidene groups and aralkylidene groups as defined in claim 1.

**23.** A compound according to any one of claims 1 to 17 or a pharmacologically acceptable salt, ester or amide thereof, wherein $R^4$ is a substituent selected from the group consisting of phenoxy, methylidene, ethylidene, propylidene and benzylidene groups.

**24.** A compound according to any one of claims 1 to 23 or a pharmacologically acceptable salt, ester or amide thereof, wherein D is a group of formula >C($R^5$)-(wherein $R^5$ is selected from the group consisting of hydrogen atoms, Substituent group $\alpha$ and Substituent group $\beta$ as defined in claim 1) and E is a nitrogen atom.

**25.** A compound according to claim 1 or a pharmacologically acceptable salt, ester or amide thereof, wherein:

$R^1$ is an aryl group which may optionally be substituted with at least one substituent selected from Substituent group $\alpha$ and Substituent group $\beta$;
$R^2$ is a 5- or 6-membered aromatic heterocyclic group which has one or two nitrogen atoms, said group optionally being substituted with at least one substituent selected from Substituent group $\alpha$ and Substituent group $\beta$; and
$R^3$ is a group of general formula (IIa) or general formula (IIb) wherein
m is 1,

D is a group of formula >C($R^5$)- (wherein $R^5$ is selected from the group consisting of hydrogen atoms, Substituent group $\alpha$ and Substituent group $\beta$) and E is a nitrogen atom, and
$R^4$ is 1 or 2 substituents which are independently selected from the group consisting of Substituent group $\alpha$, Substituent group $\beta$ and Substituent group $\gamma^1$, wherein said Substituent group $\gamma^1$ consists of oxo groups, hydroxyimino groups, lower alkoxyimino groups, lower alkylene groups, lower alkylenedioxy groups, lower alkylsulfinyl groups, lower alkylsulfonyl groups and aryl groups which may optionally be substituted with at least one substituent selected from Substituent group $\alpha$ and Substituent group $\beta$.

**26.** A compound according to claim 1 or a pharmacologically acceptable salt, ester or amide thereof, wherein:

$R^1$ is a phenyl or naphthyl group, said groups optionally being substituted with at least one substituent selected from Substituent group $\alpha$ and Substituent group $\beta$;
$R^2$ is a pyridyl or pyrimidinyl group, said groups optionally being substituted with at least one substituent selected from the group consisting of Substituent group $\alpha$ and Substituent group $\beta$; and
$R^3$ is a group of general formula (IIa) or general formula (IIb) wherein
m is 1,
D is a group of formula >C($R^5$)- (wherein $R^5$ is selected from the group consisting of hydrogen atoms, Substituent group $\alpha$ and Substituent group $\beta$) and E is a nitrogen atom, and
$R^4$ is a substituent selected from the group consisting of hydroxy groups, halogen atoms, lower alkoxy groups, lower alkylthio groups, halogeno lower alkoxy groups, lower alkyl groups, halogeno lower alkyl groups, oxo groups, aryl groups optionally substituted with at least one substituent selected from Substituent group $\alpha$ and Substituent group $\beta$, lower alkylenedioxy groups, lower alkylene groups and lower alkylsulfonyl groups.

**27.** A compound according to claim 1 or a pharmacologically acceptable salt, ester or amide thereof, wherein:

$R^1$ is a phenyl group which may optionally be substituted with at least one substituent selected from Substituent group $\alpha^1$ defined below and Substituent group $\beta^1$ defined below,
said Substituent group $\alpha^1$ consists of halogen atoms, lower alkoxy groups, halogeno lower alkoxy groups and groups of formula -$NR^aR^b$ (wherein one of $R^a$ and $R^b$ represents a hydrogen atom or a lower alkyl group, and the other represents a hydrogen atom, a lower alkyl group or an aralkyl group), said lower alkyl groups, lower alkoxy groups, halogeno lower alkoxy groups and aralkyl groups being as defined in claim 1, and
said Substituent group $\beta^1$ consists of lower alkyl groups, halogeno lower alkyl groups, hydroxyl lower alkyl groups, nitro lower alkyl groups, amino lower alkyl groups, lower alkylamino lower alkyl groups, di(lower alkyl) amino lower alkyl groups and aralkylamino lower alkyl groups, said lower alkyl groups, halogeno lower alkyl groups, alkyl moieties of said hydroxyl lower alkyl groups, nitro lower alkyl groups, amino lower alkyl groups, lower alkylamino lower alkyl groups and di(lower alkyl)amino lower alkyl groups and alkyl and aralkyl moieties of said aralkylamino lower alkyl groups being as defined in claim 1;
$R^2$ is a 4-pyridyl or 4-pyrimidinyl group, said groups optionally being substituted with at least one substituent selected from the group consisting of Substituent group $\alpha$ and Substituent group $\beta$; and
$R^3$ is a group of general formula (IIa) or general formula (IIb) wherein
m is 1,
D is a group of formula >C($R^5$)- (wherein $R^5$ is selected from the group consisting of hydrogen atoms, Substituent group $\alpha$ and Substituent group $\beta$) and E is a nitrogen atom, and
$R^4$ is a substituent selected from the group consisting of hydroxy groups, halogen atoms, lower alkoxy groups, lower alkylthio groups, halogeno lower alkoxy groups, lower alkyl groups, halogeno lower alkyl groups, oxo groups, aryl groups optionally substituted with at least one substituent selected from Substituent group $\alpha$ and Substituent group $\beta$, lower alkylenedioxy groups, lower alkylene groups and lower alkylsulfonyl groups.

**28.** A compound according to claim 1 or a pharmacologically acceptable salt, ester or amide thereof, wherein:

$R^1$ is a phenyl group which may optionally be substituted with at least one substituent selected from the group consisting of halogen atoms, halogeno lower alkyl groups as defined in claim 1 and halogeno lower alkoxy groups;
$R^2$ is a 4-pyridyl or 4-pyrimidinyl group, said groups optionally being substituted at the 2-position thereof with a substituent selected from the group consisting of Substituent group $\alpha$ and Substituent group $\beta$; and
$R^3$ is a group of general formula (IIa) wherein
m is 1,
D is a group of formula >C($R^5$)- (wherein $R^5$ is selected from the group consisting of hydrogen atoms, Substituent group $\alpha$ and Substituent group $\beta$) and E is a nitrogen atom, and

$R^4$ is a substituent selected from the group consisting of hydroxy groups, fluorine atoms, chlorine atoms, methoxy groups, ethoxy groups, propoxy groups, methyl groups, ethyl groups, propyl groups and phenyl groups which may optionally be substituted with at least one substituent selected from Substituent group a and Substituent group β.

**29.** A compound according to claim 1 or a pharmacologically acceptable salt, ester or amide thereof, wherein:

$R^1$ is a substituent selected from the group consisting of phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl and 3-trifluoromethylphenyl groups;
$R^2$ is a 4-pyridyl or 4-pyrimidinyl group, said groups optionally being substituted at the 2-position thereof with a substituent selected from the group consisting of methoxy, amino, methylamino, benzylamino, and α-methylbenzylamino groups; and
$R^3$ is a group of general formula (IIa) wherein
m is 1,
D is a group of formula >C($R^5$)- (wherein $R^5$ is selected from the group consisting of hydrogen atoms, Substituent group α and Substituent group β) and E is a nitrogen atom, and
$R^4$ is a substituent selected from the group consisting of methoxy groups, methyl groups, ethyl groups, propyl groups and phenyl groups.

**30.** A compound according to claim 1 or a pharmacologically acceptable salt, ester or amide thereof, wherein:

$R^1$ is an aryl group which may optionally be substituted with at least one substituent selected from Substituent group α and Substituent group β;
$R^2$ is a 5- or 6-membered aromatic heterocyclic group which has one or two nitrogen atoms, said group optionally being substituted with at least one substituent selected from Substituent group α and Substituent group β; and
$R^3$ is a group of general formula (IIa) or general formula (IIb) wherein
m is 1,
D is a group of formula >C($R^5$)- (wherein $R^5$ is selected from the group consisting of hydrogen atoms, Substituent group α and Substituent group β) and E is a nitrogen atom, and
$R^4$ is a substituent selected from the group consisting of aryloxy groups which may optionally be substituted with at least one substituent selected from Substituent group α and Substituent group β, alkylidene groups and aralkylidene groups.

**31.** A compound according to claim 1 or a pharmacologically acceptable salt, ester or amide thereof, wherein:

$R^1$ is a phenyl or naphthyl group, said groups optionally being substituted with at least one substituent selected from Substituent group α and Substituent group β;
$R^2$ is a pyridyl or pyrimidinyl group, said groups optionally being substituted with at least one substituent selected from the group consisting of Substituent group α and
Substituent group β; and
$R^3$ is a group of general formula (IIa) or general formula (IIb) wherein
m is 1,
D is a group of formula >C($R^5$)- (wherein $R^5$ is selected from the group consisting of hydrogen atoms, Substituent group α and Substituent group β) and E is a nitrogen atom, and
$R^4$ is a substituent selected from the group consisting of aryloxy groups which may optionally be substituted with at least one substituent selected from Substituent group α and Substituent group β, alkylidene groups and aralkylidene groups.

**32.** A compound according to claim 1 or a pharmacologically acceptable salt, ester or amide thereof, wherein:

$R^1$ is a phenyl group which may optionally be substituted with at least one substituent selected from Substituent group $\alpha^1$ defined below and Substituent group $\beta^1$ defined below,
said Substituent group $\alpha^1$ consists of halogen atoms, lower alkoxy groups, halogeno lower alkoxy groups and groups of formula $-NR^aR^b$ (wherein one of $R^a$ and $R^b$ represents a hydrogen atom or a lower alkyl group, and the other represents a hydrogen atom, a lower alkyl group or an aralkyl group), said lower alkyl groups, lower alkoxy groups, halogeno lower alkoxy groups and aralkyl groups being as defined in claim 1, and
said Substituent group $\beta^1$ consists of lower alkyl groups, halogeno lower alkyl groups, hydroxyl lower alkyl

groups, nitro lower alkyl groups, amino lower alkyl groups, lower alkylamino lower alkyl groups, di(lower alkyl) amino lower alkyl groups and aralkylamino lower alkyl groups, said lower alkyl groups, halogeno lower alkyl groups, alkyl moieties of said hydroxyl lower alkyl groups, nitro lower alkyl groups, amino lower alkyl groups, lower alkylamino lower alkyl groups and di(lower alkyl)amino lower alkyl groups and alkyl and aralkyl moieties of said aralkylamino lower alkyl groups being as defined in claim 1;
$R^2$ is a 4-pyridyl or 4-pyrimidinyl group, said groups optionally being substituted with at least one substituent selected from the group consisting of Substituent group α and Substituent group β; and
$R^3$ is a group of general formula (IIa) or general formula (IIb) wherein
m is 1,
D is a group of formula >C($R^5$)- (wherein $R^5$ is selected from the group consisting of hydrogen atoms, Substituent group α and Substituent group β) and E is a nitrogen atom, and
$R^4$ is a substituent selected from the group consisting of aryloxy groups which may optionally be substituted with at least one substituent selected from Substituent group α and Substituent group β, alkylidene groups and aralkylidene groups.

**33.** A compound according to claim 1 or a pharmacologically acceptable salt, ester or amide thereof, wherein:

$R^1$ is a phenyl group which may optionally be substituted with at least one substituent selected from the group consisting of halogen atoms, halogeno lower alkyl groups as defined in claim 1 and halogeno lower alkoxy groups;
$R^2$ is a 4-pyridyl or 4-pyrimidinyl group, said groups optionally being substituted at the 2-position thereof with a substituent selected from the group consisting of Substituent group α and Substituent group β; and
$R^3$ is a group of general formula (IIa) wherein
m is 1,
D is a group of formula >C($R^5$)- (wherein $R^5$ is selected from the group consisting of hydrogen atoms, Substituent group α and Substituent group β) and E is a nitrogen atom, and
$R^4$ is a substituent selected from the group consisting of phenoxy, methylidene, ethylidene, propylidene and benzylidene groups.

**34.** A compound according to claim 1 or a pharmacologically acceptable salt, ester or amide thereof, wherein:

$R^1$ is a substituent selected from the group consisting of phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl and 3-trifluoromethylphenyl groups;
$R^2$ is a 4-pyridyl or 4-pyrimidinyl group, said groups optionally being substituted at the 2-position thereof with a substituent selected from the group consisting of methoxy, amino, methylamino, benzylamino, and α-methylbenzylamino groups; and
$R^3$ is a group of general formula (IIa) wherein
m is 1,
D is a group of formula >C($R^5$)- (wherein $R^5$ is selected from the group consisting of hydrogen atoms, Substituent group α and Substituent group β) and E is a nitrogen atom, and
$R^4$ is a substituent selected from the group consisting of phenoxy, methylidene, ethylidene, propylidene and benzylidene groups.

**35.** A compound according to claim 1 selected from the following compounds or a pharmacologically acceptable salt or amide thereof:

2-(3-fluorophenyl)-4-[2-methyl-1,2,3,5,6,8a-hexahydroindolizin-7-yl]-3-(pyridin-4-yl)-1*H*-pyrrole,
2-(3-fluorophenyl)-4-[2-phenyl-1,2,3,5,6,8a-hexahydroindolizin-7-yl]-3-(pyridin-4-yl)-1*H*-pyrrole,
2-(3-fluorophenyl)-4-[2-methoxy-1,2,3,5,6,8a-hexahydroindolizin-7-yl]-3-(pyridin-4-yl)-1*H*-pyrrole,
2-(4-fluorophenyl)-4-[2-methyl-1,2,3,5,6,8a-hexahydroindolizin-7-yl]-3-(pyridin-4-yl)-1*H*-pyrrole,
2-(4-fluorophenyl)-4-[2-phenyl-1,2,3,5,6,8a-hexahydroindolizin-7-yl]-3-(pyridin-4-yl)-1*H*-pyrrole,
2-(4-fluorophenyl)-4-[2-methoxy-1,2,3, 5,6,8a-hexahydroindolizin-7-yl]-3-(pyridin-4-yl)-1*H*-pyrrole,
2-(3-chlorophenyl)-4-[2-methyl-1,2,3,5,6,8a-hexahydroindolizin-7-yl]-3-(pyridin-4-yl)-1*H*-pyrrole,
2-(3-chlorophenyl)-4-[2-phenyl-1,2,3,5,6,8a-hexahydroindolizin-7-yl]-3-(pyridin-4-yl)-1*H*-pyrrole,
2-(3-chlorophenyl)-4-[2-methoxy-1,2,3,5,6,8a-hexahydroindolizin-7-yl]-3-(pyridin-4-yl)-1*H*-pyrrole,
4-[2-methyl-1,2,3,5,6,8a-hexahydroindolizin-7-yl]-3-(pyridin-4-yl)-2-(3-trifluoromethylphenyl)-1*H*-pyrrole,
4-[2-phenyl-1,2,3,5,6,8a-hexahydroindolizin-7-yl]-3-(pyridin-4-yl)-2-(3-trifluoromethylphenyl)-1*H*-pyrrole,
4-[2-methoxy-1,2,3,5,6,8a-hexahydroindolizin-7-yl]-3-(pyridin-4-yl)-2-(3-trifluoromethylphenyl)-1*H*-pyrrole.

2-(4-fluorophenyl)-4-[2-methyl-3,5,6,8a-tetrahydroindolizin-7-yl]-3-(pyridin-4-yl)-1H-pyrrole,
2-(4-fluorophenyl)-4-[2-methylidene-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-2-(4-fluorophenyl)-3-(pyridin-4-yl)-1*H*-pyrrole,
4-[2-ethyl-1,2,3,5,6,8a-hexahydroindolizin-7-yl]-2-(3-fluorophenyl)-3-(pyridin-4-yl)-1*H*-pyrrole,
2-(3-chlorophenyl)-4-[2-ethyl-1,2,3,5,6,8a-hexahydroindolizin-7-yl]-3-(pyridin-4-yl)-1*H*-pyrrole,
4-[2-ethyl-1,2,3,5,6,8a-hexahydroindolizin-7-yl]-3-(pyridin-4-yl)-2-(3-trifluoromethylphenyl)-1*H*-pyrrole,
2-(4-fluorophenyl)-4-[2-propyl-1,2,3,5,6,8a-hexahydroindolizin-7-yl]-3-(pyridin-4-yl)-1*H*-pyrrole,
2-(3-fluorophenyl)-4-[2-propyl-1,2,3,5,6,8a-hexahydroindolizin-7-yl]-3-(pyridin-4-yl)-1*H*-pyrrole,
2-(3-chlorophenyl)-4-[2-propyl-1,2,3,5,6,8a-hexahydroindolizin-7-yl]-3-(pyridin-4-yl)-1*H*-pyrrole,
4-[2-propyl-1,2,3,5,6,8a-hexahydroindolizin-7-yl]-3-(pyridin-4-yl)-2-(3-trifluoromethylphenyl)-1*H*-pyrrole,
4-[2-ethyl-3,5,6,8a-tetrahydroindolizin-7-yl]-2-(4-fluorophenyl)-3-(pyridin-4-yl)-1*H*-pyrrole,
2-(4-fluorophenyl)-4-[2-propyl-3,5,6,8a-tetrahydroindolizin-7-yl]-3-(pyridin-4-yl)-1*H*-pyrrole, and
2-(4-fluorophenyl)-4-[2-phenyl-3,5,6,8a-tetrahydroindolizin-7-yl]-3-(pyridin-4-yl)-1*H*-pyrrole.

**36.** A compound according to claim 1 selected from 2-(4-fluorophenyl)-4-[(2R,8aS)-2-phenyl-1,2,3,5,6,8a-hexahydroindolizin-7-yl]-3-(pyridin-4-yl)-1*H*-pyrrole and pharmacologically acceptable salts and amides thereof.

**37.** A compound according to claim 1 selected from 2-(4-fluorophenyl)-4-[(8aS)-2-methyl-1,2,3,5,6,8a-hexahydroindolizin-7-yl]-3-(pyridin-4-yl)-1*H*-pyrrole and pharmacologically acceptable salts and amides thereof.

**38.** A compound according to claim 1 selected from 2-(4-fluorophenyl)-4-[(8aS)-2-methylidene-1,2,3,5,6,8a-hexahydroindolizin-7-yl]-3-(pyridin-4-yl)-1*H*-pyrrole and pharmacologically acceptable salts and amides thereof.

**39.** A compound according to claim 1 selected from 2-(4-fluorophenyl)-4-[(8aS)-2-methyl-3,5,6,8a-tetrahydroindolizin-7-yl]-3-(pyridin-4-yl)-1*H*-pyrrole and pharmacologically acceptable salts and amides thereof.

**40.** A compound according to claim 1 selected from 4-[(2S,8aS)-2-ethyl-1,2,3,5,6,8a-hexahydroindolizin-7-yl)-2-(4-fluorophenyl)-3-(pyridin-4-yl)-1*H*-pyrrole and pharmacologically acceptable salts and amides thereof.

**41.** A compound according to claim 1 selected from 2-(4-fluorophenyl)-4-[(2S,8aS)-2-propyl-1,2,3,5,6,8a-hexahydroindolizin-7-yl]-3-(pyridin-4-yl)-1*H*-pyrrole and pharmacologically acceptable salts and amides thereof.

**42.** A pharmaceutical composition comprising an effective amount of a pharmacologically active compound together with a carrier or diluent therefor, wherein said pharmacologically active compound is a compound of formula (I-1) or a pharmacologically acceptable salt, ester or amide thereof according to any one of claims 1 to 41.

**43.** A pharmaceutical composition according to claim 42 for use as a medicament for inhibiting the production of inflammatory cytokines in a mammal, which may be human.

**44.** A pharmaceutical composition according to claim 42 for use as a medicament for inhibiting bone resorption in a mammal, which may be human.

**45.** A pharmaceutical composition according to claim 42 for use as a medicament for the treatment or prophylaxis of inflammatory diseases in a mammal, which may be human.

**46.** A pharmaceutical composition according to claim 42 for use as a medicament for the treatment or prophylaxis of viral diseases in a mammal, which may be human.

**47.** A pharmaceutical composition according to claim 42 for use as a medicament for relieving pain or pyrexia in a mammal, which may be human.

**48.** A pharmaceutical composition according to claim 42 for use as a medicament for the treatment or prophylaxis of chronic rheumatoid arthritis in a mammal, which may be human.

**49.** A pharmaceutical composition according to claim 42 for use as a medicament for the treatment or prophylaxis of osteoarthritis in a mammal, which may be human.

**50.** A pharmaceutical composition according to claim 42 for use as a medicament for the treatment or prophylaxis of

cancer in a mammal, which may be human.

**51.** A pharmaceutical composition according to claim 42 for use as a medicament for the treatment or prophylaxis of hepatitis in a mammal, which may be human.

**52.** A pharmaceutical composition according to claim 42 for use as a medicament for the treatment or prophylaxis of a disease selected from the group consisting of allergic diseases, septicaemia, psoriasis, asthma, degenerative arthritis, Crohn's disease, systemic lupus erythematosus, osteoporosis, ulcerative colitis, diabetes, nephritis, ischemic heart disease, Alzheimer's disease and arteriosclerosis in a mammal, which may be human.

**53.** A pharmaceutical composition according to claim 42 for use as a medicament for the treatment or prophylaxis of septicaemia in a mammal, which may be human.

**54.** A compound of formula (I-1) or a pharmacologically acceptable salt, ester or amide thereof according to any one of claims 1 to 41 for use as a medicament.

**55.** The use of at least one compound of formula (I-1) or a pharmacologically acceptable salt, ester or amide thereof according to any one of claims 1 to 41 in the manufacture of a medicament for inhibiting the production of inflammatory cytokines in a mammal, which may be human.

**56.** The use of at least one compound of formula (I-1) or a pharmacologically acceptable salt, ester or amide thereof according to any one of claims 1 to 41 in the manufacture of a medicament for inhibiting bone resorption in a mammal, which may be human.

**57.** The use of at least one compound of formula (I-1) or a pharmacologically acceptable salt, ester or amide thereof according to any one of claims 1 to 41 in the manufacture of a medicament for the treatment or prophylaxis of inflammatory diseases in a mammal, which may be human.

**58.** The use of at least one compound of formula (I-1) or a pharmacologically acceptable salt, ester or amide thereof according to any one of claims 1 to 41 in the manufacture of a medicament for the treatment or prophylaxis of viral diseases in a mammal, which may be human.

**59.** The use of at least one compound of formula (I-1) or a pharmacologically acceptable salt, ester or amide thereof according to any one of claims 1 to 41 in the manufacture of a medicament for relieving pain or pyrexia in a mammal, which may be human.

**60.** The use of at least one compound of formula (I-1) or a pharmacologically acceptable salt, ester or amide thereof according to any one of claims 1 to 41 in the manufacture of a medicament for the treatment or prophylaxis of chronic rheumatoid arthritis in a mammal, which may be human.

**61.** The use of at least one compound of formula (I-1) or a pharmacologically acceptable salt, ester or amide thereof according to any one of claims I to 41 in the manufacture of a medicament for the treatment or prophylaxis of osteoarthritis in a mammal, which may be human.

**62.** The use of at least one compound of formula (I-1) or a pharmacologically acceptable salt, ester or amide thereof according to any one of claims 1 to 41 in the manufacture of a medicament for the treatment or prophylaxis of cancer in a mammal, which may be human.

**63.** The use of at least one compound of formula (I-1) or a pharmacologically acceptable salt, ester or amide thereof according to any one of claims 1 to 41 in the manufacture of a medicament for the treatment or prophylaxis of hepatitis in a mammal, which may be human.

**64.** The use of at least one compound of formula (I-1) or a pharmacologically acceptable salt, ester or amide thereof according to any one of claims 1 to 41 in the manufacture of a medicament for the treatment or prophylaxis of a disease selected from the group consisting of allergic diseases, septicaemia, psoriasis, asthma, degenerative arthritis, Crohn's disease, systemic lupus erythematosus, osteoporosis, ulcerative colitis, diabetes, nephritis, ischemic heart disease, Alzheimer's disease and arteriosclerosis in a mammal, which may be human.

**65.** The use of at least one compound of formula (I-1) or a pharmacologically acceptable salt, ester or amide thereof according to any one of claims 1 to 41 in the manufacture of a medicament for the treatment or prophylaxis of septicemia in a mammal, which may be human.

**66.** A process for the preparation of a compound according to any one of claims I to 41 comprising the following steps:

(a) subjecting a compound of formula (III)

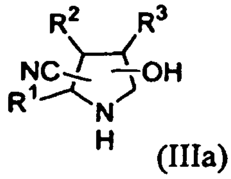

(IIIa)

wherein $R^1$, $R^2$ and $R^3$ are as defined in any one of claims 1 to 41, and the cyano and hydroxyl substituents on the pyrrolidinyl ring are on the carbon atoms of the ring which are adjacent to the nitrogen atom of the ring, to a reaction leading to the elimination of hydrogen cyanide and water from said compound of formula (IIIa) to give the desired compound of formula (I-1); and

(b) optionally, subjecting said compound of formula (I-1) obtained in step (a) to one or more reactions so as to convert one or more of said substituents $R^1$, $R^2$ and $R^3$ to other substituents falling within the definition of said groups and/or converting said compound of formula (I) to a salt, ester or amide thereof.

**67.** A process for the preparation of a compound of formula (I-I) or a pharmacologically acceptable salt, ester or amide thereof according to any one of claims 1 to 41, said process comprising the following steps:

(a) lithiating a compound of formula (IVa):

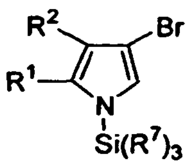

(IVa)

wherein $R^1$ and $R^2$ are as defined above and each $R^7$ is the same or different, and each represents a hydrogen atom, a lower alkyl group as defined in claim 1, an aryl group as defined in claim 1 or an aralkyl group as defined in claim 1, and then reacting the lithiated derivative thus obtained with a compound of formula $R^3$-L wherein $R^3$ is as defined above and L is a group which is capable of leaving as a nucleophilic residue, to give a compound of formula (Va):

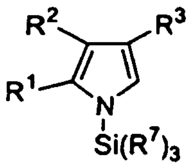

(Va)

wherein $R^1$, $R^2$, $R^3$ and $R^7$ are as defined above; and

(b) subjecting the compound of formula (Va) obtained in step (a) to a desilylation reaction to give the desired compound of formula (I-1); and

(c) optionally, subjecting said compound of formula (I-1) obtained in step (b) to one or more reactions so as to convert one or more of said substituents $R^1$, $R^2$ and $R^3$ to other substituents falling within the definition of said groups and/or converting said compound of formula (I-1) to a salt, ester or amide thereof.

**68.** A process for the preparation of a compound of formula (I-1) or a pharmacologically acceptable salt, ester or amide according to any one of claims 1 to 41, said process comprising the following steps:

(a) lithiating a compound of formula (IVa):

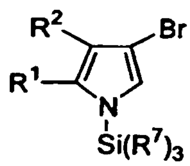

(IVa)

wherein $R^1$ and $R^2$ are as defined above and $R^7$ is as defined in claim 67, and then reacting the lithiated derivative thus obtained with a compound of formula (VI) or formula (VII):

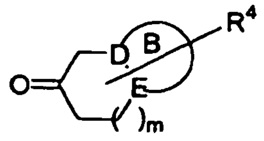

(VI)

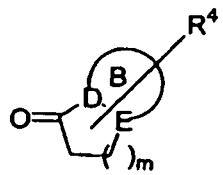

(VII)

wherein B, D, E, $R^4$ and m are as defined in any one of claims 1 to 41, to give a compound of formula (VIIIa) or (IXa):

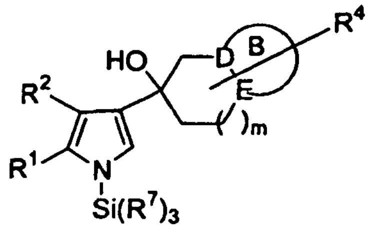

(VIIIa)

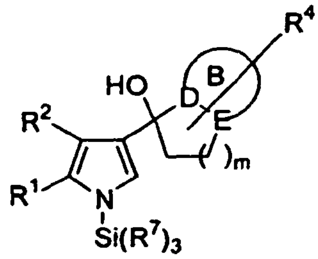

(IXa)

wherein $R^1$, $R^2$, $R^4$, $R^7$, B, D, E, and m are as defined above; and
(b) subjecting the compound of formula (VIIIa) or (IXa) obtained in step (a) to successive steps of dehydration and desilylation to give the desired compound of formula (I-1); and
(c) optionally, subjecting said compound of formula (I-1) obtained in step (b) to one or more reactions so as to convert one or more of said substituents $R^1$, $R^2$ and $R^3$ to other substituents falling within the definition of said groups and/or converting said compound of formula (I-l) to a salt, ester or amide thereof.

## Patentansprüche

**1.** Verbindung der Formel (I-1) oder ein pharmakologisch akzeptables/r Salz, Ester oder Amid davon:

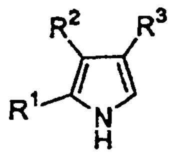

(I-1)

wobei:

$R^1$ aus der Gruppe ausgewählt ist, bestehend aus unten definierten Arylgruppen, die wahlweise mit mindestens einem Substituenten substituiert sein können, ausgewählt aus der unten definierten Substituentengruppe α und der unten definierten Substituentengruppe β, und unten definierten Heteroarylgruppen, die wahlweise mit mindestens einem Substituenten substituiert sein können, ausgewählt aus der unten definierten Substituentengruppe α und der unten definierten Substituentengruppe β;

$R^2$ eine unten definierte Heteroarylgruppe darstellt, die mindestens ein Ringstickstoffatom aufweist, wobei die Heteroarylgruppe wahlweise mit mindestens einem Substituenten substituiert ist, der aus der unten definierten Substituentengruppe α und der unten definierten Substituentengruppe β ausgewählt ist; und

$R^3$ eine Gruppe der unten gezeigten allgemeinen Formeln (IIa), (IIb) oder (IIc) darstellt:

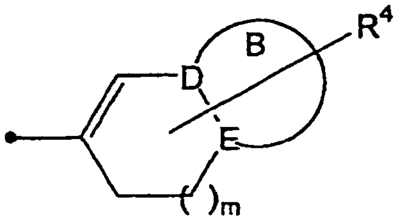

(IIa)

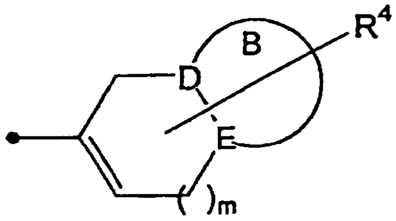

(IIb)

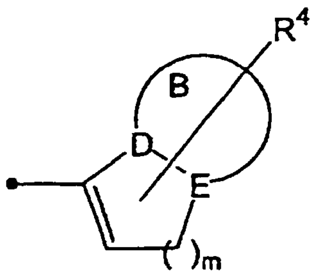

(IIc)

wobei

m 1 oder 2 darstellt,

eines von D und E ein Stickstoffatom darstellt und das andere eine Gruppe der Formel >C($R^5$)- darstellt (wobei $R^5$ aus der Gruppe ausgewählt ist, bestehend aus Wasserstoffatomen, der unten definierten Substituentengruppe α und der unten definierten Substituentengruppe β),

B einen 4- bis 7-gliedrigen heterocyclischen Ring darstellt, der mindestens ein Ringstickstoffatom aufweist (wobei der heterocyclische Ring gesättigt oder ungesättigt sein kann und wahlweise mit einer Gruppe kondensiert sein kann, die aus unten definierten Arylgruppen, unten definierten Heteroarylgruppen, unten definierten Cycloalkylgruppen und unten definierten Heterocyclylgruppen ausgewählt ist), und

$R^4$ 1 bis 3 Substituenten darstellt, die unabhängig aus der Gruppe ausgewählt sind, bestehend aus der unten definierten Substituentengruppe α, der unten definierten Substituentengruppe β und der unten definierten Substituentengruppe γ, oder

wenn B ein heterocyclischer Ring ist, der an eine Arylgruppe, eine Heteroarylgruppe, eine Cycloalkylgruppe oder eine Heterocyclylgruppe kondensiert ist, $R^4$ ein Wasserstoffatom sein kann;

wobei die Substituentengruppe α aus Hydroxygruppen, Nitrogruppen, Cyanogruppen, Halogenatomen, unten definierten Niederalkoxygruppen, unten definierten Halogenniederalkoxygruppen, unten definierten Niederalkylthiogruppen, unten definierten Halogenniederalkylthiogruppen und Gruppen der Formel $-NR^aR^b$ besteht (wobei $R^a$ und $R^b$ gleich oder voneinander verschieden sind und jeweils unabhängig aus der Gruppe ausgewählt sind, bestehend aus Wasserstoffatomen, unten definierten Niederalkylgruppen, unten definierten Niederalkenylgruppen, unten definierten Niederalkinylgruppen, unten definierten Aralkylgruppen und unten definierten Niederalkylsulfonylgruppen, oder $R^a$ und $R^b$ zusammen mit dem Stickstoffatom, an welches sie gebunden sind, eine Heterocyclylgruppe bilden);

die Substituentengruppe β aus unten definierten Niederalkylgruppen besteht, die wahlweise mit mindestens

einem Substituenten substituiert sein können, ausgewählt aus der oben definierten Substituentengruppe α, unten definierten Niederalkenylgruppen, die wahlweise mit mindestens einem Substituenten substituiert sein können, ausgewählt aus der oben definierten Substituentengruppe α, unten definierten Niederalkinylgruppen, die wahlweise mit mindestens einem Substituenten substituiert sein können, ausgewählt aus der oben definierten Substituentengruppe α, unten definierten Aralkylgruppen und unten definierten Cycloalkylgruppen;

die Substituentengruppe γ aus Oxogruppen, Hydroxyiminogruppen, unten definierten Niederalkoxyiminogruppen, unten definierten Niederalkylengruppen, unten definierten Niederalkylendioxygruppen, unten definierten Niederalkylsulfinylgruppen, unten definierten Niederalkylsulfonylgruppen, unten definierten Arylgruppen, die wahlweise mit mindestens einem Substituenten substituiert sein können, ausgewählt aus der oben definierten Substituentengruppe α und der oben definierten Substituentengruppe β, unten definierten Aryloxygruppen, die wahlweise mit mindestens einem Substituenten substituiert sein können, ausgewählt aus der oben definierten Substituentengruppe α und der oben definierten Substituentengruppe β, Niederalkylidenylgruppen und Aralkylidenylgruppen besteht;

wobei die wahlweise substituierten Arylgruppen in den Definitionen von $R^1$, des Rings B und der Substituentengruppe γ oben aromatische Kohlenwasserstoffgruppen mit 6 bis 14 Kohlenstoffatomen in ein oder mehreren Ringen sind, wobei die Arylgruppen wahlweise mit einer Cycloalkylgruppe mit 3 bis 10 Kohlenstoffatomen kondensiert sind;

wobei die wahlweise substituierten Heteroarylgruppen in der Definition von $R^1$ und des Rings B oben 5- bis 7-gliedrige aromatische heterocyclische Gruppen sind, die 1 bis 3 Heteroatome enthalten, ausgewählt aus der Gruppe, bestehend aus Schwefelatomen, Sauerstoffatomen und Stickstoffatomen, wobei die Heteroarylgruppen wahlweise mit einer anderen cyclischen Gruppe kondensiert sind, ausgewählt aus der Gruppe, bestehend aus oben definierten Arylgruppen und Cycloalkylgruppen mit 3 bis 10 Kohlenstoffatomen;

wobei die wahlweise substituierten Heteroarylgruppen mit mindestens einem Ringstickstoffatom in der Definition von $R^2$ oben 5- bis 7-gliedrige aromatische heterocyclische Gruppen sind, die mindestens ein Stickstoffatom enthalten und wahlweise ein oder zwei weitere Heteroatome enthalten, ausgewählt aus der Gruppe, bestehend aus Schwefelatomen, Sauerstoffatomen und Stickstoffatomen;

wobei die Niederalkylgruppen in den Definitionen von $R^a$, $R^b$ und der Substituentengruppe β oben und der Niederalkylrest der Niederalkylgruppen, die wahlweise mit mindestens einem Substituenten substituiert sein können, ausgewählt aus der Substituentengruppe α, in der Definition der Substituentengruppe β oben, gerade oder verzweigte Alkylgruppen mit 1 bis 6 Kohlenstoffatomen sind;

wobei die Niederalkenylgruppen in den Definitionen von $R^a$, $R^b$ und der Substituentengruppe β oben sowie der Niederalkenylrest der Niederalkenylgruppen, die wahlweise mit mindestens einem Substituenten substituiert sein können, ausgewählt aus der Substituentengruppe α, in der Definition der Substituentengruppe β oben, gerade oder verzweigte Alkenylgruppen mit 2 bis 6 Kohlenstoffatomen sind;

wobei die Niederalkinylgruppen in den Definitionen von $R^a$, $R^b$ und der Substituentengruppe β oben sowie der Niederalkinylrest der Niederalkinylgruppen, die wahlweise mit mindestens einem Substituenten substituiert sein können, ausgewählt aus der Substituentengruppe α, in der Definition der Substituentengruppe β oben, gerade oder verzweigte Alkinylgruppen mit 2 bis 6 Kohlenstoffatomen sind;

wobei die Aralkylgruppen in den Definitionen von $R^a$, $R^b$ und der Substituentengruppe β oben Niederalkylgruppen wie oben definiert sind, die mit mindestens einer oben definierten Arylgruppe substituiert sind, die wahlweise mit 1 bis 3 Substituenten substituiert sein kann, ausgewählt aus der oben definierten Substituentengruppe α und der oben definierten Substituentengruppe β;

wobei die Niederalkylsulfonylgruppen in den Definitionen von $R^a$, $R^b$ und des Substituenten γ oben Niederalkylgruppen wie oben definiert sind, die an eine Sulfonylgruppe gebunden sind;

wobei der Ring B mit einer Heterocyclylgruppe kondensiert ist, wobei die Heterocyclylgruppen 4- bis 7-gliedrige Heterocyclylgruppen sind, die 1 bis 3 Ringheteroatome enthalten, ausgewählt aus der Gruppe, bestehend aus Sauerstoffatomen, Schwefelatomen und Stickstoffatomen;

wobei $R^a$ und $R^b$ zusammen mit dem Stickstoffatom, an welches sie gebunden sind, eine Heterocyclylgruppe darstellen, wobei die Heterocyclylgruppen 4- bis 7-gliedrige Heterocyclylgruppen sind, die ein Stickstoffatom enthalten und wahlweise ein weiteres Heteroatom enthalten, ausgewählt aus der Gruppe, bestehend aus Sauerstoffatomen, Schwefelatomen und Stickstoffatomen, wobei die Heterocyclylgruppen wahlweise mit einer anderen cyclischen Gruppe kondensiert sind, ausgewählt aus der Gruppe, bestehend aus oben definierten Arylgruppen und oben definierten Heteroarylgruppen;

wobei die Niederalkoxygruppen in der Definition der Substituentengruppe α oben Gruppen sind, in welchen ein Sauerstoffatom an eine oben definierte Niederalkylgruppe gebunden ist;

wobei die Halogenniederalkoxygruppen in der Definition der Substituentengruppe α oben Gruppen sind, in welchen eine oben definierte Niederalkoxygruppe mit mindestens einem Halogenatom substituiert ist;

wobei die Niederalkylthiogruppen in der Definition der Substituentengruppe α oben Gruppen sind, in welchen

ein Schwefelatom an eine wie oben definierte Niederalkylgruppe gebunden ist;
wobei die Halogenniederalkylthiogruppen in der Definition der Substituentengruppe α oben Gruppen sind, in welchen eine oben definierte Niederalkylthiogruppe mit mindestens einem Halogenatom substituiert ist;
wobei die Cycloalkylgruppen in der Definition der Substituentengruppe β und des Rings B oben Cycloalkylgruppen mit 3 bis 7 Kohlenstoffatomen sind;
wobei die Niederalkoxyiminogruppen in der Definition der Substituentengruppe γ oben Gruppen sind, bei denen das Wasserstoffatom einer Hydroxyiminogruppe durch eine oben definierte Niederalkylgruppe ausgetauscht ist;
wobei die Niederalkylengruppen in der Definition der Substituentengruppe γ oben Alkylengruppen mit 2 bis 6 Kohlenstoffatomen sind;
wobei die Niederalkylendioxygruppen in der Definition der Substituentengruppe γ oben Gruppen sind, bei denen ein Alkylenrest, welches eine gerade oder verzweigtkettige Alkylengruppe mit 1 bis 6 Kohlenstoffatomen ist, mit 2 Oxygruppen substituiert ist;
wobei die Niederalkylsulfinylgruppen in der Definition der Substituentengruppe γ oben Gruppen sind, in welchen eine oben definierte Niederalkylgruppe an eine Sulfinylgruppe gebunden ist;
wobei die Niederalkylidenylgruppen in der Definition der Substituentengruppe γ oben gerade oder verzweigte Alkylidenylgruppen mit 1 bis 6 Kohlenstoffatomen sind;
wobei die Aralkylidenylgruppen in der Definition der Substituentengruppe γ oben Niederalkylidenylgruppen wie oben definiert sind, die mit 1 oder mehreren oben definierten Arylgruppen substituiert sind;
wobei die wahlweise substituierten Aryloxygruppen in der Definition der Substituentengruppe γ oben Gruppen sind, in welchen ein Sauerstoffatom an eine oben definierte Arylgruppe gebunden ist.

**2.** Verbindung nach Anspruch 1 oder ein pharmakologisch akzeptables/r Salz, Ester oder Amid davon, wobei $R^1$ eine Arylgruppe ist, die wahlweise mit mindestens einem Substituenten substituiert sein kann, ausgewählt aus der in Anspruch 1 definierten Substituentengruppe α und Substituentengruppe β.

**3.** Verbindung nach Anspruch 1 oder ein pharmakologisch akzeptables/r Salz, Ester oder Amid davon, wobei $R^1$ eine Phenyl- oder Naphthylgruppe ist, wobei die Gruppen wahlweise mit mindestens einem Substituenten substituiert sein können, ausgewählt aus der in Anspruch 1 definierten Substituentengruppe α und Substituentengruppe β.

**4.** Verbindung nach Anspruch 1 oder ein pharmakologisch akzeptables/r Salz, Ester oder Amid davon, wobei $R^1$ eine Phenylgruppe ist, die wahlweise durch mindestens einen Substituenten substituiert sein kann, ausgewählt aus der unten definierten Substituentengruppe $\alpha^1$ und der unten definierten Substituentengruppe $\beta^1$;
wobei die Substituentengruppe $\alpha^1$ aus Halogenatomen, Niederalkoxygruppen, Halogenniederalkoxygruppen und Gruppen der Formel $-NR^aR^b$ besteht (wobei eines von $R^a$ und $R^b$ ein Wasserstoffatom oder eine Niederalkylgruppe darstellt, und das andere ein Wasserstoffatom, eine Niederalkylgruppe oder eine Aralkylgruppe darstellt), wobei die Niederalkylgruppen, Niederalkoxygruppen, Halogenniederalkoxygruppen und Aralkylgruppen wie in Anspruch 1 definiert sind;
wobei die Substituentengruppe $\beta^1$ aus Niederalkylgruppen, Halogenniederalkylgruppen, Hydroxyniederalkylgruppen, Nitroniederalkylgruppen, Aminoniederalkylgruppen, Niederalkylaminoniederalkylgruppen, Di(niederalkyl)aminoniederalkylgruppen und Aralkylaminoniederalkylgruppen besteht, wobei die Niederalkylgruppen, Halogenniederalkylgruppen, Alkylreste der Hydroxyniederalkylgruppen, Nitroniederalkylgruppen, Aminoniederalkylgruppen, Niederalkylaminoniederalkylgruppen und Di(niederalkyl)aminoniederalkylgruppen sowie Alkyl- und Aralkylreste der Aralkylaminoniederalkylgruppen wie in Anspruch 1 definiert sind.

**5.** Verbindung nach Anspruch 1 oder ein pharmakologisch akzeptables/r Salz, Ester oder Amid davon, wobei $R^1$ eine Phenylgruppe ist, die wahlweise durch mindestens einen Substituenten substituiert sein kann, ausgewählt aus der Gruppe, bestehend aus Halogenatomen, Halogenniederalkylgruppen wie in Anspruch 1 definiert und Halogenniederalkoxygruppen wie in Anspruch 1 definiert.

**6.** Verbindung nach Anspruch 1 oder ein pharmakologisch akzeptables/r Salz, Ester oder Amid davon, wobei $R^1$ ein Substituent ist, ausgewählt aus der Gruppe, bestehend aus Phenyl-, 4-Fluorphenyl-, 3-Fluorphenyl-, 3-Chlorphenyl-, 3,4-Difluorphenyl-, 3,4,5-Trifluorphenyl-, 3-Chlor-4-fluorphenyl-, 3-Difluormethoxyphenyl- und 3-Trifluormethylphenylgruppen.

**7.** Verbindung nach einem der Ansprüche 1 bis 6 oder ein pharmakologisch akzeptables/r Salz, Ester oder Amid davon, wobei $R^2$ eine 5- oder 6-gliedrige aromatische heterocyclische Gruppe ist, die ein oder zwei Stickstoffatome aufweist, wobei die Gruppe wahlweise durch mindestens einen Substituenten substituiert ist, ausgewählt aus der in Anspruch 1 definierten Substituentengruppe α und Substituentengruppe β.

**8.** Verbindung nach einem der Ansprüche 1 bis 6 oder ein pharmakologisch akzeptables/r Salz, Ester oder Amid davon, wobei $R^2$ eine Pyridyl- oder Pyrimidinylgruppe ist, wobei die Gruppen wahlweise durch mindestens einen Substituenten substituiert sind, ausgewählt aus der Gruppe, bestehend aus der in Anspruch 1 definierten Substituentengruppe $\alpha$ und Substituentengruppe $\beta$.

**9.** Verbindung nach einem der Ansprüche 1 bis 6 oder ein pharmakologisch akzeptables/r Salz, Ester oder Amid davon, wobei $R^2$ eine 4-Pyridyl- oder 4-Pyrimidinylgruppe ist, wobei die Gruppen wahlweise durch mindestens einen Substituenten substituiert sind, ausgewählt aus der Gruppe, bestehend aus der in Anspruch 1 definierten Substituentengruppe $\alpha$ und Substituentengruppe $\beta$.

**10.** Verbindung nach einem der Ansprüche 1 bis 6 oder ein pharmakologisch akzeptables/r Salz, Ester oder Amid davon, wobei $R^2$ eine 4-Pyridyl- oder 4-Pyrimidinylgruppe ist, wobei diese Gruppen wahlweise an deren 2-Position mit einem Substituenten substituiert sind, ausgewählt aus der Gruppe, bestehend aus der in Anspruch 1 definierten Substituentengruppe $\alpha$ und Substituentengruppe $\beta$.

**11.** Verbindung nach einem der Ansprüche 1 bis 6 oder ein pharmakologisch akzeptables/r Salz, Ester oder Amid davon, wobei $R^2$ eine 4-Pyridyl- oder 4-Pyrimidinylgruppe ist, wobei diese Gruppen wahlweise an deren 2-Position mit einem Substituenten substituiert sind, ausgewählt aus der Gruppe, bestehend aus Methoxy-, Amino-, Methylamino-, Benzylamino- und $\alpha$-Methylbenzylaminogruppen.

**12.** Verbindung nach einem der Ansprüche 1 bis 11 oder ein pharmakologisch akzeptables/r Salz, Ester oder Amid davon, wobei B ein 5- oder 6-gliedriger heterocyclischer Ring ist, der ein Ringstickstoffatom aufweist und wahlweise ein weiteres Ringheteroatom oder eine Ringgruppe aufweist, ausgewählt aus einem Stickstoffatom, Sauerstoffatom, Schwefelatom, >SO und >$SO_2$ (wobei der Ring gesättigt oder ungesättigt sein kann und wahlweise mit einer Arylgruppe, Heteroarylgruppe, Cycloalkylgruppe oder Heterocyclylgruppe wie in Anspruch 1 definiert kondensiert sein kann).

**13.** Verbindung nach einem der Ansprüche 1 bis 11 oder ein pharmakologisch akzeptables/r Salz, Ester oder Amid davon, wobei B ein 5- oder 6-gliedriger heterocyclischer Ring ist, der aus der Gruppe D, der Gruppe E und drei oder vier Kohlenstoffatomen besteht (wobei der Ring gesättigt oder ungesättigt sein kann und wahlweise mit einer Arylgruppe, Heteroarylgruppe, Cycloalkylgruppe oder Heterocyclylgruppe wie in Anspruch 1 definiert kondensiert sein kann).

**14.** Verbindung nach einem der Ansprüche 1 bis 11 oder ein pharmakologisch akzeptables/r Salz, Ester oder Amid davon, wobei B ein Pyrrolidinylring oder ein Pyrrolinylring ist.

**15.** Verbindung nach einem der Ansprüche 1 bis 14 oder ein pharmakologisch akzeptables/r Salz, Ester oder Amid davon, wobei $R^3$ eine Gruppe der allgemeinen Formel (IIa) oder der allgemeinen Formel (IIb) ist.

**16.** Verbindung nach einem der Ansprüche 1 bis 14 oder ein pharmakologisch akzeptables/r Salz, Ester oder Amid davon, wobei $R^3$ eine Gruppe der allgemeinen Formel (IIa) ist.

**17.** Verbindung nach einem der Ansprüche 1 bis 16 oder ein pharmakologisch akzeptables/r Salz, Ester oder Amid davon, wobei m 1 ist.

**18.** Verbindung nach einem der Ansprüche 1 bis 17 oder ein pharmakologisch akzeptables/r Salz, Ester oder Amid davon, wobei $R^4$ 1 oder 2 Substituenten darstellt, die unabhängig aus der Gruppe ausgewählt sind, bestehend aus der in Anspruch 1 definierten Substituentengruppe $\alpha$, der in Anspruch 1 definierten Substituentengruppe $\beta$ und der Substituentengruppe $\gamma^1$, wobei die Substituentengruppe $\gamma^1$ aus Oxogruppen, Hydroxyiminogruppen, Niederalkoxyiminogruppen, Niederalkylengruppen, Niederalkylendioxygruppen, Niederalkylsulfinylgruppen, Niederalkylsulfonylgruppen und Arylgruppen, die wahlweise mit mindestens einem Substituenten substituiert sein können, ausgewählt aus der in Anspruch 1 definierten Substituentengruppe $\alpha$ und Substituentengruppe $\beta$, besteht.

**19.** Verbindung nach einem der Ansprüche 1 bis 17 oder ein pharmakologisch akzeptables/r Salz, Ester oder Amid davon, wobei $R^4$ ein Substituent ausgewählt aus der Gruppe ist, bestehend aus Hydroxygruppen, Halogenatomen, Niederalkoxygrüppen, Niederalkylthiogruppen, Halogenniederalkoxygruppen, Niederalkylgruppen, Halogenniederalkylgruppen, Oxogruppen, Arylgruppen, die wahlweise mit mindestens einem Substituenten substituiert sind, ausgewählt aus der Substituentengruppe $\alpha$ und Substituentengruppe $\beta$, Niederalkylendioxygruppen, Niederalkylen-

gruppen und Niederalkylsulfonylgruppen wie in Anspruch 1 definiert.

**20.** Verbindung nach einem der Ansprüche 1 bis 17 oder ein pharmakologisch akzeptables/r Salz, Ester oder Amid davon, wobei $R^4$ ein Substituent ausgewählt aus der Gruppe ist, bestehend aus Hydroxygruppen, Fluoratomen, Chloratomen, Methoxygruppen, Ethoxygruppen, Propoxygruppen, Methylgruppen, Ethylgruppen, Propylgruppen und Phenylgruppen, die wahlweise mit mindestens einem Substituenten substituiert sein können, ausgewählt aus der in Anspruch 1 definierten Substituentengruppe $\alpha$ und Substituentengruppe $\beta$.

**21.** Verbindung nach einem der Ansprüche 1 bis 17 oder ein pharmakologisch akzeptables/r Salz, Ester oder Amid davon, wobei $R^4$ ein Substituent ausgewählt aus der Gruppe ist, bestehend aus Methoxygruppen, Methylgruppen, Ethylgruppen, Propylgruppen und Phenylgruppen.

**22.** Verbindung nach einem der Ansprüche 1 bis 17 oder ein pharmakologisch akzeptables/r Salz, Ester oder Amid davon, wobei $R^4$ ein Substituent ausgewählt aus der Gruppe ist, bestehend aus Aryloxygruppen, die wahlweise mit mindestens einem Substituenten substituiert sein können, ausgewählt aus der Substituentengruppe $\alpha$ und Substituentengruppe $\beta$, Alkylidengruppen und Aralkylidengruppen wie in Anspruch 1 definiert.

**23.** Verbindung nach einem der Ansprüche 1 bis 17 oder ein pharmakologisch akzeptables/r Salz, Ester oder Amid davon, wobei $R^4$ ein Substituent ausgewählt aus der Gruppe ist, bestehend aus Phenoxy-, Methyliden-, Ethyliden-, Propyliden- und Benzylidengruppen.

**24.** Verbindung nach einem der Ansprüche 1 bis 23 oder ein pharmakologisch akzeptables/r Salz, Ester oder Amid davon, wobei D eine Gruppe der Formel >C ($R^5$) - ist (wobei $R^5$ aus der Gruppe ausgewählt ist, bestehend aus Wasserstoffatomen, der Substituentengruppe $\alpha$ und Substituentengruppe $\beta$ wie in Anspruch 1 definiert), und E ein Stickstoffatom ist.

**25.** Verbindung nach Anspruch 1 oder ein pharmakologisch akzeptables/r Salz, Ester oder Amid davon, wobei:

$R^1$ eine Arylgruppe ist, die wahlweise mit mindestens einem Substituenten substituiert sein kann, ausgewählt aus der Substituentengruppe $\alpha$ und Substituentengruppe $\beta$;
$R^2$ eine 5- oder 6-gliedrige aromatische heterocyclische Gruppe ist, die ein oder zwei Stickstoffatome aufweist, wobei die Gruppe wahlweise durch mindestens einen Substituenten substituiert ist, ausgewählt aus der Substituentengruppe $\alpha$ und Substituentengruppe $\beta$; und
$R^3$ eine Gruppe der allgemeinen Formel (IIa) oder der allgemeinen Formel (IIb) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe $\alpha$ und Substituentengruppe $\beta$), und E ein Stickstoffatom ist, und
$R^4$ 1 oder 2 Substituenten darstellt, die unabhängig aus der Gruppe ausgewählt ist, bestehend aus Substituentengruppe $\alpha$, Substituentengruppe $\beta$ und Substituentengruppe $\gamma^1$, wobei die Substituentengruppe $\gamma^1$ aus Oxogruppen, Hydroxyiminogruppen, Niederalkoxyiminogruppen, Niederalkylengruppen, Niederalkylendioxygruppen, Niederalkylsulfinylgruppen, Niederalkylsulfonylgruppen und Arylgruppen, die wahlweise mit mindestens einem Substituenten substituiert sein können, ausgewählt aus der Substituentengruppe $\alpha$ und Substituentengruppe $\beta$, besteht.

**26.** Verbindung nach Anspruch 1 oder ein pharmakologisch akzeptables/r Salz, Ester oder Amid davon, wobei:

$R^1$ eine Phenyl- oder Naphthylgruppe ist, wobei die Gruppen wahlweise durch mindestens einen Substituenten substituiert sind, ausgewählt aus der Substituentengruppe $\alpha$ und Substituentengruppe $\beta$;
$R^2$ eine Pyridyl- oder Pyrimidinylgruppe ist, wobei die Gruppen wahlweise mit mindestens einem Substituenten substituiert sind, ausgewählt aus der Gruppe, bestehend aus Substituentengruppe $\alpha$ und Substituentengruppe $\beta$; und
$R^3$ eine Gruppe der allgemeinen Formel (IIa) oder der allgemeinen Formel (IIb) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe $\alpha$ und der Substituentengruppe $\beta$) und E ein Stickstoffatom ist, und
$R^4$ ein Substituent ist, der aus der Gruppe ausgewählt ist, bestehend aus Hydroxygruppen, Halogenatomen, Niederalkoxygruppen, Niederalkylthiogruppen, Halogenniederalkoxygruppen, Niederalkylgruppen, Halogenniederalkylgruppen, Oxogruppen, Arylgruppen, wahlweise substituiert mit mindestens einem Substituenten,

ausgewählt aus der Substituentengruppe α und der Substituentengruppe β, Niederalkylendioxygruppen, Niederalkylengruppen und Niederalkylsulfonylgruppen.

**27.** Verbindung nach Anspruch 1 oder ein pharmakologisch akzeptables/r Salz, Ester oder Amid davon, wobei:

$R^1$ eine Phenylgruppe ist, die wahlweise mit mindestens einem Substituenten substituiert sein kann, ausgewählt aus der unten definierten Substituentengruppe $\alpha^1$ und der unten definierten Substituentengruppe $\beta^1$,
wobei die Substituentengruppe $\alpha^1$ aus Halogenatomen, Niederalkoxygruppen, Halogenniederalkoxygruppen und Gruppen der Formel $-NR^aR^b$ besteht (wobei eines von $R^a$ und $R^b$ ein Wasserstoff oder eine Niederalkylgruppe darstellt, und das andere ein Wasserstoffatom, eine Niederalkylgruppe oder eine Aralkylgruppe darstellt), wobei die Niederalkylgruppen, Niederalkoxygruppen, Halogenniederalkoxygruppen und Aralkylgruppen wie in Anspruch 1 definiert sind, und
die Substituentengruppe $\beta^1$ aus Niederalkylgruppen, Halogenniederalkylgruppen, Hydroxyniederalkylgruppen, Nitroniederalkylgruppen, Aminoniederalkylgruppen, Niederalkylaminoniederalkylgruppen, Di(niederalkyl)aminoniederalkylgruppen und Aralkylaminoniederalkylgruppen besteht, wobei die Niederalkylgruppen, Halogenniederalkylgruppen, Alkylreste der Hydroxyniederalkylgruppen, Nitroniederalkylgruppen, Aminoniederalkylgruppen, Niederalkylaminoniederalkylgruppen und Di(niederalkyl)aminoniederalkylgruppen und Alkyl- und Aralkylreste der Aralkylaminoniederalkylgruppen wie in Anspruch 1 definiert sind;
$R^2$ eine 4-Pyridyl- oder 4-Pyrimidinylgruppe ist, wobei die Gruppen wahlweise mit mindestens einem Substituenten substituiert sind, ausgewählt aus der Gruppe, bestehend aus Substituentengruppe α und Substituentengruppe β; und
$R^3$ eine Gruppe der allgemeinen Formel (IIa) oder der allgemeinen Formel (IIb) ist, wobei
m 1 ist,
D eine Gruppe der Formel $>C(R^5)-$ ist (wobei $R^5$ aus der Gruppe ausgewählt ist, bestehend aus Wasserstoffatomen, der Substituentengruppe α und der Substituentengruppe β), und E ein Stickstoffatom ist, und
$R^4$ ein Substituent ist, der aus der Gruppe ausgewählt ist, bestehend aus Hydroxygruppen, Halogenatomen, Niederalkoxygruppen, Niederalkylthiogruppen, Halogenniederalkoxygruppen, Niederalkylgruppen, Halogenniederalkylgruppen, Oxogruppen, Arylgruppen, wahlweise substituiert mit mindestens einem Substituenten, ausgewählt aus der Substituentengruppe α und der Substituentengruppe β, Niederalkylendioxygruppen, Niederalkylengruppen und Niederalkylsulfonylgruppen.

**28.** Verbindung nach Anspruch 1 oder ein pharmakologisch akzeptables/r Salz, Ester oder Amid davon, wobei:

$R^1$ eine Phenylgruppe ist, die wahlweise mit mindestens einem Substituenten substituiert sein kann, ausgewählt aus der Gruppe, bestehend aus Halogenatomen, Halogenniederalkylgruppen wie in Anspruch 1 definiert und Halogenniederalkoxygruppen;
$R^2$ eine 4-Pyridyl- oder 4-Pyrimidinylgruppe ist, wobei die Gruppen wahlweise an deren 2-Position mit einem Substituenten substituiert sind, ausgewählt aus der Gruppe, bestehend aus der Substituentengruppe α und der Substituentengruppe β; und
$R^3$ eine Gruppe der allgemeinen Formel (IIa) ist, wobei
m 1 ist,
D eine Gruppe der Formel $>C(R^5)-$ ist (wobei $R^5$ aus der Gruppe ausgewählt ist, bestehend aus Wasserstoffatomen, der Substituentengruppe α und der Substituentengruppe β), und E ein Stickstoffatom ist, und
$R^4$ ein Substituent ist, der aus der Gruppe ausgewählt ist, bestehend aus Hydroxygruppen, Fluoratomen, Chloratomen, Methoxygruppen, Ethoxygruppen, Propoxygruppen, Methylgruppen, Ethylgruppen, Propylgruppen und Phenylgruppen, die wahlweise mit mindestens einem Substituenten substituiert sein können, ausgewählt aus der Substituentengruppe α und der Substituentengruppe β.

**29.** Verbindung nach Anspruch 1 oder ein pharmakologisch akzeptables/r Salz, Ester oder Amid davon, wobei:

$R^1$ ein Substituent ist, der aus der Gruppe ausgewählt ist, bestehend aus Phenyl-, 4-Fluorphenyl-, 3-Fluorphenyl-, 3-Chlorphenyl-, 3,4-Difluorphenyl-, 3,4,5-Trifluorphenyl-, 3-Chlor-4-fluorphenyl-, 3-Difluormethoxyphenyl- und 3-Trifluormethylphenylgruppen;
$R^2$ eine 4-Pyridyl- oder 4-Pyrimidinylgruppe ist, wobei die Gruppen wahlweise an deren 2-Position mit einem Substituenten substituiert sind, ausgewählt aus der Gruppe, bestehend aus Methoxy-, Amino-, Methylamino-, Benzylamino-, und α-Methylbenzylaminogruppen; und
$R^3$ eine Gruppe der allgemeinen Formel (IIa) ist, wobei
m 1 ist,

D eine Gruppe der Formel >C($R^5$)- ist (wobei $R^5$ aus der Gruppe ausgewählt ist, bestehend aus Wasserstoffatomen, der Substituentengruppe $\alpha$ und der Substituentengruppe $\beta$), und E ein Stickstoffatom ist, und
$R^4$ ein Substituent ist, ausgewählt aus der Gruppe, bestehend aus Methoxygruppen, Methylgruppen, Ethylgruppen, Propylgruppen und Phenylgruppen.

**30.** Verbindung nach Anspruch 1 oder ein pharmakologisch akzeptables/r Salz, Ester oder Amid davon, wobei:

$R^1$ eine Arylgruppe ist, die wahlweise mit mindestens einem Substituenten substituiert sein kann, ausgewählt aus der Substituentengruppe $\alpha$ und Substituentengruppe $\beta$;
$R^2$ eine 5- oder 6-gliedrige aromatische heterocyclische Gruppe ist, die ein oder zwei Stickstoffatome aufweist, wobei die Gruppe wahlweise durch mindestens einen Substituenten substituiert ist, ausgewählt aus der Substituentengruppe $\alpha$ und Substituentengruppe $\beta$; und
$R^3$ eine Gruppe der allgemeinen Formel (IIa) oder der allgemeinen Formel (IIb) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe $\alpha$ und der Substituentengruppe $\beta$), und E ein Stickstoffatom ist, und
$R^4$ ein Substituent ist, ausgewählt aus der Gruppe, bestehend aus Aryloxygruppen, die wahlweise durch mindestens einen Substituenten substituiert sein können, ausgewählt aus der Substituentengruppe $\alpha$ und Substituentengruppe $\beta$, Alkylidengruppen und Aralkylidengruppen.

**31.** Verbindung nach Anspruch 1 oder ein pharmakologisch akzeptables/r Salz, Ester oder Amid davon, wobei:

$R^1$ eine Phenyl- oder Naphthylgruppe ist, wobei die Gruppen wahlweise durch mindestens einen Substituenten substituiert sind, ausgewählt aus Substituentengruppe $\alpha$ und Substituentengruppe $\beta$;
$R^2$ eine Pyridyl- oder Pyrimidinylgruppe ist, wobei die Gruppen wahlweise durch mindestens einen Substituenten substituiert sind, ausgewählt aus der Gruppe, bestehend aus Substituentengruppe $\alpha$ und Substituentengruppe $\beta$; und
$R^3$ eine Gruppe der allgemeinen Formel (IIa) oder der allgemeinen Formel (IIb) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe $\alpha$ und der Substituentengruppe $\beta$), und E ein Stickstoffatom ist, und
$R^4$ ein Substituent ist, ausgewählt aus der Gruppe, bestehend aus Aryloxygruppen, die wahlweise durch mindestens einen Substituenten substituiert sein können, ausgewählt aus der Substituentengruppe $\alpha$ und Substituentengruppe $\beta$, Alkylidengruppen und Aralkylidengruppen.

**32.** Verbindung nach Anspruch 1 oder ein pharmakologisch akzeptables/r Salz, Ester oder Amid davon, wobei:

$R^1$ eine Phenylgruppe ist, die wahlweise durch mindestens einen Substituenten substituiert sein kann, ausgewählt aus der unten definierten Substituentengruppe $\alpha^1$ und der unten definierten Substituentengruppe $\beta^1$, wobei die Substituentengruppe $\alpha^1$ aus Halogenatomen, Niederalkoxygruppen, Halogenniederalkoxygruppen und Gruppen der Formel $-NR^aR^b$ besteht (wobei eines von $R^a$ und $R^b$ ein Wasserstoff oder eine Niederalkylgruppe darstellt, und das andere ein Wasserstoffatom, eine Niederalkylgruppe oder eine Aralkylgruppe darstellt), wobei die Niederalkylgruppen, Niederalkoxygruppen, Halogenniederalkoxygruppen und Aralkylgruppen wie in Anspruch 1 definiert sind, und
die Substituentengruppe $\beta^1$ aus Niederalkylgruppen, Halogenniederalkylgruppen, Hydroxyniederalkylgruppen, Nitroniederalkylgruppen, Aminoniederalkylgruppen, Niederalkylaminoniederalkylgruppen, Di(niederalkyl)aminoniederalkylgruppen und Aralkylaminoniederalkylgruppen besteht, wobei die Niederalkylgruppen, Halogenniederalkylgruppen, Alkylreste der Hydroxyniederalkylgruppen, Nitroniederalkylgruppen, Aminoniederalkylgruppen, Niederalkylaminoniederalkylgruppen und Di(niederalkyl)aminoniederalkylgruppen und Alkyl- und Aralkylreste der Aralkylaminoniederalkylgruppen wie in Anspruch 1 definiert sind;
$R^2$ eine 4-Pyridyl- oder 4-Pyrimidinylgruppe ist, wobei die Gruppen wahlweise mit mindestens einem Substituenten substituiert sind, ausgewählt aus der Gruppe, bestehend aus Substituentengruppe $\alpha$ und Substituentengruppe $\beta$; und
$R^3$ eine Gruppe der allgemeinen Formel (IIa) oder der allgemeinen Formel (IIb) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe $\alpha$ und der Substituentengruppe $\beta$), und E ein Stickstoffatom ist, und
$R^4$ ein Substituent ist, ausgewählt aus der Gruppe, bestehend aus Aryloxygruppen, die wahlweise durch mindestens einen Substituenten substituiert sein können, ausgewählt aus der Substituentengruppe α und Substituentengruppe β, Alkylidengruppen und Aralkylidengruppen.

**33.** Verbindung nach Anspruch 1 oder ein pharmakologisch akzeptables/r Salz, Ester oder Amid davon, wobei:

$R^1$ eine Phenylgruppe ist, die wahlweise durch mindestens einen Substituenten substituiert sein kann, ausgewählt aus der Gruppe, bestehend aus Halogenatomen, Halogenniederalkylgruppen wie in Anspruch 1 definiert und Halogenniederalkoxygruppen;
$R^2$ eine 4-Pyridyl- oder 4-Pyrimidinylgruppe ist, wobei die Gruppen wahlweise an deren 2-Position durch einen Substituenten substituiert sind, ausgewählt aus der Gruppe, bestehend aus Substituentengruppe α und Substituentengruppe β; und
$R^3$ eine Gruppe der allgemeinen Formel (IIa) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe α und der Substituentengruppe β), und E ein Stickstoffatom ist, und
$R^4$ ein Substituent ist, ausgewählt aus der Gruppe, bestehend aus Phenoxy-, Methyliden-, Ethyliden-, Propyliden- und Benzylidengruppen.

**34.** Verbindung nach Anspruch 1 oder ein pharmakologisch akzeptables/r Salz, Ester oder Amid davon, wobei:

$R^1$ ein Substituent ist, ausgewählt aus der Gruppe, bestehend aus Phenyl-, 4-Fluorphenyl-, 3-Fluorphenyl-, 3-Chlorphenyl-, 3,4-Difluorphenyl-, 3,4,5-Trifluorphenyl-, 3-Chlor-4-fluorphenyl-, 3-Difluormethoxyphenyl- und 3-Trifluormethylphenylgruppen;
$R^2$ eine 4-Pyridyl- oder 4-Pyrimidinylgruppe ist, wobei diese Gruppen wahlweise an deren 2-Position mit mindestens einem Substituenten substituiert sind, ausgewählt aus der Gruppe, bestehend aus Methoxy-, Amino-, Methylamino-, Benzylamino- und α-Methylbenzylaminogruppen; und
$R^3$ eine Gruppe der allgemeinen Formel (IIa) ist, wobei
m 1 ist,
D eine Gruppe der Formel >C($R^5$)- ist (wobei $R^5$ aus der Gruppe ausgewählt ist, bestehend aus Wasserstoffatomen, der Substituentengruppe α und der Substituentengruppe β), und E ein Stickstoffatom ist, und
$R^4$ ein Substituent ist, ausgewählt aus der Gruppe, bestehend aus Phenoxy-, Methyliden-, Ethyliden-, Propyliden- und Benzylidengruppen.

**35.** Verbindung nach Anspruch 1, ausgewählt aus den folgenden Verbindungen, oder ein pharmakologisch akzeptables Salz oder Amid davon, wobei:

2-(3-Fluorphenyl)-4-[2-methyl-1,2,3,5,6,8a-hexahydroindolizin-7-yl]-3-(pyridin-4-yl)-1*H*-pyrrol,
2-(3-Fluorphenyl)-4-[2-phenyl-1,2,3,5,6,8a-hexahydroindolizin-7-yl]-3-(pyridin-4-yl)-1*H*-pyrrol,
2-(3-Fluorphenyl)-4-[2-methoxy-1,2,3,5,6,8a-hexahydroindolizin-7-yl]-3-(pyridin-4-yl)-1*H*-pyrrol,
2-(4-Fluorphenyl)-4-[2-methyl-1,2,3,5,6,8a-hexahydroindolizin-7-yl]-3-(pyridin-4-yl)-1*H*-pyrrol,
2-(4-Fluorphenyl)-4-[2-phenyl-1,2,3,5,6,8a-hexahydroindolizin-7-yl]-3-(pyridin-4-yl)-1*H*-pyrrol,
2-(4-Fluorphenyl)-4-[2-methoxy-1,2,3,5,6,8a-hexahydroindolizin-7-yl]-3-(pyridin-4-yl)-1*H*-pyrrol,
2-(3-Chlorphenyl)-4-[2-methyl-1,2,3,5,6,8a-hexahydroindolizin-7-yl]-3-(pyridin-4-yl)-1*H*-pyrrol,
2-(3-Chlorphenyl)-4-[2-phenyl-1,2,3,5,6,8a-hexahydroindolizin-7-yl]-3-(pyridin-4-yl)-1*H*-pyrrol,
2-(3-Chlorphenyl)-4-[2-methoxy-1,2,3,5,6,8a-hexahydroindolizin-7-yl]-3-(pyridin-4-yl)-1*H*-pyrrol,
4-[2-Methyl-1,2,3,5,6,8a-hexahydroindolizin-7-yl]-3-(pyridin-4-yl)-2-(3-trifluormethylphenyl)-1*H*-pyrrol,
4-[2-Phenyl-1,2,3,5,6,8a-hexahydroindolizin-7-yl]-3-(pyridin-4-yl)-2-(3-trifluormethylphenyl)-1*H*-pyrrol,
4-[2-Methoxy-1,2,3,5,6,8a-hexahydroindolizin-7-yl]-3-(pyridin-4-yl)-2-(3-trifluormethylphenyl)-1*H*-pyrrol,
2-(4-Fluorphenyl)-4-[2-methyl-3,5,6,8a-tetrahydroindolizin-7-yl]-3-(pyridin-4-yl)-1*H*-pyrrol,
2-(4-Fluorphenyl)-4-[2-methyliden-1,2,3,5,6,8a-hexahydroindolizin-7-yl]-3-(pyridin-4-yl)-1*H*-pyrrol,
4-[2-Ethyl-1,2,3,5,6,8a-hexahydroindolizin-7-yl]-2-(4-fluorphenyl)-3-(pyridin-4yl)-1*H*-pyrrol,
4-[2-Ethyl-1,2,3,5,6,8a-hexahydroindolizin-7-yl]-2-(3-fluorphenyl)-3-(pyridin-4-yl)-1*H*-pyrrol,
2-(3-Chlorphenyl)-4-[2-ethyl-1,2,3,5,6,8a-hexahydroindolizin-7-yl]-3-(pyridin-4-yl)-1*H*-pyrrol,
4-[(2-Ethyl-1,2,3,5,6,8a-hexahydroindolizin-7-yl]-3-(pyridin-4-yl)-2-(3-trifluormethylphenyl)-1*H*-pyrrol,
2-(4-Fluorphenyl)-4-[2-propyl-1,2,3,5,6,8a-hexahydroindolizin-7-yl]-3-(pyridin-4-yl)-1*H*-pyrrol,
2-(3-Fluorphenyl)-4-[2-propyl-1,2,3,5,6,8a-hexahydroindolizin-7-yl]-3-(pyridin-4-yl)-1*H*-pyrrol,
2-(3-Chlorphenyl)-4-[2-propyl-1,2,3,5,6,8a-hexahydroindolizin-7-yl]-3-(pyridin-4-yl)-1*H*-pyrrol,
4-[2-Propyl-1,2,3,5,6,8a-hexahydroindolizin-7-yl]-3-(pyridin-4-yl)-2-(3-trifluormethylphenyl)-1*H*-pyrrol,

4-[2-Ethyl-3,5,6,8a-tetrahydroindolizin-7-yl]-2-(4-fluorphenyl)-3-(pyridin-4-yl)-1*H*-pyrrol,
2-(4-Fluorphenyl)-4-[2-propyl-3,5,6,8a-tetrahydroindolizin-7-yl]-3-(pyridin-4-yl)-1*H*-pyrrol, und
2-(4-Fluorphenyl)-4-[2-phenyl-3,5,6,8a-tetrahydroindolizin-7-yl]-3-(pyridin-4-yl)-1*H*-pyrrol.

**36.** Verbindung nach Anspruch 1, ausgewählt aus 2-(4-Fluorphenyl)-4-[(2R,8aS)-2-phenyl-1,2,3,5,6,8a-hexahydroindolizin-7-yl]-3-(pyridin-4-yl)-1*H*-pyrrol und pharmakologisch akzeptablen Salzen und Amiden davon.

**37.** Verbindung nach Anspruch 1, ausgewählt aus 2-(4-Fluorphenyl)-4-[(8aS)-2-methyl-1,2,3,5,6,8a-hexahydroindolizin-7-yl]-3-(pyridin-4-yl)-1*H*-pyrrol und pharmakologisch akzeptablen Salzen und Amiden davon.

**38.** Verbindung nach Anspruch 1, ausgewählt aus 2-(4-Fluorphenyl)-4-[(8aS)-2-methyliden-1,2,3,5,6,8a-hexahydroindolizin-7-yl]-3-(pyridin-4-yl)-1*H*-pyrrol und pharmakologisch akzeptablen Salzen und Amiden davon.

**39.** Verbindung nach Anspruch 1, ausgewählt aus 2-(4-Fluorphenyl)-4-[(8aS)-2-methyl-3,5,6,8a-tetrahydroindolizin-7-yl]-3-(pyridin-4-yl)-1*H*-pyrrol und pharmakologisch akzeptablen Salzen und Amiden davon.

**40.** Verbindung nach Anspruch 1, ausgewählt aus 4-[(2S,8aS)-2-ethyl-1,2,3,5,6,8a-hexahydroindolizin-7-yl)-2-(4-fluorphenyl)-3-(pyridin-4-yl)-1*H*-pyrrol und pharmakologisch akzeptablen Salzen und Amiden davon.

**41.** Verbindung nach Anspruch 1, ausgewählt aus 2-(4-Fluorphenyl)-4-[(2S,8aS)-2-propyl-1,2,3,5,6,8a-hexahydroindolizin-7-yl]-3-(pyridin-4-yl)-1*H*-pyrrol und pharmakologisch akzeptablen Salzen und Amiden davon.

**42.** Pharmazeutische Zusammensetzung, umfassend eine wirksame Menge einer pharmakologisch aktiven Verbindung zusammen mit einem Träger oder Verdünnungsmittel dafür, wobei die pharmakologisch aktive Verbindung eine Verbindung der Formel (I-1) oder ein pharmakologisch akzeptables/r Salz, Ester oder Amid davon nach einem der Ansprüche 1 bis 41 ist.

**43.** Pharmazeutische Zusammensetzung nach Anspruch 42 zur Verwendung als Arzneimittel zur Hemmung der Produktion entzündlicher Zytokine in einem Säuger, der ein Mensch sein kann.

**44.** Pharmazeutische Zusammensetzung nach Anspruch 42 zur Verwendung als Arzneimittel zur Hemmung der Knochenresorption in einem Säuger, der ein Mensch sein kann.

**45.** Pharmazeutische Zusammensetzung nach Anspruch 42 zur Verwendung als Arzneimittel zur Behandlung oder Prophylaxe entzündlicher Erkrankungen in einem Säuger, der ein Mensch sein kann.

**46.** Pharmazeutische Zusammensetzung nach Anspruch 42 zur Verwendung als Arzneimittel zur Behandlung oder Prophylaxe viraler Erkrankungen in einem Säuger, der ein Mensch sein kann.

**47.** Pharmazeutische Zusammensetzung nach Anspruch 42 zur Verwendung als Arzneimittel zur Linderung von Schmerzen oder Pyrexie in einem Säuger, der ein Mensch sein kann.

**48.** Pharmazeutische Zusammensetzung nach Anspruch 42 zur Verwendung als Arzneimittel zur Behandlung oder Prophylaxe chronisch rheumatoider Arthritis in einem Säuger, der ein Mensch sein kann.

**49.** Pharmazeutische Zusammensetzung nach Anspruch 42 zur Verwendung als Arzneimittel zur Behandlung oder Prophylaxe von Osteoarthritis in einem Säuger, der ein Mensch sein kann.

**50.** Pharmazeutische Zusammensetzung nach Anspruch 42 zur Verwendung als Arzneimittel zur Behandlung oder Prophylaxe von Krebs in einem Säuger, der ein Mensch sein kann.

**51.** Pharmazeutische Zusammensetzung nach Anspruch 42 zur Verwendung als Arzneimittel zur Behandlung oder Prophylaxe von Hepatitis in einem Säuger, der ein Mensch sein kann.

**52.** Pharmazeutische Zusammensetzung nach Anspruch 42 zur Verwendung als Arzneimittel zur Behandlung oder Prophylaxe einer Erkrankung, ausgewählt aus der Gruppe, bestehend aus allergischen Erkrankungen, Septikämie, Psoriasis, Asthma, Degenerationsarthritis, Crohn'sche Krankheit, systemischem Lupus erythematodes, Osteoporose, Colitis ulcerosa, Diabetes, Nephritis, ischämischer Herzerkrankung, Alzheimer'sche Krankheit und Arteriosklerose in einem Säuger, der ein Mensch sein kann.

**53.** Pharmazeutische Zusammensetzung nach Anspruch 42 zur Verwendung als Arzneimittel zur Behandlung oder Prophylaxe von Septikämie in einem Säuger, der ein Mensch sein kann.

**54.** Verbindung der Formel (I-1) oder ein pharmakologisch akzeptables/r Salz, Ester oder Amid davon nach einem der Ansprüche 1 bis 41 zur Verwendung als Arzneimittel.

**55.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Hemmung der Produktion entzündlicher Zytokine in einem Säuger, der ein Mensch sein kann.

**56.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Hemmung der Knochenresorption in einem Säuger, der ein Mensch sein kann.

**57.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe entzündlicher Erkrankungen in einem Säuger, der ein Mensch sein kann.

**58.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe viraler Erkrankungen in einem Säuger, der ein Mensch sein kann.

**59.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Linderung von Schmerzen oder Pyrexie in einem Säuger, der ein Mensch sein kann.

**60.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe chronisch rheumatoider Arthritis in einem Säuger, der ein Mensch sein kann.

**61.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe von Osteoarthritis in einem Säuger, der ein Mensch sein kann.

**62.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe von Krebs in einem Säuger, der ein Mensch sein kann.

**63.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe von Hepatitis in einem Säuger, der ein Mensch sein kann.

**64.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe einer Erkrankung, ausgewählt aus der Gruppe, bestehend aus allergischen Erkrankungen, Septikämie, Psoriasis, Asthma, Degenerationsarthritis, Crohn'sche Krankheit, systemischem Lupus erythematodes, Osteoporose, Colitis ulcerosa, Diabetes, Nephritis, ischämischer Herzerkrankung, Alzheimer'sche Krankheit und Arteriosklerose in einem Säuger, der ein Mensch sein kann.

**65.** Verwendung mindestens einer Verbindung der Formel (I-1) oder eines pharmakologisch akzeptablen Salzes, Esters oder Amids davon nach einem der Ansprüche 1 bis 41 bei der Herstellung eines Arzneimittels zur Behandlung oder Prophylaxe von Septikämie in einem Säuger, der ein Mensch sein kann.

**66.** Verfahren zur Herstellung einer Verbindung nach einem der Ansprüche 1 bis 41, umfassend die folgenden Schritte:

(a) Unterziehen einer Verbindung der Formel (III)

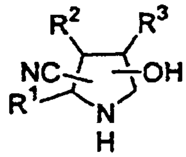

wobei $R^1$, $R^2$ und $R^3$ wie in einem der Ansprüche 1 bis 41 definiert sind, und die Cyano- und Hydroxysubstituenten an dem Pyrrolidinylring an den Kohlenstoffatomen des Rings sind, die zum Stickstoffatom des Rings benachbart sind,
einer Reaktion, die zur Entfernung von Wasserstoffcyanid und Wasser aus der Verbindung der Formel (IIIa) führt, wobei die gewünschte Verbindung der Formel (I-1) erhalten wird; und
(b) wahlweise Unterziehen der Verbindung der Formel (I-1), die in Schritt (a) erhalten wurde, einer oder mehrerer Reaktionen, um einen oder mehrere der Substituenten $R^1$, $R^2$ und $R^3$ in andere Substituenten umzuwandeln, die in die Definition dieser Gruppen fallen, und/oder die Verbindung der Formel (I) in ein Salz, Ester oder Amid davon umzuwandeln.

**67.** Verfahren zur Herstellung einer Verbindung der Formel (I-1) oder eines pharmakologisch akzeptablen Salzes, Esters oder Amids davon nach einem der Ansprüche 1 bis 41, wobei das Verfahren die folgenden Schritte umfasst:

(a) Lithiieren einer Verbindung der Formel (IVa):

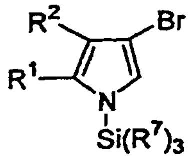

(IVa)

wobei $R^1$, $R^2$ wie oben definiert sind und jedes $R^7$ gleich oder verschieden ist und jeweils ein Wasserstoffatom, eine Niederalkylgruppe wie in Anspruch 1 definiert, eine wie in Anspruch 1 definierte Arylgruppe oder eine wie in Anspruch 1 definierte Aralkylgruppe darstellt, und dann Umsetzen des so erhaltenen lithiierten Derivats mit einer Verbindung der Formel $R^3$-L, wobei $R^3$ wie oben definiert ist und L eine Gruppe ist, die befähigt ist, als nucleophiler Rest auszutreten, um eine Verbindung der Formel (Va) zu erhalten:

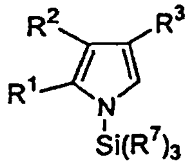

(Va)

wobei $R^1$, $R^2$, $R^3$ und $R^7$ wie oben definiert sind; und
(b) Unterziehen der in Schritt (a) erhaltenen Verbindung der Formel (Va) einer Desilylierungsreaktion, um die gewünschte Verbindung der Formel (I-1) zu erhalten; und
(c) wahlweise Unterziehen der in Schritt (b) erhaltenen Verbindung der Formel (I-1) einer oder mehrerer Reaktionen, um einen oder mehrere der Substituenten $R^1$, $R^2$ und $R^3$ in andere Substituenten umzuwandeln, die in die Definition dieser Gruppen fallen, und/oder die Verbindung der Formel (I-1) in ein Salz, Ester oder Amid davon umzuwandeln.

**68.** Verfahren zur Herstellung einer Verbindung der Formel (I-1) oder eines pharmakologisch akzeptablen Salzes, Esters oder Amids davon nach einem der Ansprüche 1 bis 41, wobei das Verfahren die folgenden Schritte umfasst:

(a) Lithiieren einer Verbindung der Formel (IVa):

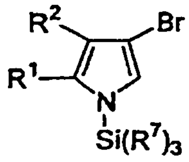

(IVa)

wobei $R^1$ und $R^2$ wie oben definiert sind und $R^7$ wie in Anspruch 67 definiert ist, und anschließend Umsetzen des so erhaltenen lithiierten Derivats mit einer Verbindung der Formel (VI) oder der Formel (VII):

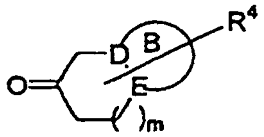

(VI)

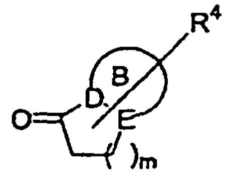

(VII)

wobei B, D, E, $R^4$ und m wie in einem der Ansprüche 1 bis 41 definiert sind, um eine Verbindung der Formel (VIIIa) oder (IXa) zu erhalten:

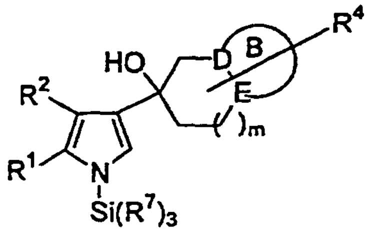

(VIIIa)

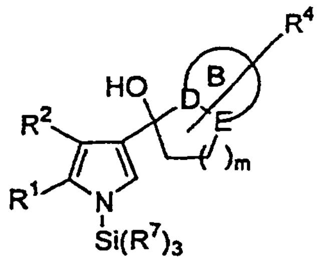

(IXa)

wobei $R^1$, $R^2$, $R^4$, $R^7$, B, D, E und m wie oben definiert sind; und

(b) Unterziehen der in Schritt (a) erhaltenen Verbindung der Formel (VIIIa) oder (IXa) nachfolgenden Schritten der Dehydratation und Desilylierung, um die gewünschte Verbindung der Formel (I-1) zu erhalten; und

(c) wahlweise Unterziehen der in Schritt (b) erhaltenen Verbindung der Formel (I-1) einer oder mehrerer Reaktionen, um einen oder mehrere der Substituenten $R^1$, $R^2$ und $R^3$ in andere Substituenten umzuwandeln, die in die Definition dieser Gruppen fallen, und/oder die Verbindung der Formel (I-1) in ein Salz, Ester oder Amid davon umzuwandeln.

## Revendications

**1.** Composé de formule (I-1), ou sel, ester ou amide pharmacologiquement acceptable de celui-ci:

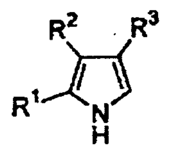

(I-1)

dans laquelle:

$R^1$ est sélectionné parmi le groupe composé des groupes aryle tels que définis ci-dessous qui peuvent éventuellement être substitués par au moins un substituant sélectionné parmi le groupe de substituants $\alpha$ et le groupe de substituants $\beta$ décrits ci-dessous,et des groupes hétéroaryle tels que définis ci-dessous qui peuvent éventuellement être substitués par au moins un substituant sélectionné parmi le groupe de substituants $\alpha$ et le groupe de substituants $\beta$ décrits ci-dessous ;

$R^2$ représente un groupe hétéroaryle tel que défini ci-dessous qui possède au moins un atome d'azote cyclique, ledit groupe hétéroalryle étant éventuellement substitué par au moins un substituant sélectionné parmi le groupe de substituants $\alpha$ et le groupe de substituants P décrits ci-dessous ; et

$R^3$ représente un groupe de formule générale (IIa), (IIb) ou (IIc) telles que représentées ci-dessous:

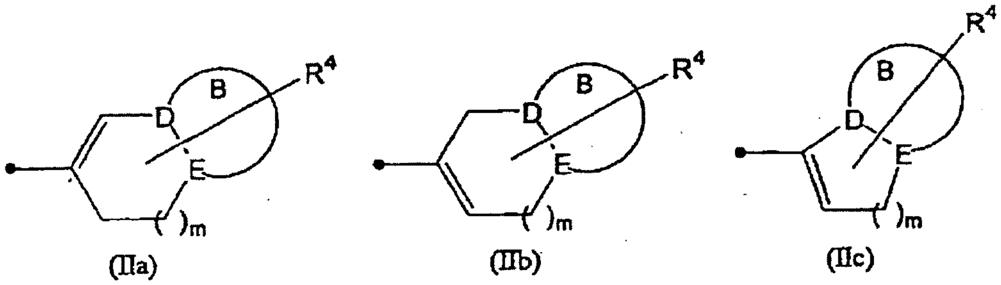

(IIa) (IIb) (IIc)

dans laquelle

m représente 1 ou 2,

l'un de D et E représente un atome d'azote et l'autre représente un groupe de formule > C($R^5$)- (dans laquelle $R^5$ est sélectionné parmi le groupe composé d'atomes d'hydrogène, du groupe de substituants $\alpha$ et du groupe de substituants P définis ci-dessous),

B représente un cycle hétérocyclique de 4 à 7 chaînons qui possède au moins un atome d'azote cyclique (ledit cycle hétérocyclique peut être saturé ou insaturé et peut éventuellement être fusionné avec un groupe choisi parmi les groupes aryle définis ci-dessous, les groupes hétéroalryle définis ci-dessous, les groupes cycloalkyle définis ci-dessous et les groupes hétérocyclyle définis ci-dessous), et

$R^4$ représente de 1 à 3 substituants sélectionnés indépendamment parmi le groupe composé du groupe de substituants $\alpha$ défini ci-dessous, du groupe de substituants $\beta$ défini ci-dessous et du groupe de substituants $\gamma$ défini ci-dessous, ou, si B est un cycle hétérocyclique qui est fusionné à un groupe aryle, un groupe hétéroaryle, un groupe cycloalkyle ou un groupe hétérocyclyle, $R^4$ peut être un atome d'hydrogène ;

le groupe de substituants $\alpha$ est composé de groupes hydroxyle, de groupes nitro, de groupes cyano, d'atomes d'halogènes, des groupes alcoxy inférieur définis ci-dessous, des groupes halogéno-alcoxy inférieur définis ci-dessous, des groupes alkylthio inférieur définis ci-dessous, de groupes halogéno-alkylthio inférieur définis ci-dessous et des groupes de formule $-NR^aR^b$ (où $R^a$ et $R^b$ sont identiques ou différents l'un de l'autre et chacun d'eux est sélectionné indépendamment parmi le groupe composé d'atomes d'hydrogène, des groupes alkyle inférieur définis ci-dessous, des groupes alcényle inférieur définis ci-dessous, des groupes alcynyle inférieur définis ci-dessous, des groupes aralkyle inférieur définis ci-dessous et des groupes alcylsulfonyle inférieur définis ci-dessous, ou $R^a$ et $R^b$, ensemble avec l'atome d'azote auquel ils sont liés, forment un groupe hétérocyclyle) ;

le groupe de substituants $\beta$ est composé de groupes alkyle inférieur définis ci-dessous qui peuvent éventuellement être substitués par au moins un substituant choisi parmi le groupe de substituants $\alpha$ défini ci-dessus, des groupes alcényle inférieur définis ci-dessous, qui peuvent éventuellement être substitués par au moins un substituant choisi

parmi le groupe de substituants α défini ci-dessus, des groupes alcynyle inférieur définis ci-dessous qui peuvent éventuellement être substitués par au moins un substituant choisi parmi le groupe de substituants α défini ci-dessus, des groupes aralkyle définis ci-dessous et des groupes cycloalkyle tels que définis ci-dessous.

le groupe de substituants γ est composé de groupes oxo, de groupes hydroxyimino, des groupes alcoxyimino inférieurs définis ci-dessous, des groupes alkylène inférieur définis ci-dessous, des groupes alkylènedioxy inférieur définis ci-dessous, des groupes alkylsulfinyle inférieur définis ci-dessous, des groupes alkylsulfonyle inférieur définis ci-dessous, des groupes aryle définis ci-dessous qui peuvent éventuellement être substitués par au moins un substituant choisi parmi le groupe substituant α défini ci-dessus et le groupe de substituants β défini ci-dessus, des groupes aryloxy définis ci-dessous qui peuvent éventuellement être substitués par au moins un substituant choisi parmi le groupe de substituants α défini ci-dessus et le groupe de substituants β défini ci-dessus, de groupes alkylidényle inférieur et de groupes aralkylidényle ;

lesdits groupes aryle éventuellement substitués dans les définitions de $R^1$, du cycle B et du groupe de substituants γ ci-dessus sont des groupes hydrocarbure aromatique possédant de 6 à 14 atomes de carbone dans un ou plusieurs cycles, lesdits groupes aryle étant éventuellement fusionnés avec un groupe cycloalkyle possédant de 3 à 10 atomes de carbone ;

lesdits groupes hétéroaryle éventuellement substitués dans la définition de $R^1$ et du cycle B ci-dessus sont des groupes hétérocycliques aromatiques de 5 à 7 chaînons contenant de 1 à 3 hétéroatomes choisis parmi le groupe composé d'atomes de soufre, d'atomes d'oxygène et d'atomes d'azote, lesdits groupes hétéroaryle étant éventuellement fusionnés avec un autre groupe cyclique choisi parmi le groupe composé des groupes aryle définis ci-dessus et de groupes cycloalkyle possédant de 3 à 10 atomes de carbone ;

lesdits groupes hétéroaryle éventuellement substitués possédant au moins un atome d'azote cyclique dans la définition de $R^2$ ci-dessus sont des groupes hétérocycliques aromatiques de 5 à 7 chaînons contenant au moins un atome d'azote et, éventuellement, un ou deux autres hétéroatomes choisis parmi le groupe composé d'atomes de soufre, d'atomes d'oxygène et d'atomes d'azote ;

lesdits groupes alkyle inférieur dans les définitions de $R^a$, $R^b$ et du groupe de substituants β ci-dessus, et le groupement alkyle inférieur desdits groupes alkyle inférieur qui peut éventuellement être substitué par au moins un substituant choisi parmi le groupe de substituants α dans la définition du groupe de substituants β ci-dessus sont des groupes alkyle linéaires ou ramifiés possédant de 1 à 6 atomes de carbone ;

lesdits groupes alcényle inférieurs dans les définitions de $R^a$, $R^b$ et du groupe de substituants β ci-dessus, et le groupement alcényle inférieur desdits groupes alcényle inférieur qui peut éventuellement être substitué par au moins un substituant choisi parmi le groupe de substituants α dans la définition du groupe de substituants β ci dessus, sont des groupes alcényle linéaires ou ramifiés possédant de 2 à 6 atomes de carbone ;

lesdits groupes alcynyle inférieurs dans les définitions de $R^a$, $R^b$ et du groupe de substituants P ci-dessus, et le groupement alcynyle inférieur desdits groupes alcynyle inférieur qui peut éventuellement être substitué par au moins un substituant choisi parmi le groupe de substituants α dans la définition du groupe de substituants P ci-dessus sont des groupes alcynyle linéaires ou ramifiés possédant de 2 à 6 atomes de carbone ;

lesdits groupes aralkyle dans les définitions de $R^a$, $R^b$ et du groupe de substituants β ci-dessus sont des groupes alkyle inférieur tels que définis ci-dessus qui sont substitués par au moins un groupe aryle comme défini ci-dessus qui peut éventuellement être substitué par de 1 à 3 substituants choisis parmi le groupe de substituants α défini ci-dessus et le groupe de substituants β défini ci-dessus ;

lesdits groupes alkylsulfonyle inférieur dans les définitions de $R^a$, $R^b$ et du groupe de substituants γ ci-dessus sont des groupes alkyle inférieur tels que définis ci-dessus qui sont liés à un groupe sulfonyle ;

si le cycle B est fusionné avec un groupe hétérocyclyle, lesdits groupes hétérocyclyle sont des groupes hétérocyclyle comprenant de 4 à 7 chaînons qui contiennent de 1 à 3 hétéroatomes cycliques sélectionnés parmi le groupe composé d'atomes de soufre, d'atomes d'oxygène et d'atomes d'azote ;

si $R^a$ et $R^b$, ensemble avec l'atome d'azote auquel ils sont liés, représentent un groupe hétérocyclyle, lesdits groupes hétérocyclyle sont des groupes hétérocyclyle de 4 à 7 chaînons qui contiennent un atome d'azote et éventuellement un autre hétéroatome sélectionné parmi le groupe composé d'atomes d'oxygène, d'atomes de soufre et d'atomes d'azote, lesdits groupes hétérocyclyle étant éventuellement fusionnés avec un autre groupe cyclique choisi parmi le groupe composé des groupes aryle définis ci-dessus et des groupes hétéroalryle définis ci-dessus ;

lesdits groupes alcoxy inférieur dans la définition du groupe de substituants α ci-dessus sont des groupes dans lesquels un atome d'oxygène est lié à un groupe alkyle inférieur tel que défini ci-dessus ;

lesdits groupes halogéno-alcoxy inférieur dans la définition du groupe de substituants α ci-dessus sont des groupes dans lesquels un groupe alcoxy inférieur tel que défini ci-dessus est substitué par au moins un atome d'halogène ;

lesdits groupes alkylthio inférieur dans la définition du groupe de substituants α ci-dessus sont des groupes dans lesquels un atome de soufre est lié à un groupe alkyle inférieur comme défini ci-dessus ;

lesdits groupes halogéno-alkylthio inférieur dans la définition du groupe de substituants α ci-dessus sont des groupes dans lesquels un groupe alkylthio inférieur tel que défini ci-dessus est substitué par au moins un atome d'halogène ;

lesdits groupes cycloalkyle dans la définition du groupe de substituants β et du cycle B ci-dessus sont des groupes cycloalkyle possédant de 3 à 7 atomes de carbone ;
lesdits groupes alcoxyimino inférieur dans la définition du groupe de substituants γ ci-dessus sont des groupes dans lesquels l'atome d'hydrogène d'un groupe hydroxyimino est remplacé par un groupe alkyle inférieur tel que définis ci-dessus ;
lesdits groupes alkylène inférieur dans la définition du groupe de substituants γ ci-dessus sont des groupes alkylène possédant de 2 à 6 atomes de carbone ;
lesdits groupes alkylènedioxy inférieur dans la définition du groupe de substituants γ ci-dessus sont des groupes dans lesquels un groupement alkylène, qui est un groupe alkylène linéaire ou ramifié possédant de 1 à 6 atomes de carbone, est substitué par 2 groupes oxy ;
lesdits groupes alkylsulfinyle inférieurs dans la définition du groupe de substituants γ ci-dessus sont des groupes dans lesquels un groupe alkyle inférieur tel que défini ci-dessus est lié à un groupe sulfinyle ;
lesdits groupes alkylidényle inférieurs dans la définition du groupe de substituants γ ci-dessus sont des groupes alkylidényle linéaires ou ramifiés possédant de 1 à 6 atomes de carbone ;
lesdits groupes aralkylidényle dans la définition du groupe de substituants γ ci-dessus sont des groupes alkylidényle inférieur tels que définis ci-dessus qui sont substitués par 1 ou plusieurs groupes aryle tels que définis ci-dessus ;
lesdits groupes aryloxy éventuellement substitués dans la définition du groupe de substituants γ ci-dessus sont des groupes dans lesquels un atome d'oxygène est lié à un groupe aryle tel que défini ci-dessus.

**2.** Composé selon la revendication 1 ou sel, ester ou amide de celui-ci pharmacologiquement acceptable, dans lequel $R^1$ est un groupe aryle qui peut éventuellement être substitué par au moins un substituant choisi parmi le groupe de substituants α et le groupe de substituants β tels que définis selon la revendication 1.

**3.** Composé selon la revendication 1 ou sel, ester ou amide de celui-ci pharmacologiquement acceptable, dans lequel $R^1$ est un groupe phényle ou naphthyle, lesdits groupes étant éventuellement substitués par au moins un substituant choisi parmi le groupe de substituants α et le groupe de substituants β tels que définis selon la revendication 1.

**4.** Composé selon la revendication 1 ou sel, ester ou amide de celui-ci pharmacologiquement acceptable, dans lequel $R^1$ est un groupe phényle qui peut éventuellement être substitué par au moins un substituant choisi parmi le groupe de substituants $\alpha^1$ défini ci-dessous et le groupe de substituants $\beta^1$ défini ci-dessous ;
ledit groupe de substituants $\alpha^1$ est composé d'atomes d'halogènes, de groupes alcoxy inférieur, de groupes halogéno alcoxy inférieur et de groupes de formule $-NR^aR^b$ (dans laquelle l'un de $R^a$ et $R^b$ représente un atome d'hydrogène ou un groupe alkyle inférieur et l'autre représente un atome d'hydrogène, un groupe alkyle inférieur ou un groupe aralkyle), lesdits groupes alkyle inférieur, groupes alcoxy inférieur, groupes halogéno-alcoxy inférieur et groupes aralkyle étant tels que définis à la revendication 1 ;
ledit groupe de substituants $\beta^1$ est composé de groupes alkyle inférieur, de groupes halogéno-alkyle inférieur, de groupes hydroxyl-alkyle inférieur, de groupes nitro-alkyle inférieur, de groupes amino-alkyle inférieur, de groupes alkyle inférieur-amino-alkyle inférieur, de groupes di(alkyle inférieur)amino-alkyle inférieur, et de groupes aralkylamino-alkyle inférieur, lesdits groupes alkyle inférieur, halogéno-alkyle inférieur, groupements alkyle desdits groupes hydroxyl-alkyle inférieur, nitro-alkyle inférieur, amino-alkyle inférieur, alkyle inférieur-amino-alkyle inférieur, et di (alkyle inférieur)amino-alkyle inférieur et groupements alkyle et aralkyle desdits groupes aralkylamino alkyle inférieur étant tels que définis à la revendication 1 ;

**5.** Composé selon la revendication 1 ou sel, ester ou amide de celui-ci pharmacologiquement acceptable, dans lequel $R^1$ est un groupe phényle qui peut éventuellement être substitué par au moins un substituant choisi parmi le groupe composé d'atomes d'halogènes, de groupes halogéno-alkyl inférieur tels que définis à la revendication 1 et de groupes halogéno alcoxy inférieur tels que définis à la revendication 1.

**6.** Composé selon la revendication 1 ou sel, ester ou amide de celui-ci pharmacologiquement acceptable, dans lequel $R^1$ est un substituant choisi parmi le groupe composé de groupes phényle, 4-fluorophényle, 3-fluorophényle, 3-chlorophényle, 3,4-difluorophényle, 3,4,5-trifluorophényle, 3-chloro-4-fluorophényle, 3-difluorométhoxyphényle, et 3-trifluorométhylphényle.

**7.** Composé selon l'une quelconque des revendications 1 à 6 ou sel, ester ou amide de celui-ci pharmacologiquement acceptable, dans lequel $R^2$ est un groupe hétérocyclique aromatique à 5 à 6 chaînons qui possède un ou deux atomes d'azote, ledit groupe étant éventuellement substitué par au moins un substituant choisi parmi le parmi le groupe de substituants α et le groupe de substituants β tels que définis à la revendication 1.

**8.** Composé selon l'une quelconque des revendications 1 à 6 ou sel, ester ou amide de celui-ci pharmacologiquement acceptable, où $R^2$ est un groupe pyridyle ou pyrimidinyle, lesdits groupes étant éventuellement substitués par au moins un substituant choisi parmi le groupe composé du groupe de substituants $\alpha$ et du groupe de substituants $\beta$ tels que définis à la revendication 1.

**9.** Composé selon l'une quelconque des revendications 1 à 6 ou sel, ester ou amide de celui-ci pharmacologiquement acceptable, dans lequel $R^2$ est un groupe 4-pyridyle ou 4-pyrimidinyle, lesdits groupes étant éventuellement substitués par au moins un substituant choisi parmi le groupe composé du groupe de substituants $\alpha$ et du groupe de substituants $\beta$ tels que définis à la revendication 1.

**10.** Composé selon l'une quelconque des revendications 1 à 6 ou sel, ester ou amide de celui-ci pharmacologiquement acceptable, dans lequel $R^2$ est un groupe 4-pyridyle ou 4-pyrimidinyle, lesdits groupes étant éventuellement substitués au niveau de leur position 2 par un substituant choisi parmi le groupe composé du groupe de substituants $\alpha$ et du groupe de substituants $\beta$ tels que définis à la revendication 1.

**11.** Composé selon l'une quelconque des revendications 1 à 6 ou sel, ester ou amide de celui-ci pharmacologiquement acceptable, dans lequel $R^2$ est un groupe 4-pyridyle ou 4-pyrimidinyle, lesdits groupes étant éventuellement substitués en position 2 par un substituant choisi parmi le groupe composé des groupes méthoxy, amino, méthylamino, benzylamino et $\alpha$-méthylbenzylamino.

**12.** Composé selon l'une quelconque des revendications 1 à 11 ou sel, ester ou amide de celui-ci pharmacologiquement acceptable, dans lequel B est un cycle hétérocyclique de 5 ou 6 chaînons qui possède un atome d'azote cyclique et éventuellement un autre hétéroatome cyclique ou un groupe cyclique choisi parmi un atome d'azote, un atome d'oxygène, un atome de soufre, >SO et $>SO_2$ (ledit cycle peut être saturé ou insaturé et peut éventuellement être fusionné avec un groupe aryle, un groupe hétéroaryle, un groupe cycloalkyle ou un groupe hétérocyclique tel que défini à la revendication 1).

**13.** Composé selon l'une quelconque des revendications 1 à 11 ou sel, ester ou amide de celui-ci pharmacologiquement acceptable, dans lequel B est un cycle hétérocyclique de 5 ou 6 chaînons qui est composé du groupe D, du groupe E et de ou quatre atomes de carbone (ledit cycle peut être saturé ou insaturé et peut éventuellement être fusionné avec un groupe aryle, un groupe hétéroaryle, un groupe cycloalkyle ou un groupe hétérocyclyle tel que défini à la revendication 1).

**14.** Composé selon l'une quelconque des revendications 1 à 11 ou sel, ester ou amide de celui-ci pharmacologiquement acceptable, dans lequel B représente un cycle pyrrolidinyle ou un cycle pyrrolinyle.

**15.** Composé selon l'une quelconque des revendications 1 à 14 ou sel, ester ou amide de celui-ci pharmacologiquement acceptable, dans lequel $R^3$ représente un groupe de formule générale (IIa) ou de formule (IIb).

**16.** Composé selon l'une quelconque des revendications 1 à 14 ou sel, ester ou amide de celui-ci pharmacologiquement acceptable, dans lequel $R^3$ représente un groupe de formule générale (IIa).

**17.** Composé selon l'une quelconque des revendications 1 à 16 ou sel, ester ou amide de celui-ci pharmacologiquement acceptable, dans lequel m représente 1.

**18.** Composé selon l'une quelconque des revendications 1 à 17 ou sel, ester ou amide de celui-ci pharmacologiquement acceptable, dans lequel $R^4$ représente 1 ou 2 substituants qui sont choisis indépendamment parmi le groupe composé du groupe de substituants $\alpha$ tel que défini selon la revendication 1, de groupe de substituants $\beta$ tel que défini à la revendication 1 et du groupe de substituants $\gamma^1$, ledit le groupe de substituants $\gamma^1$ étant composé de groupes oxo, de groupes hydroxyimino, de groupes alcoxyimino inférieur, de groupes alkylène inférieur, de groupes alkylènedioxy inférieur, de groupes alkylsulfinyle inférieur, de groupes alkylsulfonyle inférieur et de groupes aryle, qui peuvent être substitués par au moins un substituant choisi parmi le groupe de substituants $\alpha$ et le groupe de substituants $\beta$ tels que définis à la revendication 1.

**19.** Composé selon l'une quelconque des revendications 1 à 17 ou sel, ester ou amide de celui-ci pharmacologiquement acceptable, dans lequel $R^4$ est un substituant choisi parmi le groupe composé de groupes hydroxy, d'atomes d'halogènes, des groupes alcoxy inférieur, des groupes alkylthio inférieur, des groupes halogéno-alcoxy inférieur, des groupes alkyle inférieur, des groupes halogénoalkyle inférieur, des groupes oxo, des groupes aryle éventuellement substitués par au moins un substituant choisi parmi le groupe de substituants α et le groupe de substituants β, des groupes alkylène dioxy inférieur, des groupes alkylène inférieur et des groupes alkylsulfonyle inférieur tels que définis à la revendication 1.

**20.** Composé selon l'une quelconque des revendications 1 à 17 ou sel, ester ou amide de celui-ci pharmacologiquement acceptable, dans lequel $R^4$ est un substituant choisi parmi le groupe composé de groupes hydroxy, d'atomes de fluore, d'atomes de chlore, de groupes méthoxy, de groupes éthoxy, de groupes propoxy, de groupes méthyle, de groupes éthyle, de groupes propyle et de groupes phényle qui peuvent éventuellement être substitués par au moins un substituant choisi parmi le groupe de substituants α et le groupe de substituants β, tels que définis à la revendication 1.

**21.** Composé selon l'une quelconque des revendications 1 à 17 ou sel, ester ou amide de celui-ci pharmacologiquement acceptable, dans lequel $R^4$ est un substituant choisi parmi le groupe composé de groupes méthyloxy, de groupes méthyle, de groupes éthyle, de groupes propyle et de groupes phényle.

**22.** Composé selon l'une quelconque des revendications 1 à 17 ou sel, ester ou amide de celui-ci pharmacologiquement acceptable, dans lequel $R^4$ est un substituant choisi parmi le groupe composé de groupes aryloxy qui peuvent éventuellement être substitués par au moins un substituant choisi parmi le groupe de substituants α et le groupe de substituants β, des groupes alkylidène et aralkylidène tels que définis à la revendication 1.

**23.** Composé selon l'une quelconque des revendications 1 à 17 ou sel, ester ou amide de celui-ci pharmacologiquement acceptable, dans lequel $R^4$ est un substituant choisi parmi le groupe composé de groupes phénoxy, méthylidène, éthylidène, propylidène et benzylidène.

**24.** Composé selon l'une quelconque des revendications 1 à 23 ou sel, ester ou amide de celui-ci pharmacologiquement acceptable, dans lequel D est un groupe de formule >C($R^5$)- (où $R^5$ est choisi parmi le groupe composé d'atomes d'hydrogène, du groupe de substituants α et du groupe de substituants β, tels que définis à la revendication 1) et E est un atome d'azote.

**25.** Composé selon la revendication 1, ou sel, ester ou amide de celui-ci pharmacologiquement acceptable dans lequel :

$R^1$ représente un groupe aryle qui peut éventuellement être substitué par au moins un substituant choisi parmi le groupe de substituants α et le groupe de substituants β;
$R^2$ représente un groupe hétérocyclique aromatique à 5 ou 6 chaînons qui possède un ou deux atomes d'azote, ledit groupe étant éventuellement substitué par au moins un substituant choisi parmi le groupe de substituants α et le groupe de substituants β; et
$R^3$ représente un groupe de formule générale (IIa) ou de formule générale (IIb) où
m représente 1,
D représente un groupe de formule >C($R^5$) - (où $R^5$ est choisi parmi le groupe composé d'atomes d'hydrogène, du groupe de substituants α et du groupe de substituants β), et E est un atome d'azote, et
$R^4$ représente un ou deux substituants choisis indépendamment parmi le groupe composé du groupe de substituants α, du groupe de substituants β, du groupe de substituants $\gamma^1$, où ledit groupe de substituants $\gamma^1$ est composé de groupes oxo, hydroxyimino, alcoxyimino inférieur, alkylène inférieur, alkylènedioxy inférieur, alkylsulfinyl inférieur, alcylsulfonyle inférieur et aryle qui peuvent éventuellement être substitués par au moins un substituant choisi parmi le groupe de substituants α et le groupe de substituants β.

**26.** Composé selon la revendication 1, ou sel, ester ou amide de celui-ci pharmacologiquement acceptable dans lequel :

$R^1$ représente un groupe phényle ou naphthyle, lesdits groupes étant éventuellement substitués par au moins un substituant choisi parmi le groupe de substituants α et le groupe de substituants β;
$R^2$ représente un groupe pyridyle ou pyrimidinyle, lesdits groupes étant éventuellement substitués par au moins un substituant choisi parmi le groupe de substituants α et le groupe de substituants β; et
$R^3$ représente un groupe de formule générale (IIa) ou de formule générale (IIb) où
m représente 1,
D représente un groupe de formule >C($R^5$)- (où $R^5$ est choisi parmi le groupe composé d'atomes d'hydrogène, du groupe de substituants α et du groupe de substituants β), et E est un atome d'azote, et
$R^4$ représente un substituant choisi parmi le groupe composé de groupes hydroxy, d'atomes d'halogènes, de groupes alcoxy inférieur, alkylthio inférieur, de groupes halogénoalcoxy inférieur, alkyle inférieur, halogéno-alkyle inférieur, oxo et aryle qui peuvent éventuellement être substitués par au moins un substituant choisi parmi le groupe de substituants α et le groupe de substituants β, et de groupes alkylènedioxy inférieur, alkylène inférieur et alkylsulfonyle inférieur.

**27.** Composé selon la revendication 1, ou sel, ester ou amide de celui-ci pharmacologiquement acceptable dans lequel :

$R^1$ représente un groupe phényle, qui peut éventuellement être substitué par au moins un substituant choisi parmi le groupe de substituants $\alpha^1$ défini ci-dessous et le groupe de substituants $\beta^1$ défini ci-dessous;
ledit groupe de substituants $\alpha^1$ est composé d'atomes d'halogène, de groupes alcoxy inférieur, de groupes halogéno-alcoxy inférieur et de groupes de formule $-NR^aR^b$ (où l'un de $R^a$ et $R^b$ représente un atome d'hydrogène ou un groupe alkyle inférieur et l'autre représente un atome d'hydrogène, un groupe alkyle inférieur ou un groupe aralkyle), lesdits groupes alkyle inférieur, alcoxy inférieur, halogéno-alcoxy inférieur et groupe aralkyle étant tels que défini à la revendication 1 ;
ledit groupe de substituants $\beta^1$ est composé de groupes alkyle inférieur, halogéno-alkyle inférieur, hydroxyl-alkyle inférieur, nitro-alkyle inférieur, amino-alkyle inférieur, alkyle inférieur-amino-alkyle inférieur, di(alkyle inférieur)amino-alkyle inférieur et aralkylamino-alkyle inférieur, lesdits groupes alkyle inférieur, halogéno-alkyle inférieur, groupements alkyle desdits groupes hydroxyl-alkyle inférieur, nitro-alkyle inférieur, amino-alkyle inférieur, alkyle inférieur-amino-alkyle inférieur, et di(alkyle inférieur)amino-alkyle inférieur, et groupements alkyle et aralkyle desdits groupes aralkylamino alkyle inférieur étant définis à la revendication 1 ;
$R^2$ représente un groupe 4-pyridyle ou 4-pyrimidinyle, lesdits groupes étant éventuellement substitués par au moins un substituant choisi parmi le groupe de substituants α et le groupe de substituants β; et
$R^3$ représente un groupe de formule générale (IIa) ou de formule générale (IIb) dans laquelle
m représente 1,
D représente un groupe de formule $>C(R^5)-$ (où $R^5$ est choisi parmi le groupe composé d'atomes d'hydrogène, du groupe de substituants α et du groupe de substituants β), et E est un atome d'azote, et
$R^4$ représente un substituant choisi parmi le groupe composé de groupes hydroxy, d'atomes d'halogènes, de groupes alcoxy inférieur, alkylthio inférieur, halogénoalcoxy inférieur, alkyle inférieur, halogénoalkyle inférieur, oxo, et aryle qui peuvent éventuellement être substitués par au moins un substituant choisi parmi le groupe de substituants α et le groupe de substituants β, et de groupes alkylènedioxy inférieur, alkylène inférieur et alkylsulfonyle inférieur.

**28.** Composé selon la revendication 1, ou sel, ester ou amide de celui-ci pharmacologiquement acceptable dans lequel :

$R^1$ représente un groupe phényle, qui peut éventuellement être substitué par au moins un substituant choisi parmi le groupe composé d'atomes d'halogènes, de groupes halogénoalkyle inférieur comme définis à la revendication 1 et de groupes halogénoalcoxy inférieur ;
$R^2$ représente un groupe 4-pyridyle ou 4-pyrimidinyle, lesdits groupes étant éventuellement substitués en position 2 par au moins un substituant choisi parmi le groupe de substituants α et le groupe de substituants β; et
$R^3$ représente un groupe de formule générale (IIa) où
m représente 1,
D représente un groupe de formule $>C(R^5)-$ (où $R^5$ est choisi parmi le groupe composé d'atomes d'hydrogène, du groupe de substituants α et du groupe de substituants β), et E est un atome d'azote, et
$R^4$ représente un substituant choisi parmi le groupe composé de groupes hydroxy, d'atomes de fluor, d'atomes de chlore, de groupes méthoxy, éthoxy, propoxy, méthyle, éthyle, propyle et phényle qui peuvent éventuellement être substitués par au moins un substituant choisi parmi le groupe de substituants α et le groupe de substituants β.

**29.** Composé selon la revendication 1, ou sel, ester ou amide de celui-ci pharmacologiquement acceptable dans lequel :

$R^1$ représente un substituant sélectionné parmi le groupe composé de groupes phényle, 4-fluorophényle, 3-fluorophényle, 3-chlorophényle, 3,4-difluorophényle, 3,4,5-trifluorophényle, 3-chloro-4-fluorophényle, 3-difluorométhoxyphényle, et du 3-trifluorométhylphényle,
$R^2$ représente un groupe 4-pyridyle ou 4-pyrimidinyle, lesdits groupes étant éventuellement substitués en position 2 avec un substituant choisi parmi le groupe composé de groupes méthoxy, amino, méthylamino, benzylamino et α-méthylbenzylamino ; et
$R^3$ représente un groupe de formule générale (IIa) où
m représente 1,
D représente un groupe de formule $>C(R^5)-$ (où $R^5$ est choisi parmi le groupe composé d'atomes d'hydrogène, du groupe de substituants α et du groupe de substituants β), et E est un atome d'azote, et

$R^4$ représente un substituant choisi parmi le groupe composé de groupes méthoxy, méthyle, éthyle, propyle et phényle.

**30.** Composé selon la revendication 1, ou sel, ester ou amide de celui-ci pharmacologiquement acceptable dans lequel :

$R^1$ représente un groupe aryle qui peut éventuellement être substitué par au moins un substituant choisi parmi le groupe de substituants α et le groupe de substituants β;
$R^2$ représente un groupe hétérocyclique aromatique à 5 ou 6 chaînons qui possède un ou deux atomes d'azote, ledit groupe étant éventuellement substitué par au moins un substituant choisi parmi le groupe de substituants α et le groupe de substituants β; et
$R^3$ représente un groupe de formule générale (IIa) ou de formule générale (IIb) où
m représente 1,
D représente un groupe de formule >C($R^5$)- (où $R^5$ est choisi parmi le groupe composé d'atomes d'hydrogène, du groupe de substituants α et du groupe de substituants β), et E est un atome d'azote, et
$R^4$ représente un substituant choisi parmi le groupe composé des groupes aryloxy qui peuvent éventuellement être substitués par au moins un substituant choisi parmi le groupe de substituants α et le groupe de substituants β, des groupes alkylidène et des groupes aralkylidène.

**31.** Composé selon la revendication 1, ou sel, ester ou amide de celui-ci pharmacologiquement acceptable dans lequel :

$R^1$ représente un groupe phényle ou naphtyle, lesdits groupes étant éventuellement substitués par au moins un substituant choisi parmi le groupe de substituants α et le groupe de substituants β;
$R^2$ représente un groupe pyridyle ou pyrimidinyle, lesdits groupes étant éventuellement substitués par au moins un substituant choisi parmi le groupe de substituants α et le groupe de substituants β; et
$R^3$ représente un groupe de formule générale (IIa) ou de formule générale (IIb) où
m représente 1,
D représente un groupe de formule >C($R^5$)- (où $R^5$ est choisi parmi le groupe composé d'atomes d'hydrogène, du groupe de substituants α et du groupe de substituants β), et
E est un atome d'azote, et
$R^4$ est un substituant choisi parmi le groupe composé de groupes aryloxy qui peuvent éventuellement être substitués par au moins un substituant choisi parmi le groupe de substituants α et le groupe de substituants β, de groupes alkylidène et de groupes aralkylidène.

**32.** Composé selon la revendication 1, ou sel, ester ou amide de celui-ci pharmacologiquement acceptable dans lequel :

$R^1$ représente un groupe phényle, qui peut éventuellement être substitué par au moins un substituant choisi parmi le groupe de substituants $\alpha^1$ défini ci-dessous et le groupe de substituants $\beta^1$ défini ci-dessous;
ledit groupe de substituants $\alpha^1$ est composé d'atomes d'halogènes, de groupes alcoxy inférieurs, de groupes halogéno alcoxy inférieur et de groupes de formule $-NR^aR^b$ (où l'un de $R^a$ et $R^b$ représente un atome d'hydrogène ou un groupe alkyle inférieur et l'autre représente un atome d'hydrogène, un groupe alkyle inférieur ou un groupe aralkyle), lesdits groupes alkyle inférieur, alcoxy inférieur, halogéno-alcoxy inférieur et aralkyle étant tels que définis à la revendication 1 ;
ledit groupe de substituants $\beta^1$ est composé de groupes alkyle inférieur, halogéno-alkyle inférieur, hydroxyl-alkyle inférieur, nitro-alkyle inférieur, amino-alkyle inférieur, alkyle inférieur-amino-alkyle inférieur, di(alkyle inférieur)amino-alkyle inférieur et aralkylamino-alkyle inférieur, lesdits groupes alkyle inférieur, halogéno-alkyle inférieur, groupements alkyle desdits groupes hydroxyl-alkyle inférieur, nitro-alkyle inférieur, amino-alkyle inférieur, alkyle inférieur-amino-alkyle inférieur, et di(alkyle inférieur)amino-alkyle inférieur, et groupements alkyle et aralkyle desdits groupes aralkylamino alkyle inférieur étant définis à la revendication 1 ;
$R^2$ représente un groupe 4-pyridyle ou 4-pyrimidinyle, lesdits groupes étant éventuellement substitués par au moins un substituant choisi parmi le groupe composé par le groupe de substituants α et le groupe de substituants β; et
$R^3$ représente un groupe de formule générale (IIa) ou de formule générale (IIb) où
m représente 1,
D représente un groupe de formule >C($R^5$)- (où $R^5$ est choisi parmi le groupe composé d'atomes d'hydrogène, du groupe de substituants α et du groupe de substituants β), et
E est un atome d'azote, et
$R^4$ représente un substituant choisi parmi le groupe composé de groupes aryloxy qui peuvent éventuellement être substitués par au moins un substituant choisi parmi le groupe de substituants α et le groupe de substituants

β, de groupes alkylidène et de groupes aralkylidène.

**33.** Composé selon la revendication 1, ou sel, ester ou amide de celui-ci pharmacologiquement acceptable dans lequel :

$R^1$ représente un groupe phényle qui peut éventuellement être substitué par au moins un substituant choisi parmi le groupe composé d'atomes d'halogènes, des groupes halogénoalkyle inférieurs tels que définis à la revendication 1 et de groupes halogéno-alcoxy inférieurs ;
$R^2$ représente un groupe 4-pyridyle ou 4-pyrimidinyle, lesdits groupes étant éventuellement substitués en position 2 avec un substituant choisi parmi le groupe de substituants α et le groupe de substituants β; et
$R^3$ est un groupe de formule générale (IIa) dans laquelle
m représente 1,
D représente un groupe de formule >C($R^5$)- (où $R^5$ est choisi parmi le groupe composé d'atomes d'hydrogène, du groupe de substituants α et du groupe de substituants β), et
E est un atome d'azote, et
$R^4$ représente un substituant choisi parmi le groupe composé de groupes phénoxy, méthylidène, éthylidène, propylidène et benzylidène.

**34.** Composé selon la revendication 1, ou sel, ester ou amide de celui-ci pharmacologiquement acceptable dans lequel :

$R^1$ représente un substituant sélectionné parmi le groupe composé des groupes phényle, 4-fluorophényle, 3-fluorophényle, 3-chlorophényle, 3,4-difluorophényle, 3,4,5-trifluorophényle, 3-chloro-4-fluorophényle, 3-difluorométhoxyphényle, et 3-trifluorométhylphényle ;
$R^2$ représente un groupe 4-pyridyle ou 4-pyrimidinyle, lesdits groupes étant éventuellement substitués en position 2 avec un substituant choisi parmi le groupe composé de groupes méthoxy, amino, méthylamino, benzylamino et α-méthylbenzylamino ;et
$R^3$ représente un groupe de formule générale (IIa) dans laquelle
m représente 1,
D représente un groupe de formule >C($R^5$)- (où $R^5$ est choisi parmi le groupe composé d'atomes d'hydrogène, du groupe de substituants α et du groupe de substituants β), et
E est un atome d'azote, et
$R^4$ représente un substituant choisi parmi le groupe composé de groupes phénoxy, méthylidène, éthylidène, propylidène et benzylidène.

**35.** Composé selon la revendication 1, choisi parmi les composants suivants ou un sel ou un amide de ceux-ci pharmacologiquement acceptable dans lequel :

2- (3-fluorophényl)-4-[2-méthyl-1,2,3,5,6,8a-hexahydroindolizin-7-yl]-3-(pyridin-yl)-1*H*-pyrrole,
2- (3-fluorophényl)-4-[2-phényl-1,2,3,5,6,8a-hexahydroindolizin-7-yl]-3-(pyridin-yl)-1*H*-pyrrole,
2- (3-fluorophényl)-4-[2-méthoxy-1,2,3,5,6,8a-hexahydroindolizin-7-yl]-3-(pyridin-4-yl)-1*H*-pyrrole,
2- (4-fluorophényl)-4-[2-méthyl-1,2,3,5,6,8a-hexahydroindolizin-7-yl]-3-(pyridin-4-yl)-1*H*-pyrrole,
2- (4-fluorophényl)-4-[2-phényl-1,2,3,5,6,8a-hexahydroindolizin-7-yl]-3-(pyridin-4-yl)-1*H*-pyrrole,
2- (4-fluorophényl)-4-[2-méthoxy-1,2,3,5,6,8a-hexahydroindolizin-7-yl]-3-(pyridin- 4-yl)-1H-pyrrole,
2- (3-chlorophényl)-4-[2-méthyl-1,2,3,5,6,8a-hexahydroindolizin-7-yl]-3-(pyridin-4-yl)-1*H*-pyrrole,
2- (3-chlorophényl)-4-[2-phényl-1,2,3,5,6,8a-hexahydroindolizin-7-yl]-3-(pyridin-4 -yl)-1*H*-pyrrole,
2- (3-chlorophényl)-4-[2-méthoxy-1,2,3,5,6,8a-hexahydroindolizin-7-yl]-3- (pyridin-4-yl)-1*H*-pyrrole,
4-[2-méthyl-1,2,3,5,6,8a-hexahydroindolizin-7-yl]-3-(pyridin-4-yl)-2-(trifluorométhylphényl)-1*H*-pyrrole,
4-[2-phényl-1,2,3,5,6,8a-hexahydroindolizin-7-yl]-3-(pyridin-4-yl)-2-(trifluorométhylphényl)-1*H*-pyrrole,
4-[2-méthoxy-1,2,3,5,6,8a-hexahydroindolizin-7-yl]-3-(pyridin-4-yl)-2-(3-trifluorométhylphényl)-1*H*-pyrrole,
2- (4-fluorophényl)-4-[2-méthyl-3,5,6,8a-tétrahydroindolizin-7-yl]-3-(pyridin-4-yl)-1*H*-pyrrole,
2- (4-fluorophényl)-4-[2-méthylidène-1,2,3,5,6,8a-hexahydroindolizin-7-yl]-3-(pyridin-4-yl)-1*H*-pyrrole,
4-[2-éthyl-1,2,3,5,6,8a-hexahydroindolizin-7-yl]-2-(4-fluorophényl)-3-(pyridin-4-yl)-1*H*-pyrrole,
4-[2-éthyl-1,2,3,5,6,8a-hexahydroindolizin-7-yl]-2-(3-fluorophényl)-3-(pyridin-4-yl)-1*H*-pyrrole,
2- (3-chlorophényl)-4-[2-éthyl-1,2,3,5,6,8a-hexahydroindolizin-7-yl]-3-(pyridin-4-yl)-1*H*-pyrrole,
4-[2-éthyl-1,2,3,5,6,8a-hexahydroindolizin-7-yl]-3-(pyridin-4-yl)-2-(3trifluorométhylphényl)-1*H*-pyrrole,
2- (4-fluorophényl)-4-[2-propyl-1,2,3,5,6,8a-hexahydroindolizin-7-yl]-3-(pyridin-4-yl)-1*H*-pyrrole,
2- (3-fluorophényl)-4-[2-propyl-1,2,3,5,6,8a-hexahydroindolizin-7-yl]-3-(pyridin-4-yl)-1*H*-pyrrole,
2- (3-chlorophényl)-4-[2-propyl-1,2,3,5,6,8a-hexahydroindolizin-7-yl]-3-(pyridin-4-yl)-1*H*-pyrrole,
4-[2-propyl-1,2,3,5,6,8a-hexahydroindolizin-7-yl]-3-(pyridin-4-yl)-2-(3-trifluorométhylphényl)-1*H*-pyrrole,

4- [2-éthyl-3,5,6,8a-tetrahydroindolizin-7-yl]-2-(4-fluorophényl)-3-(pyridin-4-yl)-1*H*-pyrrole,
2- (4-fluorophényl)-4-[2-propyl-3,5,6,8a-tétrahydroindolizin-7-yl]-3-(pyridin-4-yl)-1*H*-pyrrole, et
2- (4-fluorophényl)-4-[2-phényl-3,5,6,8a-tétrahydroindolizin-7-yl]-3-(pyridin-4-yl)-1*H*-pyrrole.

**36.** Composé selon la revendication 1, choisi parmi le 2- (4-fluorophényl)-4-[(2R,8aS)-2-phényl-1,2,3,5,6,8a-hexahydroindolizin-7-yl]-3-(pyridin-4-yl)-1*H*-pyrrole et ses sels et amides pharmacologiquement acceptables.

**37.** Composé selon la revendication 1, choisi parmi le 2-(4-fluorophényl)-4-[(8aS)-2-méthyl-1,2,3,5,6,8a-hexahydroindolizin-7-yl]-3-(pyridin-4-yl)-1*H*-pyrrole et ses sels et amides pharmacologiquement acceptables.

**38.** Composé selon la revendication 1, choisi parmi le 2-(4-fluorophényl)-4-[(8aS)-2-méthylidène-1,2,3,5,6,8a-hexahydroindolizin-7-yl]-3-(pyridin-4-yl)-1*H*-pyrrole et ses sels et amides pharmacologiquement acceptables.

**39.** Composé selon la revendication 1, choisi parmi le 2-(4-fluorophényl)-4-[(8aS)-2-méthyl-3,5,6,8a-tétrahydroindolizin-7-yl]-3-(pyridin-4-yl)-1*H*-pyrrole et ses sels et amides pharmacologiquement acceptables.

**40.** Composé selon la revendication 1, choisi parmi le 4-[(2S,8aS)-2-éthyl-1,2,3,5,6,8a-hexahydroindolizin-7-yl)-2-(4-fluorophényl)-3-(pyridin-4-yl)-1*H*-pyrrole et ses sels et amides pharmacologiquement acceptables.

**41.** Composé selon la revendication 1, choisi parmi le 2-(4-fluorophényl)-4-[(2S,8aS)-2-propyl-1,2,3,5,6,8a-hexahydroindolizin-7-yl]-3-(pyridin-4-yl)-1H-pyrrole et ses sels et amides pharmacologiquement acceptables.

**42.** Composition pharmaceutique comprenant une quantité efficace d'un composé pharmacologiquement actif et un support ou un diluant approprié, dans laquelle ledit composé pharmacologiquement actif est un composé de formule (I-1) ou un sel, un ester ou un amide de celui-ci pharmacologiquement acceptable selon l'une quelconque des revendications 1 à 41.

**43.** Composition pharmaceutique selon la revendication 42 destinée à être utilisée comme médicament pour inhiber la production de cytokines inflammatoires chez un mammifère qui peut être un être humain.

**44.** Composition pharmaceutique selon la revendication 42 destinée à être utilisée comme médicament pour inhiber la résorption osseuse chez un mammifère qui peut être un être humain.

**45.** Composition pharmaceutique selon la revendication 42 destinée à être utilisée comme médicament pour le traitement ou la prophylaxie des maladies inflammatoires chez un mammifère qui peut être un être humain.

**46.** Composition pharmaceutique selon la revendication 42 destinée à être utilisée comme médicament pour le traitement ou la prophylaxie de maladies virales chez un mammifère qui peut être un être humain.

**47.** Composition pharmaceutique selon la revendication 42 destinée à être utilisée comme médicament pour soulager la douleur ou la pyréxie chez un mammifère qui peut être un être humain.

**48.** Composition pharmaceutique selon la revendication 42 destinée à être utilisée comme médicament pour le traitement ou la prophylaxie de la polyarthrite rhumatoïde chronique chez un mammifère qui peut être un être humain.

**49.** Composition pharmaceutique selon la revendication 42 destinée à être utilisée comme médicament pour le traitement ou la prophylaxie de l'arthrose chez un mammifère qui peut être un être humain.

**50.** Composition pharmaceutique selon la revendication 42 destinée à être utilisée comme médicament pour le traitement ou la prophylaxie du cancer chez un mammifère qui peut être un être humain.

**51.** Composition pharmaceutique selon la revendication 42 destinée à être utilisée comme médicament pour le traitement ou la prophylaxie de l'hépatite chez un mammifère qui peut être un être humain.

**52.** Composition pharmaceutique selon la revendication 42 destinée à être utilisée comme médicament pour le traitement ou la prophylaxie d'une maladie choisie parmi le groupe composé des maladies allergiques, de la septicémie, du psoriasis, de l'asthme, de l'arthrose déformante, de la maladie de Crohn, du lupus érythémateux disséminé, de l'ostéoporose, de la colite ulcéreuse, du diabète, de la néphrite, de la maladie cardiaque ischémique, de la maladie

d'Alzheimer et de l'artériosclérose chez un mammifère qui peut être un être humain.

**53.** Composition pharmaceutique selon la revendication 42 destinée à être utilisée comme médicament pour le traitement ou la prophylaxie de la septicémie chez un mammifère qui peut être un être humain.

**54.** Composé de formule (I-1) un sel, ester ou amide pharmacologiquement acceptable de celui-ci selon l'une quelconque des revendications 1 à 41 destiné à être utilisé en tant que médicament.

**55.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné à inhiber la production de cytokines inflammatoires chez un mammifère qui peut être un être humain.

**56.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné à inhiber la résorption osseuse chez un mammifère qui peut être un être humain.

**57.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de maladies inflammatoires chez un mammifère qui peut être un être humain.

**58.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de maladies virales chez un mammifère qui peut être un être humain

**59.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné à lutter contre la douleur ou la pyréxie chez un mammifère qui peut être un être humain

**60.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de la polyarthrite rhumatoïde chronique chez un mammifère qui peut être un être humain.

**61.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de l'arthrose chez un mammifère qui peut être un être humain.

**62.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie du cancer chez un mammifère qui peut être un être humain.

**63.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de l'hépatite chez un mammifère qui peut être un être humain.

**64.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de la maladie choisie parmi le groupe composé des maladies allergiques, de la septicémie, du psoriasis, de l'asthme, de l'arthrose déformante, de la maladie de Crohn, du lupus érythémateux disséminé, de l'ostéoporose, de la colite ulcéreuse, du diabète, de la néphrite, de la maladie cardiaque ischémique, de la maladie d'Alzheimer et de l'artériosclérose chez un mammifère qui peut être un être humain.

**65.** Utilisation d'au moins un composé de formule (I-1) ou d'un sel, un ester ou un amide pharmacologiquement acceptable de celui-ci, selon l'une quelconque des revendications 1 à 41, dans la fabrication d'un médicament destiné au traitement ou à la prophylaxie de la septicémie chez un mammifère qui peut être un être humain.

**66.** Procédé de préparation d'un composé selon l'une quelconque des revendications 1 à 41, comprenant les étapes suivantes consistant à:

(a) soumettre un composé de formule (III)

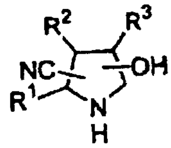

dans laquelle $R^1$, $R^2$ et $R^3$ sont tels que défini dans l'une quelconque des revendications 1 à 41, et les substituants cyano et hydroxyle sur le cycle pyrrolidinyle se trouvent sur les atomes de carbone du cycle qui sont adjacents à l'atome d'azote du cycle,

à une réaction aboutissant à l'élimination de cyanure d'hydrogène et d'eau dudit composé de formule (IIIa) pour donner le composé désiré de formule (I-1) ; et

(b) éventuellement, soumettre ledit composé désiré de formule (I-1) obtenu à l'étape (a) à une ou plusieurs réactions de manière à transformer un ou plusieurs desdits substituants $R^1$, $R^2$ et $R^3$ en d'autres substituants couverts par la définition desdits groupes et/ou à convertir ledit composé de formule (I) en sel, en ester ou en amide de celui-ci.

**67.** Procédé de préparation d'un composé de formule (I-1) ou d'un sel, un ester ou un amide de celui-ci pharmacologiquement acceptable, selon l'une quelconque des revendications 1 à 41, ledit procédé comprenant les étapes suivantes :

(a) la lithiation d'un composé de formule (IVa)

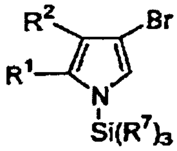

(IVa)

dans laquelle $R^1$ et $R^2$ sont tels que défini ci-dessus et chaque radical $R^7$ est identique ou différent, et représente un atome d'hydrogène, un groupe alkyle inférieur tel que défini à la revendication 1, un groupe aryle tel que défini à la revendication 1 ou un groupe aralkyle tel que défini à la revendication 1, puis la réaction du dérivé lithié ainsi obtenu avec un composé de formule $R^3$-L dans laquelle $R^3$ est tel que défini ci-dessus et L est un groupe susceptible de partir sous forme de résidu nucléophile, pour donner un composé de formule (Va) :

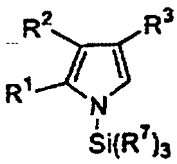

(Va)

dans laquelle $R^1$, $R^2$, $R^3$ et $R^7$ sont tels que définis ci-dessus et

(b) l'exposition du composé de formule (Va) obtenu à l'étape (a) à une réaction de désilylation pour donner le composé désiré de formule (I-1) ; et

(c) éventuellement, l'exposition dudit composé de formule (I-1) obtenu à l'étape (b) à une ou plusieurs réactions afin de convertir un ou plusieurs desdits substituants $R^1$, $R^2$ et $R^3$ en d'autres substituants couverts par la définition desdits groupes et/ou à convertir ledit composé de formule (I-1) en un sel, un ester ou un amide de

celui-ci.

**68.** Procédé de préparation d'un composé de formule (I-1) ou d'un sel, un ester ou un amide de celui-ci pharmacologiquement acceptable, selon l'une quelconque des revendications 1 à 41, ledit procédé comprenant les étapes suivantes :

(a) la lithiation d'un composé de formule (IVa)

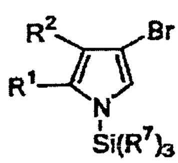

(IVa)

dans laquelle $R^1$ et $R^2$ sont tels que défini ci-dessus et $R^7$ est tel que défini à la revendication 67, puis la réaction du dérivé lithié ainsi obtenu avec un composé de formule (VI) ou de formule (VII) :

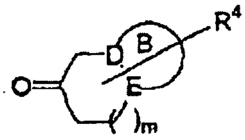

(VI)

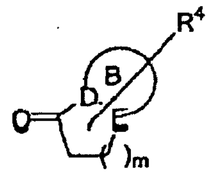

(VII)

dans laquelle B, D, E, $R^4$ et m sont définis à l'une quelconque des revendications 1 à 41, pour donner un composé de formule (VIIIa) ou (IXa) :

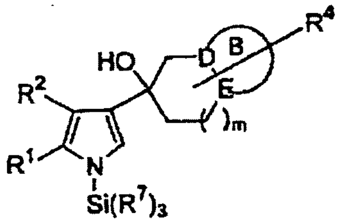

(VIIIa)

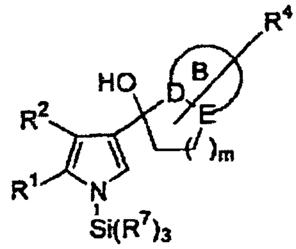

(IXa)

dans laquelle $R^1$, $R^2$, $R^4$, $R^7$, B, D et m sont tels que défini ci-dessus, et
(b) l'exposition du composé de formule (Villa) ou (IXa) obtenu à l'étape (a) à des étapes successives de déshydratation et de désilylation pour donner le composé désiré de formule (I-1) ; et
(c) éventuellement, l'exposition dudit composé de formule (I-1) obtenu à l'étape (b) à une ou plusieurs réactions de manière à convertir un ou plusieurs desdits substituants $R^1$, $R^2$ et $R^3$ en d'autres substituants couverts par la définition desdits groupes et/ou la conversion dudit composé de formule (I-1) en un sel, un ester ou un amide de celui-ci.